# EUROPEAN PATENT APPLICATION

(11) **EP 2 368 902 A2**
(43) Date of publication of application: **28.09.2011**
(21) Application number: 10186251.4
(22) Date of filing: 29.03.2000
(51) Int. Cl.: C07K 7/08, A61K 38/00, G01N 33/74

(54) **Insulin and IGF-1 receptor agonists and antagonists**

(62) Divisional of application: 00918510.9
(71) Applicant: DGI Bio Technologies LLC, Edison, NJ 08818 (US); NOVO Nordisk A/S, 2880 Bagsvaerd (DK)
(72) Inventor: Beasley, James, Franklin Park, NJ 08823 (US); Blume, Arthur J., Annandale, NJ 08801 (US); Schäffer, Lauge, 2100 Copenhagen (DK); Pillutla, Renuka, Bridgewater, NJ 08807 (US); Brandt, Jakob, 2700 Broenshoej (DK); Brissette, Renee, Edison, NJ 08817 (US); Spetzler, Jane, DK-2700 Brønshøj (DK); Cheng, Weiqing, Highland Park, NJ 08904 (US); Östergaard, Sören, 2700 Broenshoej (DK); Mandecki, Wlodek S., Princeton Junction NJ 08550 (US); Hansen, Per Hertz, 2800 Lyngby (DK); Ravera, Mark, Chatham, NJ 07928 (US); Hsiao, Ku-chaun, Edison, NJ 08820 (US)
(74) Representative: Finnie, Isobel Lara

(57) **Abstract**

Peptide sequences capable of binding to insulin and/or insulin-like growth factor receptors with either agonist or antagonist activity and identified from various peptide libraries are disclosed. This invention also identifies at least two different binding sites which are present on insulin and insulin-like growth factor receptors which selectively bind the peptides of this invention. As agonists, certain of the peptides of this invention may be useful for development as therapeutics to supplement or replace endogenous peptide hormones. The antagonists may also be developed as therapeutics.
In one embodiment, the receptor-binding peptides comprise the core motive B6:
X₆X₇X₈X₉X₁₀X₁₁X₁₂X₁₃ wherein X₆ and X₇ are aromatic, and X₁₀ and X₁₃ are hydrophobic residues.

## Description

### I. FIELD OF THE INVENTION

This invention relates to the field of hormone receptor activation or inhibition. More specifically, this invention relates to the identification of molecular structures, especially peptides, which are capable of acting at either the insulin or insulin-like growth factor receptors as agonists or antagonists. Also related to this invention is the field of molecular modeling whereby useful molecular structures are derived from known structures.

### II. BACKGROUND OF THE INVENTION

Insulin is a potent metabolic and growth promoting hormone that acts on cells to stimulate glucose, protein, and lipid metabolism, as well as RNA and DNA synthesis. A well-known effect of insulin is the regulation of the level of glucose at a whole body level. This effect by insulin occurs predominantly in liver, fat, and muscle. In liver, insulin stimulates glucose incorporation into glycogen and inhibits the production of glucose. In muscle and fat, insulin stimulates glucose uptake, storage, and metabolism. Disruptions of glucose utilization are very common in the population in giving rise to diabetes.

Signal transduction in target cells is initiated by binding of insulin to a specific cell-surface receptor, the insulin receptor (IR). The binding leads to conformational changes in the.extracellular domain of the receptor, which are transmitted across the cell membrane and result in activation of the receptor's tyrosine kinase activity. This, in turn, leads to autophosphorylation of the insulin receptor's tyrosine kinase, and the binding of soluble effector molecules that contain SH2 domains such as phophoinositol-3-kinase, Ras GTPase-activating protein, and phospholipase Cγ to IR (Lee and Pilch, 1994).

Insulin-like growth factor 1 (IGF-1) is a small, single-chain protein (MW = 7,500 Da) that is involved in many aspects of tissue growth and repair, and recently has been implicated in various forms cancer including prostrate, breast, colorectal, and ovarian. It is similar in size, sequence and structure to insulin, but has 100-1,000-fold lower affinity for the insulin receptor (Mynarcik *et al*., 1997).

Clinically, recombinant human IGF-1 has been investigated for the treatment of several diseases, including type I diabetes (Carroll *et al*., 1997; Crowne *et al.,* 1998), amyotropic lateral sclerosis (Lai *et al*., 1997), and diabetic motor neuropathy (Apfel and Kessler, 1996). Other potential therapeutic applications of IGF-1 such as osteoporosis (Canalis, 1997), immune modulation (Clark, 1997) and nephrotic syndrome (Feld and Hirshberg, 1996) are being examined.

A number of studies have analyzed the role of natural IGF-1 in various disease states. Most interestingly, several reports have shown that IGF-1 promotes the growth of normal and cancerous prostate cells both *in vitro* and *in vivo* (Angelloz-Nicoud and Binoux, 1995; Figueroa *et al.,* 1995; Torring *et al.,* 1997). Additionally, elevated serum IGF-1 levels have been connected with increased risks of prostate cancer, and may be an earlier predictor of cancer than is prostate-specific antigen (PSA) (Chan *et al.,* 1998). Recent studies have indicated a connection between IGF-1 and other cancers such as breast, colorectal, and ovarian. Serum IGF-1 levels are regulated by the presence of IGF binding proteins (IGFBP) which bind to IGF-1 and prevent its interaction with the IGF-1 R (reviewed in Conover, 1996 and Rajaram *et al*., 1997). Interestingly, PSA has been shown to be a protease that cleaves IGFBP-3, resulting in an increase of free IGF-1 in serum (Cohen *et al.,* 1992; Cohen *et al*., 1994; Lilja, 1995). Clearly, regulation of IGF-1R activity can play an important role in several disease states, indicating that there are potential clinical applications for both IGF-1 agonists and antagonists.

The type-1 insulin-like growth-factor receptor (IGF-1R) and insulin receptor (IR) are rotated members of the tyrosine-kinase receptor superfamily of growth factor receptors. Both types of receptors are composed of two α and two β subunits which form a disulfide-linked heterotetramer (β-α-α-β). They have an extracellular ligand binding domain, a single transmembrane domain, and a cytoplasmic domain displaying the tyrosine kinase activity. The extracellular domain is composed of the entire subunits and a portion of the N-terminus of the β subunits, while the intracellular portion of the β subunits contains the tyrosine kinase domain. Besides IR and IGF-1R, the other known member of the IR family is the insulin-related receptor (IRR), for which no natural ligand is known.

While similar in structure, IGF-1 and insulin receptors serve different physiological functions. The IR is primarily involved in metabolic functions whereas the IGF-1R mediates growth and differentiation. However, both insulin and IGF-1 can induce both mitogenic and metabolic effects. Whether each ligand elicits both activities via its own receptor, or whether insulin exerts its mitogenic effects through its weak affinity binding to the IGF-1 receptor, and IGF-1 its metabolic effects through the insulin receptor, remains controversial. (De Meyts, 1994).

The insulin receptor is a glycoprotein having molecular weight of 350-400 kDa (depending of the level of glycosylation). It is synthesized as a single polypeptide chain and proteolytically cleaved yielding the disulfide-linked monomer α-β insulin receptor. Two α-β monomers are linked by disulfide bonds between the α-subunits to form a dimeric form of the receptor (β-α-α-β-type configuration). The α subunit is comprised of 723 amino acids, and it can be divided into two large homologous domains, L1 (amino acids 1-155) and L2 (amino acids 313-468), separated by a cysteine rich region (amino acids 156-312) (Ward *et al.,* 1995). Many determinants of insulin binding seem to reside in the α-subunit. A unique feature of the insulin receptor is that it is dimeric in the absence of ligand.

The sequence of IR is highly homologous to the sequence of the type-1 insulin-like growth factor receptor (IGF-1R). The homology level varies from about 40% to 70%, depending on the position within the α-subunit. The three-dimensional structures of both receptors may therefore be similar. The crystal structure of the first three domains of IGF-1 R has been determined (Garrett *et al.,* 1998). The L domains consist of a single-stranded right-handed β-helix (a helical arrangement of β-strands), while the cysteine-rich region is composed of eight disulfide-bonded modules.

The β-subunit of the insulin receptor has 620 amino acid residues and three domains: extracellular, transmembrane, and cytosolic. The extracellular domain is linked by disulfide bridges to the α-subunit. The cytosolic domain includes the tyrosine kinase domain, the three-dimensional structure of which has been solved (Hubbard *et al*., 1994).

To aid in drug discovery efforts, a soluble form of a membrane-bound receptor was constructed by replacing the transmembrane domain and the intracellular domain of IR with constant domains from immunoglobulin Fc or λ subunits (Bass *et al.,* 1996). The recombinant gene was expressed in human embryonic kidney 293 cells. The expressed protein was a fully processed heterotetramer and the ability to bind insulin was similar to that of the full-length holoreceptor.

IGF-1 and insulin competitively cross-react with IGF-1R and IR. (Schäffer, 1994). Despite 45% overall amino acid homology, insulin and IGF-1 bind only weakly to each other's receptor. The affinity of each peptide for the non-cognate receptor is about 3 orders of magnitude lower than that for the cognate receptor. (Mynarcik, *et al.,* 1997). The differences in binding affinities may be partly explained by the differences in amino acids and unique domains which contribute to unique tertiary structures of ligands. (Blakesley *et al.,* 1996).

Both insulin and IGF-1 are expressed as precursor proteins comprising, among other regions, contiguous A, B, and C peptide regions, with the C peptide being an intervening peptide connecting the A and B peptides. A mature insulin molecule is composed of the A and B chains connected by disulfide bonds, whereas the connecting C peptide has been removed during post-translational processing. IGF-1 retains its smaller C-peptide as well as a small D extension at the C-terminal end of the A chain, making the mature IGF-1 slightly larger than insulin. (Blakesley, 1996). The C region of human insulin-like growth factor (IGF-1) appears to be required for high affinity binding to the type I IGF receptor. (Pietrzkowski *et al*., 1992). Specifically, tyrosine 31 located within this region appears to be essential for high affinity binding. Furthermore, deletion of the D domain of IGF-1 increased the affinity of the mutant IGF-1 for binding to the IR, while decreasing its affinity for the IGF-1 R receptor. (Pietrzkowski *et al.,* 1992). A further structural distinction between the two hormones is that, unlike insulin, IGF-1 has very weak self-association and does not hexamerize. (De Meyts, 1994).

The α-subunits, which contain the ligand binding region of the IR and IGF-1R, demonstrate between 47-67% overall amino acid homology. Three general domains have been reported for both receptors from sequence analysis of the α subunits, L1-Cys-rich-L2. The cysteine residues in the C-rich region are highly conserved between the two receptors; however, the cysteine-rich domains have only 48% overall amino acid homology.

Despite the similarities observed between these two receptors, the role of the domains in specific ligand binding are distinct. Through chimeric receptor studies, (domain swapping of the IR and IGF-1R α-subunits), researchers have reported that the sites of interaction of the ligands with their specific receptors differ. (Blakesley, *et al.,* 1996). For example, the cysteine-rich domain of the IGF-1R (amino acids 191-290) was determined to be essential for high-affinity IGF binding, but not insulin binding by introducing this IGF-1R region into the corresponding region of the IR (amino acids 198-300) and observing that the IR demonstrated high affinity binding of IGF-1 while maintaining high-affinity insulin binding. Conversely, when the corresponding region of the IR was introduced into the IGF-1R, the affinity for IGF-1 was not detectable while the affinity for insulin remained undisturbed.

A further distinction between the binding regions of their and IGF-1 R is their differing dependence on the N-terminal and C-terminal regions. Both the N-terminal and C-terminal regions (located within the putative L1 and L2 domains) of the IR are important for high-affinity insulin binding but appear to have little effect on IGF-1 binding. Replacing residues in the N-terminus of IGF-1R (amino acids 1-62) with the corresponding residues of IR (amino acids 1-68) confers insulin-binding ability on IGF-1R. Within this region residues Phe-39, Arg-41 and Pro-42 are reported as major contributors to the interaction with insulin. (Williams *et al.,* 1995). When these residues are introduced into the equivalent site of the IGF-1R, the affinity for insulin is markedly increased, whereas, substitution of these residues by alanine in the IR results in markedly decreased insulin affinity. Similarly, the region between amino acids 704-717 of the C-terminus of IR has been shown to play a major role in insulin specificity. Substitution of these residues with alanine also disrupts insulin binding. (Mynarcik *et a*/., 1996).

Further studies of alanine scanning of the receptors suggest that insulin and IGF-1 may use some common contacts to bind to the IGF-1 receptor but that those contacts differ from those that insulin utilizes to bind to the insulin receptor. (Mynarcik *et al.,* 1997). Hence, the data in the literature has led one commentator to state that even though "the binding interfaces for insulin and IGF-1 on their respective receptors may be homologous within this interface the side chains which make actual contact and determine specificity may be quite different between the two ligand-receptor systems." (De Meyts, 1994).

The identification of molecular structures having a high degree of specificity for one or the other receptor is important to developing efficacious and safe therapeutics. For example, a molecule developed as an insulin agonist should have little or no IGF-1 activity in order to avoid the mitogenic activity of IGF-1 and a potential for facilitating neoplastic growth.

It is therefore important to determine whether insulin and IGF-1 share common three-dimensional structures but which have sufficient differences to confer selectivity for their respective receptors. Similarly, it would be desirable to identify other molecular structures which mimic the active binding regions of insulin and/or IGF-1 and which impart selective agonist or antagonist activity.

Although certain proteins are important drugs, their use as therapeutics presents several difficult problems, including the high cost of production and formulation, administration usually via injection and limited stability in the bloodstream. Therefore, replacing proteins, including insulin or IGF-1, with small molecular weight drugs has received much attention. However, none of these efforts has resulted in finding a successful drug.

Peptides mimicking functions of protein hormones have been previously reported. Yanofsky *et al.* (1996) reports the isolation of a monomer peptide antagonistic to IL-1 with nanomolar affinity for the IL-1 receptor. This effort required construction and use of many phage displayed peptide libraries and sophisticated phage panning procedures.

Wrighton *et al.* (1996) and Livnah *et al*. (1996) reported dimer peptides that bind to the erythropoietin (EPO) receptor with full agonistic activity *in vivo.* These peptides are cyclical and have intra-peptide disulfide bonds; like the IL-1 receptor antagonist, they show no significant sequence identity to the natural ligand. Importantly, X-ray crystallography revealed that it was the spontaneous formation of non-covalent peptide homodimers which enabled the dimerization two EPO receptors.

Most recently, Cwirla *et al.* (1997) reported the identification of two families of peptides that bind to the human thrombopoietin (TPO) receptor and are competed by the binding of the natural TPO ligand. The peptide with the highest affinity, when dimerized by chemical means proved to be as potent an *in vivo* agonist as TPO, the natural ligand .

WO 96/04557 reports the use of peptides and antibodies which bind to active sites of biological targets and which are then used in competition assays to identify small molecules which are agonist or antagonists at the biological targets.

### III. SUMMARY OF THE INVENTION

This invention relates to the identification of amino acid sequences that specifically recognize sites involved in IR and/or IGF-1R activation. Specific amino acid sequences are identified and their agonist or antagonist activity at IR or IGF-1R has been determined. Such sequences may be developed as potential therapeutics or as lead compounds to develop other more efficacious ones. In addition, these sequences may be used in high-throughput screens to identify and provide information on small molecules which bind at these sites and mimic or antagonize the functions of insulin or IGF-1. Furthermore, the peptide sequences provided by this invention can be used to design secondary peptide libraries, which can be used to identify sequence variants that increase or modulate the binding and/or activity of the original peptide at IR or IGF-1R.

In one aspect of this invention large numbers of peptides have been screened for their IR or IGF-1R binding and activity characteristics. Analysis of their amino acid sequences has identified certain consensus sequences which may be used themselves or as core sequences in larger amino acid sequences conferring upon them agonist or antagonist activity. At least ten generic amino acid sequences have been identified which bind IR and IGF-1 R with varying degrees of agonist or antagonist activity depending on the specific sequence of the various peptides identified within each motif group. Also provided are amino or carboxyl terminal extensions capable of modifying the affinity and/or pharmacological activity of the consensus sequences when part of a larger amino acid sequence.

The amino acid sequences of this invention which bind IR and/or IGF-1R include:
a. X₁ X₂ X₃ X₄ X₅ wherein X₁, X₂, X₄ and X₅ are aromatic amino acids, and X₃ is any polar amino acid;
b. X₆ X₇ X₈ X₉ X₁₀ X₁₁ X₁₂ X₁₃ wherein X₆ and X₇ are aromatic amino acids, X₈, X₉, X₁₁ and X₁₂ are any amino acid, and X₁₀ and X₁₃ are hydrophobic amino acids;
c. X₁₄ X₁₅ X₁₆ X₁₇ X₁₈ X₁₉ X₂₀ X₂₁ wherein X₁₄, and X₁₇ are hydrophobic amino acids, X₁₅, X₁₆, X₁₈ and X₁₉ are any amino acid, and X₂₀ and X₂₁ are aromatic amino acids.
d. X₂₂ X₂₃ X₂₄ X₂₅ X₂₆ X₂₇ X₂₈ X₂₉ X₃₀ X₃₁ X₃₂ X₃₃ X₃₄ X₃₅ X₃₆ X₃₇ X₃₈ X₃₉ X₄₀ X₄₁ wherein X₂₂, X₂₅, X₂₈, X₂₉, X₃₀, X₃₃, X₃₄, X₃₅, X₃₆, X₃₇, X₃₈, X₄₀, and X₄₁ are any amino acid, X₃₅ and X₃₇ may be any amino acid for binding to IR, whereas X₃₅ is preferably a hydrophobic amino acid and X₃₇ is preferably glycine for binding to IGF-1 R and possess agonist or antagonist activity. X₂₃ and X₂₆ are hydrophobic amino acids. This sequence further comprises at least two cysteine residues, preferably at X₂₅ and X₄₀ X₃₁ and X₃₂ are small amino acids.
e. X₄₂ X₄₃ X₄₄ X₄₅ X₄₆ X₄₇ X₄₈ X₄₉ X₅₀ X₅₁ X₅₂ X₅₃ X₅₄ X₅₅ X₅₆ X₅₇ X₅₈ X₅₉ X₆₀ X₆₁ wherein X₄₂, X₄₃, X₄₄, X₄₅, X₅₃, X₅₅, X₅₆, X₅₈, X₆₀ and X₆₁ may be any amino acid, X₄₃, X₄₆, X₄₉, X₅₀, X₅₄ are hydrophobic amino acids, X₄₇ and X₅₉ are preferably cysteines, X₄₈ is a polar amino acid, and X₅₁, X₅₂ and X₅₇ are small amino acids.
f. X₆₂ X₆₃ X₆₄ X₆₅ X₆₆ X₆₇ X₆₈ X₆₉ X₇₀ X₇₁ X₇₂ X₇₃ X₇₄ X₇₅ X₇₆ X₇₇ X₇₈ X₇₉ X₈₀ X₈₁ wherein X₆₂, X₆₅, X₆₈, X₆₉, X₇₁, X₇₃, X₇₆, X₇₇, X₇₈, X₈₀, and X₈₁ may be any amino acid; X₆₃, X₇₀, X₇₄ are hydrophobic amino acids; X₆₄ is a polar amino acid, X₆₇ and X₇₅ are aromatic amino acids and X₇₂ and X₇₉ are preferably cysteines capable of forming a loop.
g. H X₈₂ X₈₃ X₈₄ X₈₅ X₈₆ X₈₇ X₈₈ X₈₉ X₉₀ X₉₁ X₉₂ wherein X₈₂ is proline or alanine, X₈₃ is a small amino acid, X₈₄ is selected from leucine, serine or threonine, X₈₅ is a polar amino acid, X₈₆, X₈₈, X₈₉ and X₉₀ are any amino acid, and X₈₇, X₉₁ and X₉₂ are an aliphatic amino acid.
h. X₁₀₄ X₁₀₅ X₁₀₆ X₁₀₇ X₁₀₈ X₁₀₉ X₁₁₀ X₁₁₁ X₁₁₂ X₁₁₃ X₁₁₄ wherein at least one of the amino acids of X₁₀₆ through X₁₁₁, and preferably two, are tryptophan separated by three amino acids, and wherein at least one of X₁₀₄, X₁₀₆ and X₁₀₆ and at least one of X₁₁₂, X₁₁₃ and X₁₁₄ are cysteine; and
i. an amino acid sequence comprising the sequence DYKDLCQSWGVRIGWLAGLCPKK.
j. WX₁₂₃ GYX₁₂₄ WX₁₂₆ X₁₂₆, wherein X₁₂₃ is selected from proline, glycine, serine, arginine, alanine or leucine, but more preferably proline; X₁₂₄ is any amino acid, but preferably a charged or aromatic amino acid; X₁₂₅ is a hydrophobic amino acid preferably leucine or phenylalanine, and most preferably leucine. X₁₂₆ is any amino acid, but preferably a small amino acid.

In one embodiment, preferred amino acid sequences FYX₃WF ("A6" motif) and FYX₈ X₉ L/IX₁₁ X₁₂ L ("B6" motif) have been identified which competitively bind to sites on IR and IGF-1R and possess either agonist or antagonist activity. Surprisingly FYX₃WF which possesses agonist activity at IGF-1R, can possess agonist or antagonist activity at IR. Similarly, FY X₈ X₉ L/IX₁₁ X₁₂ L, which is an antagonist at IGF-1R, possesses agonist activity at IR.

This invention also identifies at least two distinct binding sites on IR and IGF-1 R based on the differing ability of certain of the peptides to compete with one another and insulin or IGF-1 for binding to IR and IGF-1 R. Accordingly, this invention provides amino acid sequences which bind specifically to one or both sites of IR and/or IGF-1R. Furthermore, specific amino acid sequences are provided which have either agonist or antagonist characteristics based on their ability to bind to the specific sites of IR.

In another embodiment of this invention, amino acid sequences which bind to one or more sites of IR or IGF-1R may be covalently linked together to form multivalent ligands. These multivalent ligands are capable of forming complexes with a plurality of IR or IGF-1R. Either the same or different amino acid sequences may be covalently bound together to form homo- or heterocomplexes. Dimers of the same amino acid sequence, for example, may be used to form receptor complexes bound through the same corresponding sites. Alternatively, heterodimers may be used to bind to different sites on one receptor or to cause receptor complexing through different sites.

The present invention also provides assays for identifying compounds that mimic the binding characteristics of insulin. Such compounds may act as antagonists or agonists of insulin function in cell based assays.

This invention also provides amino acid sequences such as peptides and recombinant antibody variable regions (rVab) that inhibit binding of insulin to the insulin receptor. Such amino acid sequences and rVabs are used in the assays of the invention to identify compounds that mimic insulin.

This invention also provides kits for identifying compounds that bind to the insulin receptor. The invention further provides therapeutic compounds that bind the insulin receptor.

In another embodiment, this invention provides assays for identifying compounds which mimic the binding characteristics of IGF-1. Such compounds act as antagonists or agonists of IGF-1 hormone function in cell based assays.

The invention also provides amino acid sequences such as peptides and rVabs which inhibit binding of IGF-1 to IGF-1R. Such amino acid sequences and rVabs are used in the assays of the invention to identify compounds which mimic IGF-1.

Another embodiment of this invention is the nucleic acid sequences encoding the amino acid sequences of the invention. Also within the scope of this invention are vectors containing the nucleic acids and host cells which express the genes encoding the amino acid sequences which bind at IR or IGF-1R and possess agonist or antagonist activity.

It is an object of this invention to provide amino acid sequences which bind to active sites of IR and/or IGF-1R and to identify structural criteria for conferring agonist or antagonist activity at IR and/or IGF-1R.

It is a further object of this invention to provide specific amino acid sequences which possess agonist, partial agonist or antagonist activity at either IR or IGF-1R. Such amino acid sequences are potentially useful as therapeutics themselves or may be used to identify other molecules, especially small organic molecules, which possess agonist or antagonist activity at IR or IGF-1R.

Another object of this invention is to provide structural information derived from the amino acid sequences of this invention which may be used to construct other molecules possessing the desired activity at the relevant IR of IGF-1R binding site.

### IV. BRIEF DESCRIPTION OF THE DRAWINGS

Figures 1A-10G. Amino acid sequences comprising the motif of Formulas 1 through 10. Sequences were identified by panning peptide libraries against IGF-1R and/or IR. The amino acids are represented by their one-letter abbreviation. The ratios over background are determined by dividing the signal at 405 nm (E-Tag, IGF-1R, or IR) by the signal at 405 nm for non-fat milk. The IGF-1R/IR Ratio Comparison is determined by dividing the ratio of IGF-1R by the ratio of IR. The IR/IGF-1R Ratio Comparison is determined by dividing the ratio of IR by the ratio of IGF-1R.

The design of each library is shown in the first line in bold. In the design, symbol 'X' indicates a random position, an underlined amino acid indicates a doped position at the nucleotide level, and other positions are held constant. Additional abbreviations in the B6H library are: 'O' indicates an NGY codon where Y is C or T; 'J' indicates an RHR codon where R is A or G, and H is A, C, or T; and 'U' indicates an WY codon where V is A, C, or G, and Y is C or T. The 'h' in the 20E2 libraries indicates an NTN codon.

Symbols in the listed sequences are: Q - TAG Stop; # -TAA Stop; *-TGA Stop; and ? - Unknown Amino Acid. It is believed that a W replaces the TGA Stop Codon when expressed. Except for the 20C, A6L, and B6L libraries, all libraries are designed with the short FLAG Epitope DYKD (Hopp *et al.,* 1988) at the N-terminus of the listed sequence and AAAGAP at the C-terminus. The 20C, A6L, and B6L libraries have the full length FLAG epitope DYKDDDDDK.
Figure 1A: Formula 1 motif peptide sequences obtained from a random 40mer library panned against IR.
Figure 1B: Formula 1 motif peptide sequence obtained from a random 40mer library panned against IGF-1R.
Figure 1C: Formula 1 motif peptide sequences obtained from a random 20mer library panned against IR.
Figure 1D: Formula 1 motif peptide sequences obtained from a random 20mer library panned against IGF-1R.
Figure 1E: Formula 1 motif peptide sequences obtained from a 21 mer library constructed to contain X₁₋₁₀NFYDWFVX₁₈₋₂₁ (also referred to as "A6S") panned against IR.
Figure 1F: Formula 1 motif peptide sequences obtained from a 21mer library constructed to contain X₁₋₁₀NFYDWFVX₁₈₋₂₁ (also referred to as "A6S") panned against IGF-1R.
Figure 1G: Formula 1 motif peptide sequences obtained from a library constructed to contain variations outside the consensus core of the A6 peptide as indicated (referred to as "A6L") panned against IR.
Figure 1H: Formula 1 motif peptide sequences obtained from a library constructed to contain variations outside the consensus core of the A6 peptide as indicated (referred to as "A6L") panned against IGF-1R.
Figure 1I: Formula 1 motif peptide sequences obtained from a library constructed to contain variations in the consensus core of the E4D peptide (as indicated) panned against IR.
Figure 1J: Formula 1 motif peptide sequences obtained from a library constructed to contain variations in the consensus core of the E4D peptide (as indicated) panned against IGF-1R.
Figure 1K: Formula 1 motif peptide sequences obtained from a library constructed using the sequence X₁₋₆FHENFYDWFVRQVSX₂₁₋₂₆ (H2C-A) panned against IR.
Figure 1L: Formula 1 motif peptide sequences obtained from a library constructed using the sequence X₁₋₆FHENFYDWFVRQVSX₂₁₋₂₆ (H2C-A) panned against IGF-1R.
Figure 1M: Formula 1 motif peptide sequences obtained from a library constructed using the sequence X₁₋₆FHXXFYXWFX₁₆₋₂₁ (H2C-B) and panned against IR.
Figure 1N: Formula 1 motif peptide sequences obtained from a library constructed using the sequence X₁₋₆FHXXFYXWFX₁₆₋₂₁ (H2C-B) and panned against IGF-1R.
Figure 1O: Formula 1 motif peptide sequences obtained from other libraries panned against IR.
Figure 2A: Formula 2 motif peptide sequence identified from a random 40mer library panned against IR.
Figure 2B: Formula 2 motif peptide sequences identified from a random 40mer library panned against IGF-1 R.
Figure 2C: Formula 2 motif peptide sequences identified from a random 20mer library panned against IR.
Figure 2D: Formula 2 motif peptide sequences identified from a random 20mer library panned against IGF-1R.
Figure 2E: Formula 2 motif peptide sequences identified from a X₁-₄CX₆₋₂₀ library panned against IGF-1R.
Figure 2F: Formula 2 motif peptide sequences identified from a library constructed to contain variations outside the consensus core of the B6 peptide as indicated (referred to as "B6L") and panned against IR.
Figure 2G: Formula 2 motif peptide sequences identified from a library constructed to contain variations outside the consensus core of the B6 peptide as indicated (referred to as "B6L") and panned against IGF-1R.
Figure 2H: Formula 2 motif peptide sequences identified from a library constructed to contain a helix-turn-helix based on the B6 peptide as indicated (referred to as "B6H") and panned against IR.
Figure 2I: Formula 2 motif peptide sequences identified from a library constructed to contain a helix-turn-helix based on the B6 peptide as indicated (referred to as "B6H") and panned against IGF-1R.
Figure 2J: Formula 2 motif peptide sequences identified from a library constructed to contain variations in the consensus core of B6 peptide as indicated (referred to as "B6C") and panned against IR.
Figure 2K: Formula 2 motif peptide sequences identified from a library constructed to contain variations in the consensus core of B6 peptide as indicated (referred to as "B6C") and panned against IGF-1R.
Figure 2L: Formula 2 motif peptide sequences identified from a library constructed using the sequence X₁₋₆FYDAIDQLVX₁₆₋₂₁ (20E2-A) panned against IR.
Figure 2M: Formula 2 motif peptide sequences identified from a library constructed using the sequence X₁₋₆FYDAIDQLVX₁₆₋₂₁ (20E2-A) panned against IGF-1R.
Figure 2N: Formula 2 motif peptide sequences identified from a library constructed using the sequence X₁₋₆FYXXhXXhhX₁₆₋₂₁ (20E2-B) panned against IR.
Figure 20: Formula 2 motif peptide sequences identified from a library constructed using the sequence X₁₋₆FYXXhXXhhX₁₆₋₂₁ (20E2-B) panned against IGF-1R.

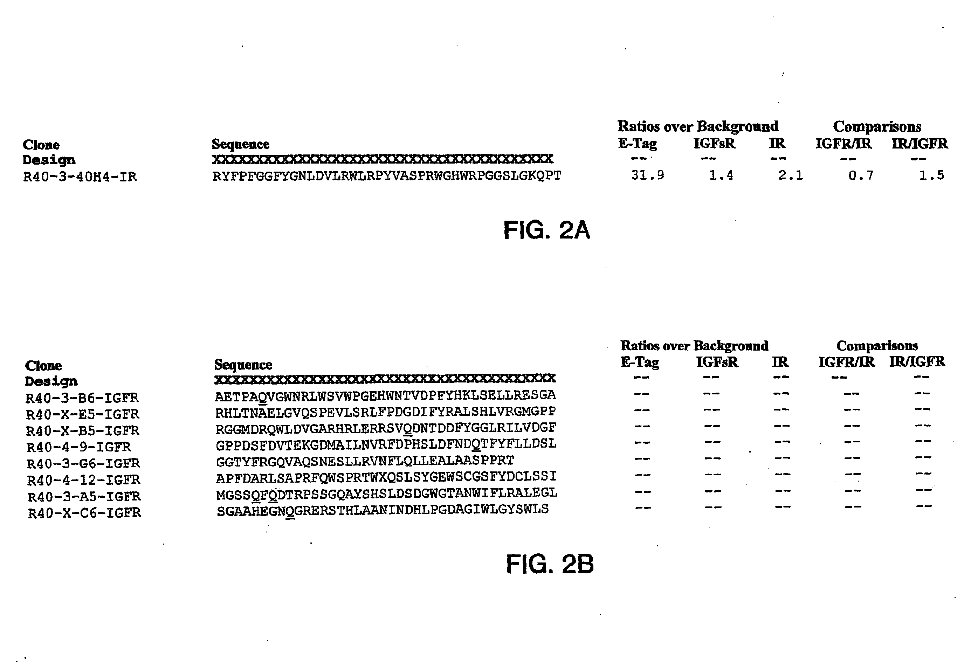

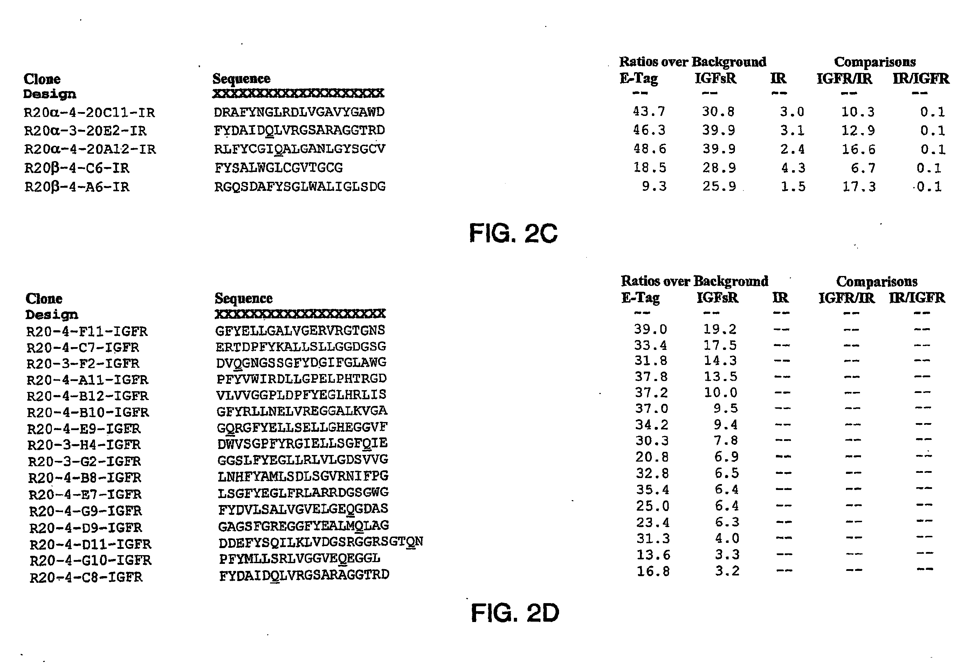

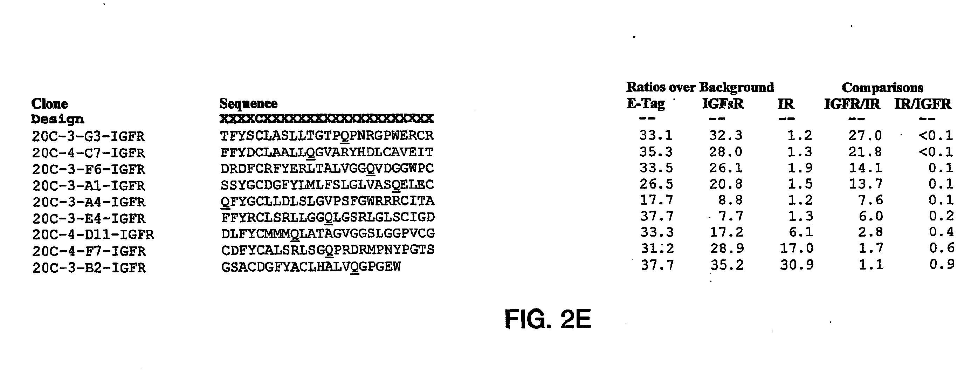

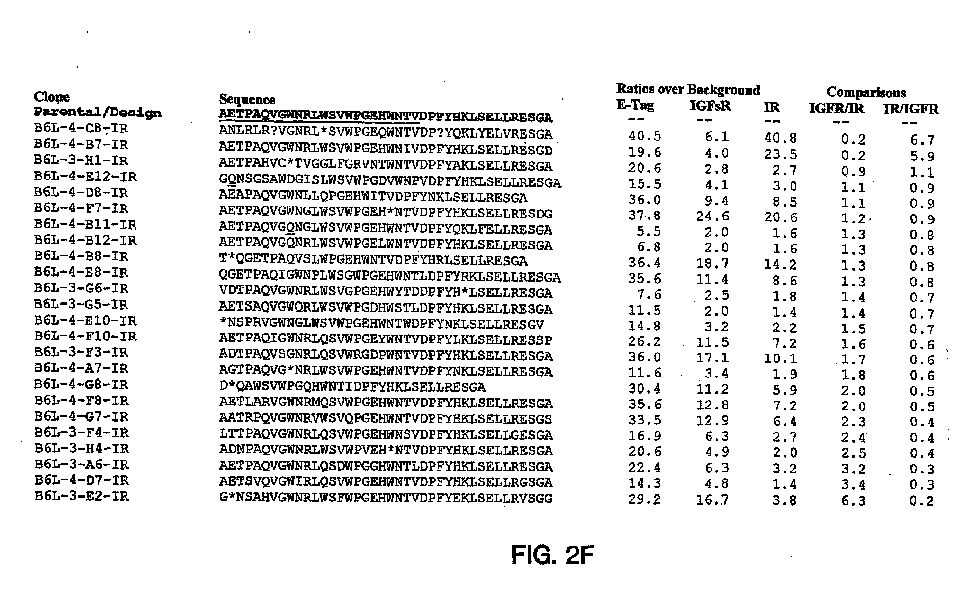

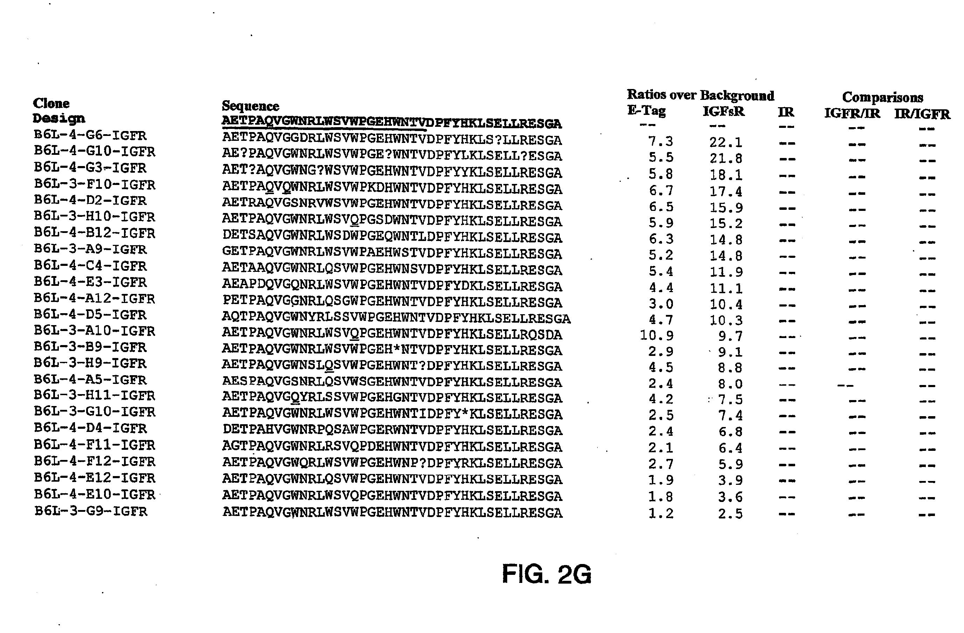

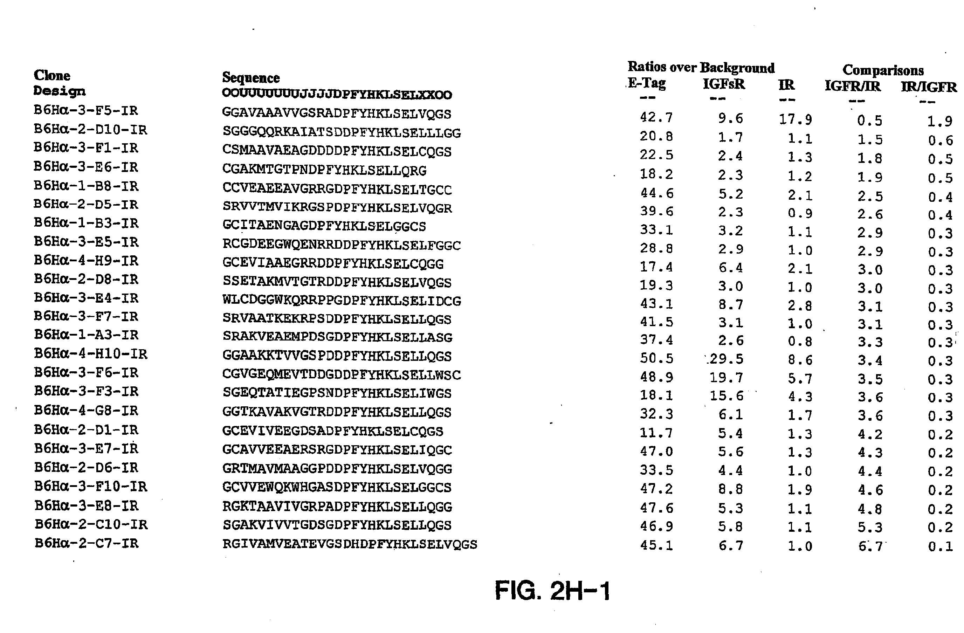

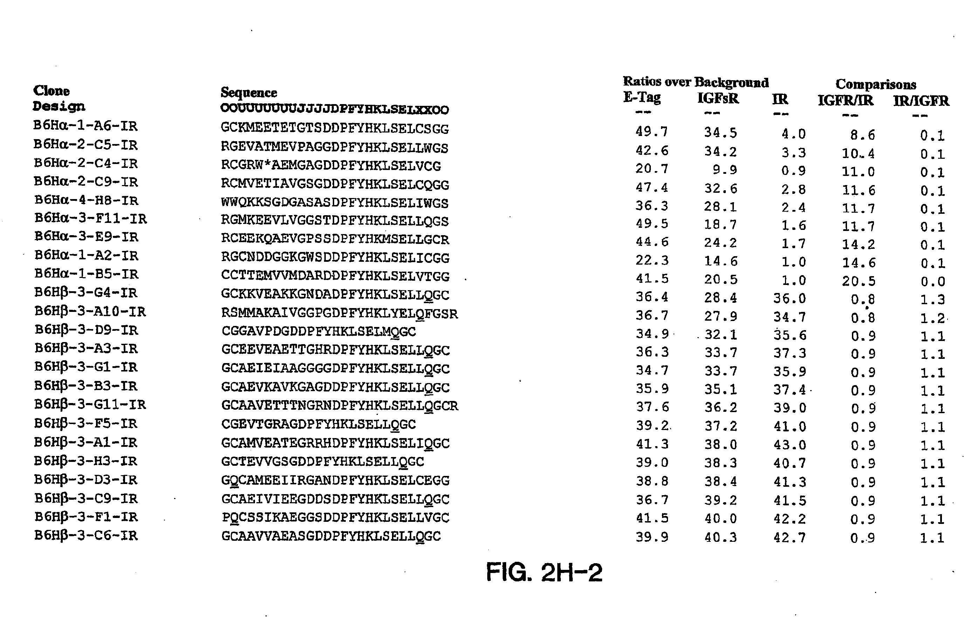

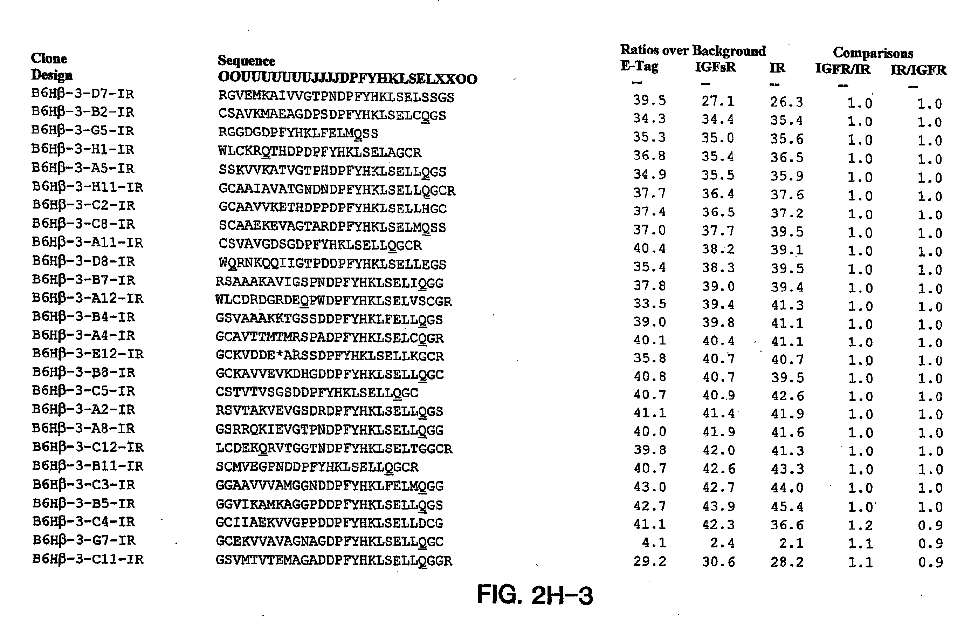

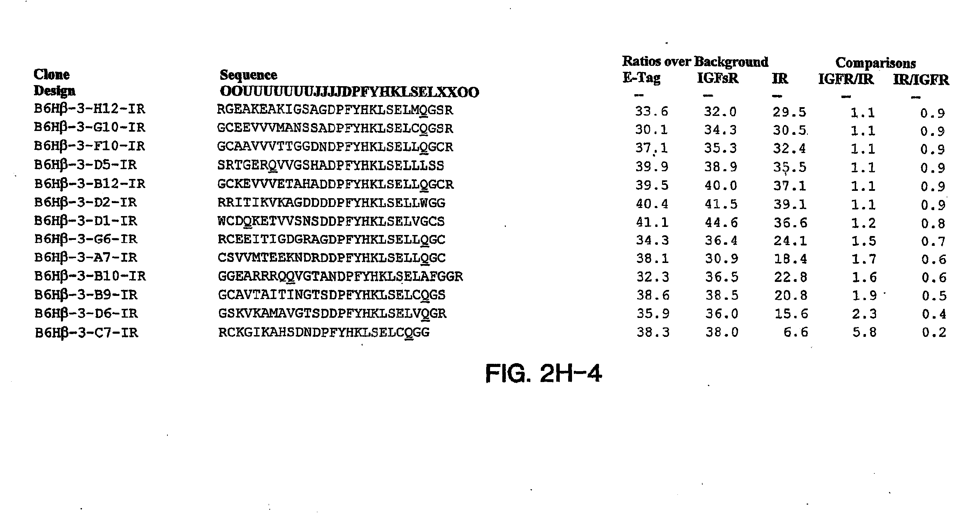

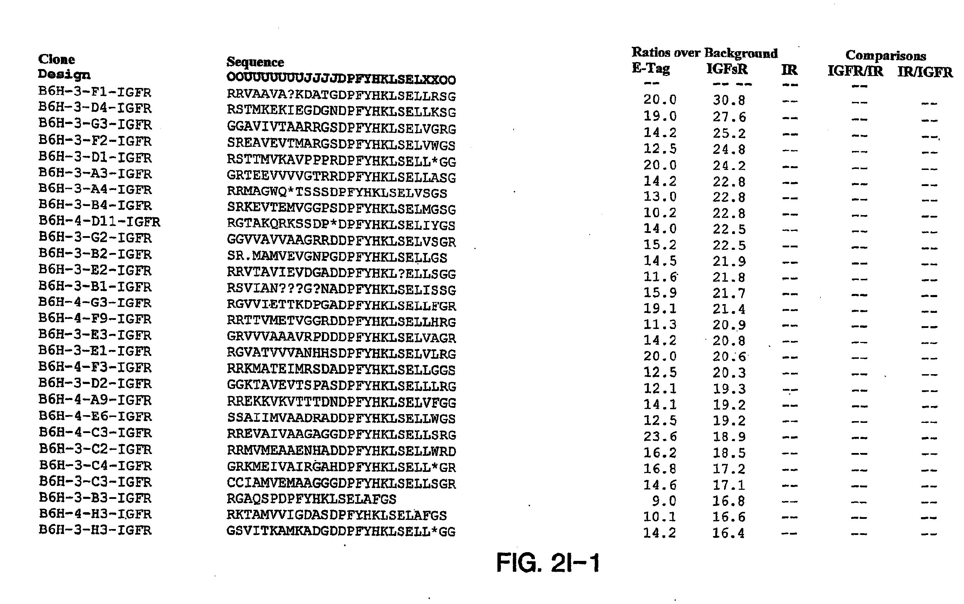

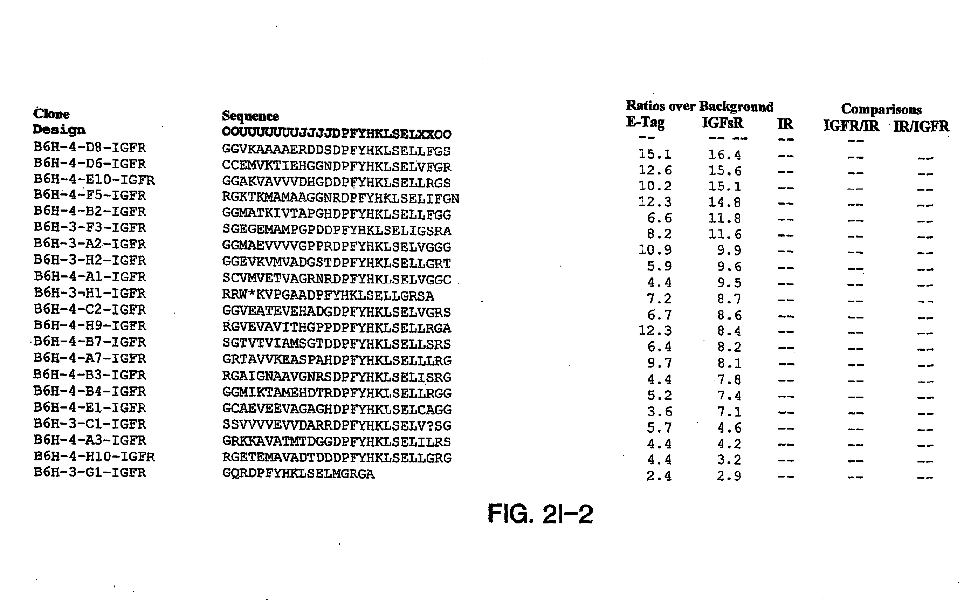

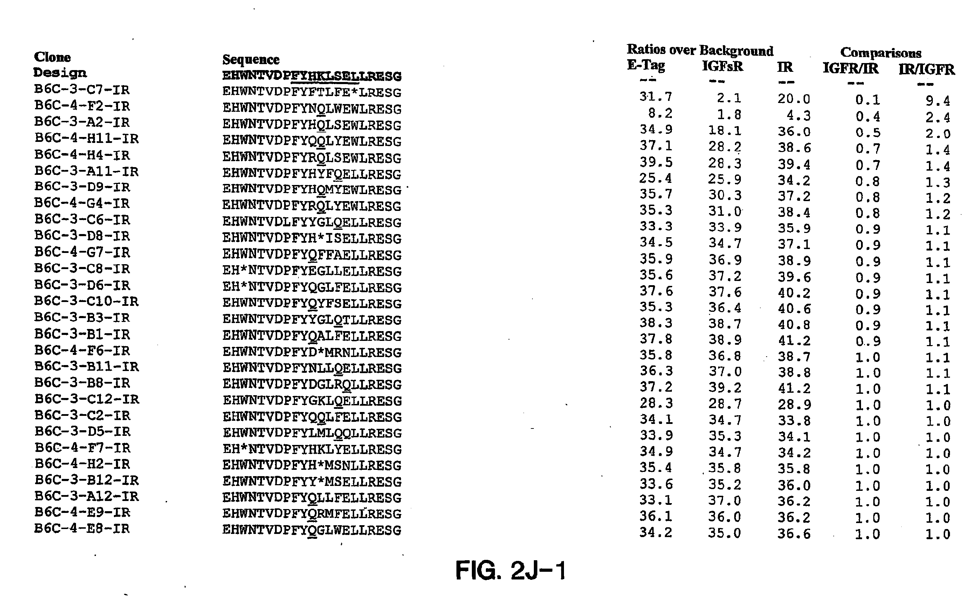

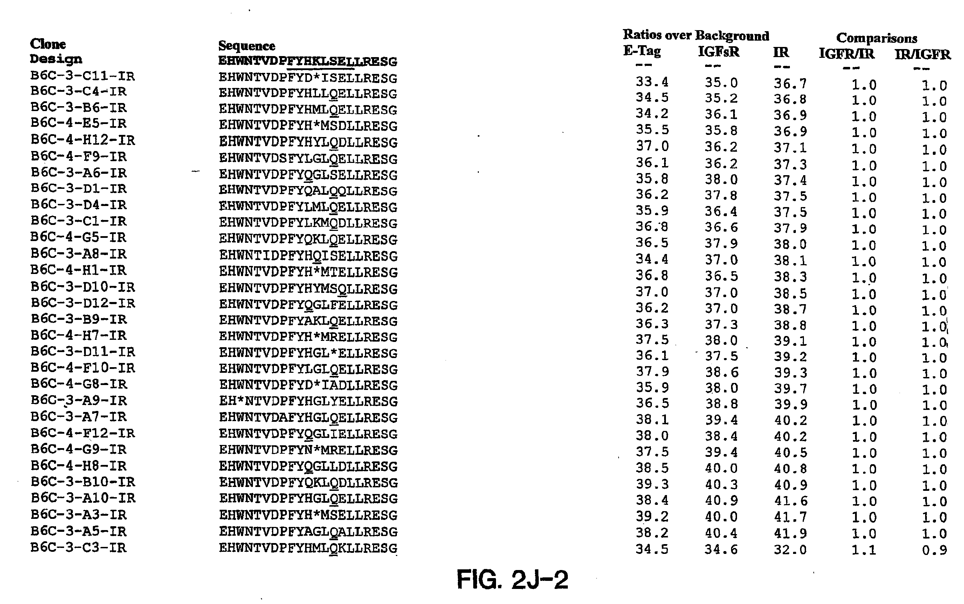

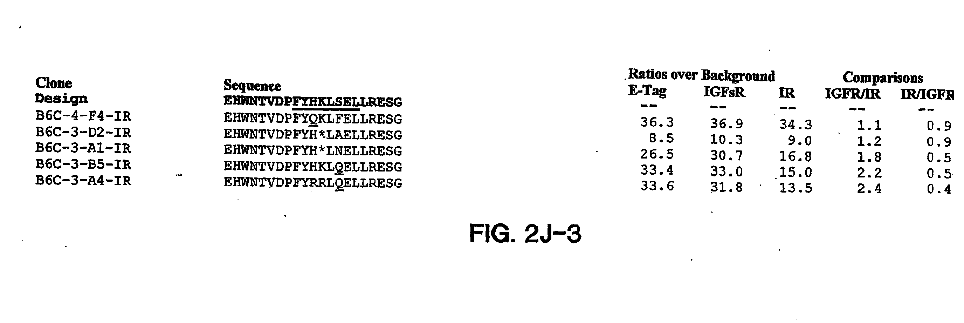

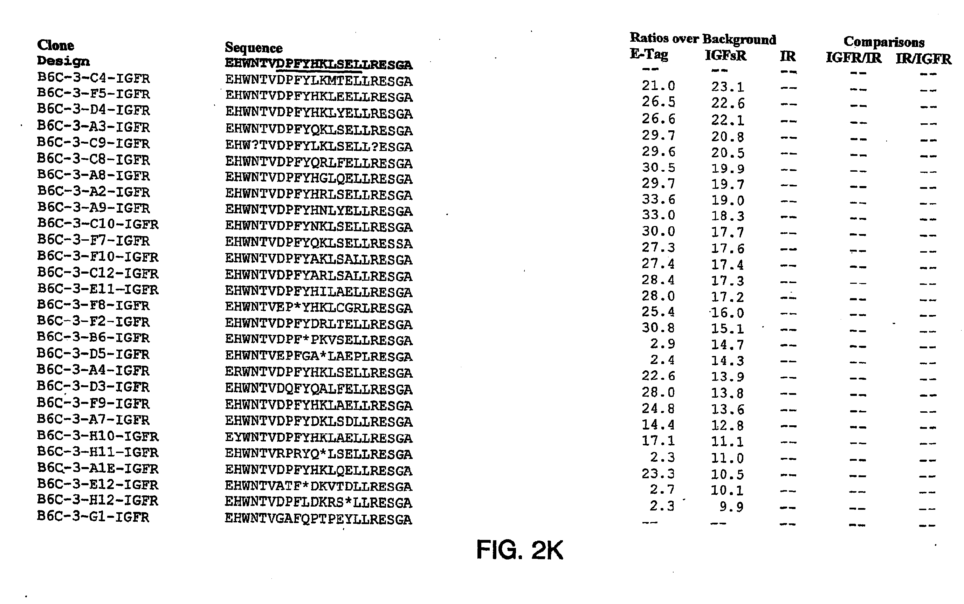

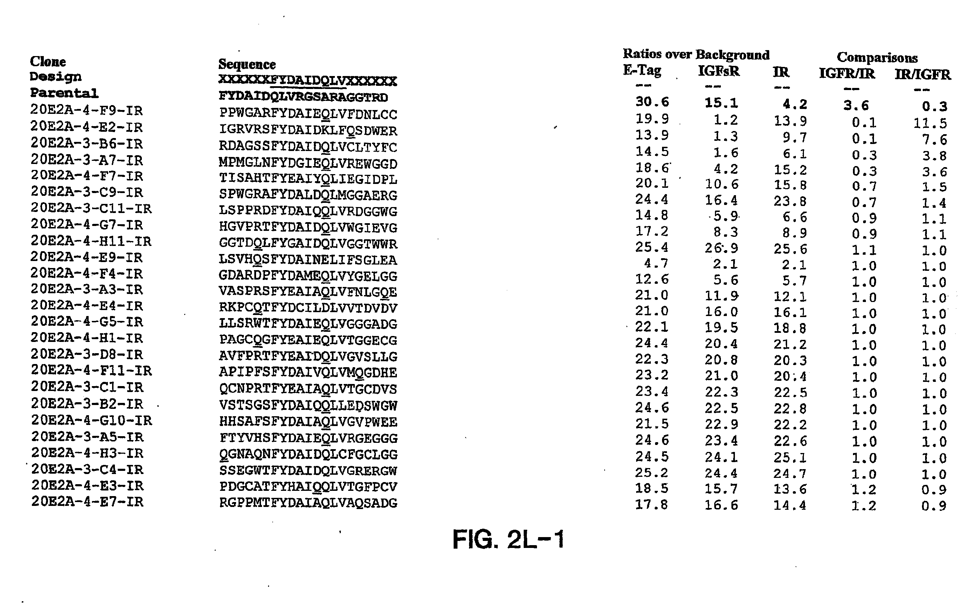

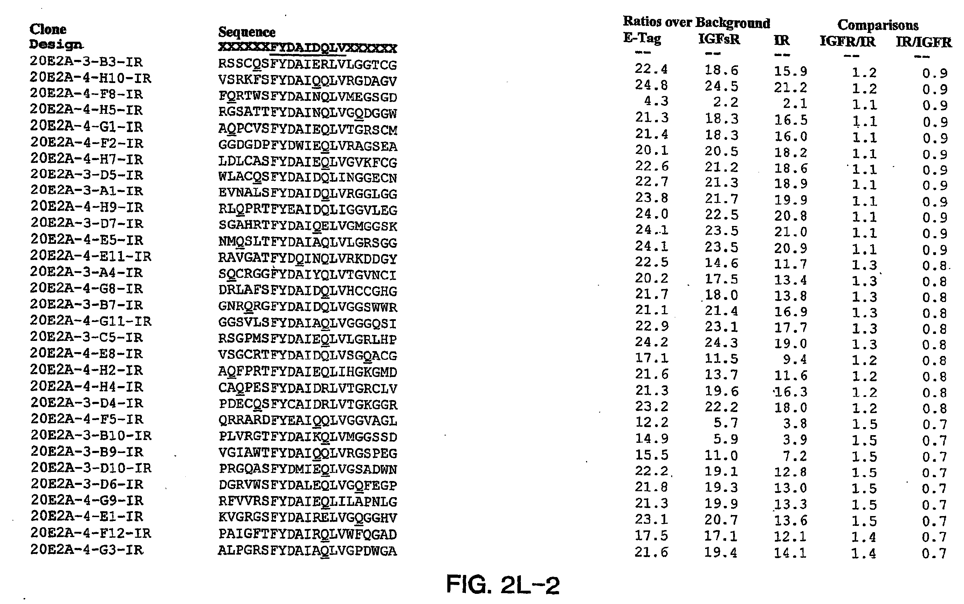

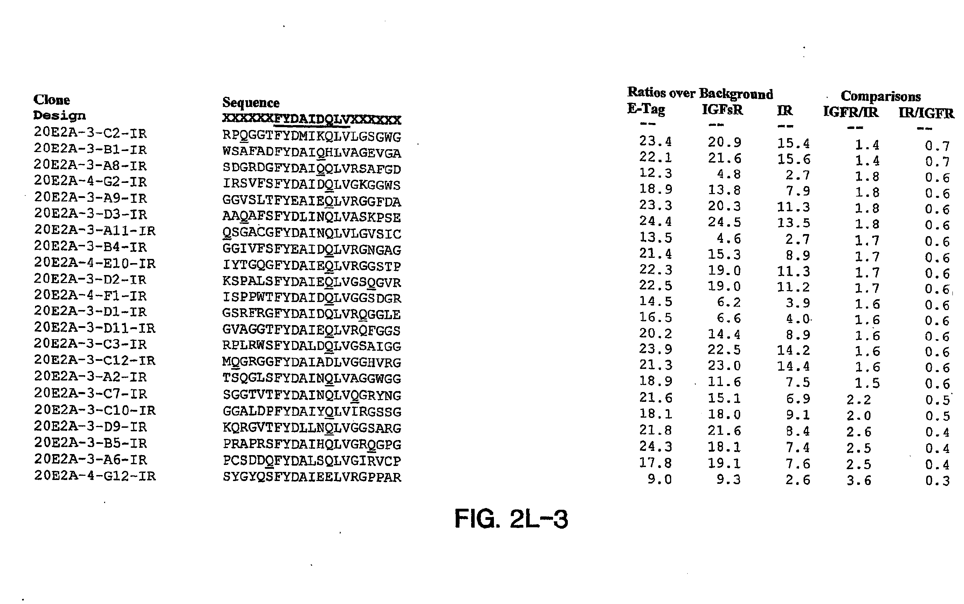

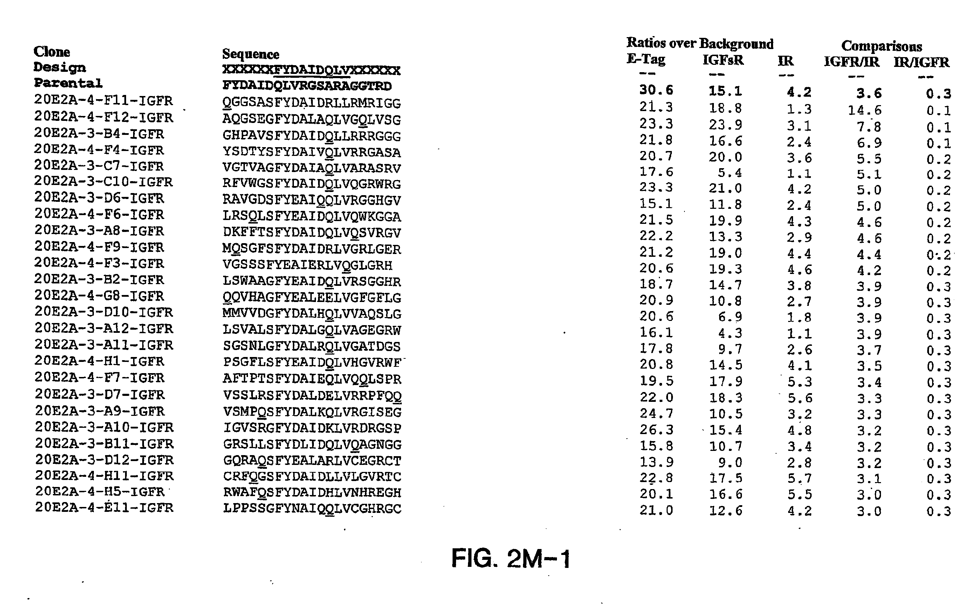

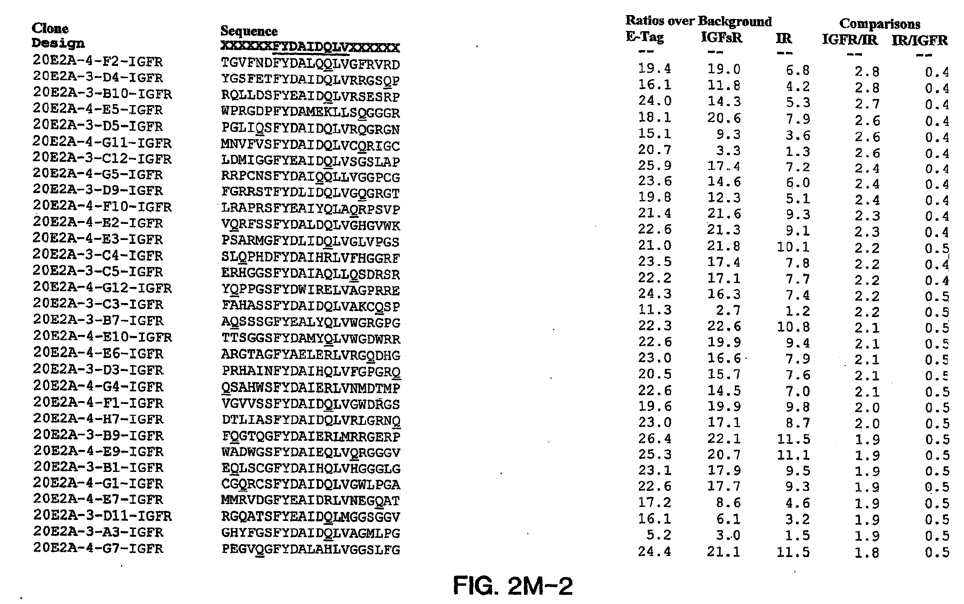

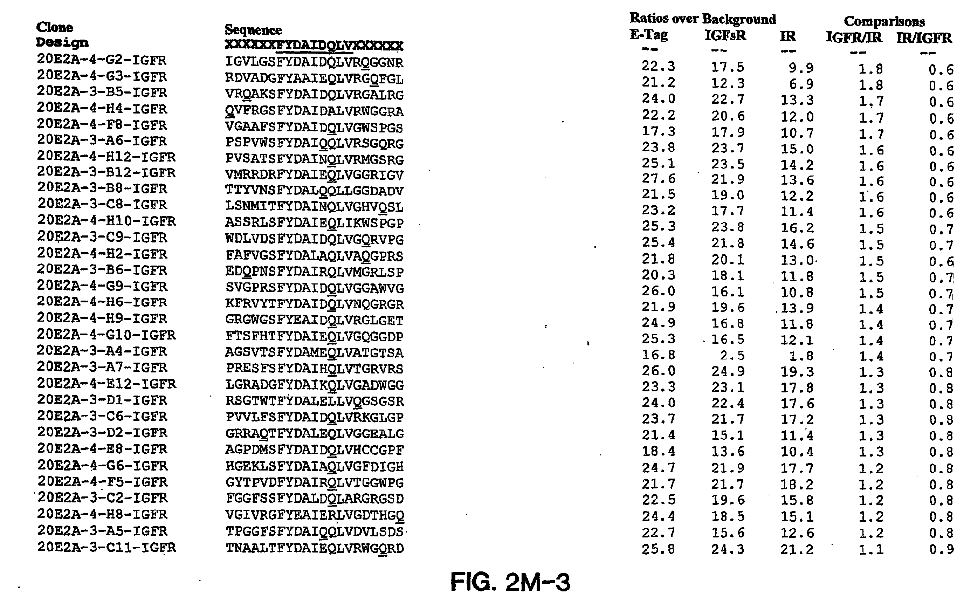

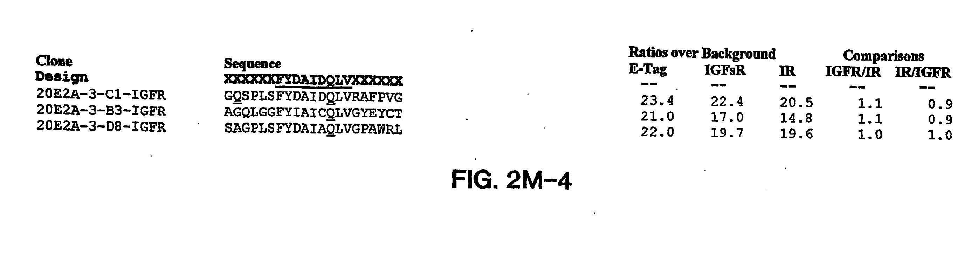

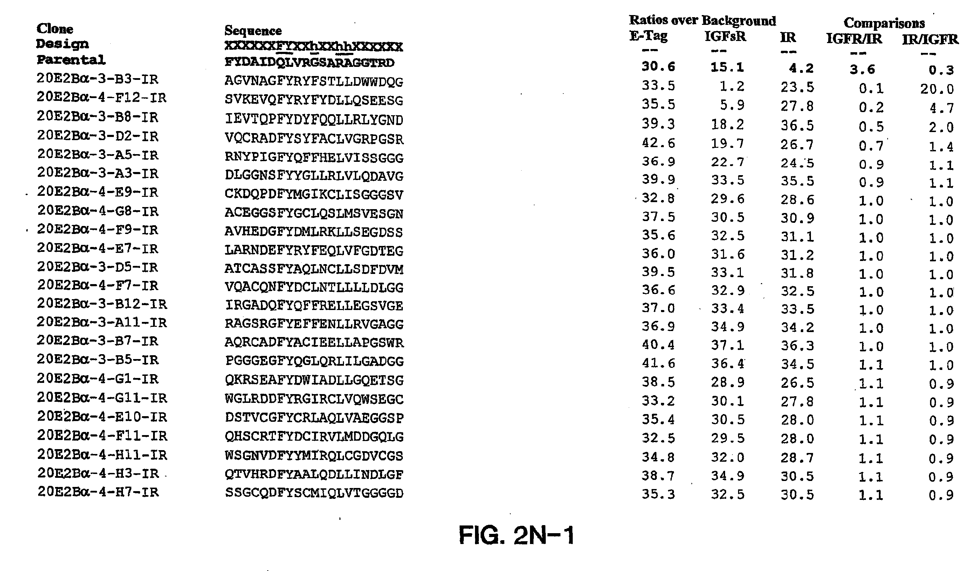

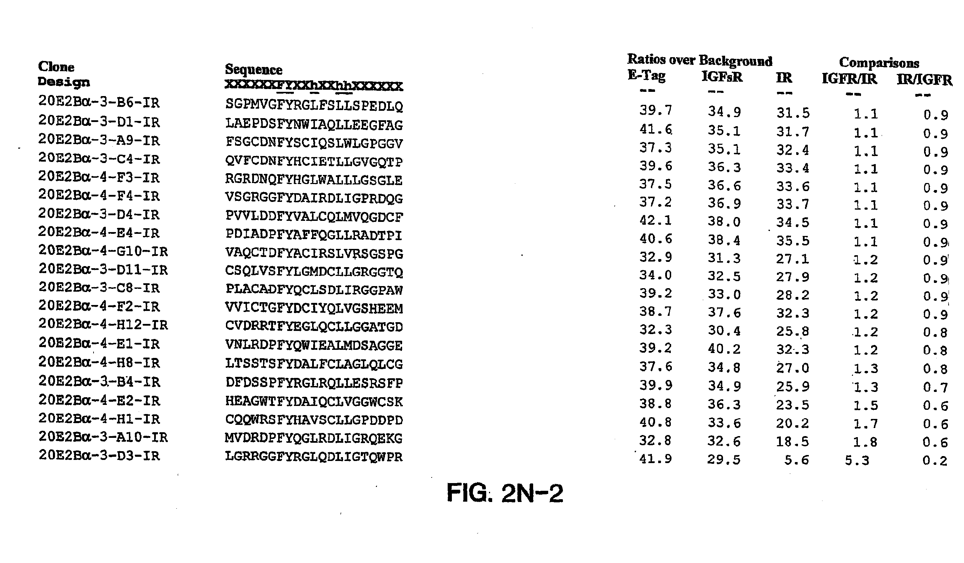

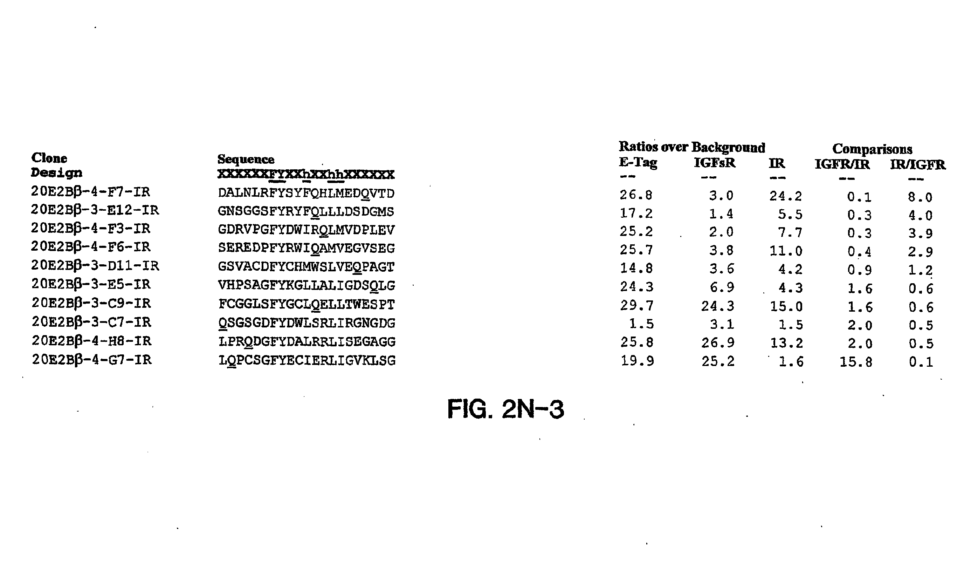

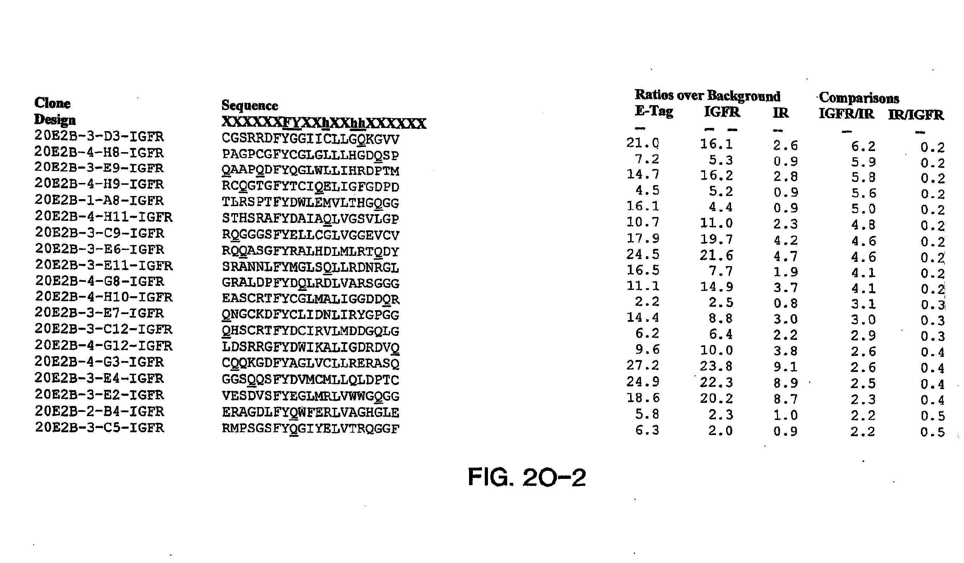

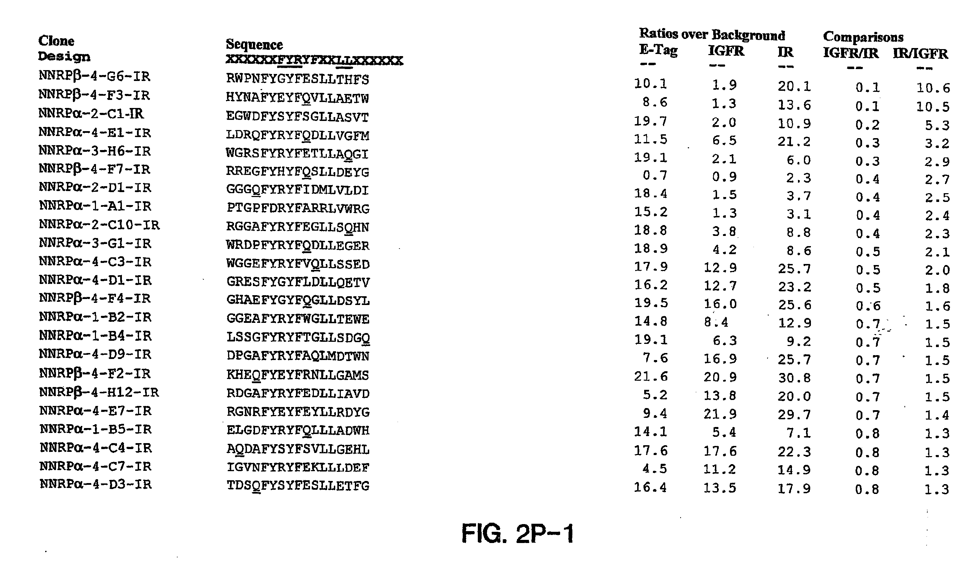

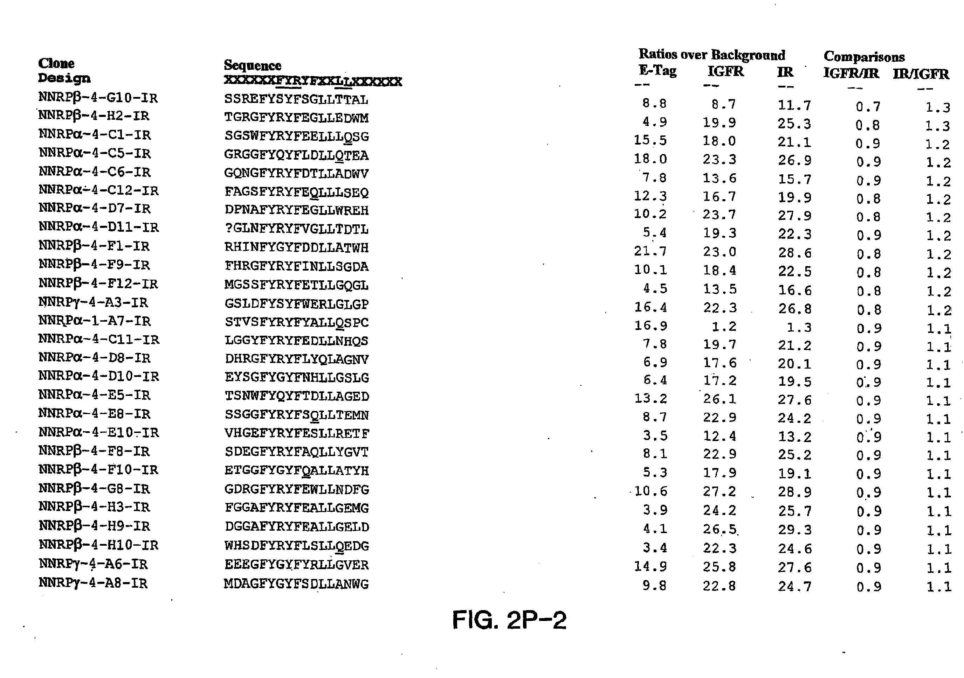

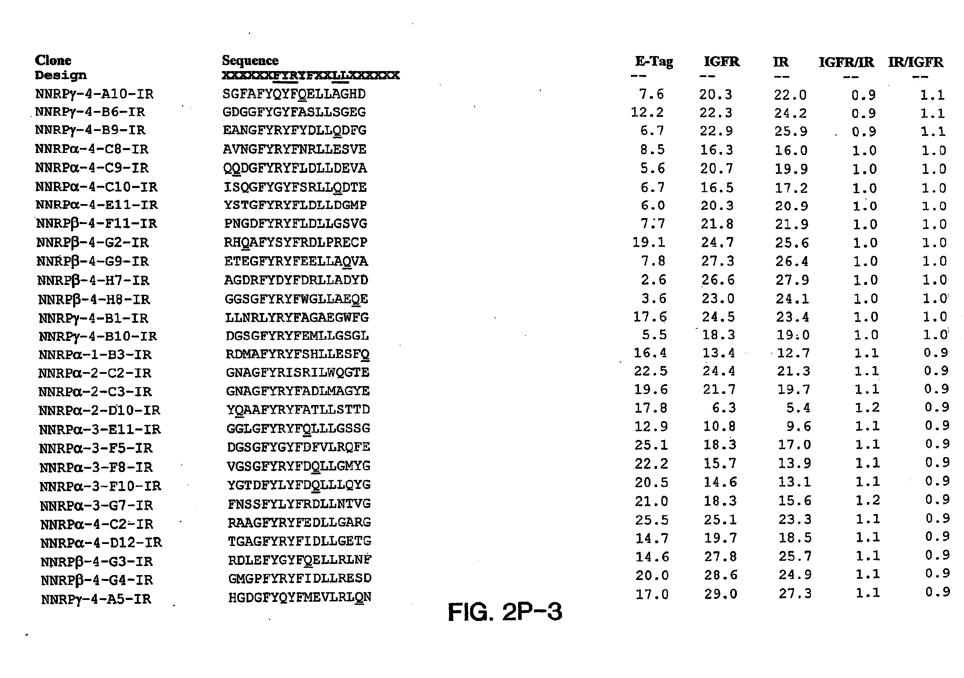

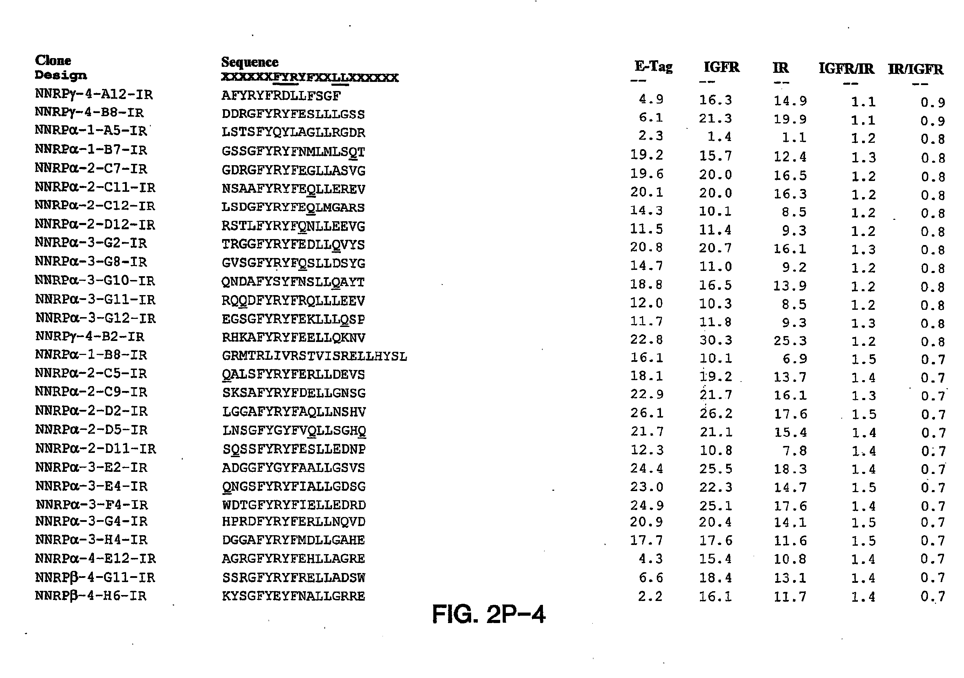

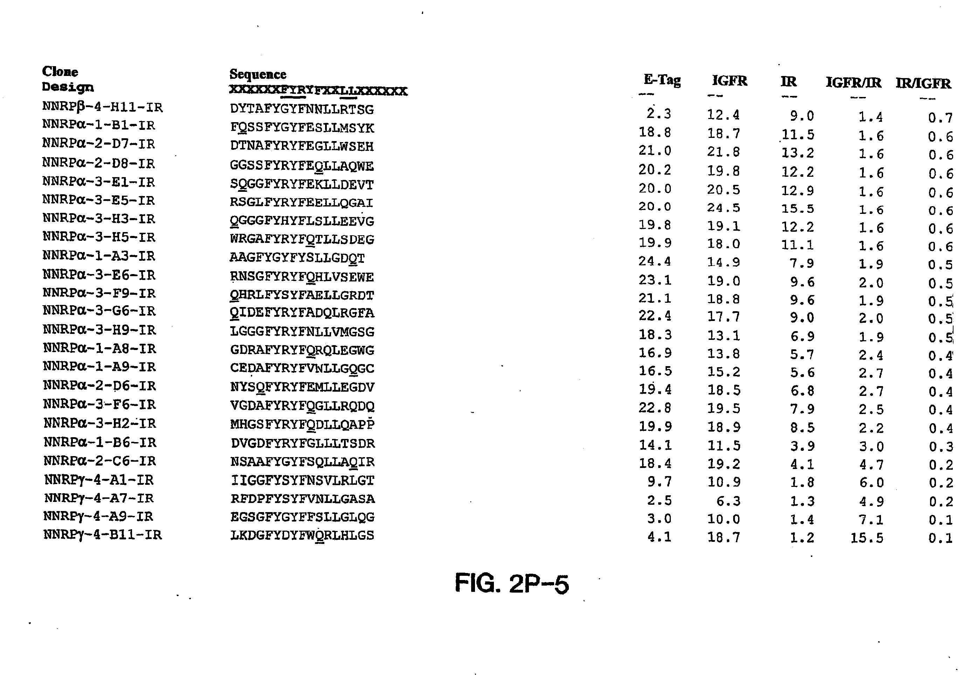

Figure 2P: Formula 2 motif peptide sequences identified from a library constructed using the sequence X₁₋₆FYRYFXXLLX₁₆₋₂₁ (NNRP) panned against IR.
Figure 3A: Formula 3 motif peptide sequences identified from a random 20mer library panned against IGF-1R.
Figure 3B: Formula 3 motif peptide sequences identified from a X_{1- 4}CX₆₋₂₀ library panned against IGF-1R.
Figure 3C: Formula 3 motif peptide sequences identified from a library constructed using the sequence X₃LXXLXXYFX₁₂₋₁₇ (reverse B6; rB6) panned against IR.
Figure 3D: Formula 3 motif peptide sequences identified from a library constructed using the sequence X₃LXXLXXYFX₁₂₋₁₇ (reverse B6; rB6) panned against IGF-1R.
Figure 4A: Formula 4 motif peptide sequences identified from a random 20mer library panned against IR.
Figure 4B: Formula 4 motif peptide sequences identified from a library constructed to contain variations in the F8 peptide as indicated (15% dope; referred to as "F815") panned against IR.
Figure 4C: Formula 4 motif peptide sequences identified from a library constructed to contain variations in the F8 peptide as indicated (15% dope; referred to as "F815") panned against IGF-1R.
Figure 4D: Formula 4 motif peptide sequences identified from a library constructed to contain variations in the F8 peptide as indicated (20% dope; referred to as "F820") panned against IR.
Figure 4E: Formula 4 motif peptide sequences identified from other libraries panned against IR.
Figure 5: Formula 5 motif peptide sequences identified from a library constructed to contain variations in the F8 peptide as indicated (15% dope; referred to as "F815") panned against IGF-1R.
Figure 6A: Formula 6 motif peptide sequences identified from a random 20mer library and panned against IR.
Figure 6B: Formula 6 motif peptide sequences identified from a library constructed to contain variations in the D8 peptide as indicated (15% dope; referred to as "D815") panned against IR.
Figure 6C: Formula 6 motif peptide sequences identified from a library constructed to contain variations in the D8 peptide as indicated (20% dope; referred to as "D820") panned against IR.
Figure 6D: Formula 6 motif peptide sequences identified from a library constructed to contain variations in the D8 peptide as indicated (20% dope; referred to as "D820") panned against IGF-1R.
Figure 6E: Formula 6 motif peptide sequences identified from other libraries panned against IR.
Figure 7: Formula 7 motif peptide sequences.
Figure 8: Formula 8 motif peptide sequences identified from a commercial phage display peptide library and synthetic sequences. Small letters denote D-amino acids. Unnatural amino acids are denoted with a 3-letter abbreviation in certain sequences. K_{d} values greater than 2 x 10⁻⁵ are approximate.
Figure 9A: Formula 9 motif peptide sequences identified from a library constructed to contain variations in the H5 peptide as indicated (referred to as "H5") panned against IGF-1R.
Figure 9B: Formula 9 motif peptide sequences identified from a library constructed to contain variations in the JBA5 peptide as indicated (referred to as "JBA5") panned against IGF-1R.
Figure 9C: Formula 9 motif peptide sequences identified from a library constructed to contain variations in the JBA5 peptide as indicated (referred to as "JBA5") panned against IR.
Figure 10A: Formula 10 motif peptide sequences identified from random 20mer libraries panned against IGF-1R.
Figure 10B: Formula 10 motif peptide sequences identified from random 20mer libraries panned against IR.
Figure 10C: Miscellaneous peptide sequences identified from a random 20mer library panned against IR.
Figure 10D: Miscellaneous peptide sequences identified from a random 40mer library panned against IR.
Figure 10E: Miscellaneous peptide sequences identified from a random 20mer library panned against IGF-1R.
Figure 10F: Miscellaneous peptide sequences identified from a X₁-₄CX₆₋₂₀ and panned against IGF-1R.
Figure 10G: Miscellaneous peptide sequences identified from a library constructed to contain variations of the F8 peptide as indicated (F815) panned against IGF-1R.
Figure 10H: Miscellaneous peptide sequences identified from a library constructed to contain variations in the F8A11 peptide as indicated (referred to as "NNKH") panned against IR.
Figure 10I: Miscellaneous peptide sequences identified from a library constructed to contain variations in the F8A11 peptide as indicated (referred to as "NNKH") panned against IGF-1R.

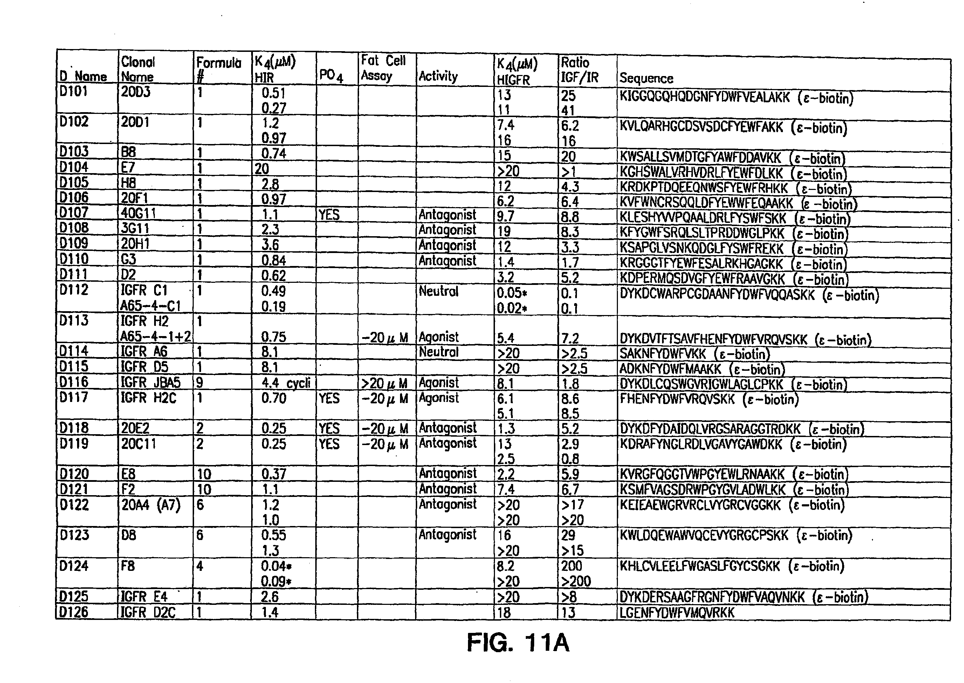

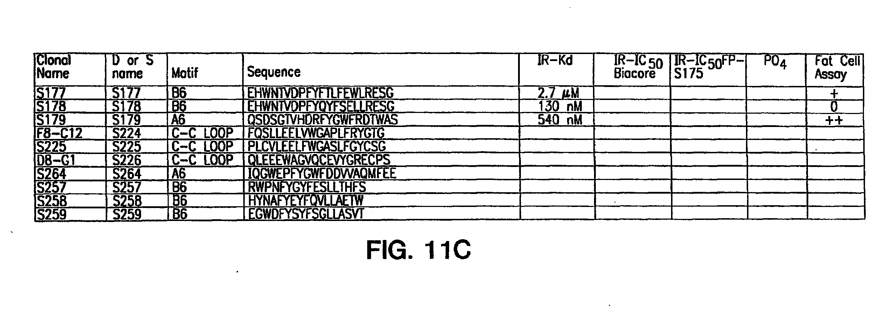

Figure 11A: Summary of specific representative amino acid sequences from Formulas 1 through 11.

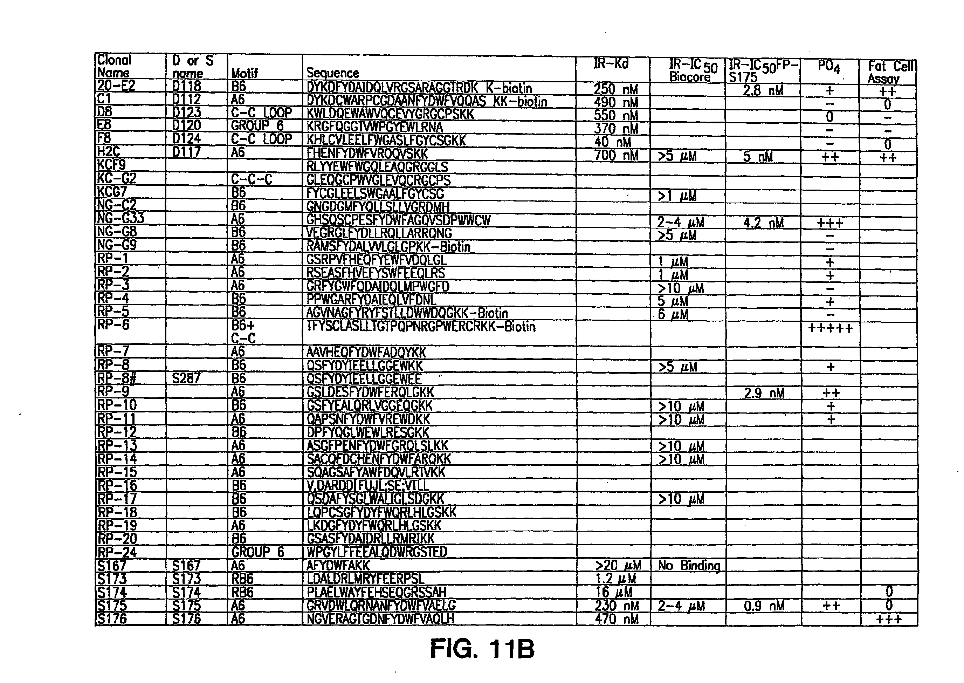

Figure 11 B: Summary of specific representative amino acid sequences from Formulas 1 through 11.

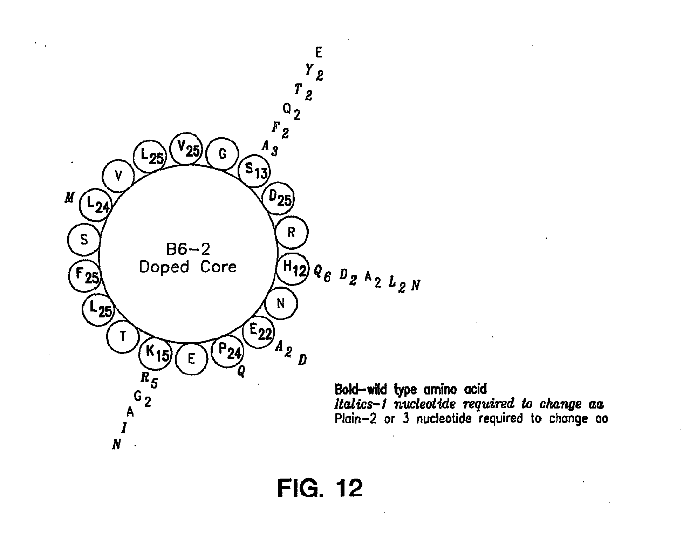

Figure 12: Illustration of helix wheels applied to Formula 2 and 3 motifs.

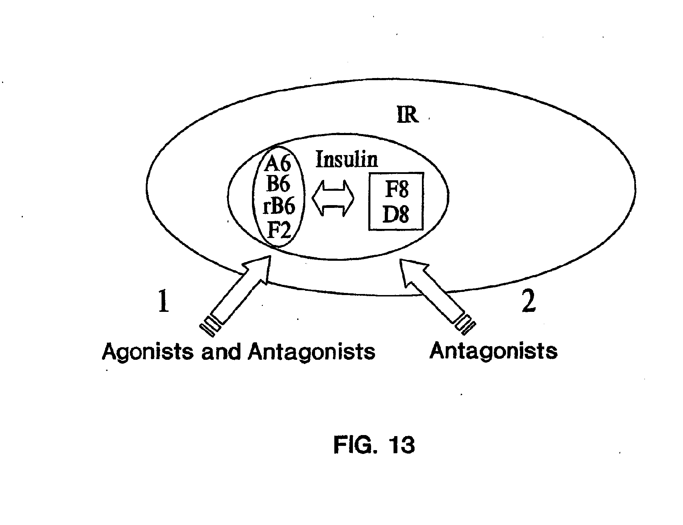

Figure 13: Illustration of 2 binding site domains on IR based on competition data.

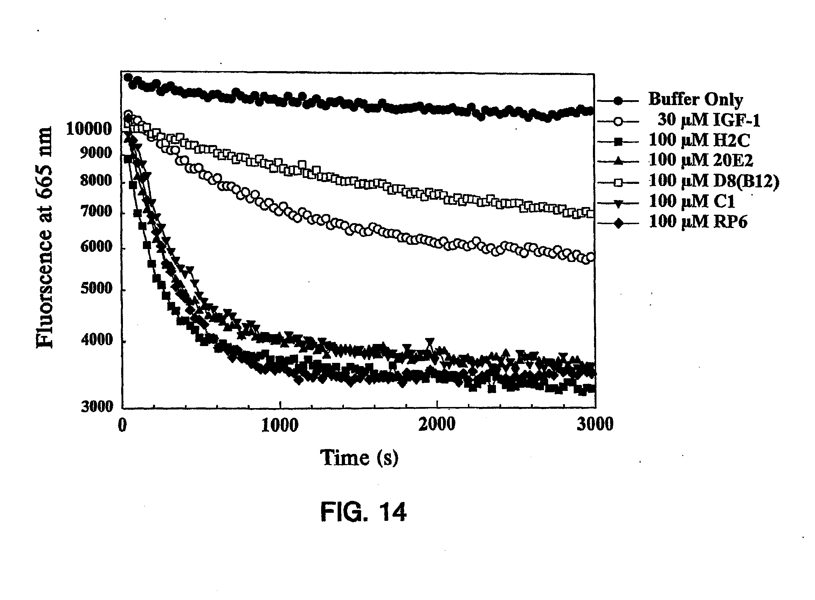

Figure 14: Dissociation of 20E2 peptide from IGF-1R in the presence of buffer (filled circle), 30 µM IGF-1 (open circle), 100 µM H2C (filled square), 100 µM 20E2 (filled triangle), 100 µM D8 (B12; open square), 100 µM C1 (filled, inverted triangle) and 100 µM RPG (filled diamond).

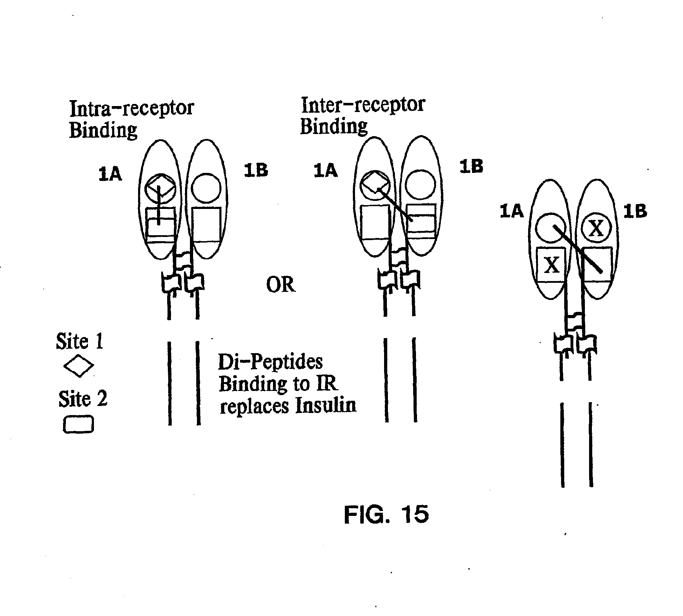

Figure 15: Schematic illustration of potential binding schemes to the multiple binding sites on IR.

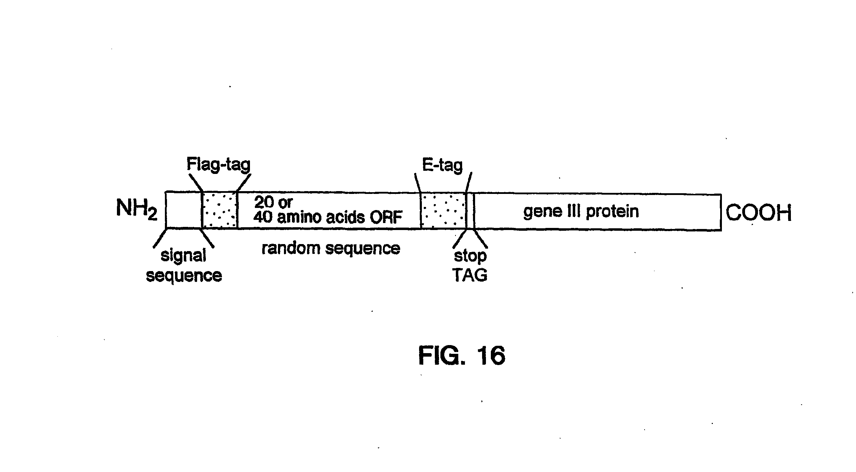

Figure 16: Schematic diagram of the phage-displayed peptide library. The peptide is displayed as a protein fusion to the N-terminus of gene *III* encoding the minor coat protein of the phage.

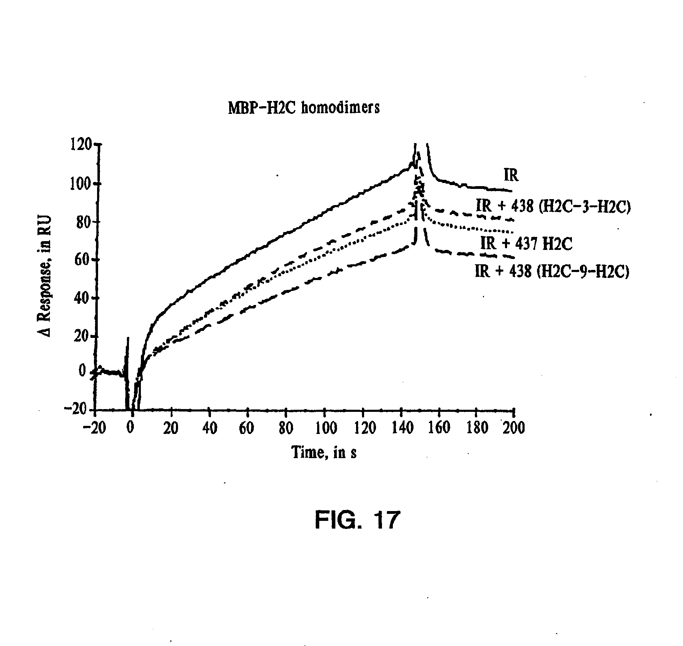

Figure 17: BIAcore analysis of competition binding between IR and MBP fusion H2C-9-H2C, H2C and H2C-3-H2C.

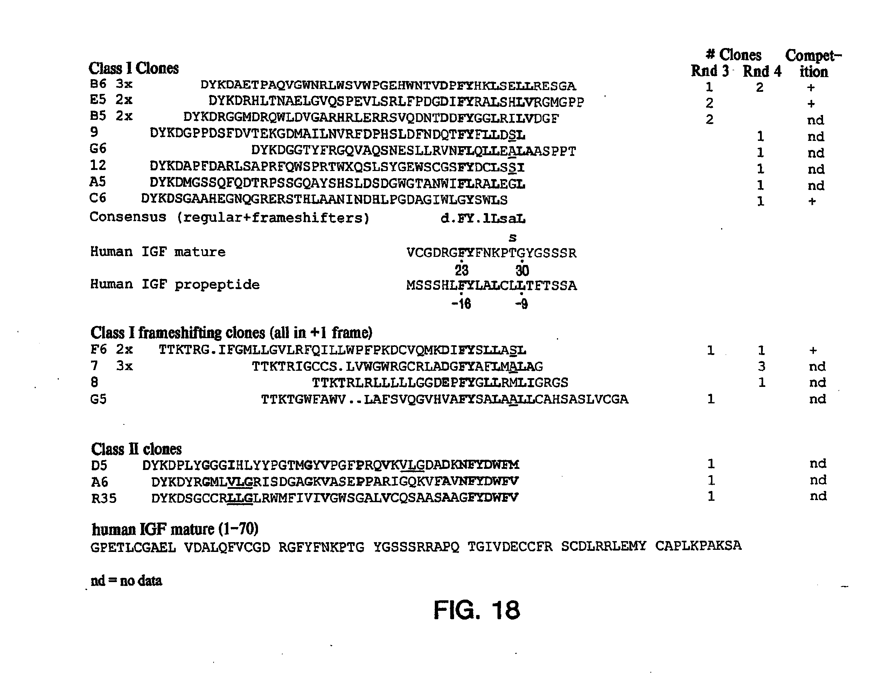

Figure 18: Sequence alignments of Class I and Class II peptides.
   The Class I peptides have been shown to be IGF-1R antagonists, while the Class II peptides are IGF-1R agonists.

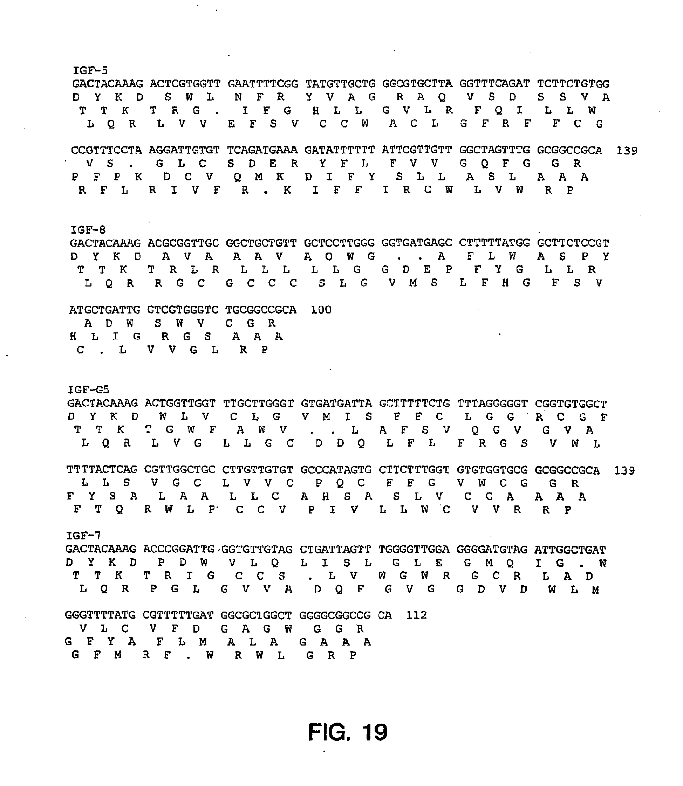

Figure 19: DNA sequences of the frameshifted clones.

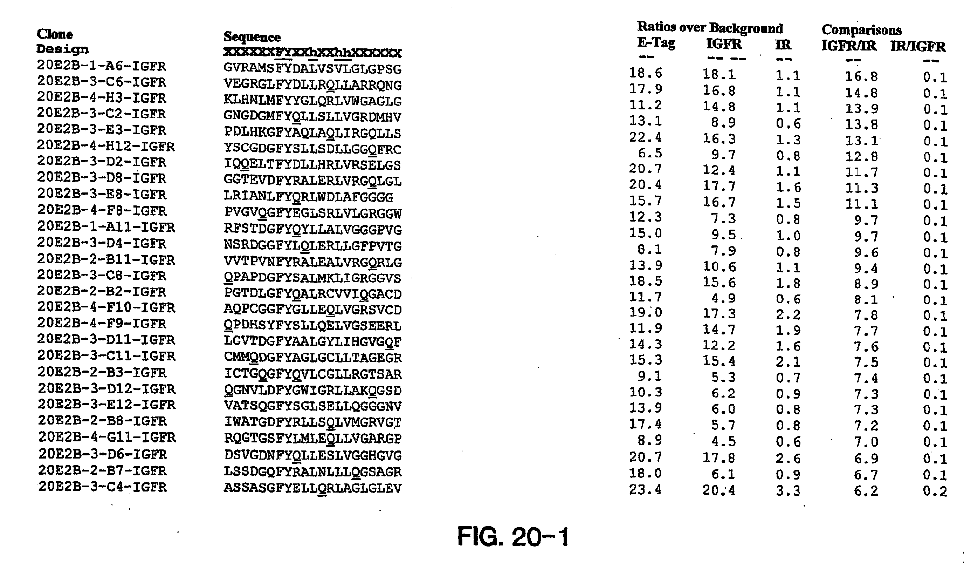

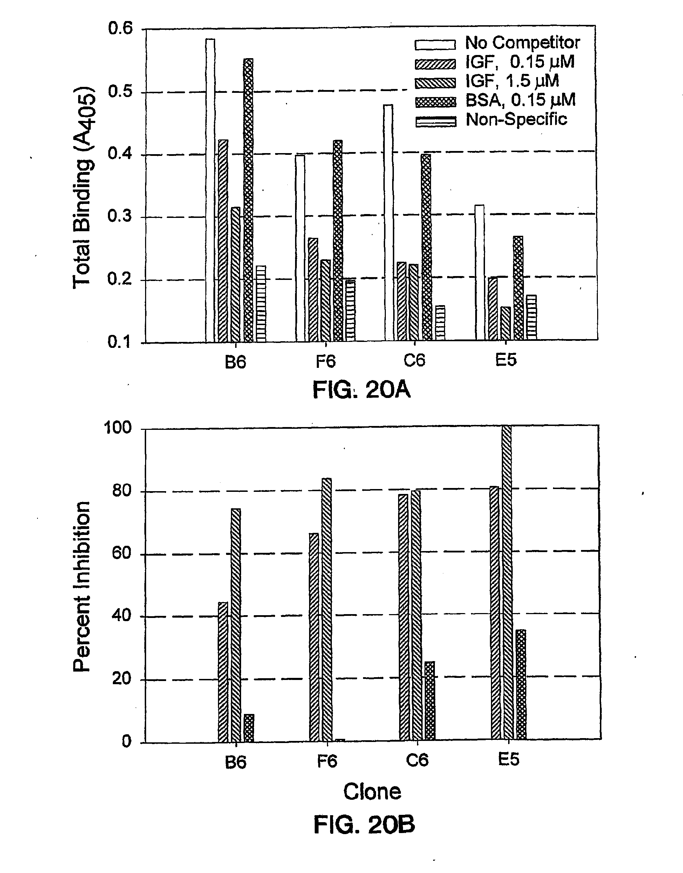

Figures 20A and 20B: Results of the phage ELISA for binding to IGF-1R. Wells were coated with 100 ng/well IGF-1R and blocked. Competitor, the IGF-1 native ligand, was present prior (1 h) and during the phage incubation (1 h). Phage were detected with HRP-anti M13 phage antibody and reported as OD₄₀₅ as described. Total Binding is shown in Figure 20A and Percent Inhibition is shown in Figure 20B.

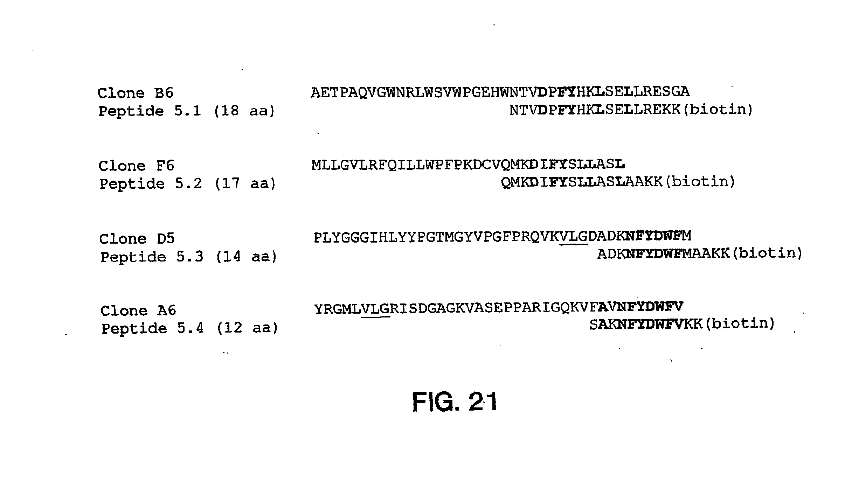

Figure 21: Sequences of the designed IGF-1R-specific synthetic peptides.

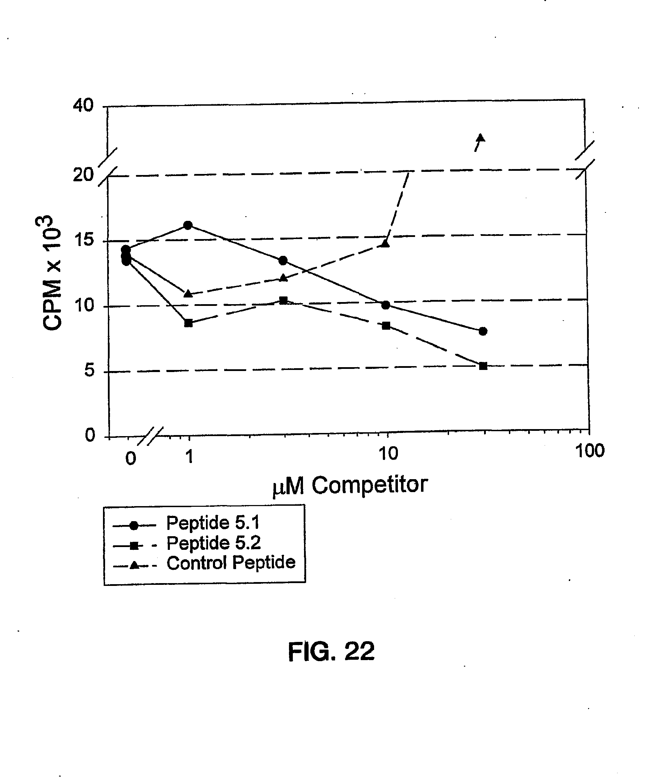

Figure 22: Assay results showing that Motif 2 peptides (5.1 and 5.2) antagonize the effects of IGF-1 on IGF-1R⁺ cells.

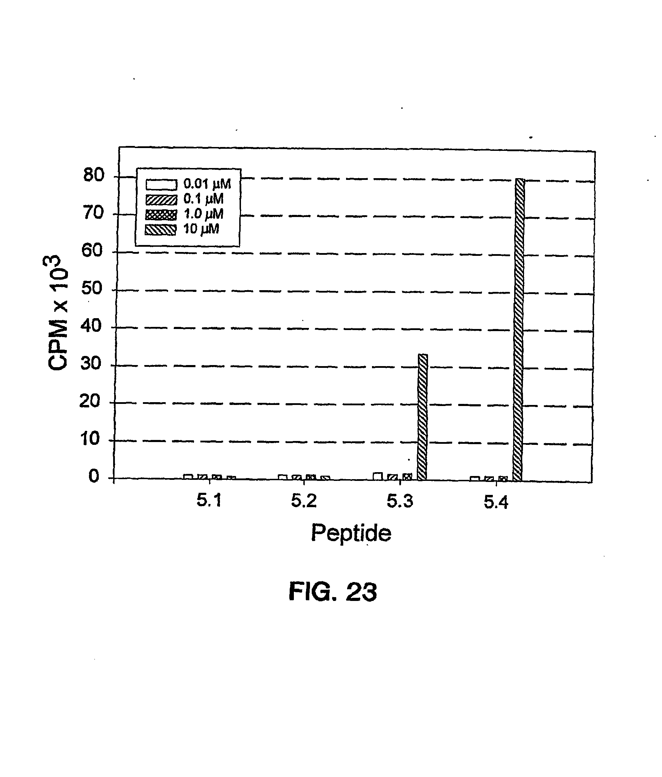

Figure 23: Assay results showing that Motif 1 peptides (5.3 and 5.4) stimulate growth of IGF-1R⁺ cells. Cells expressing human IGF-1R (30,000 cells per well) were incubated with the 5.4 peptide for 42 h at 37°C. Experiments were done in triplicate. Background signal A₄₅₀=0.15. Proliferation was measured using WST-1 reagent (Boehringer Mannheim Biochemicals/Roche Molecular Biochemicals, Indianapolis, IN).

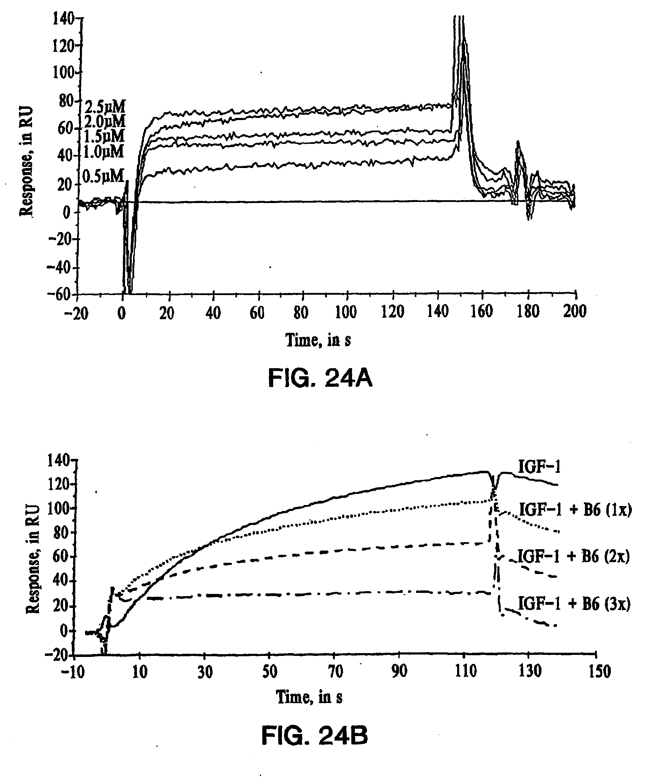

Figures 24A and 24B: Demonstration of binding of peptide 5.1 to IGF-1R using BIAcore. Figure 24A: Binding as a function of the peptide concentration. Figure 24B: Inhibition of IGF-1 binding by peptide 5.1. RU - refractive units.

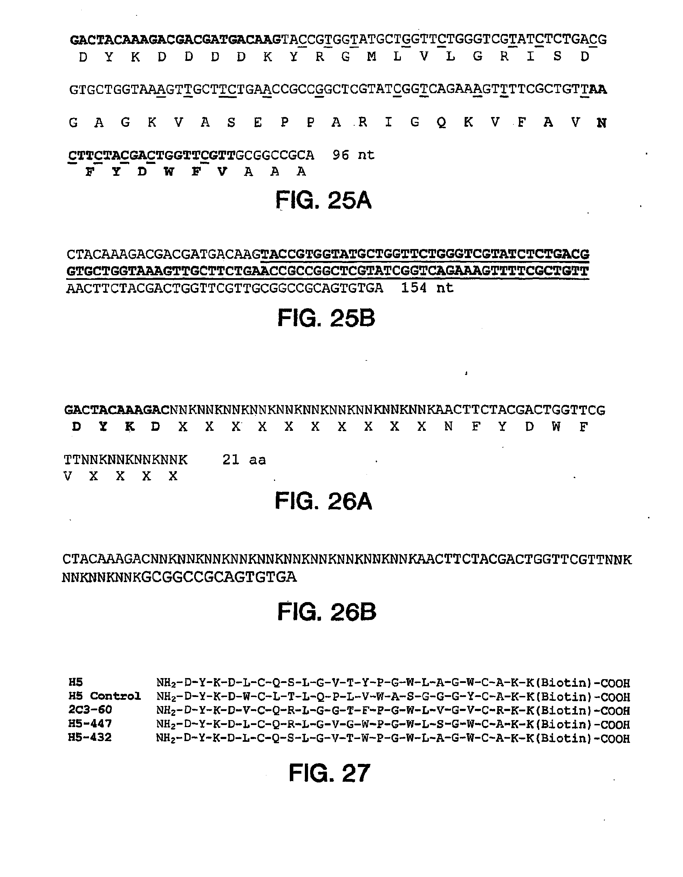

Figures 25A and 25B: Design of the secondary phage library A6L based on the Class II peptide sequences. Figure 25A: Design of the sequence of the gene. Underlined residues indicate positions mutated to optimize the codons for expression in *E*. *coli.* Figure 25B: Synthetic oligonucleotide for the A6L secondary library. Underlined residues were doped in the chemical DNA synthesis. Definitions of mixes (all mixes are equimolar) are as follows: N = A, C, G, or T; K = G or T. Nucleosides were premixed in the bottle (and not line mixed) to improve the accuracy of nucleoside mixes: The sequence of the FLAG epitope is shown in bold.
Figures 26A and 26B: Design of the secondary phage library A6S based on the Motif 1 peptide sequences. Figure 26A: Sequence design for the A6S secondary phage library. Figure 26B: Synthetic oligonucleotide for the A6L secondary library. Definitions of mixes (all mixes are equimolar) are as follows: N = A, C, G, or T; K = G or T. Nucleosides were premixed in the bottle (and not line mixed) to improve the accuracy of nucleoside mixes.

The sequence of the FLAG epitope is shown in bold.
Figure 27: Sequences of the five H5-like peptides that show agonistic activity toward IGF-1R. The C-terminal lysine contains a biotin moiety linked to the amino group of the side chain.

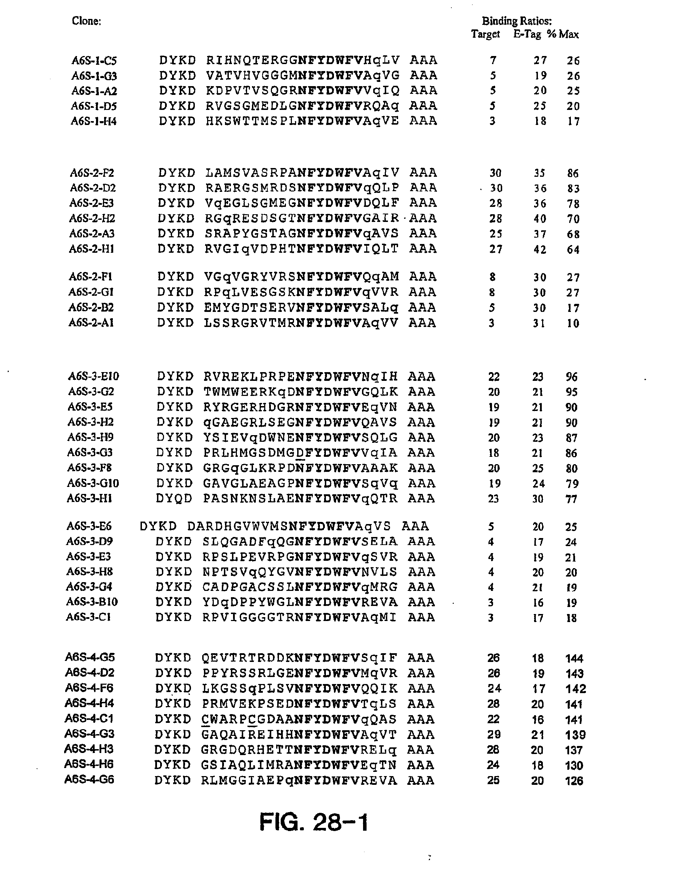

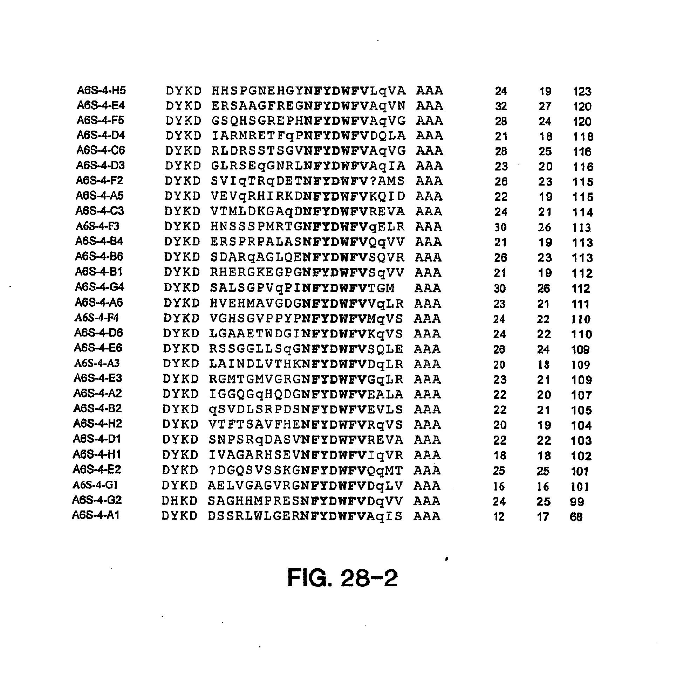

Figure 28: Listing of amino acid sequences obtained from panning with the A6S library.

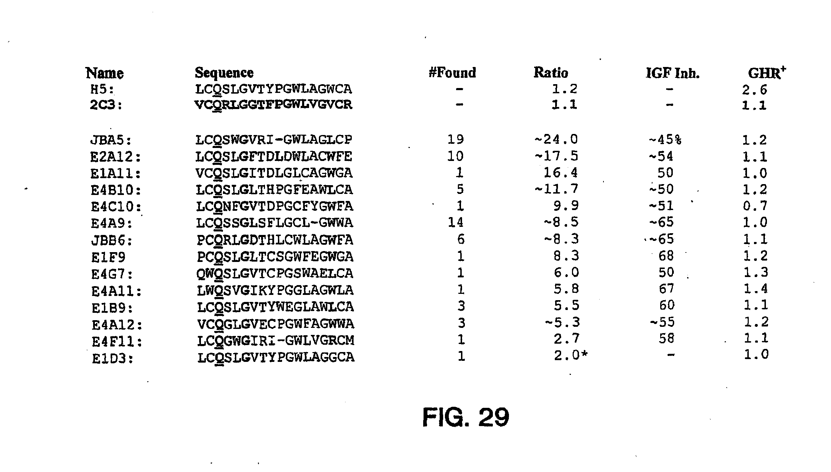

Figure 29: Listing of amino acid sequences obtained from panning with the H5 secondary phage library.

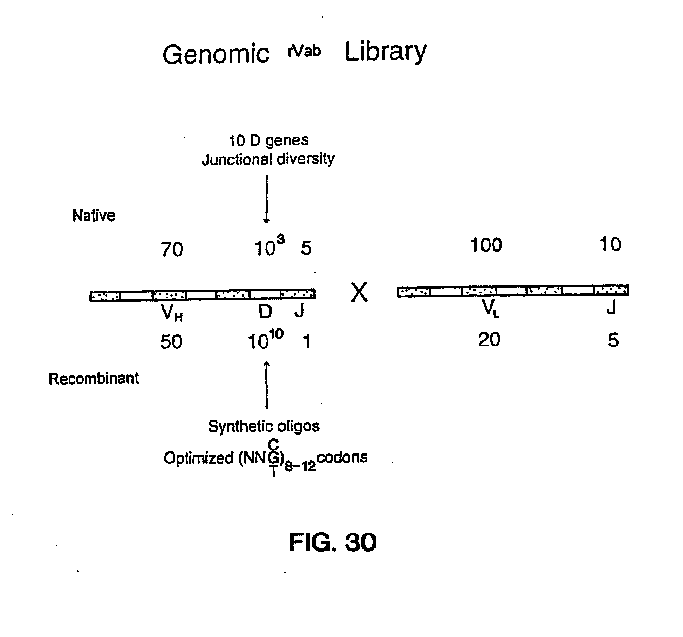

Figure 30: Schematic of the genomic rVab library.

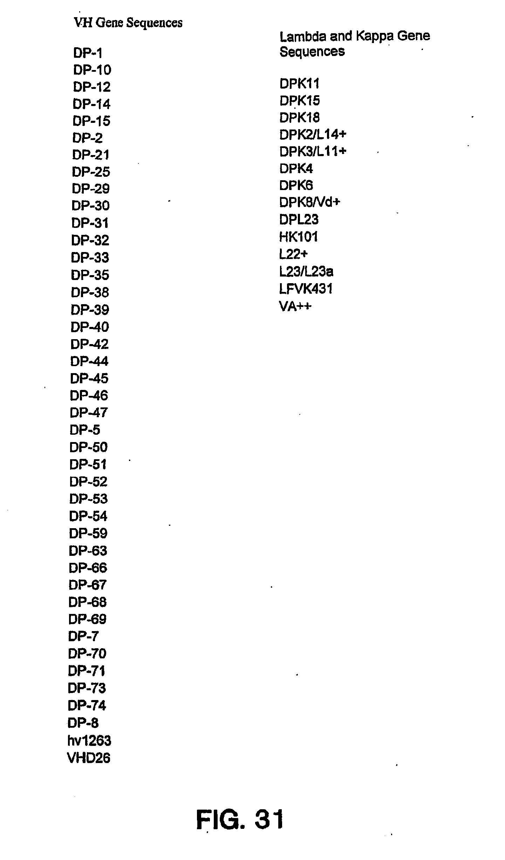

Figure 31: Listing of the V_{H}, kappa and lambda genes used to assemble the rVab antibody library for IGF-1 R binders.

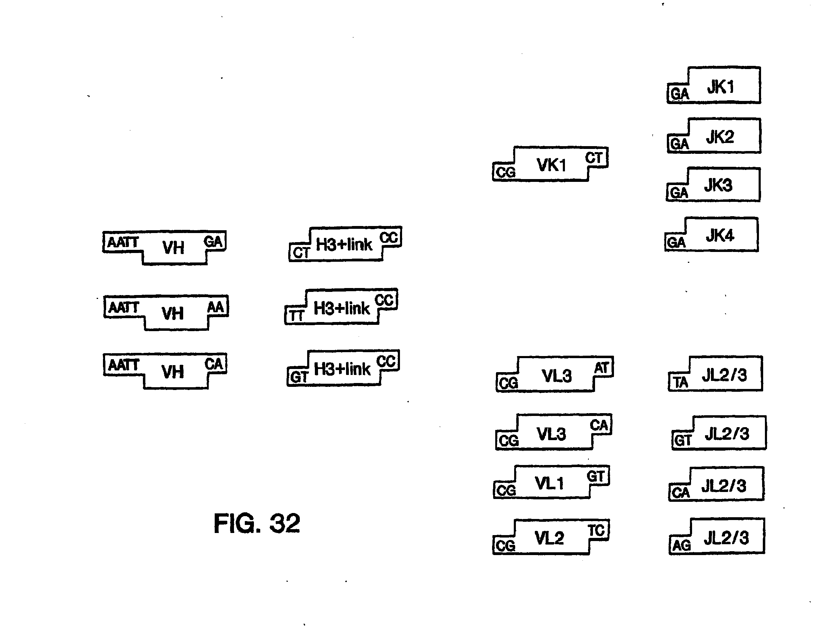

Figure 32. Schematic of the assembly of the single-chain IGF-I and insulin antibody libraries from restriction fragments.

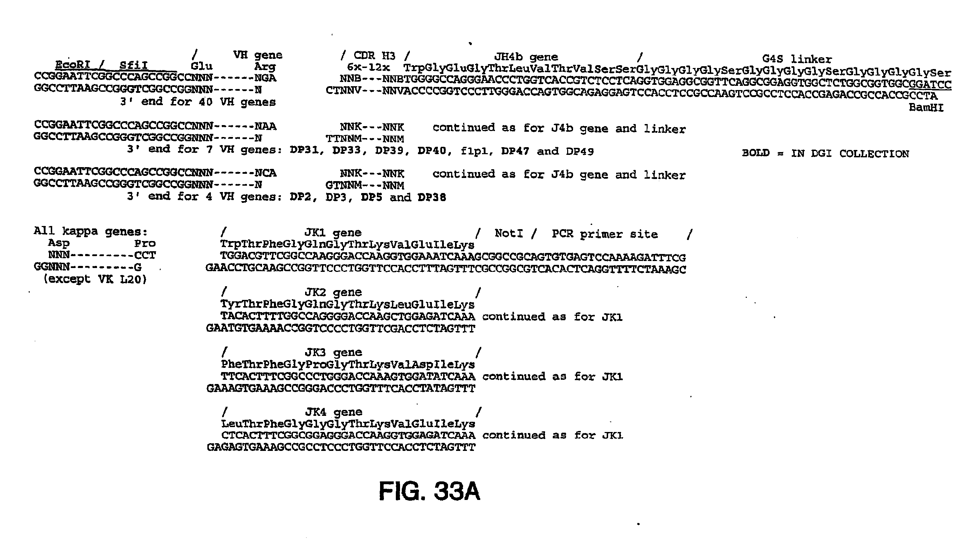

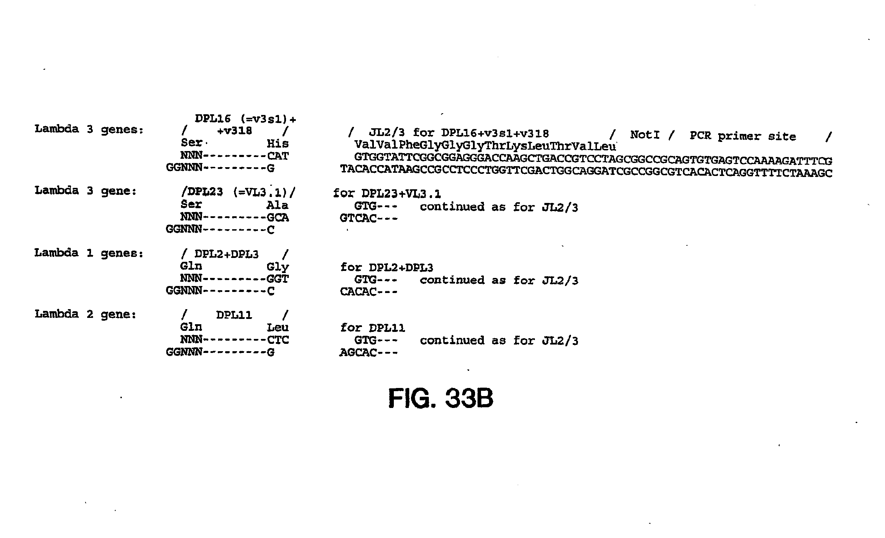

Figure 33: Sequences of the restriction fragments used to assemble the rVab libraries.

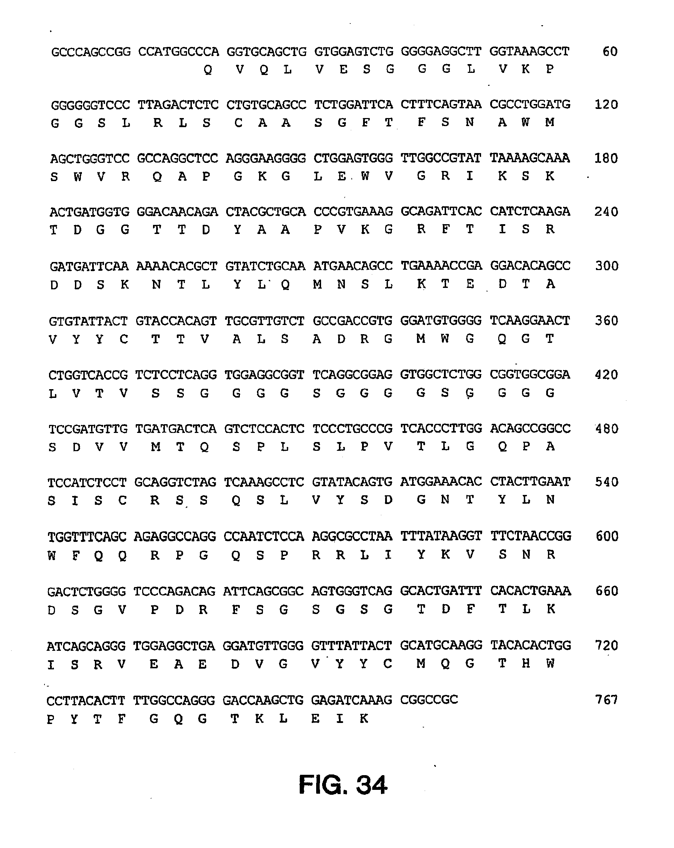

Figure 34: Nucleotide sequence of the gene encoding the 43G7 rVab specific for IGF-1R. The predicted amino acid sequence of the rVab is shown below the nucleic acid sequence.

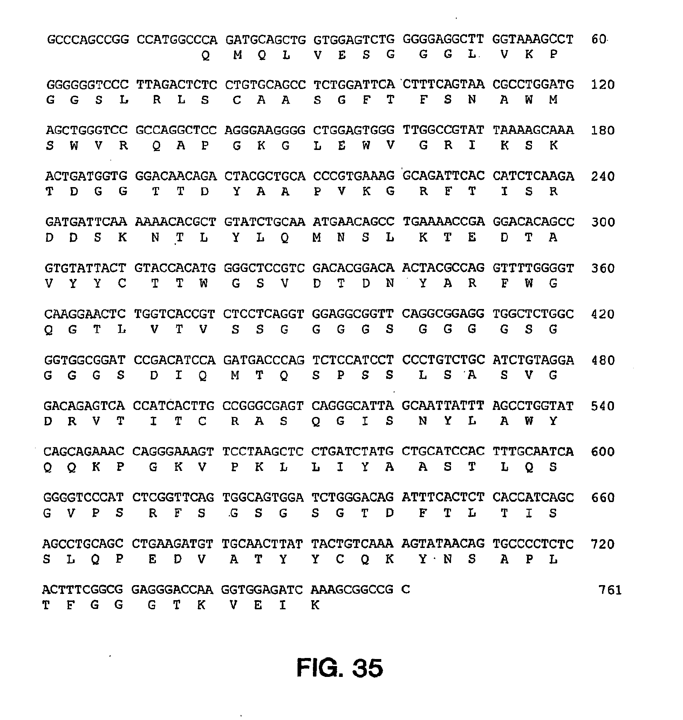

Figure 35: Nucleotide sequence of the gene encoding the 1 G2P rVab specific for IGF-1R. The predicted amino acid sequence of the rVab is shown below the nucleic acid sequence.

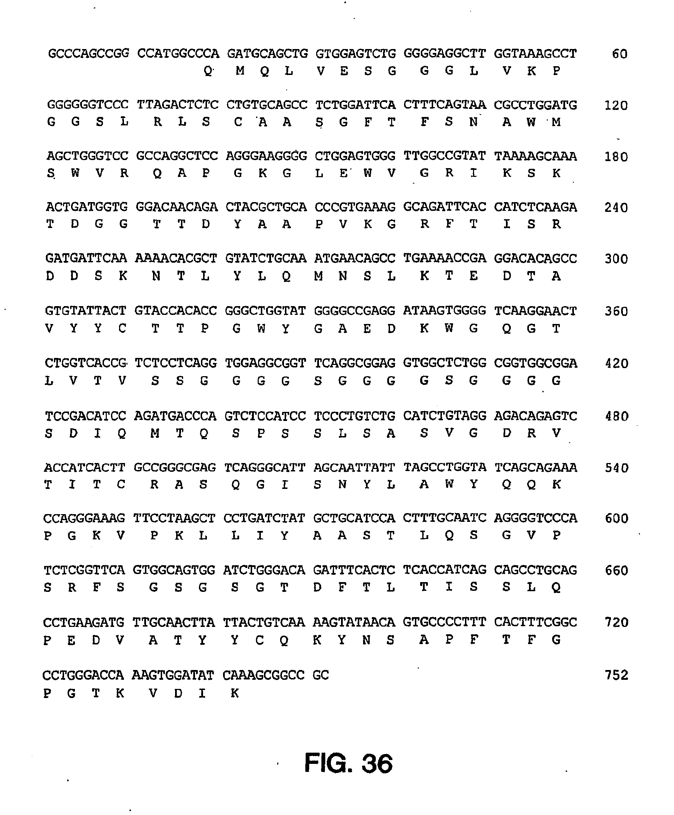

Figure 36: Nucleotide sequence of gene encoding the 39F7 rVab specific for IGF-1R. The predicted amino acid sequence of the rVab is shown below the nucleic acid sequence.

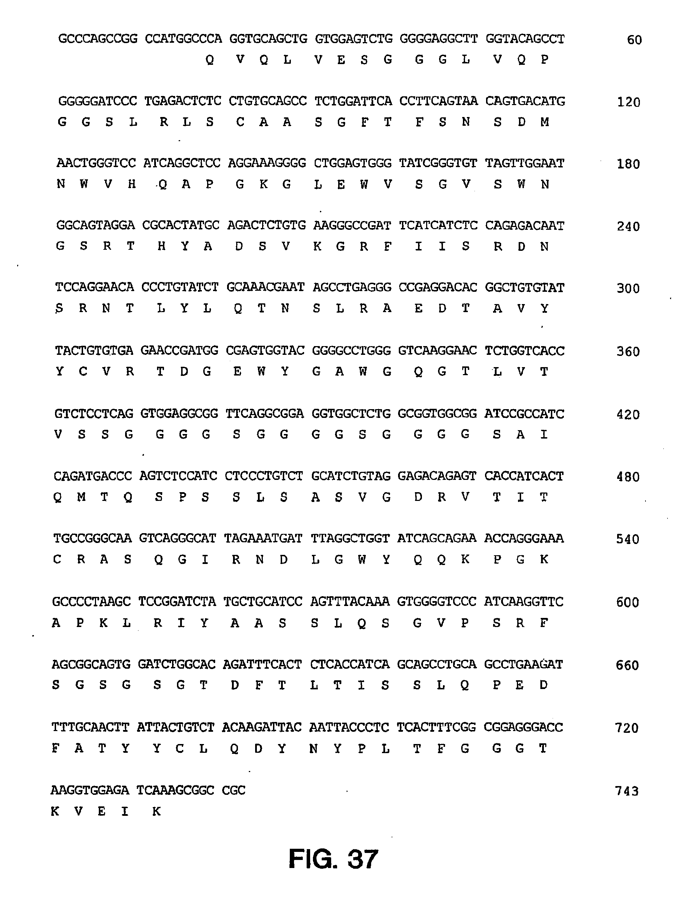

Figure 37: Nucleotide sequence of gene encoding the M100 rVab specific for IGF-1R. The predicted protein sequence of the rVab is shown below the nucleic acid sequence.

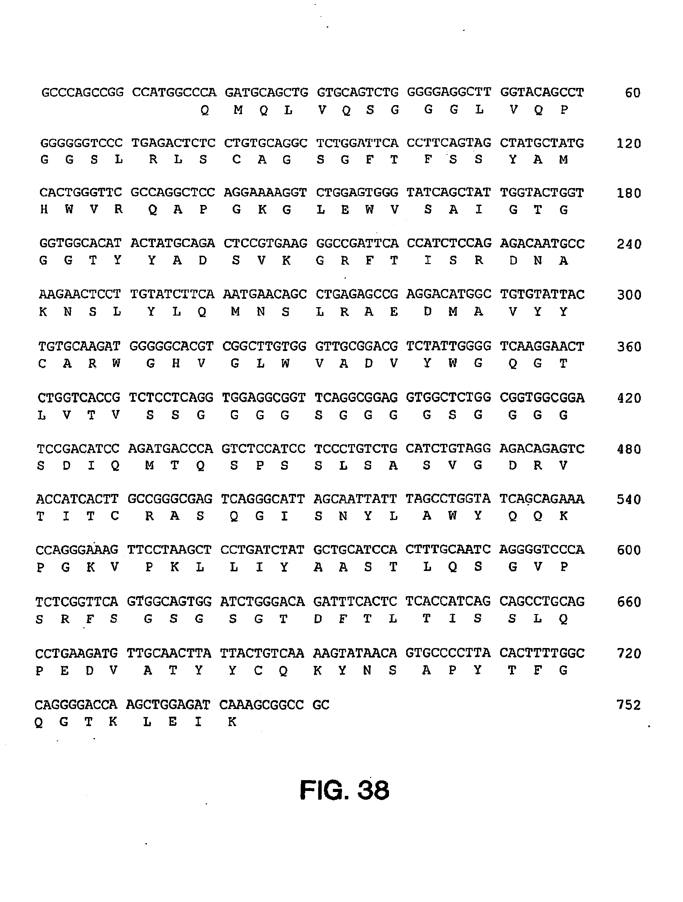

Figure 38: Nucleotide sequence of gene encoding the 46A7 rVab specific for IGF-1R. The predicted protein sequence of the rVab is shown below the nucleic acid sequence.

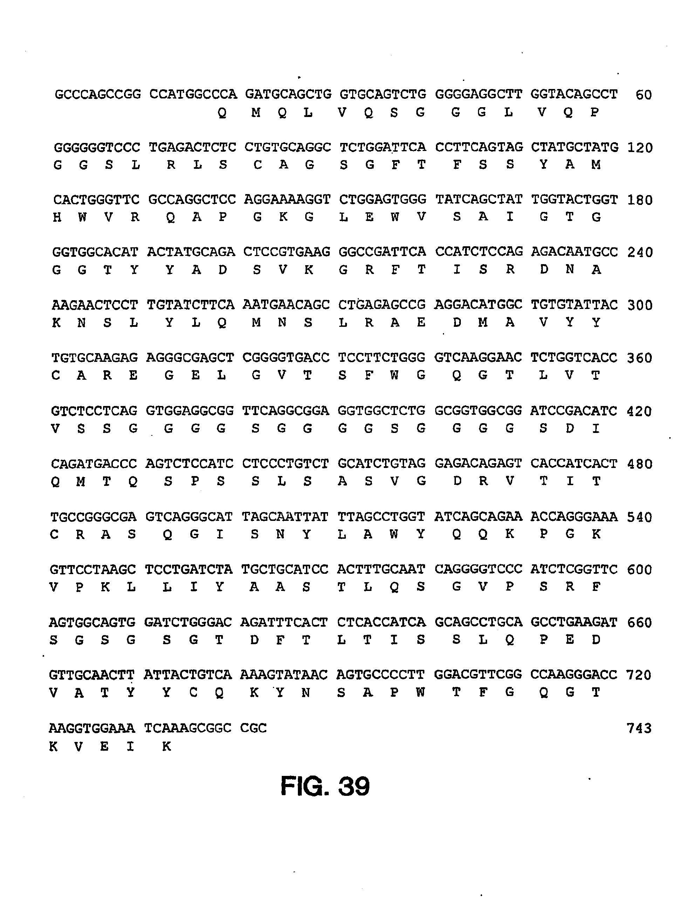

Figure 39: Nucleotide sequence of gene encoding the 49E8 rVab specific for IGF-1R. The predicted protein sequence of the rVab is shown below the nucleic acid sequence.

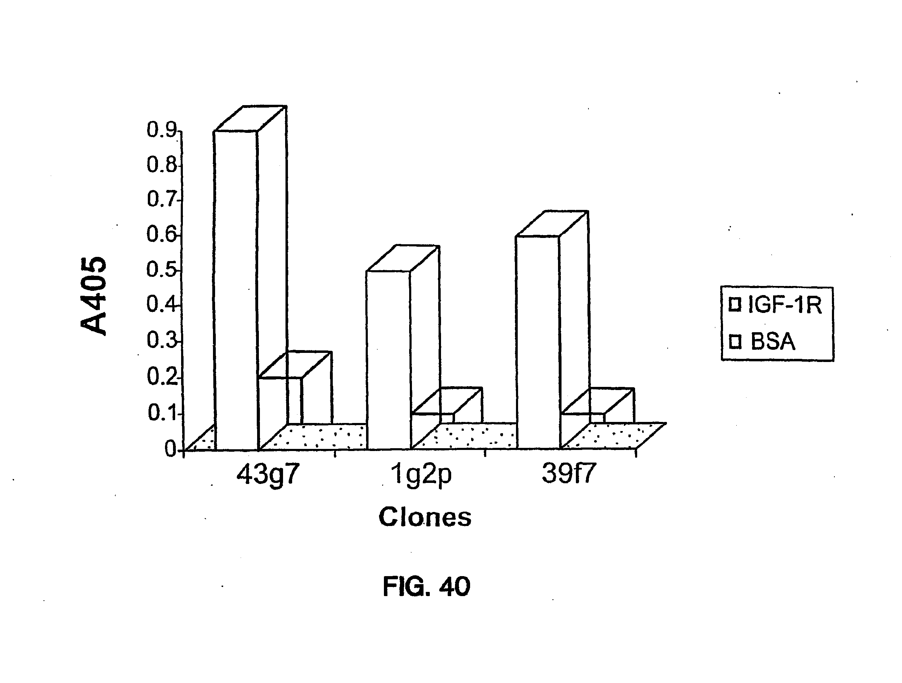

Figure 40: Assay results demonstrating the binding of soluble forms of three rVabs to IGF-1 R.

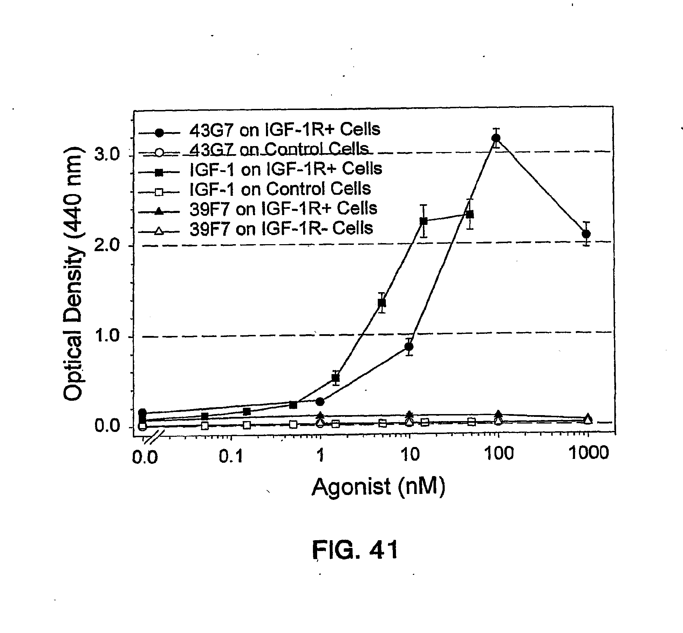

Figure 41: Assay results showing that the 43G7 rVab stimulates growth of IGF-1R⁺ cells.

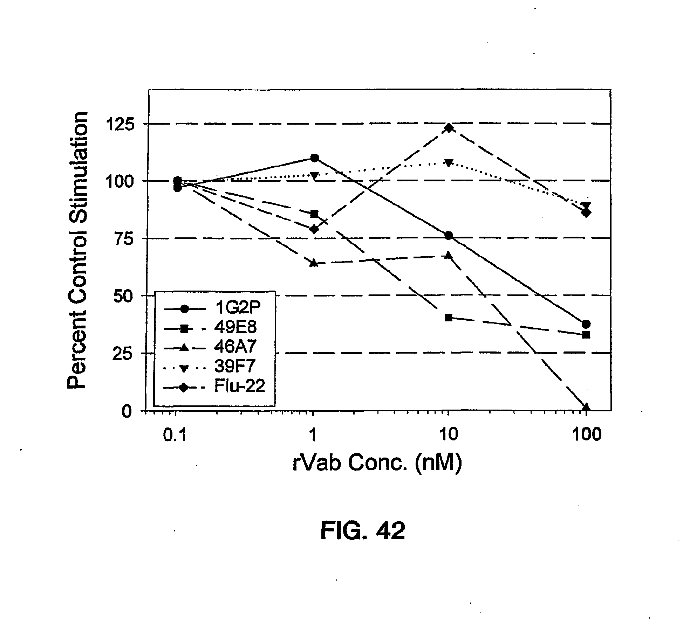

Figure 42: Assay results showing that the stimulation by rVab 43G7 is antagonized by the 1 G2P, 49E8, and 46A7 rVabs. The assay was done on IGF-1R⁺ cells.

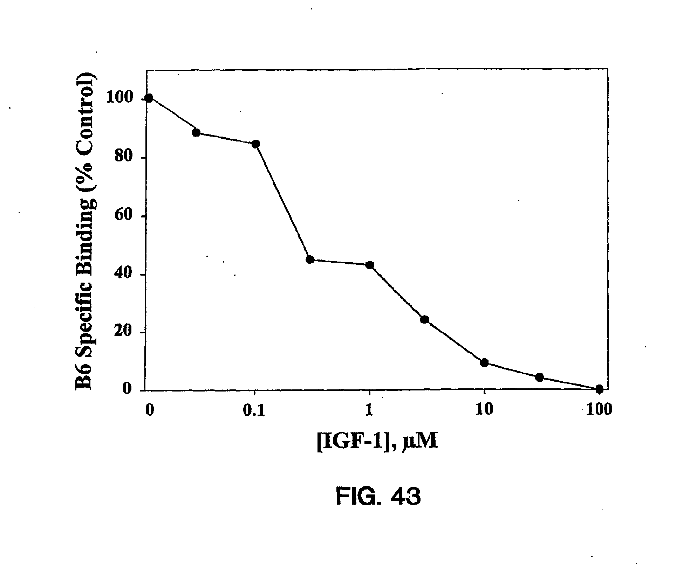

Figure 43: Eu-based fluorescence assay results showing that the binding of peptide 5.1 to IGF-1R can be competed by the IGF-1 ligand.

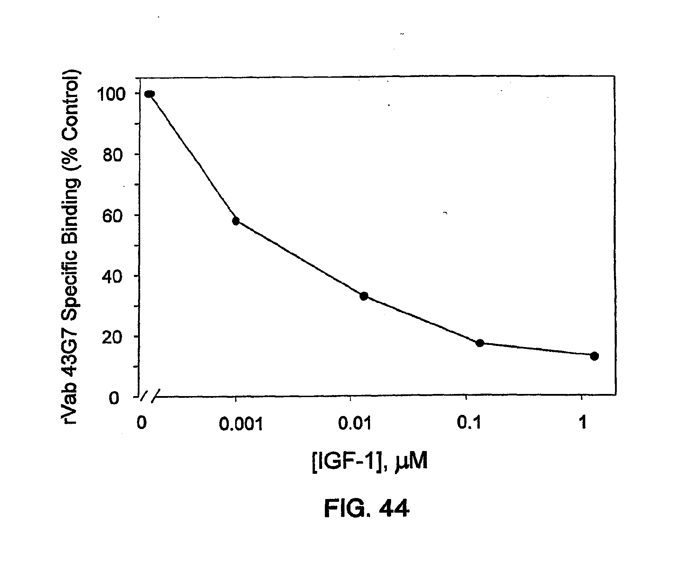

Figure 44: Results of the time-resolved fluorescence assay showing that the binding of 43G7 rVab to IGF-1R is effectively competed by IGF-1.

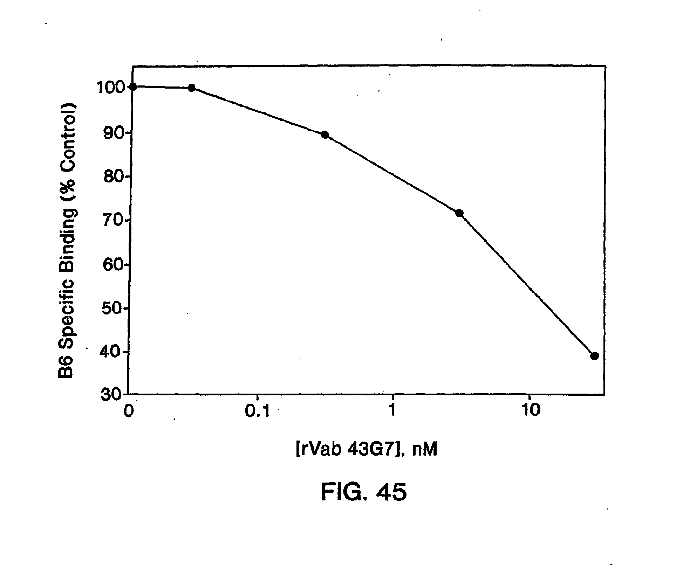

Figure 45: Eu-based fluorescence assay showing that the binding of the B6 peptide to IGF-1 R is effectively competed by the 43G7 rVab.

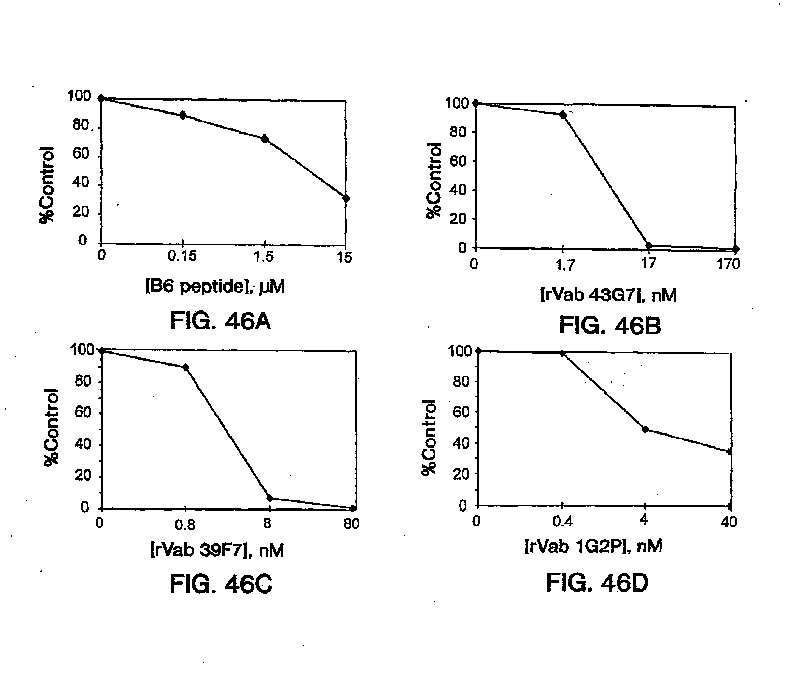

Figures 46A-46D: Results of the Eu-based fluorescence assay showing that the binding of the europium-labeled 43G7 rVab to IGF-1R is effectively competed by selected scAbs specific for IGF-1R.

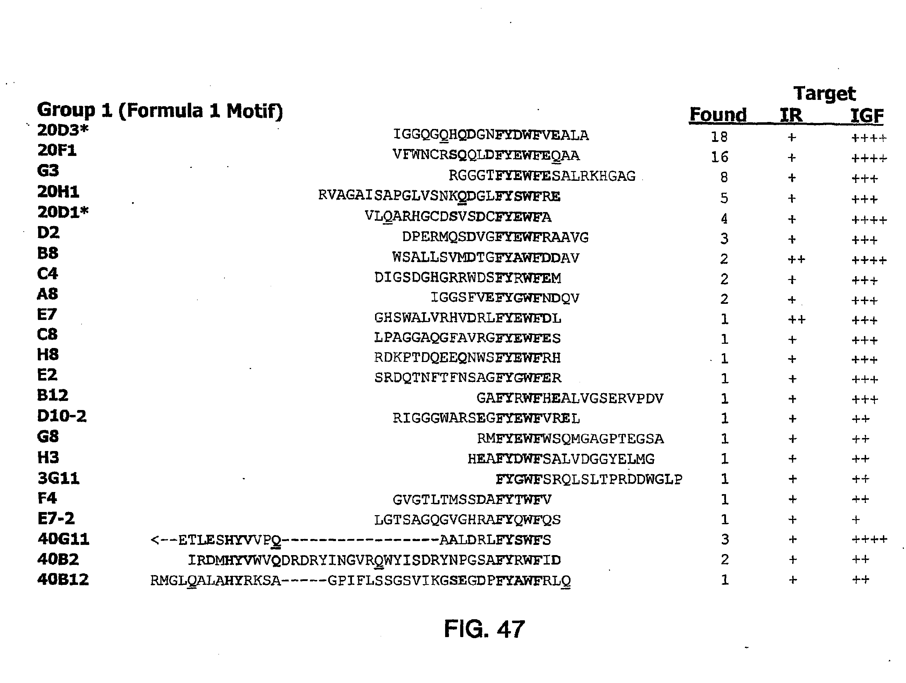

Figure 47: Biopanning results and sequence alignments of Group 1 of IR-binding peptides. The number of sequences found is indicated on the right side of the figure together with data on the phage binding to either IR or IGF-1R receptor. Absorbance signals are indicated by: ++++, >30X over background; +++, 15-30X; ++, 5-15X; +,2-5X; and; 0, <2X.

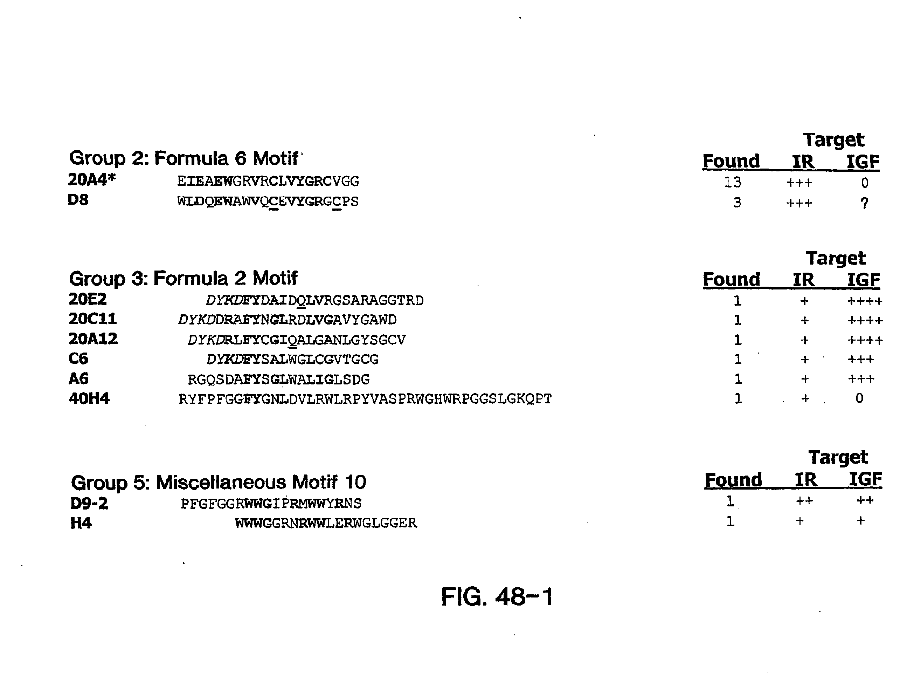

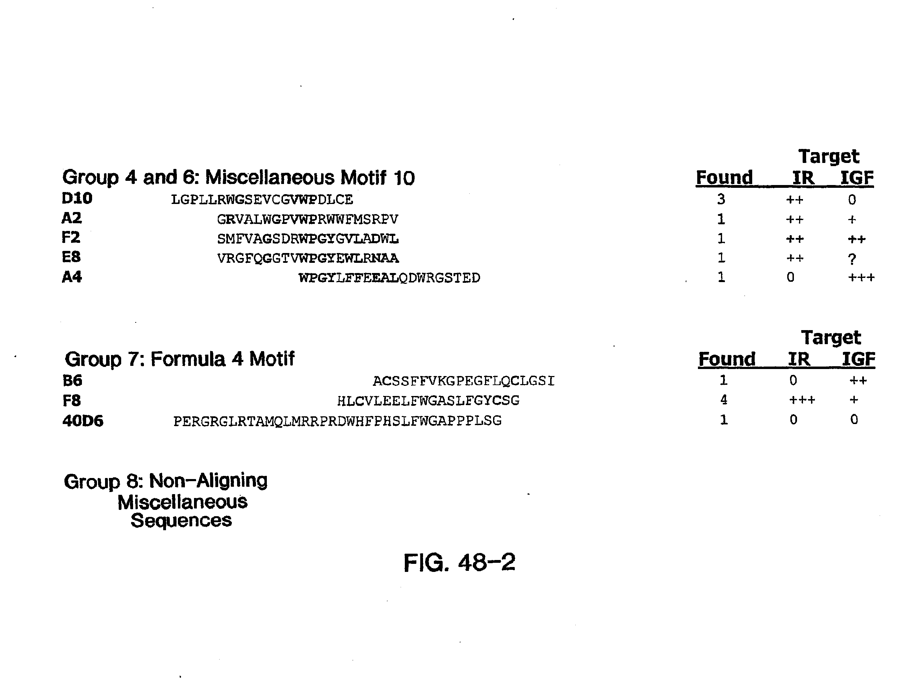

Figure 48: Biopanning results and sequence alignments of Groups 2 through 7 of IR-binding peptides. The number of sequences found is indicated on the right side of the figure together with data on the phage binding to either IR or IGF-1R receptor.

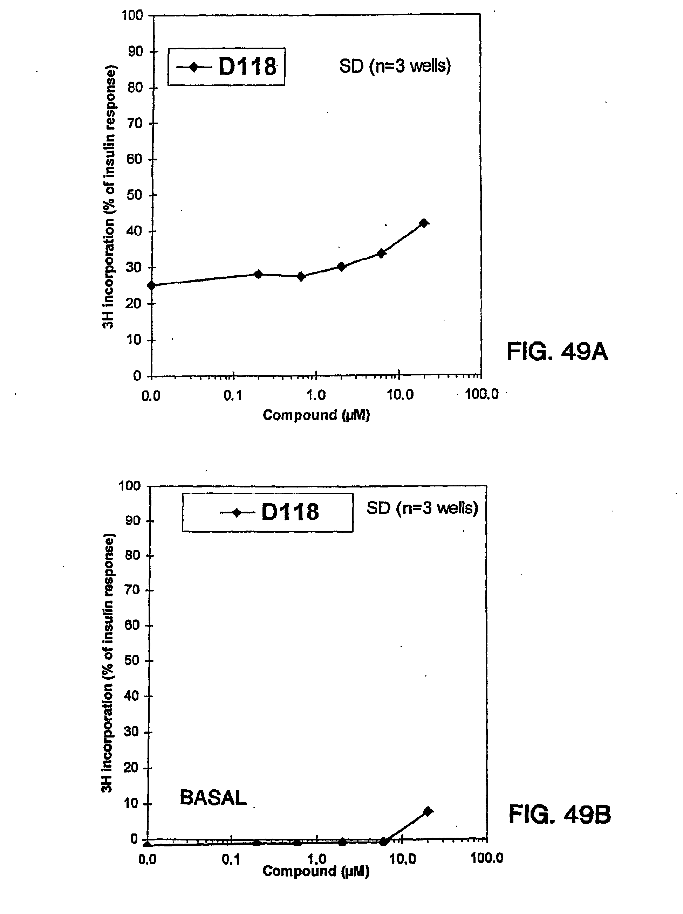

Figure 49A-49D: Dose response curve of D118 peptide (Formula 2 motif) stimulated increase of ³H-glucose into mouse adipocytes.

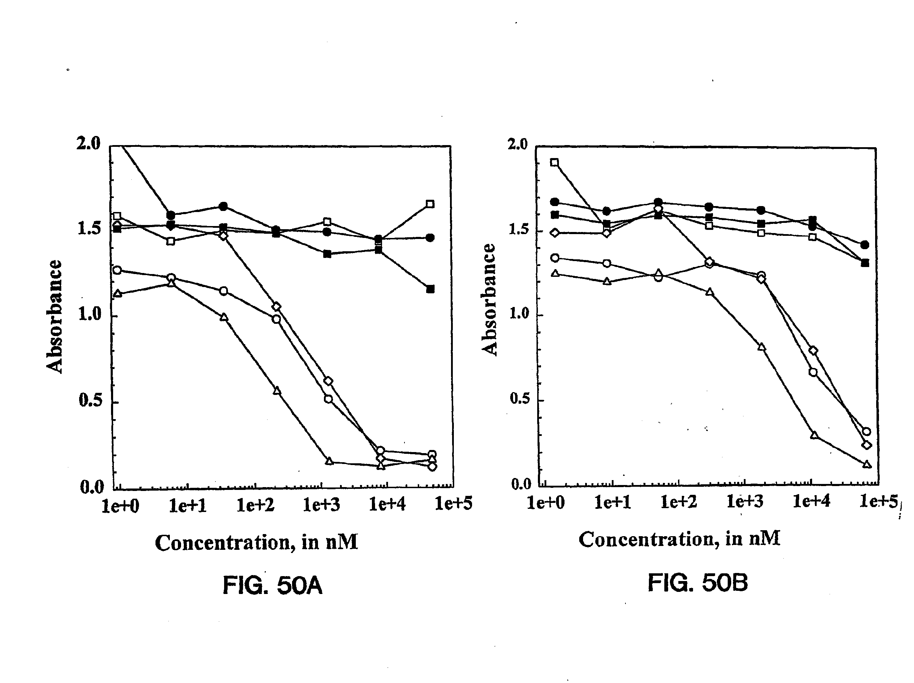

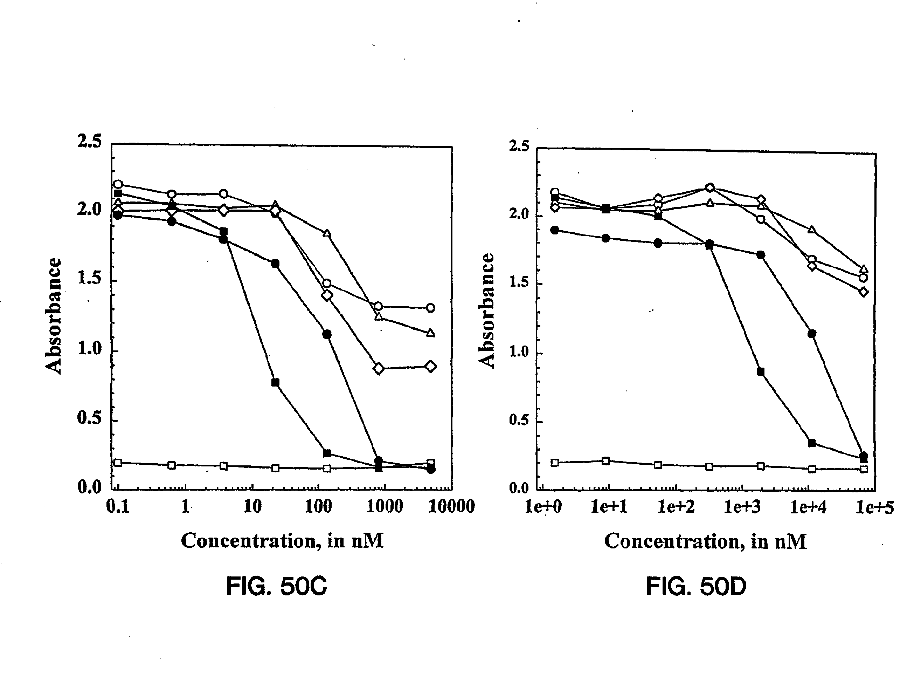

Figures 50A-50D: Titration of the synthetic peptides C1 (Figures 50A, 50C) or B6 (Figures 50B, 50D) against constant concentration of phage bound to IR (Figures 50A, 50B) or IGF-1R (Figures 50C, 50D). Phage are represented by: open circle - 20D3; open square - 20A4; open triangle - 20E2; open diamond - F2; filled circle - F8; and filled square - D8.

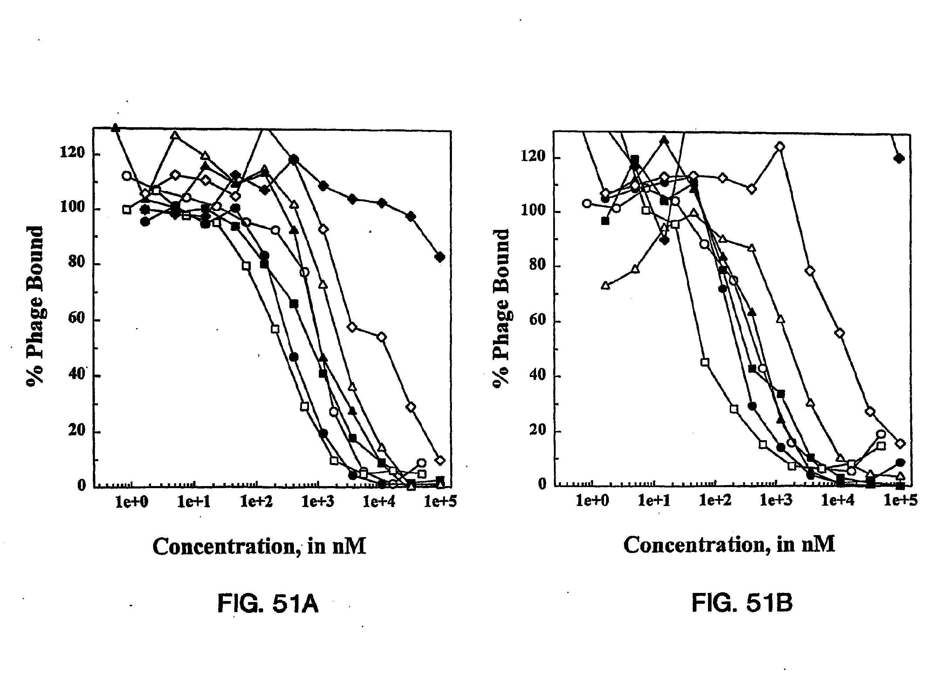

Figure 51A-51D: Titration of the IGF-1R synthetic peptides against constant concentration of phage. Symbols for the peptides are: open circles - H2; filled circles - H2C; open square - C1; filled square - C1C; open triangle - D2C; filled triangle - E4; open diamond - A6; and filled diamond p53.

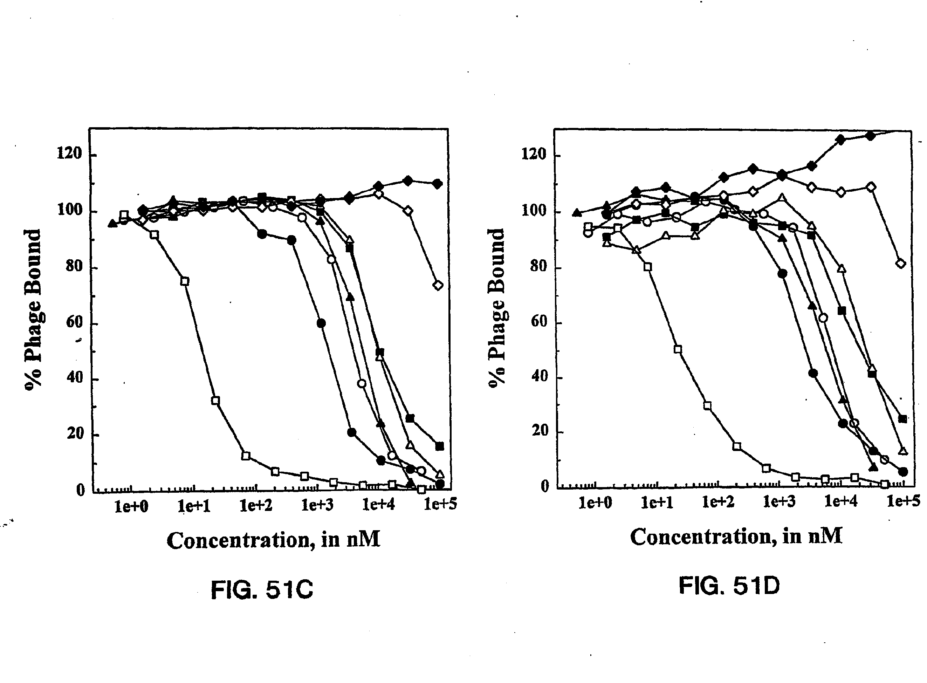

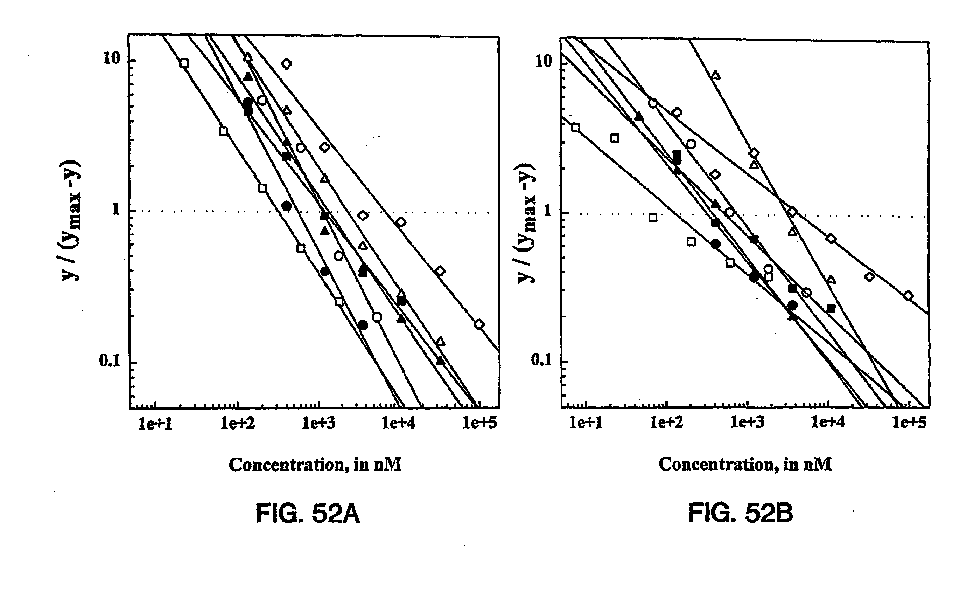

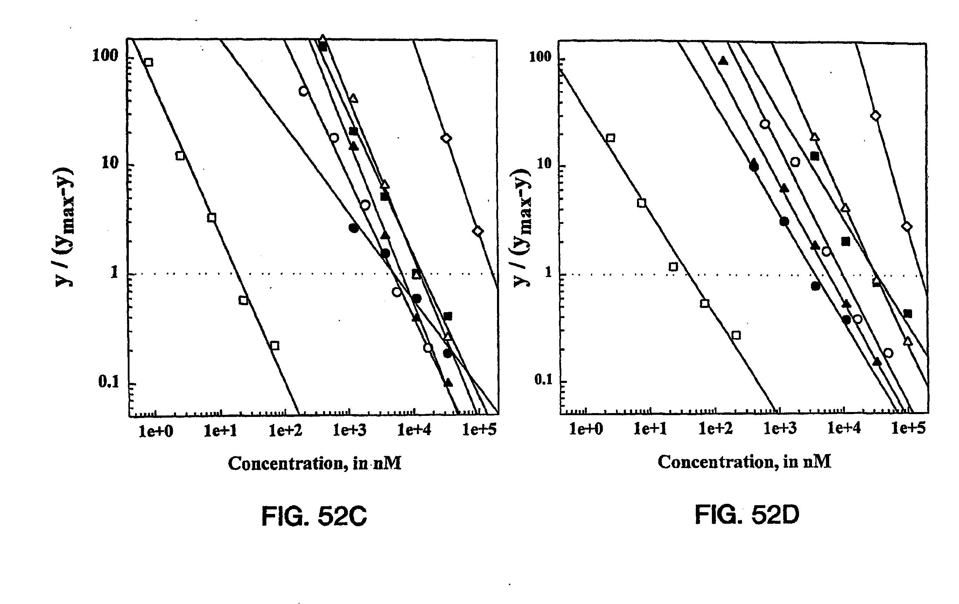

Figure 52A-52D: Hill plot analysis of phage clones. The detailed data are provided in Table 7. Symbols are the same as in Figure 51.

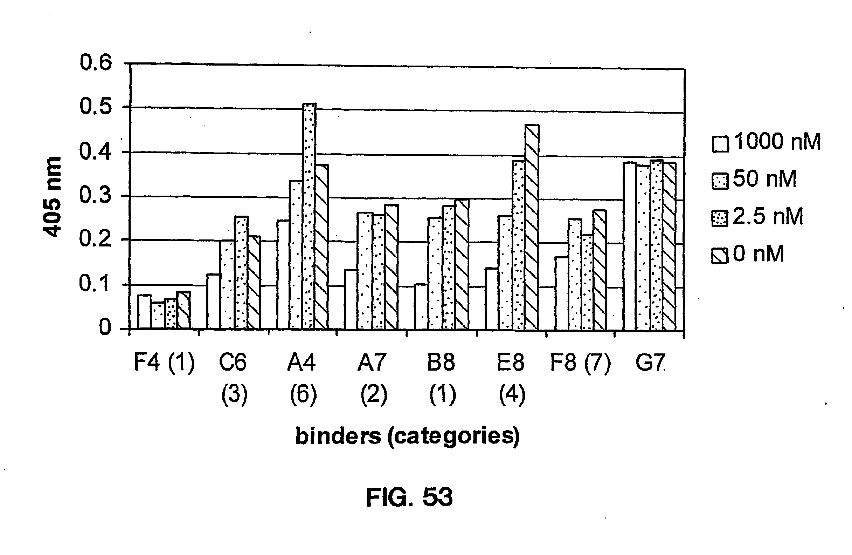

Figure 53: Competition between the insulin and the IR-binding phage. The results for seven different groups (categories) of phage binders are shown.

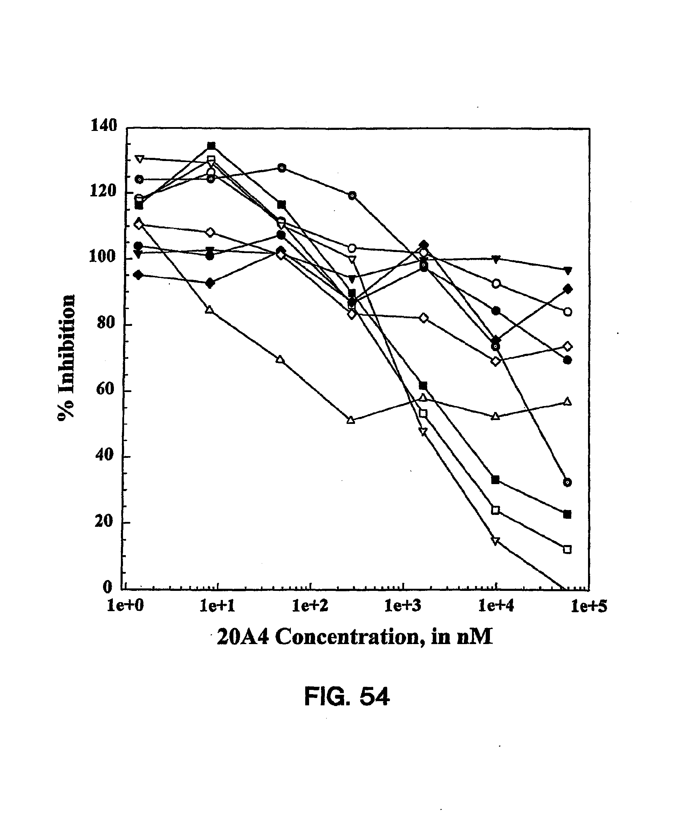

Figure 54: Titration of the synthetic peptide 20A4 against constant concentration of phage. Phage binding to IR are represented by: open circle - 20D3; filled circle B8; open square - 20A4; filled square - D8; open up triangle - 20E2; open down triangle - D10; filled down triangle - A2; open diamond - F2; filled diamond - E8; and cross-filled circle - F8.

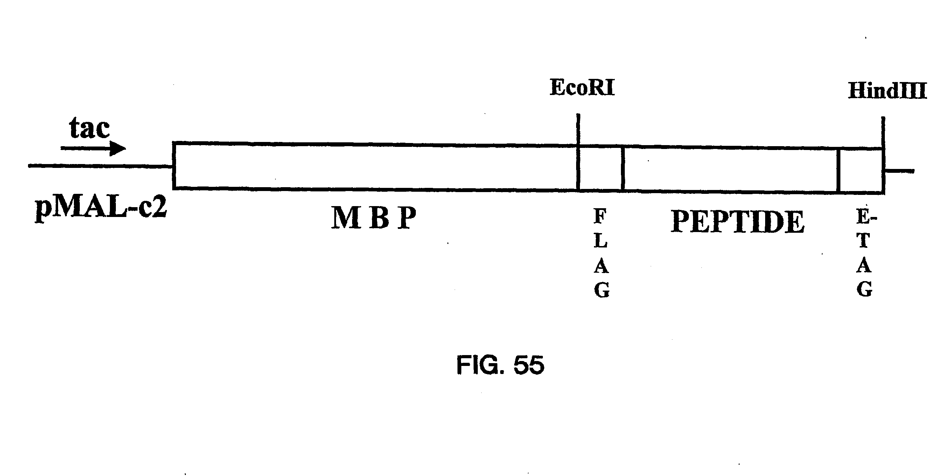

Figure 55: A schematic drawing for the construction of protein fusions of the maltose binding protein and peptides from phage libraries.

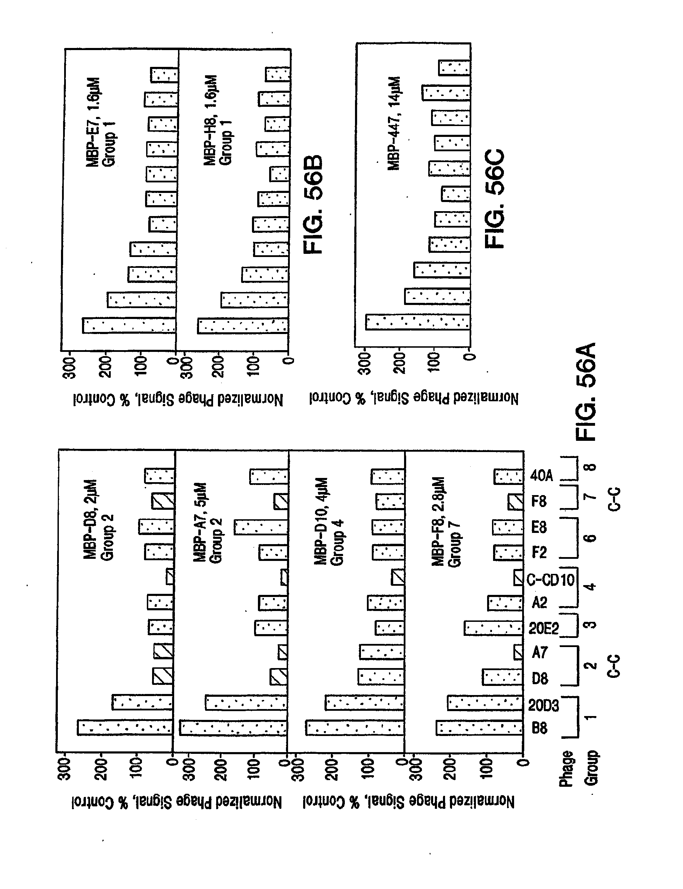

Figure 56A-56C: Insulin Receptor Competition ELISA-using MBP-Peptide Fusion Proteins. Figure 54A. Competition with fusion proteins containing cysteine residues. The hatched bars indicate value is ≤ 54 % control value. Figure 54B. Competition with fusion proteins containing the consensus sequence. The notation, c-c, indicates phage displayed peptides with cysteine residues. Figure 54C. Competition with fusion protein containing a control peptide.

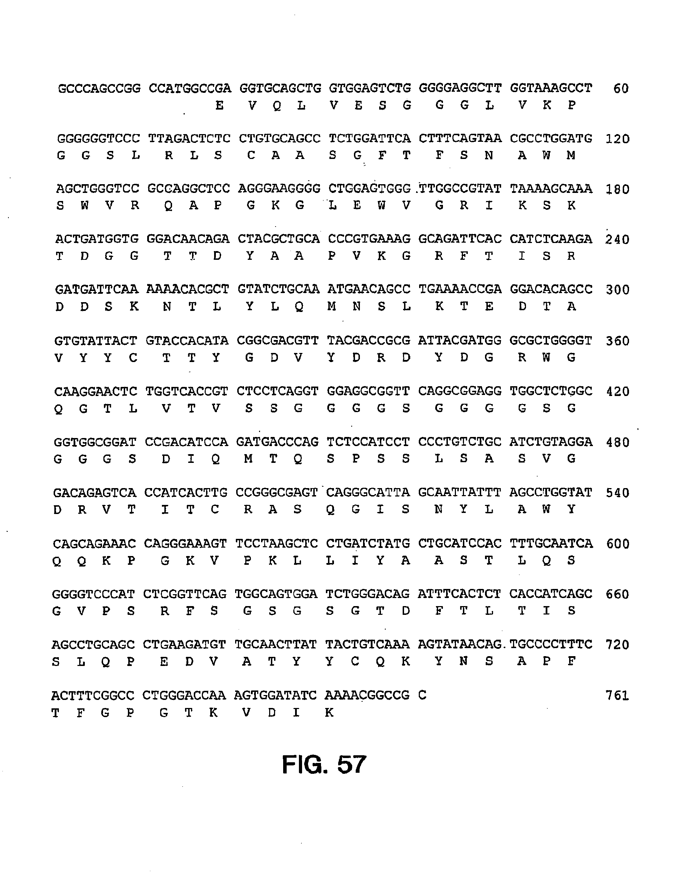

Figure 57: Nucleotide and predicted amino acid sequence of the gene encoding the 6f6 rVab that binds to IR.

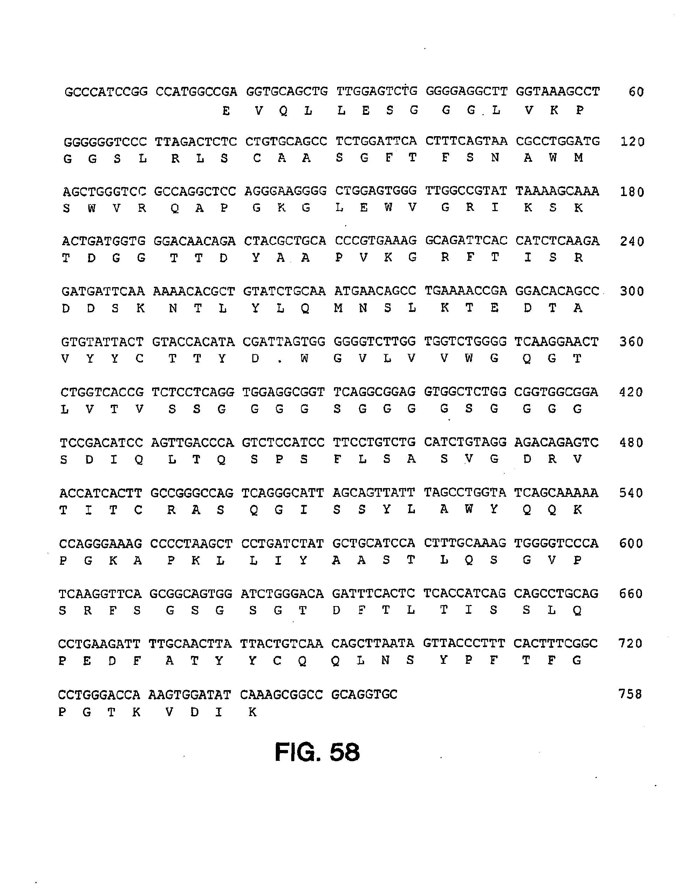

Figure 58: Nucleotide and predicted amino acid sequence of the gene encoding the 14c8 rVab that binds to IR.

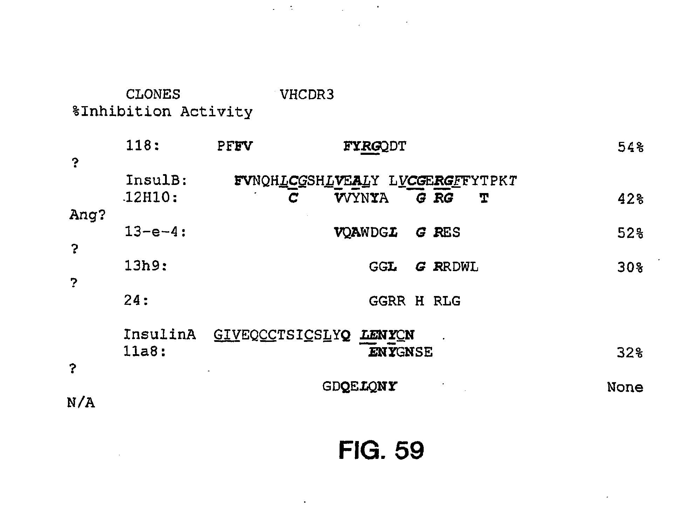

Figure 59: Comparison of the VH CDR3 sequences of different rVabs that bind to IR, and competitions of these rVabs and insulin for binding to IR.

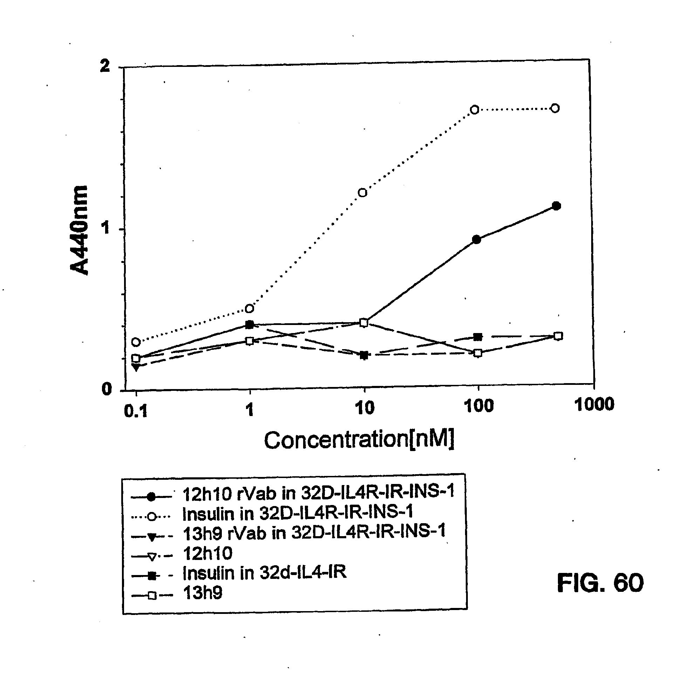

Figure 60: Biological response of insulin, rVab 12h10, and rVab 13h9 in 32D cells expressing or not expressing IR.

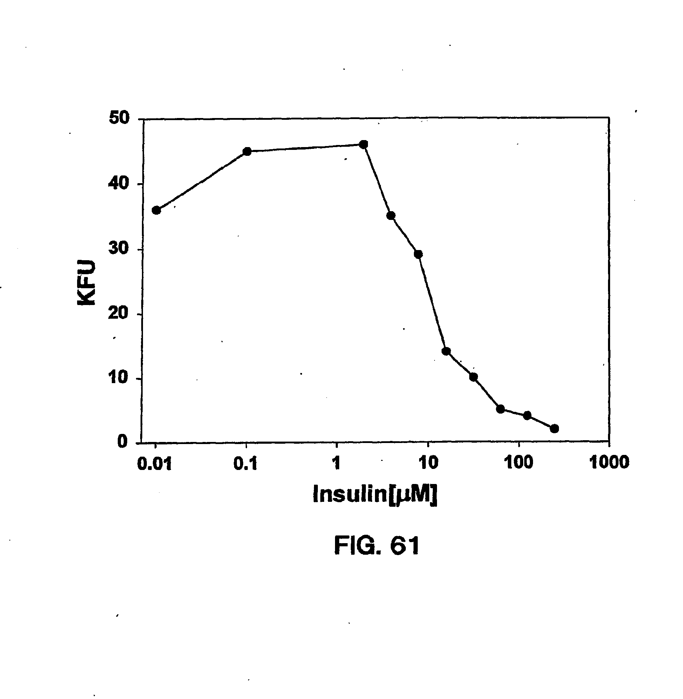

Figure 61: Competition of rVab 6f6 and insulin for binding to IR.

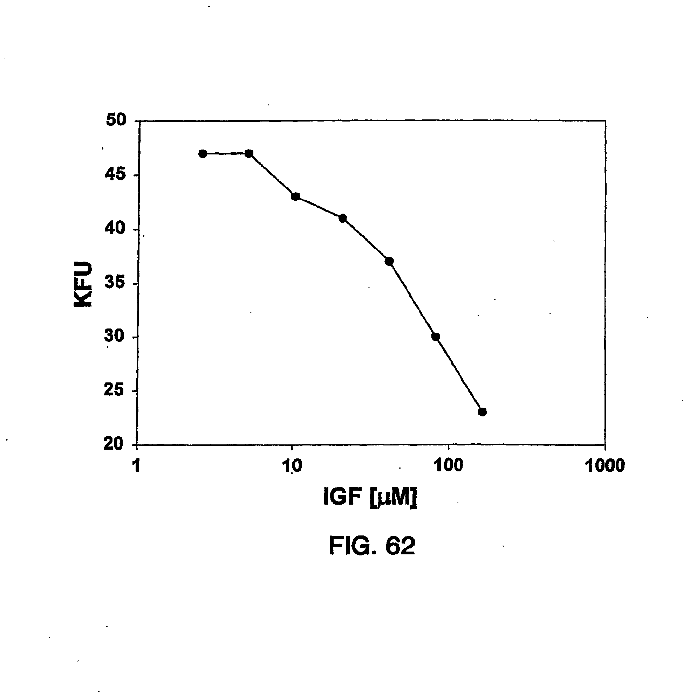

Figure 62: Competition of rVab 6f6 and IGF-1 for binding to IR.

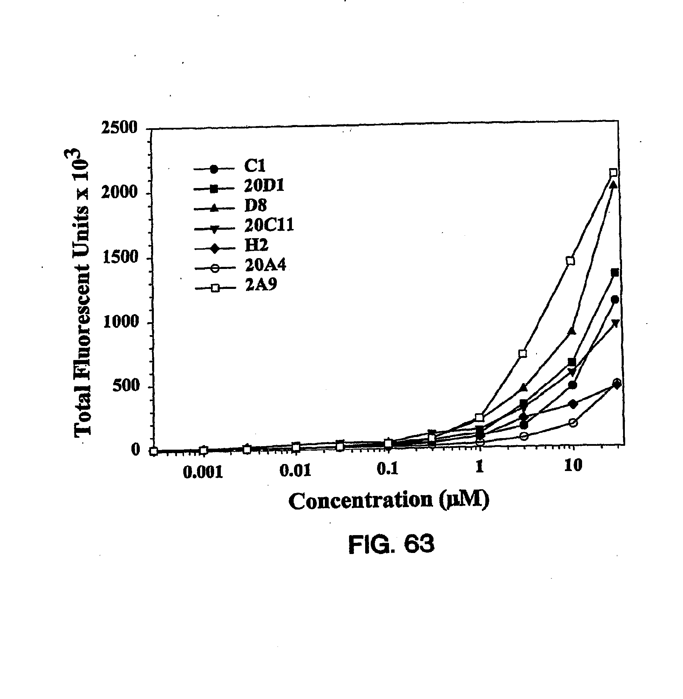

Figure 63: Competition of synthetic peptides and soluble rVab antibodies for binding of biotinylated peptides to insulin receptor. Synthetic peptides or soluble rVab at indicated concentrations were incubated with biotinylated peptides overnight using the heterogeneous TRFA.

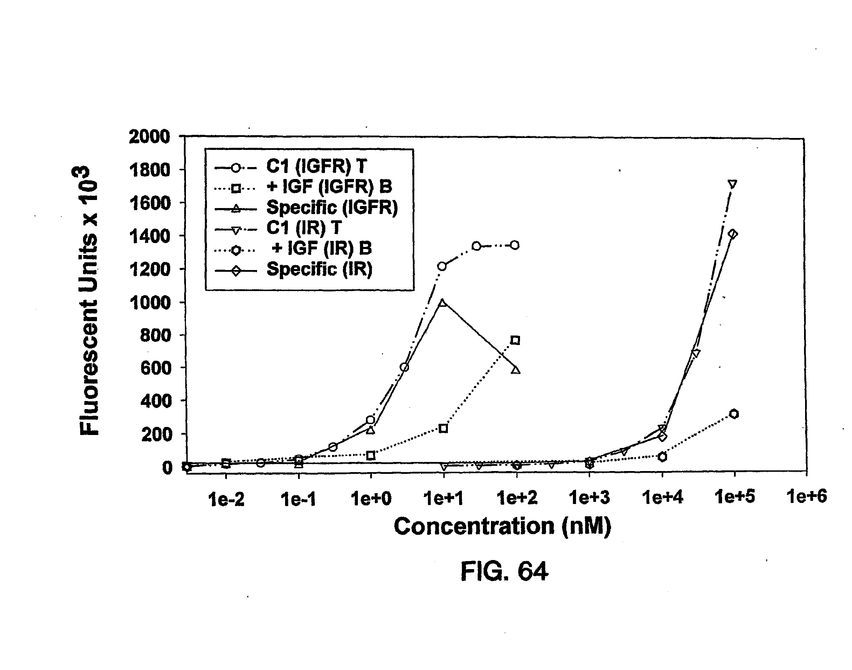

Figure 64: Binding of C1 to IR and IGF-1R.

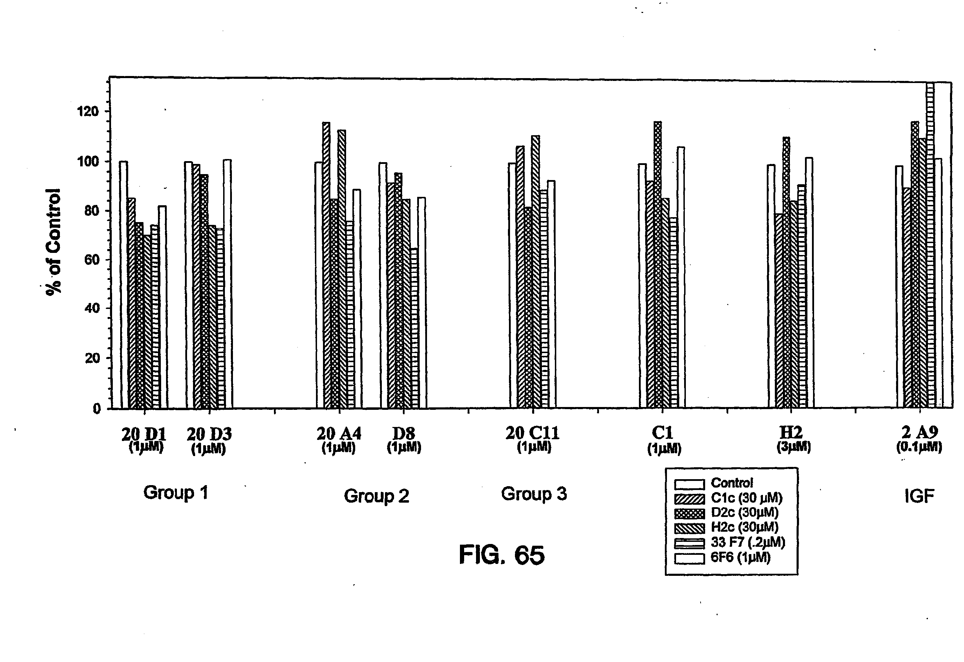

Figure 65: Competition of peptides for binding to IR.

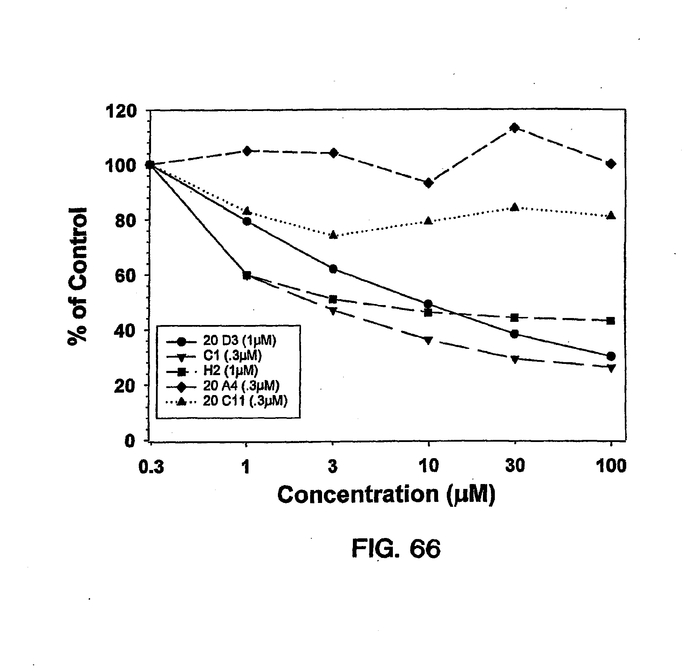

Figure 66: H2C competition for b-peptide binding to IR. Biotinylated peptides at indicated concentrations were competed by increasing concentrations of H2C for binding to IR using the heterogeneous TRFA.

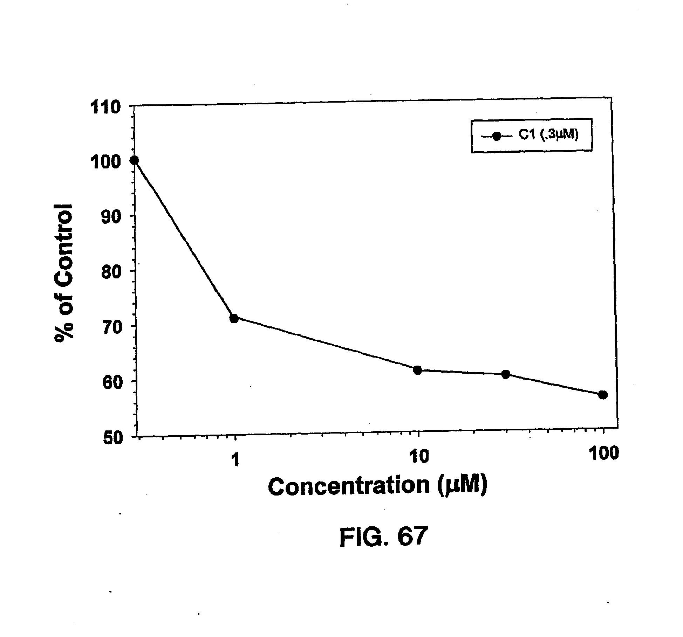

Figure 67: C1 C competition for b-C1 binding to IR. Biotinylated C1 peptide at 0.3 µM was competed by increasing concentrations of C1C for binding to IR using the heterogeneous TRFA.

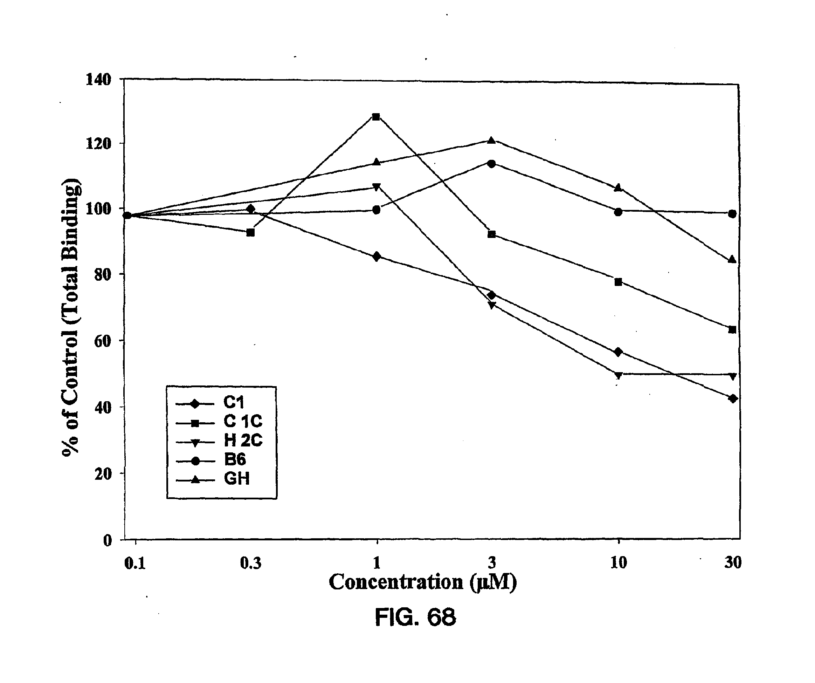

Figure 68: Competition of peptides for binding of rVab 12H10 to insulin receptor. Synthetic peptides at indicated concentration were incubated with rVab 12H10 overnight using the heterogeneous TRFA.

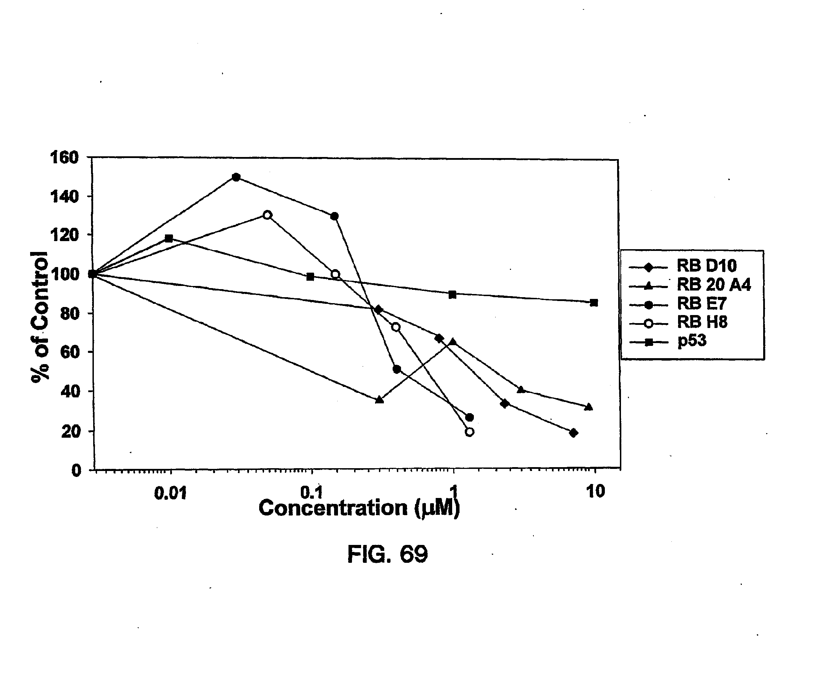

Figure 69: Competition of MBP-peptide fusion proteins to rVab 12H10 binding to insulin receptor. Four MBP-peptides fusion proteins at indicated concentrations were incubated with rVab 12H10 overnight using the heterogeneous TRFA.

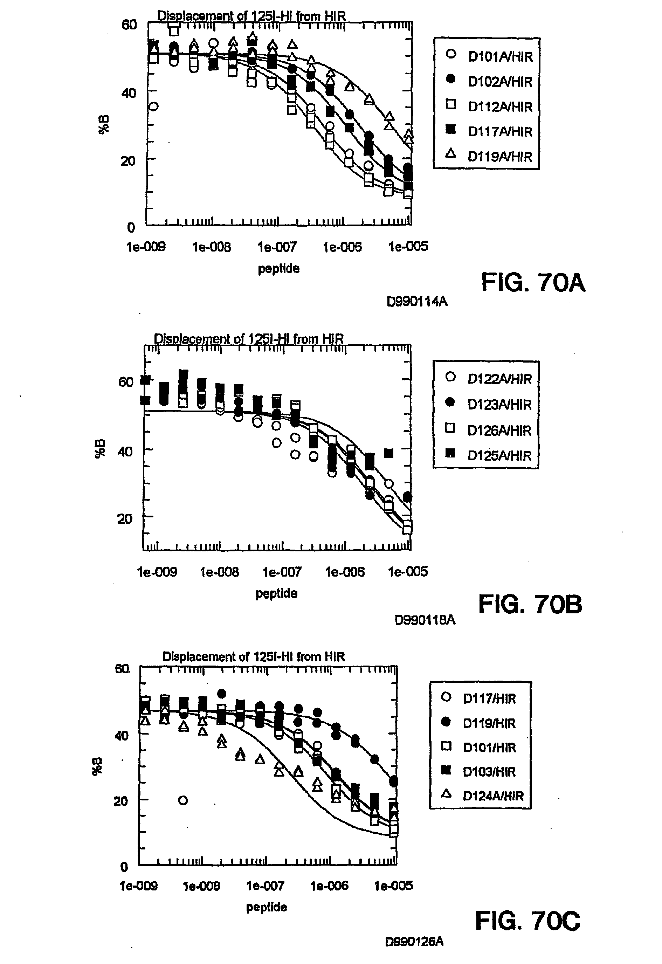

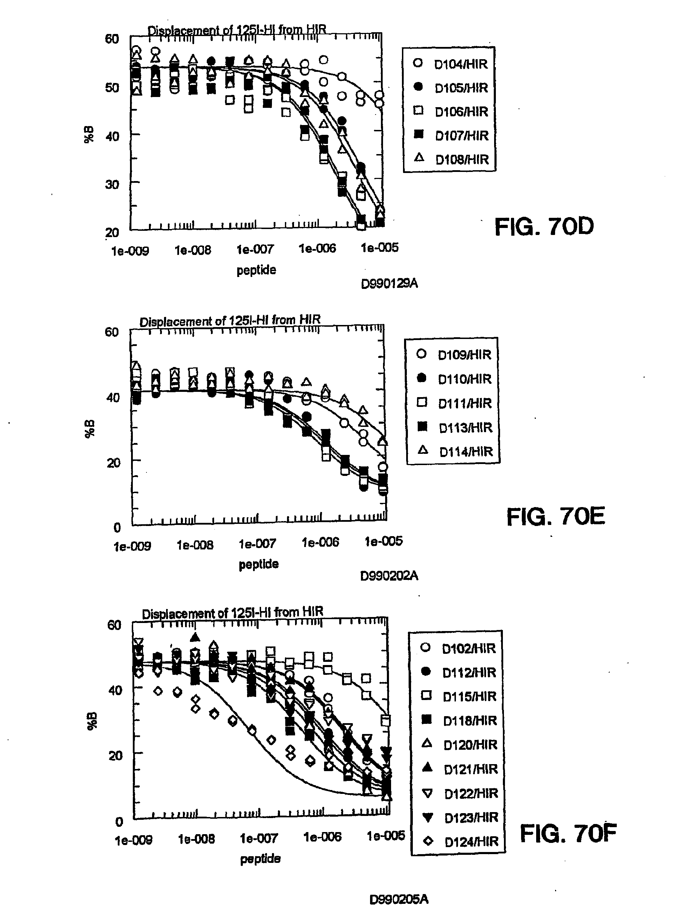

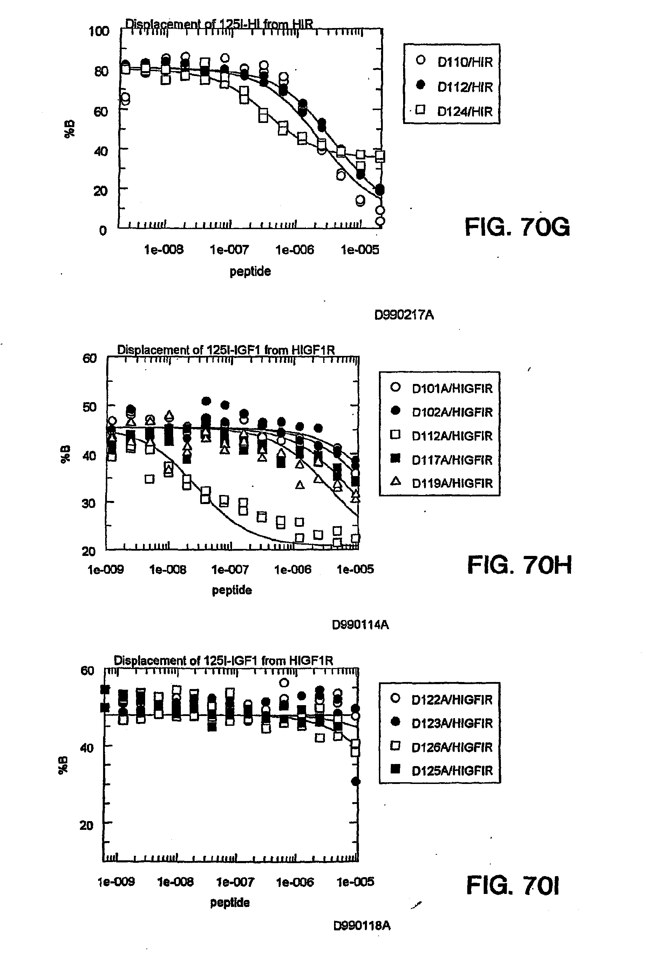

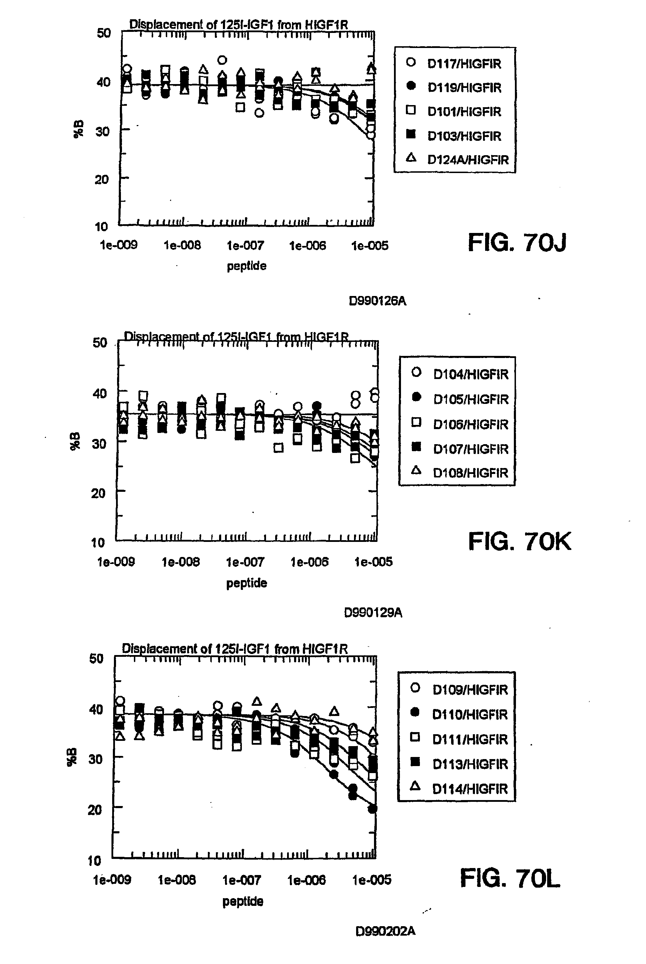

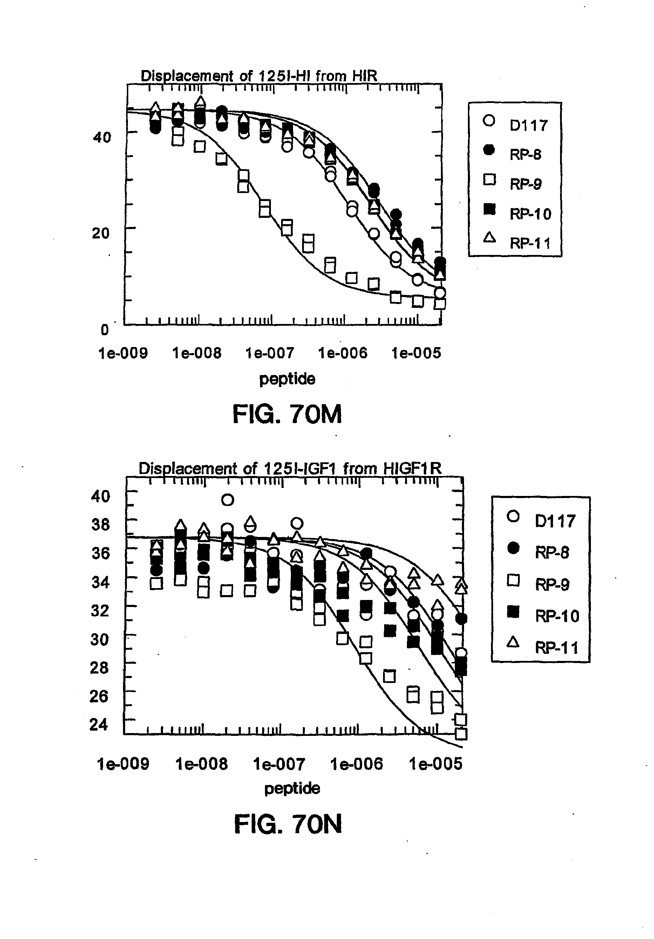

Figures 70A-70N: Peptide binding displacement curves showing the displacement of ¹²⁵insulin or ¹²⁵lGF-1 from HIR or HIGF-1 R in the presence of various peptides.

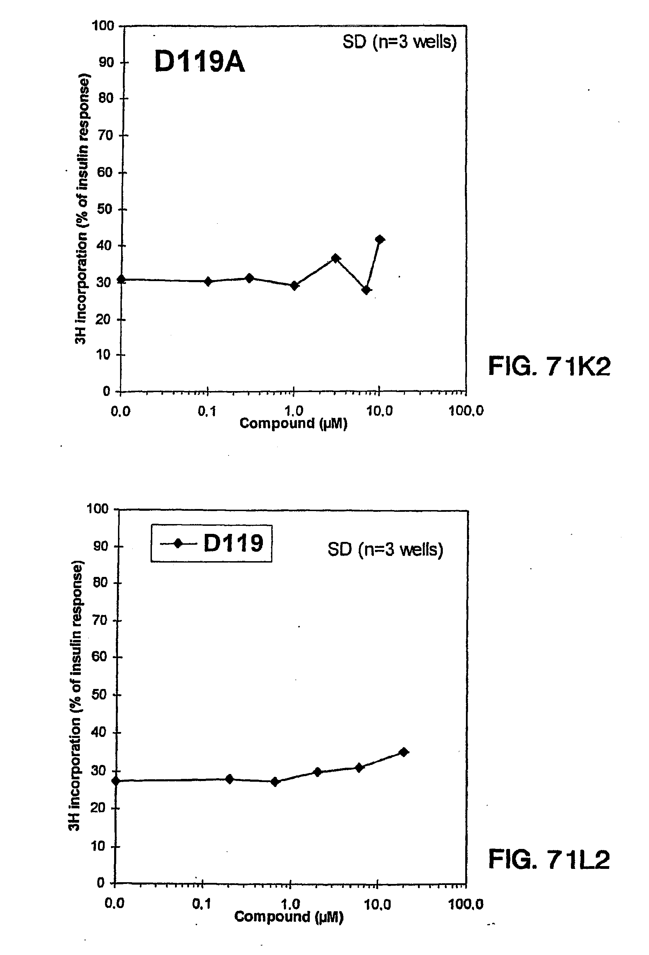

Figures 71A-71Z; 71A2-71Z2; 71A3-71B3: Concentration dependent modulation of ³H-glucose into adipocytes by various peptides. Formula 1 motif peptide responses are shown in Figures 71A-71V; 71A2-71J2; Formula 9 motif peptide response is shown in Figures 71W-71Z; Formula 2 motif peptide response is shown in Figures 71 K2-71 L2; Miscellaneous peptide motif 10 peptide responses are shown in Figures 71 M2-71 P2; Formula 6 motif peptide response is shown in Figure 71 Q2-Figure 71 R2; and Formula 4 motif peptide response is shown in Figure 71S2-Figure 71W2. Formula 1 and Formula 2 motif peptide response is shown in Figure 71X2-Figure 71A3. Fusion peptide S291 response is shown in Figure 71B3.

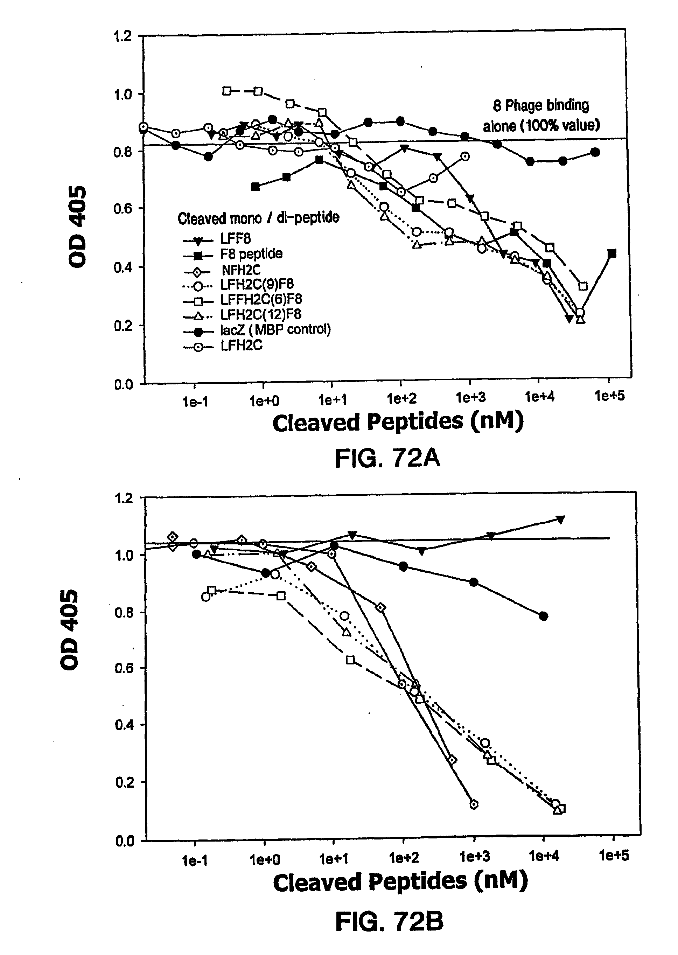

Figures 72A and 72B: Competition of Site 1 (Figure 72B) and Site 2 (Figure 72A) phage displayed peptides with recombinant cleaved dipeptides.

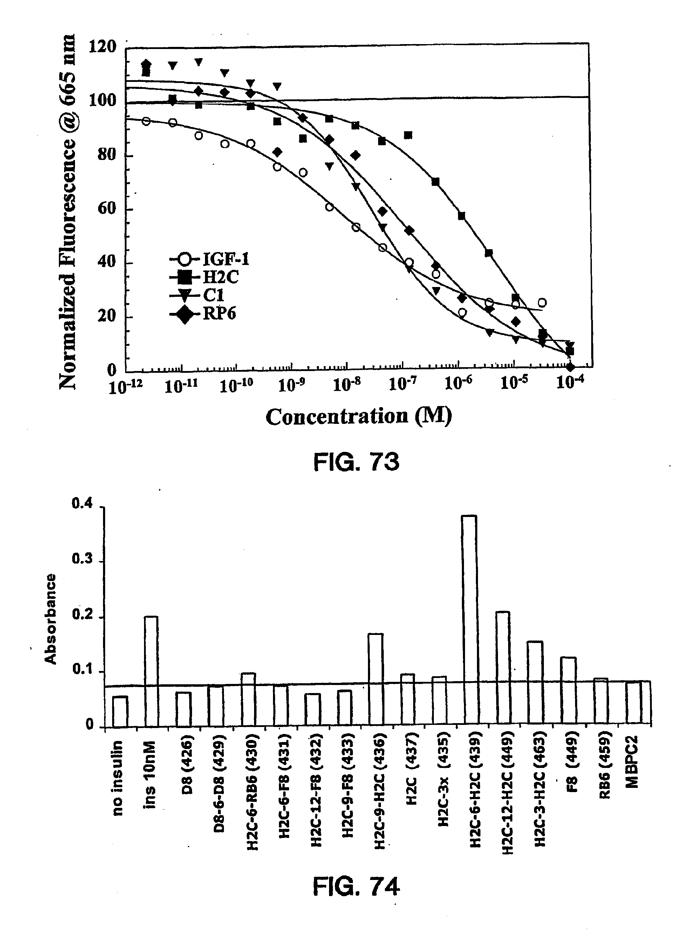

Figure 73: Competition of IGF-1R, peptide H2C (D117), peptide C1 (D112), and peptide RP6 (20C-3-G3-IGFR) in a homogeneous fluorescent-resonance energy transfer assay based on the binding of IGF-1R to peptide 20E2 (D118).
Figure 74: Stimulation of IR autophosphorylation *in vivo* by MBP-fusion peptides.

### V. DETAILED DESCRIPTION OF THE INVENTION

This invention relates to amino acid sequences comprising motifs which bind to the IGF-1 receptor (IGF-1R) and/or the insulin receptor (IR). In addition to binding to IR and IGF-1R; the amino acid sequences also possess either agonist, partial agonist or antagonist activity at one or both of these receptors. Based on the differing regions of IR and IGF-1R which are reported to be important for binding and activity, this invention surprisingly provides amino acid sequences which define common binding motifs on IR and IGF-1 R which are capable of conferring agonist and/or antagonist activity at these receptors. In addition, this invention identifies multiple binding sites (Sites 1 and 2) on IR and IGF-1R which appear to be allosterically coupled.

Although capable of binding to IR and/or IGF-1 R at sites which participate in conferring agonist or antagonist activity, the amino acid sequences are neither based on insulin or IGF-1 native sequences, nor do they reflect an obvious homology to any such sequence.

The amino acid sequences of the invention may be peptides, polypeptides, or proteins. These terms as used herein should not be considered limiting with respect to the size of the various amino acid sequences referred to herein and which are encompassed within this invention. Thus, any amino acid sequence comprising at least one of the IR or IGF-1R binding motifs disclosed herein, and which binds to one of the receptors is within the scope of this invention. In preferred embodiments, the amino acid sequences confer insulin or IGF agonist or antagonist activity. The amino acid sequences of the invention are typically artificial, i.e. non-naturally occurring peptides or polypeptides. Amino acid sequences useful in the invention may be obtained through various means such as chemical synthesis, phage display, cleavage of proteins or polypeptides into fragments, or by any means which amino acid sequences of sufficient length to possess binding ability may be made or obtained.

The amino acid sequences provided by this invention should have an affinity for IR or IGF-1 R sufficient to provide adequate binding for the intended purpose. Thus, for use as a therapeutic, the peptide, polypeptide or protein provided by this invention should have an affinity (K_{d}) of between about 10⁻⁷ to about 10⁻¹⁵ M. More preferably the affinity is 10⁻⁸ to about 10⁻¹² M. Most preferably, the affinity is10⁻⁹,to about 10⁻¹¹ M. For use as a reagent in a competitive binding assay to identify other ligands, the amino acid sequence preferably has affinity for the receptor of between about 10⁻⁵ to about 10⁻¹² M.

A further consideration in identifying peptides provided by this invention for use as therapeutics is the relative activity at either IR or IGF-IR. Thus, a peptide which has efficacy at IR and clinically insignificant activity of IGF-IR may be a useful therapeutic even though such a peptide may bind IGF-IR with relatively high affinity.

At least ten different binding motifs have been identified which bind to active sites on IR; at least four of these also bind to IGF-1 R. The binding motifs are defined based on the analysis of several different amino acid sequences and analyzing the frequency that particular amino acids or types of amino acids occur at a particular position of the amino acid sequence.

For the purposes of this invention, the amino acids are grouped as follows: amino acids possessing alcohol groups are serine (S) and threonine (T). Aliphatic amino acids are isoleucine (I), leucine (L), valine (V), and methionine (M). Aromatic amino acids are phenylalanine (F), histidine (H), tryptophan (W), and tyrosine (Y). Hydrophobic amino acids are alanine (A), cysteine (C), phenylalanine (F), glycine (G), histidine (H), isoleucine (I), lysine (L), methionine (M), arginine (R), threonine (T), valine (V), tryptophan (W), and tyrosine (Y). Negative amino acids are aspartic acid (D) and glutamic acid (E). The following amino acids are polar amino acids: cysteine (C), aspartic acid (D), glutamic acid (E), histidine (H), lysine (K), asparagine (N), glutamine (Q), arginine (R), serine (S), and threonine (T). Positive amino acids are histidine (H), lysine (K), and arginine (R). Small amino acids are alanine (A), cysteine (C), aspartic acid (D), glycine (G), asparagine (N), proline (P), serine (S), threonine (T), and valine (V). Very small amino acids are alanine (A), glycine (G) and serine (S). Amino acids likely to be involved in a turn formation are alanine (A), cysteine (C), aspartic acid (D), glutamic acid (E), glycine (G), histidine (H), lysine (K), asparagine (N), glutamine (Q), arginine=(R)serine (S), proline (P), and threonine (T).

The amino acids within each of these defined groups may be substituted for each other in the motifs described below, subject to the specific preferences stated herein. In addition, synthetic or non-naturally occurring amino acids may also be used in accordance with this invention.

Also included within the scope of this invention are amino acid sequences containing substitutions, additions, or deletions based on the teachings disclosed herein and which bind to IR or IGF-1R with the same or altered affinity. For example, amino acid residues located at the carboxy and amino terminal regions of the consensus motifs described below, which amino acid residues are not associated with a strong preference for a particular amino acid, may optionally be deleted providing for truncated sequences. Certain amino acids such as lysine which promote the stability of the amino acids sequences may be deleted depending on the use of the sequence, as for example, expression of the sequence as part of a larger sequence which is soluble, or linked to a solid support.

Peptides that bind to IGF-1 R, and methods and kits for identifying such peptides, have been disclosed by Beasley et al., U.S. Application Serial No. 09/146,127, filed September 2, 1998, which is incorporated by reference in its entirety.

### A. Consensus Motifs

The following motifs have been identified as conferring binding activity to IR and/or IGF-1 R:
1. X₁X₂X₃X₄X₅ (Formula 1, the A6 motif) wherein X₁, X₂, X₄ and X₅ are aromatic amino acids, preferably, phenylalanine or tyrosine. Most preferably, X₁ and X₅ are phenylalanine and X₂ is tyrosine. X₃ may be any small polar amino acid, but is preferably selected from aspartic acid, glutamic acid, glycine, or serine, and is most preferably aspartic acid or glutamic acid. X₄ is most preferably tryptophan, tyrosine, or phenylalanine and most preferably tryptophan. Particularly preferred embodiments of the A6 motif are FYDWF and FYEWF. The A6 motif possesses agonist activity at IGF-1 R, but agonist or antagonist activity at IR depending on the identity of amino acids flanking A6. See Figure 11A. Two amino acid sequences comprising the A6 motif possess agonist activity at IR are FHENFYDWFVRQVSKK (D117; H2C) and GRVDWLQRNANFYDWFVAELG-NH₂ (S175). Nonlimiting examples of Formula 1 amino acid sequences are shown in Figures 1A-1O.
2. X₆X₇X₈X₉X₁₀X₁₁X₁₂X₁₃ (Formula 2, the B6 motif) wherein X₆ and X₇ are aromatic amino acids, preferably, phenylalanine or tyrosine. Most preferably, X₆ is phenylalanine and X₇ is tyrosine. X₈, X₉, X₁₁ and X₁₂ may be any amino acid. X₁₀ and X₁₃ are hydrophobic amino acids, preferably leucine, isoleucine, phenylalanine, tryptophan or methionine, but more preferably leucine or isoleucine. X₁₀ is most preferably isoleucine for binding to IR and leucine for binding to IGF-1 R. X₁₃ is most preferably leucine. Amino acid sequences of Formula 2 may function as an antagonist at the IGF-1R, or as an agonist at the IR. Preferred consensus sequences of the Formula 2 motif are FYX₈ X₉ L X₁₁ X₁₂L, FYX₈ X₉ IX₁₁ X₁₂ L FYX₈ AIX₁₁ X₁₂L, and FYX₈ YFX₁₁ X₁₂ L.
   Another Formula 2 motif for use with this invention comprises FYX₈ YFX₁₁ X₁₂L and is shown as Formula 2A ("NNRP") below: X₁₁₅ X₁₁₆ X₁₁₇ X₁₁₈ FY X₈ YF X₁₁ X₁₂ L X₁₁₉ X₁₂₀ X₁₂₁ X₁₂₂, wherein X₁₁₅-X₁₁₈ and X₁₁₈-X₁₂₂ may be any amino acid which allows for binding to IR or IGF-1R. X₁₁₅ is preferably selected from the group consisting of tryptophan, glycine, aspartic acid, glutamic acid and arginine. Aspartic acid, glutamic acid, glycine, and arginine are more preferred. Tryptophan is most preferred. The preference for tryptophan is based on its presence in clones at a frequency three to five fold higher than that expected over chance for a random substitution, whereas aspartic acid, glutamic acid and arginine are present about two fold over the frequency expected for random substitution.
   X₁₁₆ preferably is an amino acid selected from the group consisting of aspartic acid, histidine, glycine, and asparagine. X₁₁₇ and X₁₁₈ are preferably glycine, aspartic acid, glutamic acid, asparagine or alanine. More preferably X₁₁₇ is glycine, aspartic acid, glutamic acid and asparagine whereas X₁₁₈ is more preferably glycine, aspartic acid, glutamic acid or alanine.
   X₈ when present in the Formula 2A motif is preferably arginine, glycine, glutamic acid, or serine.
   X₁₁ when present in the Formula 2A motif is preferably glutamic acid, asparagine, glutamine, or tryptophan, but most preferably glutamic acid.
   X₁₂ when present in the Formula 2A motif is preferably aspartic acid, glutamic acid, glycine, lysine or glutamine, but most preferably aspartic acid.
   X₁₁₉ is preferably glutamic acid, glycine, glutamine, aspartic acid or alanine, but most preferably glutamic acid.
   X₁₂₀ is preferably glutamic acid, aspartic acid, glycine or glutamine, but most preferably glutamic acid.
   X₁₂₁ is preferably tryptophan, tyrosine, glutamic acid, phenylalanine, histidine, or aspartic acid, but most preferably tryptophan or tyrosine.
   X₁₂₂ is preferably glutamic acid, aspartic acid or glycine; but most preferably glutamic acid.
   Preferred amino acid residue are identified based on their frequency in clones over two fold over that expected for a random event, whereas the more preferred sequences occur about 3-5 times as frequently as expected.
   Nonlimiting examples of amino acid sequences having the Formula 2 and 2A motifs are described in Figures 2A-2P.
3. X₁₄X₁₅X₁₆X₁₇X₁₈X₁₉X₂₀X₂₁ (Formula 3, reverse B6, revB6), wherein X₁₄ and X₁₇ are hydrophobic amino acids; X₁₄, X₁₇ are preferably leucine, isoleucine, and valine, but most preferably leucine; X₁₅, X₁₆, X₁₈ and X₁₉ may be any amino acid; X₂₀ is an aromatic amino acid, preferably tyrosine or histidine, but most preferably tyrosine; and X₂₁ is an aromatic amino acid, but preferably phenylalanine or tyrosine, and most preferably phenylalanine. For use as an IGF-1R binding ligand, an-aromatic amino acid is strongly preferred at X₁₈. See Figures 3A-3D for nonlimiting examples of Formula 3 amino acid sequences.
4. X₂₂X₂₃X₂₄X₂₅X₂₆X₂₇X₂₈X₂₉X₃₀X₃₁X₃₂X₃₃X₃₄X₃₅X₃₆X₃₇X₃₈X₃₉X₄₀X₄₁ (Formula 4, "F8") wherein X₂₂, X₂₅, X₂₆, X₂₈, X₂₉, X₃₀, X₃₃, X₃₄, X₃₅,X₃₆, X₃₇, X₃₈, X₄₀, and X₄₁ are any amino acid. X₃₅ and X₃₇ may be any amino acid when the F8 motif is used as an IR binding ligand or as a component of an IR binding ligand, however for use as an IGF-1R binding ligand, glycine is strongly preferred at X₃₇ and a hydrophobic amino acid, particularly, leucine, is preferred at X₃₅. X₂₃ is a hydrophobic amino acid. Methionine, valine, leucine or isoleucine are preferred amino acids for X₂₃, however, leucine which is most preferred for preparation of an IGF-1R binding ligand is especially preferred for preparation of an IR binding ligand. At least one cysteine is located at X₂₄ through X₂₇, and one at X₃₉ or X₄₀. Together the cysteines are capable of forming a cysteine cross-link to create a looped amino acid sequence. In addition, although a spacing of 14 amino acids in between the two cysteine residues is preferred, other spacings may also be used provided binding to IGF-1R or IR is maintained. Accordingly, other amino acids may be substituted for the cysteines at positions X₂₄ and X₃₉ if the cysteines occupy other positions. In one embodiment, for example, the cysteine at position X₂₄ may occur at position X₂₇ which will produce a smaller loop provided that the cysteine is maintained at position X₃₉. These smaller looped peptides are described herein as Formula 5, infra. X₂₇ is any polar amino acid, but is preferably selected from glutamic acid, glutamine, aspartic acid, asparagine, or as discussed above cysteine. The presence of glutamic acid at position X₂₇ decreases binding to IR but has less of an effect on binding to IGF-1R. X₃₁ is any aromatic amino acid and X₃₂ is any small amino acid. For binding to IGF-1 R, glycine or serine are preferred at position X₃₁, however, tryptophan is highly preferred for binding to IR. At position X₃₂, glycine is preferred for both IGF-1R and IR binding. X₃₆ is an aromatic amino acid. A preferred consensus sequence for F8 is X₂₂ LC X₂₅ X₂₆ E X₂₈ X₂₉ X₃₀ WG X₃₃ X₃₄ X₃₅ X₃₆ X₃₇ X₃₈ C X₄₀ X₄₁ whereas the amino acids are defined above. A more preferred F8 sequence is HLCVLEELFWGASLFGYCSG ("F8"). Amino acid sequences comprising the F8 sequence motif preferably bind to IR over IGF-1R. Figures 4A-4E list nonlimiting examples of Formula 4 amino acid sequences.
5. X₄₂ X₄₃ X₄₄ X₄₅ X₄₆ X₄₇ X₄₈ X₄₉ X₅₀ X₅₁ X₅₂ X₅₃ X₅₄ X₅₅ X₅₆X₅₇ X₅₈ X₅₉ X₆₀ X₆₁ ("mini F8", Formula 5) wherein X₄₂, X₄₃, X₄₄, X₄₅, X₅₃, X₅₅, X₅₆, X₅₈, X₆₀ and X₆₁ are any amino acid. X₄₃, X₄₆, X₄₉, X₅₀ and X₅₄ are hydrophobic amino acids, however, X₄₃ and X₄₆ are preferably leucine, whereas X₅₀ is preferably phenylalanine or tyrosine but most preferably phenylalanine. X₄₇ and X₅₉ are cysteines. X₄₈ is preferably a polar amino acid, i.e. aspartic acid or glutamic acid, but most preferably glutamic acid. Use of the small amino acid at position 54 may confer IGF-1R specificity. X₅₁, X₅₂ and X₅₇ are small amino acids, preferably glycine. A preferred consensus sequence for mini F8 is X₄₂ X₄₃ X₄₄ X₄₅ LCEX₄₉ FGGX₅₃ X₅₄ X₅₅ X₅₆ GX₅₈ CX₆₀ X₆₁. Amino acid sequences comprising the sequence of Formula 5 preferably bind to IGF-1R or IR. Nonlimiting examples of Formula 5 amino acid sequences are described in Figure 5.
6. X₆₂ X₆₃ X₆₄ X₆₅ X₆₆ X₆₇ X₆₈ X₆₉ X₇₀ X₇₁ X₇₂ X₇₃ X₇₄ X₇₅ X₇₆ X₇₇ X₇₈ X₇₉ X₈₀ X₈₁ (Formula 6, "D8") wherein X₆₂, X₆₅, X₆₈, X₆₉, X₇₁, X₇₃, X₇₆, X₇₇, X₇₈, X₈₀ and X₈₁ may be any amino acid. X₆₆ may also be any amino acid, however, there is a strong preference for glutamic acid. Substitution of X₆₆ with glutamine or valine may result in attenuation of binding. X₆₃, X₇₀, and X₇₄ are hydrophobic amino acids. X₆₃ is preferably leucine, isoleucine, methionine, or valine, but most preferably leucine. X₇₀ and X₇₄ are preferably valine, isoleucine, leucine, or methionine. X₇₄ is most preferably valine. X₆₄ is a polar amino acid, more preferably aspartic acid or glutamic acid, and most preferably glutamic acid. X₆₇ and X₇₅ are aromatic amino acids. Whereas tryptophan is highly preferred at X₆₇, X₇₅ is preferably tyrosine or tryptophan but most preferably tyrosine. X₇₂ and X₇₉ are cysteines which again are believed to form a loop which position amino acid may be altered by shifting the cysteines in the amino acid sequence. D8 is most useful as an amino acid sequence having a preference for binding to IR as only a few D8 sequences capable of binding to IGF-1R over background have been detected. A preferred sequence for binding to IR is X₆₂ L X₆₄ X₆₅ X₆₆ W X₆₈ X₆₉ X₇₀ X₇₁ C X₇₃ X₇₄ X₇₅ X₇₆ X₇₇ X₇₈ C X₈₀ X₈₁ Nonlimiting examples of Formula 6 amino acid sequences are described in Figures 6A-6E.
7. HX₈₂, X₈₃, X₈₄ X₈₅ X₈₆ X₈₇ X₈₈ X₈₉ X₉₀ X₉₁ X₉₂ (Formula 7) wherein X₈₂ is proline or alanine but most preferably proline; X₈₃ is a small amino acid more preferably proline, serine or threonine and most preferably proline; X₈₄ is selected from leucine, serine or threonine but most preferably leucine; X₈₅ is a polar amino acid preferably glutamic acid, serine, lysine or asparagine but more preferably serine; X₈₆ may be any amino acid but is preferably a polar amino acid such as histidine, glutamic acid, aspartic acid, or glutamine; X₈₇ is an aliphatic amino acid preferably leucine, methionine or isoleucine and most preferably leucine; amino acid X₈₈, X₈₉ and X₉₀ may be any amino acids; X₉₁ is an aliphatic amino acid with a strong preference for leucine as is X₉₂. Phenylalanine may also be used at position 92. A preferred consensus sequence of Formula 7 is HPPLSX₈₆LX₈₈X₈₉X₉₀LL. The Formula 7 motif binds to IR with little or no binding to lGF-1R. Nonlimiting examples of Formula 7 amino acid sequences are described in Figure 7.
8. Another sequence is X₁₀₄, X₁₀₅ X₁₀₆ X₁₀₇ X₁₀₈ X₁₀₉ X₁₁₀ X₁₁₁ X₁₁₂ X₁₁₃ X₁₁₄. (Formula 8) which comprises eleven amino acids wherein at least one, and preferably two of the amino acids of X₁₀₆ through X₁₁₁ are tryptophan. In addition, it is also preferred that when two tryptophan amino acids are present in the sequence they are separated by three amino acids, which are preferably, in sequential order proline, threonine and tyrosine with proline being adjacent to the tryptophan at the amino terminal end. Accordingly, the most preferred sequence for X₁₀₇ X₁₀₈ X₁₀₉ X₁₁₀ X₁₁₁ is WPTYW. At least one of the three amino acids on the amino terminal (X_{104,} X₁₀₅ X₁₀₆) and at least one of the amino acids carboxy terminal (X₁₁₂ X₁₁₃ X₁₁₄) ends immediately flanking X₁₀₇-X₁₁₁ are preferably a cysteine residue, most preferably at X₁₀₅ and X₁₁₃ respectively. Without being bound by theory, the cysteines are preferably spaced so as to allow for the formation of a loop structure. X₁₀₄ and X₁₁₄ are both small amino acids such as, for example, alanine and glycine. Most preferably, X₁₀₄ is alanine and X₁₁₄ is glycine. X₁₀₅ may be any amino acid but is preferably valine. X₁₁₂ is preferably asparagine. Thus, the most preferred sequence is ACVWPTYWNCG. The IR binding displayed amino acid sequences are described in Figure 8.
9. An amino acid sequence comprising DYKDLCQSWGVRIGWLAGLCPKK (Formula 9, JBA5). The Formula 9 motif is another motif believed to form a cysteine loop which possesses agonist activity at both IR and IGF-1R. Although IR binding is not detectable by ELISA, binding of Formula 9 to IR is competed by insulin and is agonistic. See Figure 11A. Binding of Formula 9 through IGF-1R is detected by ELISA. Nonlimiting examples of Formula 9 amino acid sequences are described in Figures 9A-9C.
10. WX₁₂₃ GYX₁₂₄ WX₁₂₅ X₁₂₆ (Formula 10, Group 6 Secondary Library) wherein X₁₂₃ is selected from proline, glycine, serine, arginine, alanine or leucine, but more preferably proline; X₁₂₄ is any amino acid, but preferably a charged or aromatic amino acid; X₁₂₅ is a hydrophobic amino acid preferably leucine or phenylalanine, and most preferably leucine. X₁₂₆ is any amino acid, but preferably a small amino acid. Nonlimiting examples of Formula 10 amino acid sequences are described in Figures 10A-10B.
11. Other Motifs
   Another motif for use with this invention includes WPGY. Examples of specific peptide sequences comprising this motif include
   KVRGFQGGTVWPGYEWLRNAAKK (E8), and
   KSMFVAGSDRWPGYGVLADWLKK (F2).
   Various amino acid sequences which bind lR and/or IGF-1R have been identified through panning of various libraries designed to identify preferred IR or IGF-1R consensus sequences which do not correspond to one of the motifs described above. Such sequences are described in Figures 10C-10I.

### B. Amino And Carboxyl Terminal Extensions Modulate Activity of Motifs

In addition to the motifs stated above, the invention also provides preferred sequences at the amino terminal or carboxyl terminal ends which are capable of enhancing binding of the motifs to either IR, IGF-1 R, or both. In addition, the use of the extensions described below does not preclude the possible use of the motifs with other substitutions, additions or deletions which allow for binding to IR, IGF-1R or both.

### 1. Formula 1

Any amino acid sequence may be used for extensions of the amino terminal end of A6, although certain amino acids in amino terminal extensions may be identified which modulate activity. Preferred carboxy terminal extensions for A6 are A6 X₉₃ X₉₄ X₉₅ X₉₆ X₉₇ wherein X₉₃ may be any amino acid, but is preferably selected from the group consisting of alanine, valine, aspartic acid, glutamic acid, and arginine, and X₉₄ and X₉₇ are any amino acid; X₉₅ is preferably glutamine, glutamic acid, alanine or lysine but most preferably glutamine. The presence of glutamic acid at X₉₅ however may confer some IR selectivity. Further, the failure to obtain sequences having an asparagine or aspartic acid at position X₉₅ may indicate that these amino acids should be avoided to maintain or enhance sufficient binding to IR and IGF-1R. X₉₆ is preferably a hydrophobic or aliphatic amino acid, more preferably leucine, isoleucine, valine, or tryptophan but most preferably leucine. Hydrophobic residues, especially tryptophan at X₉₆ may be used to enhance IR selectivity.

### 2. Formula 2

B6 with amino terminal and carboxy terminal extensions may be represented as X₉₈ X₉₉ B6 X₁₀₀. X₉₈ is optionally aspartic acid and X₉₉ is independently an amino acid selected from the group consisting of glycine, glutamine, and proline. The presence of an aspartic acid at X₉₈ and a proline at X₉₉ is associated with an enhancement of binding for both IR and IGF-1 R. A hydrophobic amino acid is preferred for the amino acid at X₁₀₀, an aliphatic amino acid is more preferred. Most preferably leucine, for IR and valine for IGF-1R. Negatively charged amino acids are preferred at both the amino and carboxy terminals of Formula 2A.

### 3. Formula 3

An amino terminal extension of Formula 3 defined as X₁₀₁ X₁₀₂ X₁₀₃ revB6 wherein X₁₀₃ is a hydrophobic amino acid, preferably leucine, isoleucine or valine, and X₁₀₂ and X₁₀₁ are preferably polar amino acids, more preferably aspartic acid or glutamic acid may be useful for enhancing binding to IR and IGF-1R. No preference is apparent for the amino acids at the carboxy terminal end of Formula 3.

### C. Secondary Structure

Without being bound by theory, it is believed that the B6 and reverse B6 motifs participate in alpha helix formation such that the most highly preferred residues at positions X₆, X₇, X₁₀ and X₁₃ (B6) and X₁₄ X₁₇ X₂₀ and X₂₁ (rB6) reside on the same side of a helix. See Figure 12. Because both B6 and RB6 motifs form structurally analogous motifs from their palindrome sequences, the use of D-amino acids instead of typical L-amino acids would be expected to produce amino acid sequences having similar properties to the L-amino acid sequences. D-amino acids may be advantageous, as the resultant sequences may be more resistant to enzymatic degradation than L-amino acid sequences. In addition, to maintain the appropriate orientation of highly preferred amino acid sequences on the appropriate side of the helix, it is important to maintain the spacing of those residues along the amino acid sequence. For example, the second and third amino acids of B6 (X₇ and X₈) are oriented at opposite sides of the helix. See Figure 12.

### D. IR Binding Preferences

As indicated above, the amino acid sequences containing the motifs of this invention may be constructed to have enhanced selectivity for either IR or IGF-1R by choosing appropriate amino acids at specific positions of the motifs or the regions flanking them. By providing amino acid preferences for IR or IGF-1R, this invention provides the means for constructing amino acid sequences with minimized activity at the non-cognate receptor. For example, the amino acid sequences disclosed herein with high affinity and activity for IR and low affinity and activity for IGF-1R are desirable as IR agonist as their propensity to promote undesirable cell proliferation, an activity of IGF-1 agonists, is reduced. Ratios of IR binding affinity to IGF-1R binding affinity for specific sequences are provided in Figures 1A-10I. As an insulin therapeutic, the IR/IGF-1R binding affinity ratio is preferably greater than 100. Conversely, for use as an IGF-1R therapeutic, the IR/IGF-1R ratio should be less than 0.01. Examples of peptides that selectively bind to IGF-1R are shown below.

| **IGF-1R-SELECTIVE SEQUENCES** | | | | | | |
|---|---|---|---|---|---|---|
| **MOTIF 1 (A6-like):** | | **Ratios over Background** | | | **Comparisons** | |
| **Clone** | **Sequence** | **E-Tag** | **IGF-1R** | **IR** | **IGF-1R/IR** | **IR/IGF-1R** |
| A6L-0-E6-IR | YRGMLVLGRSSDGAGKVAFERPARIGQTVFAVNFYDWFV | 31.0 | 31.0 | 1.8 | 17.0 | 0.1 |
| H2CA-4-G9-IGFR | GIISQSCPESFYDWFAGQVSDPWWCW | 8.6 | 9.5 | 0.6 | 16.0 | 0.1 |
| H2CA-4-H6-IGFR | VGRASGFPENFYDWFGRQLSLQSGEQ | 4.9 | 10.5 | 0.7 | 14.6 | 0.1 |
| A6L-0-E4-IR | YRGMLVLGRISDGAG#VASEPPARIGRKVFAVNFYDWFV | 26.0 | 16.0 | 1.3 | 13.0 | 0.1 |
| A6L-0-H3-IR | YRGMLVLGRISGGAGKAASERPARIGQKVSAVNFYDWFV | 27.0 | 26.0 | 2.0 | 13.0 | 0.1 |
| H2CA-4-F5-IGFR | VGYQGQGDENFYDWFIRQVSGRLGVQ | 5.5 | 9.7 | 0.8 | 12.3 | 0.1 |
| H2CA-4-H8-IGFR | SACQFDCHENFYDWFARQVSGGAAYG | 5.6 | 9.2 | 1.0 | 9.4 | 0.1 |
| H2CA-4-F11-IGFR | SAAQLFFQESFYDWFLRQVAESSQPN | 3.5 | 6.8 | 1.0 | 6.7 | 0.1 |
| H2CA-4-F6-IGFR | AVRATRFDEAFYDWFVRQISDGQGNK | 3.9 | 7.3 | 1.1 | 6.4 | 0.2 |
| H2CA-4-F10-IGFR | VNQSGSIHENFYDWFERQVSHQRGVR | 4.9 | 5.7 | 1.0 | 5.9 | 0.2 |
| H2CA-1-A3-IGFR | APDPSDFQEIFYDWFVRQVSRMPGGG | 7.7 | 3.8 | 0.8 | 5.1 | 0.2 |
| H2CA-3-C8-IGFR | SSCDGAGHESFYEWFVRQVSGCRSV | 15.1 | 5.6 | 1.2 | 4.8 | 0.2 |
| H2CA-2-B9-IGFR | RAGSSDFHEDFYEWFVRQVSLSLKGK | 9.3 | 7.0 | 1.7 | 4.2 | 0.2 |
| H2CA-4-H4-IGFR | QAVQPGFHEEFYDWFVRQVSTGVGGG | 3.9 | 4.1 | 1.0 | 4.2 | 0.2 |
| E4Dα-4-H2-IR | GFREGNFYEWFQAQVT | 37.8 | 33.9 | 8.2 | 4.1 | 0.2 |
| H2CA-4-F7-IGFR | SSIGGGFHENFYDWFSRQLSQSPPLK | 1.5 | 3.2 | 0.8 | 4.1 | 0.2 |
| H2CA-3-D6-IGFR | QSPVGSSHEDFYDWFFRQVAQSGAHQ | 8.3 | 9.0 | 2.2 | 4.0 | 0.3 |
| H2CA-3-D8-IGFR | NYRRQVFNGNFYDWFDRQVFSLVTPG | 10.9 | 7.2 | 1.8 | 4.0 | 0.3 |
| H2CA-4-G11-IGFR | TLDGGSFEEQFYDWFVRQLSYRTNPD | 10.8 | 9.5 | 2.5 | 3.9 | 0.3 |
| H2CA-4-FI-IGFR | FYVQQWGHENFYDWFDRQVSQSGGAG | 5.8 | 3.5 | 0.9 | 3.8 | 0.3 |
| H2CA-3-D7-IGFR | LRRQAPVEENFYDWFVRQVSGDRVGG | 13.3 | 3.0 | 0.8 | 3.7 | 0.3 |
| H2CA-1-A7-IGFR | RCGRELYHSTFYDWFDRQVAGRTCPS | 8.0 | 2.2 | 0.6 | 3.7 | 0.3 |
| H2CA-2-B4-IGFR | CCLLCRFQQNFYDWFVCQGISRLRPL | 3.5 | 4.1 | 1.1 | 3.6 | 0.3 |
| H2CA-2-B3-IGFR | PPLASDLDVQFYGWFVQQVS PPGRGG | 7.7 | 3.8 | 1.0 | 3.6 | 0.3 |

| | | **Ratios over Background** | | | **Comparisons** | |
|---|---|---|---|---|---|---|
| **Clone** | **Sequence** | **E-Tag** | **IGF-1R** | **IR** | **IGF-1R/IR** | **IR/IGF-1R** |
| H2CA-2-B2-IGFR | GAPVDQLHEDFYDWFVRQVSQAATG | 4.1 | 3.4 | 1.0 | 3.5 | 0.3 |
| E4Dα-2-D11-IR | GFREGSFYDWFQAQVT | 40.2 | 11.1 | 3.3 | 3.4 | 0.3 |
| 20E2Bβ-4 -G6-IR | SQAGSAFYAWFDQVLRTVHSA | 22.4 | 6.2 | 1.9 | 3.3 | 0.3 |
| H2CA-4-H9-IGFR | RGAVAGFHDQFYDWFDRQVSRVHKFG | 8.7 | 5.6 | 1.9 | 3.0 | 0.3 |
| H2CA-2-B11-IGFR | AICDAGFHEHFYDWFALQVSDCGRQS | 11.9 | 4.6 | 1.6 | 3.0 | 0.3 |
| H2CA-3-E8-IGFR | LGYQEPFQQNFYDWFVRQVSGAENAG | 13.2 | 6.3 | 2.2 | 2.9 | 0.3 |
| A6S-2-D11-IR | EAASLGSQDRNFYDWFVRQW | 48.4 | 37.4 | 13.5 | 2.8 | 0.4 |
| A6S-2-D1-IR | VERSASSQDGNFYDWFWQIR | 37.8 | 30.6 | 12.0 | 2.6 | 0.4 |
| A6S-3-E2-IR | TSEVQRRSQDNFYDWFVAQVA | 33.1 | 24.7 | 9.8 | 2.5 | 0.4 |
| H2CA-3-E11-IGFR | HLADGQFHEKFYDWFERQISSRCNDC | 4.7 | 2.2 | 1.0 | 2.2 | 0.5 |
| H2CA-3-C11-IGFR | FRTLAAQHDSFYDWFDRQVSGAAGER | 9.3 | 3.3 | 1.6 | 2.1 | 0.5 |
| | | | | | | |
| A6-PD1-IGFR | SFHEDFYDWFDRQVSGSLKK | | | | | |
| H2C-PD1-IGFR (RP9) | GSLDESFYDWFERQLGKK | | | | | |

| **MOTIF 2 (B6-like):** | | | | | | |
|---|---|---|---|---|---|---|
| | | **Ratios over Background** | | | **Comparisons** | |
| **Clone** | **Sequence** | **E-Tag** | **IGF-1R** | **IR** | **IGF-1 R/IR** | **IR/IGF-1R** |
| 20C-3-G3-IGFR | TFYSCLASLLTGTPQPNRGPWERCR | 33.1 | 32.3 | 1.2 | 27.0 | <0.1 |
| 20C-4-C7-IGFR | FFYDCLAALLQGVARYHDLCAVEIT | 35.3 | 28.0 | 1.3 | 21.8 | <0.1 |
| B6Hα-1-B5-IR | CCTTEMWMDARDDPFYHKLSELVTGG | 41.5 | 20.5 | 1.0 | 20.5 | 0.0 |
| R20β-4-A6-IR | RGQSDAFYSGLWALIGLSDG | 9.3 | 25.9 | 1.5 | 17.3 | 0.1 |
| 20E2B-1-A6-IGFR | GVRAMSFYDALVSVLGLGPSG | 18.6 | 18.1 | 1.1 | 16.8 | 0.1 |
| R20α-4-20A12-IR | RLFYCGIQALGANLGYSGCV | 48.6 | 39.9 | 2.4 | 16.6 | 0.1 |
| 20E2Bβ-4-G7-IR | LQPCSGFYECIERLIGVKLSG | 19.9 | 25.2 | 1.6 | 15.8 | 0.1 |
| NNRPγ-4-B11-IR | LKDGFYDYFWQRLHLGS | 4.1 | 18.7 | 1.2 | 15.5 | 0.1 |
| 20E28-3-C6-IGFR | VEGRGLFYDLLRQLLARRQNG | 17.9 | 16.8 | 1.1 | 14.8 | 0.1 |
| B6Hα-1-A2-IR | RGCNDDGGKGWSDDPFYHKLSELICGG | 22.3 | 14.6 | 1.0 | 14.6 | 0.1 |
| 20E2A-4-F11-IGFR | QGGSASFYDAIDRLLRMRIGG | 21.3 | 18.8 | 1.3 | 14.6 | 0.1 |
| B6Hα-3-E9-IR | RCEEKQAEVGPSSDPFYHKMSELLGCR | 44.6 | 24.2 | 1.7 | 14.2 | 0.1 |
| 20C-3-F6-IGFR | DRDFCRFYERLTALVGGQVDGGWPC | 33.5 | 26.1 | 1.9 | 14.1 | 0.1 |
| 20E2B-4-H3-IGFR | KLHNLMFYYGLQRLVWGAGLG | 11.2 | 14.8 | 1.1 | 13.9 | 0.1 |
| 20E2B-3-C2-IGFF2 | GNGDGMFYQLLSLLVGRDMHV | 13.1 | 8.9 | 0.6 | 13.8 | 0.1 |
| 20C-3-A1-IGFR | SSYGCDGFYLMLFSLGLVASQELEC | 26.5 | 20.8 | 1.5 | 13.7 | 0.1 |
| 20E2B-3-E3-IGFR | PDLHKGFYAQLAQLIRGQLLS | 22.4 | 16.3 | 1.3 | 13.1 | 0.1 |
| R20α-3-20E2-IR | FYDAIDQLVRGSARAGGTRD | 46.3 | 39.9 | 3.1 | 12.9 | 0.1 |
| 20E2B-4-H12-IGFR | YSCGDGFYSLLSDLLGGQFRC | 6.5 | 9.7 | 0.8 | 12.8 | 0.1 |
| B6Hα-3-F11-IR | RGMKEEVLVGGSTDPFYHKLSELLQGS | 49.5 | 18.7 | 1.6 | 11.7 | 0.1 |
| 20E2B-3-D2-IGFR | IQQELTFYDLLHRLVRSELGS | 20.7 | 12.4 | 1.1 | 11.7 | 0.1 |
| 20E2B-3-D8-IGFR | GGTEVDFYRALERLVRGQLGL | 20.4 | 17.7 | 1.6 | 11.3 | 0.1 |
| 20E2B-3-E8-IGFR | LRIANLFYQRLWDLAFGGGG | 15.7 | 16.7 | 1.5 | 11.1 | 0.1 |
| B6Hα-2-C4-IR | RCGRW*AEMGAGDDPFYHKLSELVCG | 20.7 | 9.9 | 0.9 | 11.0 | 0.1 |
| R20α-4-2OC11-IR | DRAFYNGLRDLVGAVYGAWD | 43.7 | 30.8 | 3.0 | 10.3 | 0.1 |
| 20E28-4-F8-IGFR | PVGVQGFYEGLSRLVLGRGGW | 12.3 | 7.3 | 0.8 | 9.7 | 0.1 |

| | | **Ratios over Background** | | | **Comparisons** | |
|---|---|---|---|---|---|---|
| **Clone** | **Sequence** | **E-Tag** | **IGF-1R** | **IR** | **IGF-1R/IR** | **IR/IGF-1R** |
| 20E2B-1-A11-IGFR | RFSTDGFYQYLLALVGGGPVG | 15.0 | 9.5 | 1.0 | 9.7 | 0.1 |
| 20E2B-3-D4-IGFR | NSRDGGFYLQLERLLGFPVTG | 8.1 | 7.9 | 0.8 | 9.6 | 0.1 |
| 20E2B-2-B11-IGFR | VVTPVNFYRALEALVRG.RLG | 13.9 | 10.6 | 1.1 | 9.4 | 0.1 |
| 20E2B-3-C8-IGFR | QPAPDGFYSALMKLIGRGGVS | 18.5 | 15.6 | 1.8 | 8.9 | 0.1 |
| 20E2B-2-B2-IGFR | PGTDLGFYQALRCVVIQGACD | 11.7 | 4.9 | 0.6 | 8.1 | 0.1 |
| 20E2B-4-F10-IGFR | AQPCGGFYGLLEQLVGRSVCD | 19.0 | 17.3 | 2.2 | 7.8 | 0.1 |
| 20E2B-4-F9-IGFR | QPDHSYFYSLLQELVGSEERL | 11.9 | 14.7 | 1.9 | 7.7 | 0.1 |
| 20C-3-A4-IGFR | QFYGCLLDLSLGVPSFGWRRRCITA | 17.7 | 8.8 | 1.2 | 7.6 | 0.1 |
| 20E2B-3-D11-IGFR | LGVTDGFYAALGYLIHGVGQF | 14.3 | 12.2 | 1.6 | 7.6 | 0.1 |
| 20E2B-3-C11-IGFR | CMM.DGFYAGLGCLLTAGEGR | 15.3 | 15.4 | 2.1 | 7.5 | 0.1 |
| 20E2B-2-B3-IGFR | ICTGQGFYQVLCGLLRGTSAR | 9.1 | 5.3 | 0.7 | 7.4 | 0.1 |
| 20E2B-3-D12-IGFR | QGNVLDFYGWIGRLLAKQGSD | 10.3 | 6.2 | 0.9 | 7.3 | 0.1 |
| 20E2B-3-E12-IGFR | VATSQGFYSGLSELLQGGGNV | 13.9 | 6.0 | 0.8 | 7.3 | 0.1 |
| 20E2B-2-B8-IGFR | IWATGDFYRLLSQLVMGRVGT | 17.4 | 5.7 | 0.8 | 7.2 | 0.1 |
| NNRPγ-4-A9-IR | EGSGFYGYFFSLLGLQG | 3.0 | 10.0 | 1.4 | 7.1 | 0.1 |
| 20E2B-4-G11-IGFR | RQGTGSFYLMLEQLLVGARGP | 8.9 | 4.5 | 0.6 | 7.0 | 0.1 |
| 20E2B-3-D6-IGFR | DSVGDNFYQLLESLVGGHGVG | 20.7 | 17.8 | 2.6 | 6.9 | 0.1 |
| B6Hα-2-C7-IR | RGIVAMVEATEVGSDHDPFYHKLSELVQGS | 45.1 | 6.7 | 1.0 | 6.7 | 0.1 |
| 20E2B-2-B7-IGFR | LSSDGQFYRALNLLLQGSAGR | 18.0 | 6.1 | 0.9 | 6.7 | 0.1 |
| 20E2B-3-C4-IGFR | ASSASGFYELLQRLAGLGLEV | 23.4 | 20.4 | 3.3 | 6.2 | 0.2 |
| 20C-3-E4-IGFR | FFYRCLSRLLGGQLGSRLGLSCIGD | 37.7 | 7.7 | 1.3 | 6.0 | 0.2 |
| NNRPγ-4-A1-IR | IIGGFYSYFNSVLRLGT | 9.7 | 10.9 | 1.8 | 6.0 | 0.2 |
| 20E2B-4-H8-IGFR | PAGPCGFYCGLGLLLHGDQSP | 7.2 | 5.3 | 0.9 | 5.9 | 0.2 |
| 20E2B-4-H9-TGFR | RCQGTGFYTCIQELIGFGDPD | 4.5 | 5.2 | 0.9 | 5.6 | 0.2 |
| B6Hα-2-CIO-IR | SGAKVIVVTGDSGDPFYHKLSELLQGS | 46.9 | 5.8 | 1.1 | 5.3 | 0.2 |
| 20E2A-3-C7-IGFR | VGTVAGFYDAIAQLVARASRV | 17.6 | 5.4 | 1.1 | 5.1 | 0.2 |
| 20E2B-1-A8-IGFR | TLRSPTFYDWLEMVLTHGQGG | 16.1 | 4.4 | 0.9 | 5.0 | 0.2 |
| NNRPγ-4-A7-IR | RFDPFYSYFVNLLGASA | 2.5 | 6.3 | 1.3 | 4.9 | 0.2 |
| B6Hα-3-E8-IR | RGKTAAVIVGRPADPFYHKLSELLQGG | 47.6 | 5.3 | 1.1 | 4.8 | 0.2 |
| B6Hα-3-F10-IR | GCVVEWQKWHGASDPFYHKLSELGGCS | 47.2 | 8.8 | 1.9 | 4.6 | 0.2 |
| B6Hα-2-D6-IR | GRTMAVMAAGGPDDPFYHICLSELVQGG | 33.5 | 4.4 | 1.0 | 4.4 | 0.2 |
| B6Hα-3-E7-IR | GCAVVEEAERSRGDPFYHKLSELIQGC | 47.0 | 5.6 | 1.3 | 4.3 | 0.2 |
| B6Hα-2-D1-IR | GCEVIVEEGDSAUPFYHKLSELCQGS | 11.7 | 5.4 | 1.3 | 4.2 | 0.2 |
| 20E2A-3-D10-IGFR | MMVVDGFYDALHQLWAQSLG | 20.6 | 6.9 | 1.8 | 3.9 | 0.3 |
| 20E2A-3-A12-IGFR | LSVALSFYDALGQLVAGEGRW | 16.1 | 4.3 | 1.1 | 3.9 | 0.3 |
| B6Hα-4-G8-IR | GGTKAVAKVGT`RDDPFYHKLSELLQGS | 32.3 | 6.1 | 1.7 | 3.6 | 0.3 |
| B6L-4-D7-IR | AETSVQVGWIRLQSVWPGEHWNTVDPFYHKLSELLRGSGA | 14.3 | 4.8 | 1.4 | 3.4 | 0.3 |
| B6Hα-1-A3-IR | SRAKVEAEMPDSGDPFYHKLSELLASG | 37.4 | 2.6 | 0.8 | 3.3 | 0.3 |
| S6Hα-3-F7-IR | SRVAATKEKRPSDDPFYHKLSELLQGS | 41.5 | 3.1 | 1.0 | 3.1 | 0.3 |
| B6Hα-2-D8-IR | SSETAKMVTGTRDDPFYHKLSELVQGS | 19.3 | 3.0 | 1.0 | 3.0 | 0.3 |
| B6Hα-1-B3-IR | GCITAENGAGDPFYHKLSELGGCS | 33.1 | 3.2 | 1.1 | 2.9 | 0.3 |
| B6Hα-3-E5-IR | RCGDEEGWQENRRDDPFYHKLSEIfFGGC | 28.8 | 2.9 | 1.0 | 2.9 | 0.3 |
| 20E2A-4-G11-IGFR 20E2Bβ-3C7-IR | MNVFVSFYDAIDQLVCQRIGC | 20.7 | 3.3 | 1.3 | 2.6 | 0.4 |
| | QSGSGDFYDWLSRLIRGNGDG | 1.5 | 3.1 | 1.5 | 2.0 | 0.5 |
| B6Hα-3-E6-IR | CGAKMTGTPNDPFYHKLSELLQRG | 18.2 | 2.3 | 1.2 | 1.9 | 0.5 |
| 20E2A-3-A3-IGFR | GHYFGSFYDAIDQLVAGMLPG | 5.2 | 3.0 | 1.5 | 1.9 | 0.5 |
| B6L-4-A7-IR | AGTPAQVG*NRLWSVWPGEHNNTVDPFYNKLSELLRESGA | 11.6 | 3.4, | 1.9 | 1.8 | 0.6 |
| B6Hα-3-F1-IR | CSMAAVAEAGDDDDPFYHKLSELCQGS | 22.5 | 2.4 | 1.3 | 1.8 | 0.5 |
| B6L-3-G6-IR | VDTPAQVGWNRLWSVGPGEHWYTDDPFYH*LSELLRESGA | 7.6 | 2.5 | 1.8 | 1.4 | 0.7 |
| B6L-3-G5-IR | AETSAQVGWQRLWSVWPGDHWSTLDPFYHKLSELLRESGA | 11.5 | 2.0 | 1.4 | 1.4 | 0.7 |
| 20E2A-3-A4-IGFR | AGSVTSFYDAMEQLVATGTSA | 16.8 | 2.5 | 1.8 | 1.4 | 0.7 |
| | | | | | | |
| B6-PD1-IGFR | TDDGFYDALEQLVQGSKK | | | | | |
| 20E2-PD1-IGFR(RP10) | GSFYEALQRLVGGEQGKK | | | | | |

| **MOTIF 10 (Group6):** | | | | | | |
|---|---|---|---|---|---|---|
| | | **Ratios over Background** | | | **Comparisons** | |
| **Clone** | **Sequence** | **E-Tag** | **IGF-1R** | **IR** | **IGF-1 R/IR** | **IR/IGF-1R** |
| R20β-4-E8-IR | VRGFQGGTVWPGYEWLRNAA | 41.0 | 34.9 | 3.6 | 9.7 | 0.1 |
| 40F-4-D1-IGFR | LSCLAYSRHGIWRPSTDLGLGRSVGEGSVSTRWRGYDWFE | 4.9 | 4.6 | 0.3 | 13.1 | 0.1 |
| 40F-4-B1-IGFR | GLDHSDAVGVHLGFAWPAQARGRWEAGGLEDTWAGYDWL | 4.1 | 3.0 | 0.2 | 13.1 | 0.1 |
| 40F-4-D10-IGFR | W.GYAWLS | 4.9 | 4.5 | 0.4 | 11.7 | 0.1 |
| R20β-4-E8-IR | VRGFQGGTVWPGYEWLRNAA | 41.0 | 3.6 | 34.9 | 0.1 | 9.7 |

Besides relative binding at IR or IGF-1R, relative efficacy at the cognate receptor is another important consideration for choosing a potential therapeutic. Thus, a sequence which is efficacious at IR but has little or no significant activity at IGF-1R may also be considered as an important IR therapeutic, irrespective of the relative binding affinities at IR and IGF-1R.

A6 selectivity for IR may be enhanced by including glutamic acid in a carboxyl terminal extension at position X₉₅. IR selectivity of the B6 motif may be enhanced by having a tryptophan or phenylalanine at X₁₁. Tryptophan at X₁₃ also favors selectivity of IR. A tryptophan amino acid at X₁₃ rather than leucine at that position also may be used to enhance selectivity for IR. In the reverse B6 motif, a large amino acid at X₁₅ favors IR selectivity. Conversely, small amino acids may confer specificity for IGF-1R. In the F8 motif, an L in position X₂₃ is essentially required for IR binding. In addition, tryptophan at X₃₁ is also highly preferred. At X₃₂, glycine is preferred for IR selectivity.

### E. Multiple Binding Sites On IR And IGF-1R

The competition data disclosed herein reveals that at least two separate binding sites are present on IR and IGF-1 R which recognize the different sequence motifs provided by this invention.

As shown in Figure 13, competition data (See Example 15) indicates that peptides comprising the A6, B6, revB6, and F2 motifs compete for binding to the same site on IR (Site 1) whereas the F8 and D8 motifs compete for a second site (Site 2). Similarly, the decrease of dissociation of B6 motif peptide (20E2) from IGF-1 R by a D8 ligand indicates multiple interacting binding sites.

The identification of peptides which bind to separate binding sites on IR and IGF-1R provides for various schemes of binding to IR or IGF-1R to increase or decrease its activity. Examples of such schemes for IR are illustrated in Figure 15.

The table below shows sequences based on their groups, which bind to Site 1 or Site 2.

| **REPRESENTATIVE SITE 1 PEPTIDES** | | |
|---|---|---|
| **A6-like (FYxWF):** | | |
| Clone | | Sequence |
| G3 | | KRGGGTFYEWFESALRKHGAGKK |
| H2 | | VTFTSAVFHENFYDWFVRQVSKK |
| H2C | | FHENFYDWFVRQVSKK |
| A6S-IR3-E12 | | GRVDWLQRNANFYDWFVAELG |
| A6S-IR4-G1 | | NGVERAGTGDNFYDWFVAQLH |
| H2CB-R3-B12 | | QSDSGTVHDRFYGWFRDTWAS |
| | | |
| 20E2A-R3-B11 | | GRFYGWFQDAIDQLMPWGFDP |
| rB6-F6 | | RYGRWGLAQQFYDWFDR |
| E4Dα-1-B8-IR- | | GFREGQRWYWFVAQVT |
| H2CA-4-F11-IR | | TYKARFLHENFYDWFNRQVSQYFGRV |
| H2CB-R3-D2 | | WTDVDGFHSGFYRWFQNQWER |
| H2CB-R3-D12 | | VASGHVLHGQFYRWFVDQFAL |
| H2CB-R4-H5 | | QARVGNVHQQFYEWFREVMQG |
| H2C-B-E8* | | TGHRLGLDEQFYWWFRDALSG |
| H2CB-3-B6-IR- | | VGDFCVSHDCFYGWFLRESMQ |
| A6S-IR2-C1 | | RMYFSTGAPQNFYDWFVQEWD |
| | | |

| **B6-like (FYxxLxxL):** | | |
|---|---|---|
| Clone | | Sequence |
| 20C11 | | KDRAFYNGLRDLVGAVYGAWDKK |
| 20E2 | | DYKDFYDAIDQLVRGSARAGGTRDKK |
| B62-R3-C7 | | EHWNTVDPFYFTLFEWLRESG |
| B62-R3-C10 | | EHWNTVDPFYQYFSELLRESG |
| | | |
| 20E2B-3-B3-IR | | AGVNAGFYRYFSTLLDWWDQG |
| 20E2-B-E3* | | IQGWEPFYGWFDDVVAQMFEE |
| 20E2A-R4-F9 | | PPWGARFYDAIEQLVFDNLCC |
| RPNN-4-G6-HOLO* | | RWPNFYGYFESLLTHFS |
| RPNN-4-F3-HOLO* | | HYNAFYEYFQVLLAETW |
| 20E2A-R4-E2 | | IGRVRSFYDAIDKLFQSDWER |
| RPNN-2-C1-IR* | | EGWDFYSYFSGLLASVT |
| 20E2B-4-F12-IR | | SVKEVQFYRYFYDLLQSEESG |
| 20E2-B-E12 | | GNSGGSFYRYFQLLLDSDGMS |
| 20E2A-R3-B6 | | RDAGSSFYDAIDQLVCLTYFC |
| | | |

| **Reverse B6-like (LxxLxxYF):** | | |
|---|---|---|
| Clone | | Sequence |
| rB6-A12 | | LDALDRLMRYFEERPSL |
| rB6-F9 | | PLAELWAYFEHSEQGRSSAH |
| | | |
| rB6-4-E7-IR | | LDPLDALLQYFWSVPGH |
| rB6-4-F9-IR | | RGRLGSLSTQFYNWFAE |
| rB6-E6 | | ADELEWLLDYFMHQPRP |
| rB6-4-F12-IR | | DGVLEELFSYFSATVGP |
| | | |

| **Group 6 (WPxYxWL):** | | |
|---|---|---|
| Clone | | Sequence |
| R20β-4-A4-IR | WPGYLFFEEALQDWRGSTED | |
| | | |

| **Peptides by design**:** | | |
|---|---|---|
| Clone | | Sequence |
| H2C-PD1-IR | | AAVHEQFYDWFADQYKK |
| A6S-PD1-IR- | | QAPSNFYDWFVREWDKK |
| 20E2-PDI-IR- | | QSFYDYIEELLGGEWKK |
| B6C-PD1-IR- | | DPFYQGLWEWLRESGKK |

| **REPRESENTATIVE SITE 2 PEPTIDES (C-C LOOPS)** | |
|---|---|
| **F8-derived (Long C-C loop):** | |
| Clone | Sequence |
| F8 | HLCVLEELFWGASLFGYCSG |
| F8-C12 | FQSLLEELVWGAPLFRYGTG |
| F8-Des2 | PLCVLEELFWGASLFGYCSG |
| | |
| F8-F12 | PLCVLEELFWGASLFGQCSG |
| F8-B9 | HLCVLEELFWGASLFGQCSG |
| F8-B12 | DLRVLCELFGGAYVLGYCSE |
| NNKH-2B3 | HRSVLKQLSWGASLFGQWAG |
| NNKH-2F9- | HLSVGEELSWWVALLGQWAR |
| NNKH-4H4- | APVSTEELRWGALLFGQWAG |
| | |

| **D8-derived (Small C-C loop):** | |
|---|---|
| Clone | Sequence |
| D8 | KWLDQEWAWVQCEVYGRGCPSKK |
| D8-G1 | QLEEEWAGVQCEVYGRECPS |
| D8-B5~ | ALEEEWAWVQVRSIRSGLPL |
| D8-A7 | SLDQEWAWVQCEVYGRGCLS |
| D8-F1- | WLEHEWAQIQCELYGRGCTY |
| | |

| **Midi C-C loop:** | |
|---|---|
| Clone | Sequence |
| D8-F10 | GLEQGCPWVGLEVQCRGCPS |
| F8-B12- | DLRVLCELFGGAYVLGYCSE |
| F8-A9 | PLWGLCELFGGASLFGYCSS |

| | |
|---|---|
| **Based on analysis of entire panning data, amino acid preferences at each position were calculated to define these "idealized" peptides. * Peptides synthesized and currently being purified - Peptides planned | |

### F. Multivalent Ligands

This invention provides ligands which preferentially bind different sites on IR and IGF-1R. The amino acid motifs which bind IR at one site (Site 1, Figure 13) are A6, B6, revB6, and F2. A second in site (Site 2, Figure 13) binds F8 and D8. Accordingly, multimeric ligands may be prepared according to the invention by covalently linking amino acid sequences. Depending on the purpose intended for the multivalent ligand, amino acid sequences which bind the same or different sites may be combined to form a single molecule. Where the multivalent ligand is constructed to bind to the same corresponding site on different receptors, or different subunits of a receptor, the amino acid sequences of the ligand for binding to the receptors may be the same or different, provided that if different amino acid sequences are used, they both bind to the same site.

Multivalent ligands may be prepared by either expressing amino acid sequences which bind to the individual sites separately and then covalently linking them together, or by expressing the multivalent ligand as a single amino acid sequence which comprises within it the combination of specific amino acid sequences for binding.

Various combinations of amino acid sequences may be combined to produce multivalent ligands having specific desirable properties. Thus, agonists may be combined with agonists, antagonists combined with antagonists, and agonists combined with antagonists. Combining amino acid sequences which bind to the same site to form a multivalent ligand may be useful to produce molecules which are capable of cross-linking together multiple receptor units. Multivalent ligands may also be constructed to combine amino acid sequences which bind to different sites (Figure 15).

In view of the discovery disclosed herein of monomers having agonist properties at IR or IGF-1R, preparation of multivalent ligands may be useful to prepare ligands having more desirable pharmacokinetic properties due to the presence of multiple bind sites on a single molecule. In addition, combining amino acid sequences which bind to different sites with different affinities provides a means for modulating the overall potency and affinity of the ligand for IR or IGF-1R.

### 1. Construction of Hybrids

In one embodiment, hybrids of at least two peptides may be produced as recombinant fusion polypeptides which are expressed in any suitable expression system. The polypeptides may bind the receptor as either fusion constructs containing amino acid sequences besides the ligand binding sequences or as cleaved proteins from which signal sequences or other sequences unrelated to ligand binding are removed. Sequences for facilitating purification of the fusion protein may also be expressed as part of the construct. Such sequences optionally may be subsequently removed to produce the mature binding ligand. Recombinant expression also provides means for producing large quantities of ligand. In addition, recombinant expression may be used to express different combinations of amino acid sequences and to vary the orientation of their combination, i.e., amino to carboxyl terminal orientation.

Whether produced by recombinant gene expression or by conventional chemical linkage technology, the various amino acid sequences may be coupled through linkers of various lengths. Where linked sequences are expressed recombinantly, and based on an average amino acid length of about 4 angstroms, the linkers for connecting the two amino acid sequences would typically range from about 3 to about 12 amino acids corresponding to from about 12 to about 48 A. Accordingly, the preferred distance between binding sequences is from about 2 to about 50 A. More preferred is 4 to about 40. The degree of flexibility of the linker between the amino acid sequences may be modulated by the choice of amino acids used to construct the linker. The combination of glycine and serine is useful for producing a flexible, relatively unrestrictive linker. A more rigid linker may be constructed by using amino acids with more complex side chains within the linkage sequence.

In a preferred embodiment shown below (Figure 16)
MBP-FLAG-PEPTIDE-(G,S)n-PEPTIDE-E-TAG
a fusion construct producing a dipeptide comprises a maltose binding protein amino acid sequence (MBP) or similar sequence useful for enabling the affinity chromatography purification of the expressed peptide sequences. This purification facilitating sequence may then be attached to a flag sequence to provide a cleavage site to remove the initial sequence. The peptide dimer then follows which includes the intervening linker and a tag sequence may be included at the carboxyl terminal portion to facilitate identification/purification of the expression of peptide. In the representative construct illustrated above, G and S are glycine and serine residues, which make up the linker sequence.

In addition to producing the dimer peptides by recombinant protein expression, dimers may also be produced by peptide synthesis whereby a synthetic technique such as Merrifield synthesis (Merrifield, 1997), may be used to construct the entire peptide.

Other methods of constructing dimers include introducing a linker molecule which activates the terminal end of a peptide so that it can covalently bind to a second peptide. Examples of such linkers include diaminoproprionic acid activated with an oxyamino function. A preferred linker is a dialdehyde having the formula

O=CH-(CH₂)ₙ-CH=O,

Wherein n is 2 to 6, but is preferably 6 to produce a linker of about 25 to 30 angstroms in length. Linkers may be used to link dimers either to the carboxyl terminal or the amino terminal.

### 2. Characterization Of Specific Dimers

Specific dimers which bind with high affinity to Site 1, Site 2, or both Site 1 and Site 2 of the insulin receptor are shown in Table 1. Although agonist activity has been observed for the Site 1-Site 1 dimers, the Site 1-Site 2 dimers may also possess desirable properties.

**TABLE 1**

| **Fusion** | **Seq.** | **Action** | **Site** | **Fusion Concentration** | **MW (kDa)** | **K_{d}(HIR)** |
|---|---|---|---|---|---|---|
| 426 | D8 | N | 2 | 0.76 | 52.2 | 1.4x10⁻⁶ |
| 429 | D8-6aa-D8 | N-N | 2-2 | 3.2 | 55.3 | 1.3 x 10⁻⁶ |
| 430 | H2C-6aa-RB6 | A- | 1-1 | 0.17 | 54.5 | 2.1 x 10⁻⁶ |
| 431 | H2C-6aa-F8 | A-N | 1-2 | 3.3 | 54.8 | 4.7 x 10⁻⁸ |
| 432 | H2C-12aa-F8 | A-N | 1-2 | 2.9 | 55.5 | 3.5 x 10⁻⁸ |
| 433 | H2C-9aa-F8 | A-N | 1-2 | 2.8 | 55.2 | 2.1 x 10⁻⁸ |
| 434 | G3-12aa-G3 | N-N | 1-1 | 0.01 | 56 | 3.2 x 10⁻⁶ |
| 436 | H2C-9aa-H2C | A | 1-1 | 1.1 | 54.2 | 4.1 x 10⁻⁷ |
| 437 | H2C | N-N | 1 | 0.3 | 51.5 | 8.3x10⁻⁶ |
| 427 | G3-6aa-G3 | N-N | 1-1 | 0.02 | 55.3 | 3.3 x 10⁻⁶ |
| 435 | H2C-3-H2C-3-H2C | A-A-A | 1-1-1 | 2.1 | 55.5 | 2:0 x 10⁻⁶ |
| 439 | H2C-6aa-H2C | A-A | 1-1 | 1.4 | 53.9 | 5.5 x 10⁻⁷ |
| 449 | H2C-12aa-H2C | | 1-1 | 1.5 | 51.8 | 6.2 x 10⁻⁷ |
| 452 | G3 | | 1 | 0.15 | 48.8 | 7.8 x 10⁻⁷ |
| 463 | H2C-3aa-H2C | A-A | 1-1 | 1.8 | 50.1 | 9.6 x 10⁻⁷ |
| 464 | LF-H2C | | 1 | 0.045 | 48.4 | 3.9 x 10⁻⁸ |
| 446 | LF-F8 | | 2 | 1.9 | 49.1 | 7.7x10⁻⁷ |
| 459 | SF-RB6 | | | 0.069 | 48.1 | 7.7 x 10⁻⁸ |
| MBP* | lacZ | | | 5.1 | 50 | > 1 x 10⁻⁵ |

| | | | | | | |
|---|---|---|---|---|---|---|
| *MBP (negative control for the fusions) is fused to a small fragment of beta-galactosidase (lacZ). N = Antagonist A = Agonist **LF = Long FLAG** epitope **(DYKDDDDK)** **SF = Short FLAG** epitope **(DYKD)** | | | | | | |

| Additional binding data for the fusion peptides are shown below: | | |
|---|---|---|
| **Fusion** | **Highest conc. tested (µM)** | **Kd (HIR) µM** |
| 431- | 0.2 | 0.033 |
| 431+ | 0.2 | 0.0074 |
| 432- | 0.2 | 0.02 |
| 432+ | 0.2 | 0.0038 |
| 433- | 0.2 | 0.03 |
| 433+ | 0.2 | 0.004 |

The concentrations of these fusions vary depending on the expression quality. There are 2 sets of each fusion: uncleaved (-) and cleaved with factor Xa (+). The fusion proteins are in Tris buffer (20 mM Tris, 200 mM NaCl, 1 mM EDTA, 50 mM maltose, pH 7.5) and the cleaved fusions (+) are in the same Tris buffer (500 µl) + 12 µg Factor Xa. (Source of Factor Xa: New England Biolabs).

Other combinations of peptides are within the scope of this invention and may be determined as demonstrated in the examples described herein.

Regarding preparation of a Site 1 agonist comprising two D117 (H2C) peptides, a linker of only 3 amino acids (12 A) provided a ligand of greater affinity for Site 1 of IR than a corresponding ligand prepared with a 9 amino acid (36 A) linking region. Figure 17.

Notably, several fusion peptides show IR agonist activity as determined by an IR autophosphorylation assay (see Example 20). Figure 74. In particular, fusion peptides 439, 436, 449, and 463 show significant IR agonist activity (Figure 74).

### G. Peptide Synthetic Techniques

Many conventional techniques in molecular biology, protein biochemistry, and immunology may be used to produce the amino acid sequences for use with this invention.

### 1. Recombinant Synthesis

To obtain recombinant peptides, the corresponding DNA sequences may be cloned into any suitable vectors for expression in intact host cells or in cell-free translation systems by methods well known in the art (see Sambrook *et al.,* 1989). The particular choice of the vector, host, or translation system is not critical to the practice of the invention.

Cloning vectors for the expression of recombinant peptides include, but are not limited to, pUC, pBluescript (Stratagene, La Jolla, CA), pET (Novagen, Inc., Madison, WI), pMAL (New England Biolabs, Beverly, MA), or pREP (Invitrogen Corp., San Diego, CA) vectors. Vectors can contain one or more replication and inheritance systems for cloning or expression, one or more markers for selection in the host (e.g. antibiotic resistance), and one or more expression cassettes. The inserted coding sequences can be synthesized by standard methods, isolated from natural sources, or prepared as hybrids, etc. Ligation of the coding sequences to transcriptional regulatory elements and/or to other amino acid coding sequences can be carried out using established methods. DNA sequences can be optimized, if desired, for more efficient expression in a given host organism. For example, codons can be altered to conform to the preferred codon usage in a given host cell or cell-free translation system using techniques routinely practiced in the art.

Suitable cell-free systems for expressing recombinant peptides include, for example, rabbit reticulocyte lysate, wheat germ extract, canine pancreatic microsomal membranes, *Escherichia coli* (*E. coli*) S30 extract, and coupled transcription/translation systems (Promega Corp., Madison, WI). Such systems allow expression of recombinant polypeptides upon the addition of cloning vectors, DNA fragments, or RNA sequences containing coding regions and appropriate promoter elements.

Host cells for cloning vectors include bacterial, archebacterial, fungal, plant, insect and animal cells, especially mammalian cells. Of particular interest are *E. coli, Bacillus subtilis, Staphylococcus aureus, Saccharomyces cerevisiae, Schizosaccharomyces pombe, Neurospora crassa,* SF9, C129, 293, NIH 3T3, CHO, COS, and HeLa cells. These cells can be transformed, transfected, or transduced, as appropriate, by any suitable method including electroporation, CaCl₂-, LiCl-, LiAc/PEG-, spheroplasting-, Ca-Phosphate, DEAE-dextran, liposome-mediated DNA uptake, injection, microinjection, microprojectile bombardment, or other established methods.

For some purposes, it may be preferable to produce peptides in a recombinant system in which they carry additional sequence tags to facilitate purification. Non-limiting examples of tags include c-myc, haemagglutinin (HA), polyhistidine (6X-HIS), GLU-GLU, and DYKDDDDK (FLAG®) epitope tags. Epitope tags can be added to peptides by a number of established methods. DNA sequences of epitope tags can be inserted into peptide coding sequences as oligonucleotides or through primers used in PCR amplification. As an alternative, peptide coding sequences can be cloned into specific vectors that create fusions with epitope tags; for example, pRSET vectors (Invitrogen Corp., San Diego, CA). The expressed, tagged peptides can then be purified from a crude lysate of the cell-free translation system or host cell by chromatography on an appropriate solid-phase matrix.

Methods for directly purifying peptides from natural sources such as cellular or extracellular lysates are well known in the art (see Harris and Angal, 1989). Such methods include, without limitation, sodium dodecylsulfate-polyacrylamide gel electrophoresis (SDS-PAGE), preparative disc-gel electrophoresis, isoelectric focusing, high-performance liquid chromatography (HPLC), reversed-phase HPLC, gel filtration, ion exchange and partition chromatography, countercurrent distribution, and combinations thereof. Naturally occurring peptides can be purified from many possible sources, for example, plasma, body tissues, or body fluid lysates derived from human or animal, including mammalian, bird, fish, and insect sources.

Antibody-based methods may also be used to purify naturally occurring or recombinantly produced peptides. Antibodies that recognize these peptides or fragments derived therefrom can be produced and isolated. The peptide can then be purified from a crude lysate by chromatography on an antibody-conjugated solid-phase matrix (see Harlow and Lane, 1998).

### 2. Chemical Synthesis Of Peptides

Alternately, peptides may be chemically synthesized by commercially available automated procedures, including, without limitation, exclusive solid phase synthesis, partial solid phase methods, fragment condensation or classical solution synthesis. The polypeptides are preferably prepared by solid-phase peptide synthesis; for example, as described by Merrifield (1965; 1997). In addition, recombinant and synthetic methods of polypeptide production can be combined to produce semi-synthetic polypeptides.

### H. Screening Assays

In another embodiment of this invention, screening assays to identify pharmacologically active ligands at IR and/or IGF-1R are provided. The screening assays provided in accordance with this invention are based on those disclosed in International application WO 96/04557 which is incorporated herein in its entirety. Briefly, WO 96/04557 discloses the use of reporter peptides which bind to active sites on targets and possess agonist or antagonist activity at the target. These reporters are identified from recombinant libraries and are either peptides with random amino acid sequences or variable antibody regions with at least one CDR region which has been randomized (rVab). The reporter peptides may be expressed in cell recombinant expression systems, such as for example in *E. coli,* or by phage display. See WO 96/04557 and Kay *et al.* (1996), both of which are incorporated herein by reference. The reporters identified from the libraries may then be used in accordance with this invention either as therapeutics themselves, or in competition binding assays to screen for other molecules, preferably small, active molecules, which possess similar properties to the reporters and may be developed as drug candidates to provide agonist or antagonist activity. Preferably, these small organic molecules are orally active.

The basic format of an *in vitro* competitive receptor binding assay as the basis of a heterogeneous screen for small organic molecular replacements for insulin may be as follows: occupation of the active site of IR is quantified by time-resolved fluorometric detection (TRFD) with streptavidin-labeled europium (saEu) complexed to biotinylated peptides (bP). In this assay, saEu forms a ternary complex with bP and IR (i.e., IR:bP:saEu complex). The TRFD assay format is well established, sensitive, and quantitative (Tompkins *et al.,* 1993). The assay can use a single-chain antibody or a biotinylated peptide. Furthermore, both assay formats faithfully report the competition of the biotinylated ligands binding to the active site of IR by insulin.

In these assays, soluble IR is coated on the surface of microtiter wells, blocked by a solution of 0.5% BSA and 2% non-fat milk in PBS, and then incubated with biotinylated peptide or rVab. Unbound bP is then washed away and saEu is added to complex with receptor-bound bP. Upon addition of the acidic enhancement solution, the bound europium is released as free Eu³⁺ which rapidly forms a highly fluorescent and stable complex with components of the enhancement solution. The IR:bP bound saEu is then converted into its highly fluorescent state and detected by a detector such as Wallac Victor II (EG&G Wallac, Inc.)

The designing of mimetics to a known pharmaceutically active compound is a known approach to the development of pharmaceuticals based on a "lead" compound. This might be desirable where the active compound is difficult or expensive to synthesize or where it is unsuitable for a particular method of administration, e.g. peptides are generally unsuitable active agents for oral compositions as they tend to be quickly degraded by proteases in the alimentary canal. Mimetic design, synthesis and testing is generally used to avoid randomly screening large number of molecules for a target property.

There are several steps commonly taken in the design of a mimetic from a compound having a given target property. First, the particular parts of the compound that are critical and/or important in determining the target property are determined. In the case of a peptide, this can be done by systematically varying the amino acid residues in the peptide (e.g. by substituting each residue in turn). These parts or residues constituting the active region of the compound are known as its "pharmacophore".

Once the pharmacophore has been found, its structure is modeled according to its physical properties (e.g. stereochemistry, bonding, size and/or charge), using data from a range of sources (e.g. spectroscopic techniques, X-ray diffraction data and NMR). Computational analysis, similarity-mapping (which models the charge and/or volume of a pharmacophore, rather than the bonding between atoms), and other techniques can be used in this modeling process.

In a variant of this approach, the three dimensional structure of the ligand and its binding partner are modeled. This can be especially useful where the ligand and/or binding partner change conformation on binding, allowing the model to take account of this in the design of the mimetic.

A template molecule is then selected onto which chemical groups which mimic the pharmacophore can be grafted. The template molecule and the chemical groups grafted on to it can conveniently be selected so that the mimetic is easy to synthesize, is likely to be pharmacologically acceptable, does not degrade *in vivo,* and retains the biological activity of the lead compound. The mimetics found are then screened to ascertain the extent they exhibit the target property, or to what extent they inhibit it. Further optimization or modification can then be carried out to arrive at one or more final mimetics for *in vivo* or clinical testing.

This invention provides specific IR and IGF-1R amino acid sequences which function as either agonists or antagonists at IR and/or IGF-1R. Examples of phage display libraries suitable for use in this invention include one such library containing randomized 40 amino acid peptides (RAPIDLIB™, Figure 16), another library containing rVab derived from human genomic antibody DNA (GRABLIB™, Figure 30). Details of the construction and analyses of these libraries, as well as the basic procedures for biopanning and selection of binders, have been described elsewhere (WO 96/04557; Mandecki *et al*., 1997; Ravera *et al.,* 1998; Scott and Smith, 1990); Grihalde *et al.,* 1995; Chen *et al.,* 1996; Kay *et al*., 1993, Carcamo *et al*., 1998, all of which are incorporated herein by reference). Another phage display library suitable for use with this invention is available commercially from New England Biolabs (Ph.D. C7C Disulfide Constrained Peptide Library). Additional sequences may be obtained in accordance with the procedures described herein.

### 1. Use of the Peptides Provided by this Invention

The IR and IGF-1R agonist and antagonist peptides provided by this invention are useful as potential therapeutics in pharmaceutical compositions, lead compounds for identifying other more potent or selective therapeutics, assay reagents for identifying other useful ligands by, for example, competition screening assays, and as research tools for further analysis of IR and IGF-1R. In particular, the peptide sequences provided by this invention can be used to design secondary peptide libraries, which include members that bind to Site 1 and/or Site 2 of IR or IGF-1R. Such libraries can be used to identify sequence variants that increase or modulate the binding and/or activity of the original peptide at IR or IGF-1R.

IR agonist amino acid sequences provided by this invention are useful as insulin analogs and may therefore be developed as treatments for diabetes or other diseases associated with a decreased response or production of insulin. For use as an insulin supplement or replacement, preferred amino acid sequence are: FHENFYDWFVRQVSK (D117, H2C),
DYKDFYDAIQLVRSARAGGTRDKK (D118, 20E2),
KDRAFYNGLRDLVGAVYGAWDKK (D119, 20C11),
DYKDLCQSWGVRIGWLAGLCPKK (D116, JBA5),
DYKDVTFTSAVFHENFYDWFVRQVSKK (D113, H2), and
GRVDWLQRNANFYDWFVAELG (S175). More preferred IR agonists are: FHENFYDWFVRQVSK (D117, H2C) and GRVDWLQRNANFYDWFVAELG (S175). Most preferred is GRVDWLQRNANFYDWFVAELG (S175). Preferred dimer sequences are represented by S170, S171, S172, S232, S300 sequences (see Table 15).

IGF-1 R antagonist amino acid sequences provided by this invention are useful as treatments for cancers, including, but not limited to, breast and prostate cancers. Human and breast cancers are responsible for over 40,000 deaths per year, as present treatments such as surgery, chemotherapy, radiation therapy, and immunotherapy show limited success. The IGF-1R antagonist amino acid sequences disclosed herein are also useful for the treatment or prevention of diabetic retinopathy. Recent reports have shown that a previously identified IGF-1 R antagonist can suppress retinal neovascularization, which causes diabetic retinopathy (Smith *et al.,* 1999).

IGF-1 R agonist amino acid sequences provided by this invention are useful for development as treatments for neurological disorders, including stroke and diabetic neuropathy. Reports of several different groups implicate IGF-1R in the reduction of global brain ischemia, and support the use of IGF-1 for the treatment of diabetic neuropathy (reviewed in Auer *et al*., 1998; Apfel, 1999).

### J. Methods of Administration

The amino acid sequences of this invention may be administered as pharmaceutical compositions comprising standard carriers known in the art for delivering proteins and peptides and by gene therapy. Due to the labile nature of the amino acid sequences parenteral administration is preferred. Preferred modes of administration include aerosols for nasal or bronchial absorption; suspensions for intravenous, intramuscular, intrasternal or subcutaneous, injection; and compounds for oral administration. Other modes of administration and examples of suitable formulative components for use with this embodiment are discussed below. Other modes of administration include intranasal, intrathecal, intracutaneous, percutaneous, enteral, and sublingual. For injectable administration, the composition is in sterile solution or suspension or may be emulsified in pharmaceutically- and physiologically-acceptable aqueous or oleaginous vehicles, which may contain preservatives, stabilizers, and material for rendering the solution or suspension isotonic with body fluids (i.e. blood) of the recipient. Excipients suitable for use are water, phosphate buffered saline, pH 7.4, 0.15 M aqueous sodium chloride solution, dextrose, glycerol, dilute ethanol, and the like, and mixtures thereof. Illustrative stabilizers are polyethylene glycol, proteins, saccharides, amino acids, inorganic acids, and organic acids, which may be used either on their own or as admixtures. The amounts or quantities, as well as routes of administration, used are determined on an individual basis, and correspond to the amounts used in similar types of applications or indications known to those of skill in the art.

The constructs as described herein may also be used in gene transfer and gene therapy methods to allow the expression of one or more amino acid sequences of the present invention. Using the amino acid sequences of the present invention for gene therapy may provide an alternative method of treating diabetes which does not rely on the administration or expression of insulin. Expressing insulin for use in gene therapy requires the expression of a precursor product, which must then undergo processing including cleavage and disulfide bond formation to form the active product. The amino acid sequences of this invention, which possess activity, are relatively small, and thus do not require the complex processing steps to become active. Accordingly, these sequences provide a more suitable product for gene therapy.

Gene transfer systems known in the art may be useful in the practice of the invention. Both viral and non-viral methods are suitable. Examples of such transfer systems include, but are not limited to, delivery via liposomes or via viruses, such as adeno-associated or vaccinia virus. Numerous viruses have been used as gene transfer vectors, including papovaviruses (e.g., SV40, adenovirus, vaccinia virus, adeno-associated virus, herpes viruses, including HSV and EBV, and retroviruses of avian, murine, and human origin). As is appreciated by those in the art, most human gene therapy protocols have been based on disabled murine retroviruses. Recombinant retroviral DNA can also be employed with amphotrophic packaging cell lines capable of producing high titer stocks of helper-free recombinant retroviruses (e.g., Cone and Mulligan, 1984).

A recombinant retroviral vector may contain the following parts: an intact 5' LTR from an appropriate retrovirus, such as MMTV, followed by DNA containing the retroviral packaging signal sequence; the insulator element placed between an enhancer and the promoter of a transcription unit containing the gene to be introduced into a specific cell for replacement gene therapy; a selectable gene as described below; and a 3' LTR which contains a deletion in the viral enhancer region, or deletions in both the viral enhancer and promoter regions. The selectable gene may or may not have a 5' promoter that is active in the packaging cell line, as well as in the transfected cell.

The recombinant retroviral vector DNA can be transfected into the amphotrophic packaging cell line Ψ-AM (see Cone and Mulligan, 1984) or other packaging cell lines which are capable of producing high titer stocks of helper-free recombinant retroviruses. After transfection, the packaging cell line is selected for resistance to G418, present at appropriate concentration in the growth medium. Adenoviral vectors (e.g. DNA virus vectors), particularly replication-defective adenovirus vectors, or adeno-associated vectors, have been described in the art (Kochanek *et al.,* 1996; Ascadi *et al.,* 1994; Ali *et* al., 1994).

Nonviral gene transfer methods known in the art include chemical techniques, such as calcium phosphate co-precipitation, direct DNA uptake and receptor-mediated DNA transfer, and mechanical means, such as microinjection and membrane fusion-mediated liposomal transfer. In addition, viral-mediated gene transfer can be combined with direct *in vivo* gene transfer using liposomes, thereby allowing the delivery or the viral vectors to tumor cells, for example, and not to surrounding non-proliferating cells. A description of various liposomes which are stated as being useful for transferring DNA or RNA into cells is present in United States Patents 5,283,185 and 5,795,587. The retroviral vector producer cell line can also be injected directly into specific cell types, e.g., tumors, to provide a continuous source of viral particles, such as has been approved for use in patients afflicted with inoperable brain tumors.

Receptor-mediated gene transfer methods allow targeting of the DNA in the construct directly to particular tissues. This is accomplished by the conjugation of DNA (frequently in the form of a covalently closed supercoiled plasmid) to a protein ligand via polylysine. The appropriate or suitable ligands are selected on the basis of the presence of the corresponding ligand receptors on the cell surface of the target cell or tissue type. These ligand-DNA conjugates can be injected directly into the blood, if desired, and are directed to the target tissue where receptor binding and DNA-protein complex internalization occur. Co-infection with adenovirus to disrupt endosome function can be used to overcome the problem of intracellular destruction of DNA.

An approach that combines biological and physical gene transfer methods utilizes plasmid DNA of any size combined with a polylysine-conjugated antibody specifically reactive with the adenovirus hexon protein. The resulting complex is bound to an adenovirus vector. The trimolecular complex is then used to infect cells. The adenovirus vector allows efficient binding to the cell, internalization, and degradation of the endosome before the coupled DNA can be damaged.

Many types of cells and cell lines (e.g. primary cell lines or established cell lines) and tissues are capable of being stably transfected by or receiving the constructs of the invention. Examples of cells that may be used include, but are not limited to, stem cells, B lymphocytes, T lymphocytes, macrophages, other white blood lymphocytes (e.g. myelocytes, macrophages, monocytes), immune system cells of different developmental stages, erythroid lineage cells, pancreatic cells, lung cells, muscle cells, liver cells, fat cells, neuronal cells, glial cells, other brain cells, transformed cells of various cell lineages corresponding to normal cell counterparts (e.g. K562, HEL, HL60, and MEL cells), and established or otherwise transformed cells lines derived from all of the foregoing. In addition, the constructs of the present invention may be transferred by various means directly into tissues, where they would stably integrate into the cells comprising the tissues. Further, the constructs containing the DNA sequences of the peptides of the invention can be introduced into primary cells at various stages of development, including the embryonic and fetal stages, so as to effect gene therapy at early stages of development.

The described constructs may be administered in the form of a pharmaceutical preparation or composition containing a pharmaceutically acceptable carrier and a physiological excipient, in which preparation the vector may be a viral vector construct, or the like, to target the cells, tissues, or organs of the recipient organism of interest, including human and non-human mammals. The composition may be formed by dispersing the components in a suitable pharmaceutically acceptable liquid or solution such as sterile physiological saline or other injectable aqueous liquids. The amounts of the components to be used in such compositions may be routinely determined by those having skill in the art. The compositions may be administered by parenteral routes of injection, including subcutaneous, intravenous, intramuscular, and intrasternal.

The following non-limiting examples illustrate various aspects and embodiments of the invention and should not be contrived as limiting the scope of the invention.

### VI. EXAMPLES

The following materials were used in the examples described below. Soluble IGF-1R was obtained from R&D Systems (Cat. # 391-GR/CF). Insulin receptor was prepared according to Bass *et al.,* 1996. The insulin is either from Sigma (Cat. # I-0259) or Boehringer. The IGF-1 is from PeproTech (Cat. # 100-11). All synthetic peptides were synthesized by Novo Nordisk, AnaSpec, Inc. (San Jose, CA), PeptioGenics (Livermore, CA), or Research Genetics (Huntsville, AL) at >80% purity. The Maxisorb Plates are from Nunc via Fisher (Cat. # 12565347). The HRP/Anti-M13 Conjugate is from Pharmacia (Cat. # 27-9421-01). The ABTS solution is from BioF/X (Cat. # ABTS-0100-04).

### Example 1

### A. Construction of Phage Library for Identifying IGF-1R and IR Binding Ligands

The schematic for the peptide library "RAPIDLIB™" on filamentous phage is shown in Figure 16. DNA fragments coding for peptides containing 40 random amino acids were generated in the following manner. A 145 base oligonucleotide was synthesized to contain the sequence (NNK)₄₀, where N = A, C, T, or G, and K = G or T. This oligonucleotide was used as the template in a PCR amplification along with two shorter oligonucleotide primers, both of which were biotinylated at their 5' ends. The resulting 190 bp product was purified and concentrated with QIAquick spin columns (QIAGEN, Inc. Valencia, CA), then digested with *Sfi*l and *Not*l. Streptavidin-agarose (GibcoBRL Life Technologies, Inc., Rockville, MD) was added to the digestion mixture to remove the cleaved ends of the PCR product as well as any uncut DNA. The resulting 150 bp fragment was again purified over QIAquick spin columns. The phagemid pCANTAB5E (Amersham Pharmacia Biotech, Inc., Piscataway, NJ) was digested with *Sfi*l and *Not*l*,* followed by phosphatase treatment. The digested DNA was purified using a 1% agarose gel followed by QIAEX II (QIAGEN). The vector and insert were ligated overnight at 15°C. The ligation product was purified using QIAquick spin columns (QIAGEN). Electroporations were performed at 1500 v in an electroporation cuvette (0.1 mm gap; 0.5 ml volume) containing 12.5 µg of DNA and 500 µl of TG1 electrocompetent cells (see below). Immediately after the pulse, 12.5 ml of pre-warmed (40°C) 2xYT medium containing 2% glucose (2xYT-G) was added and the transformants were grown at 37°C for 1 h. Cell transformants were pooled, the volume measured, and an aliquot was plated onto 2xYT-G containing 100 g/ml ampicillin (2xYT-AG) plates to determine the total number of transformants.

Sequence analysis of randomly selected clones indicated that 54% of all clones are in-frame (Mandecki *et al.,* 1997). The FLAG sequence (Hopp *et al.,* 1988) was incorporated into the library as an immunoaffinity tag as shown in Figure 16.

Another phage library expressing 20mer peptides, was constructed according to a similar procedure. The diversity of the library is 1.1 x 10¹¹ different clones.

### B. Preparation of Electrocompetent Cells

To prepare electrocompetent cells, an overnight culture of *E. coli* TG1 cells (F'_*traD*36 *lacl*^{q} Δ(*lacZ*)M15 *proAB*) / *supE* Δ(*hsdM-mcrB*)₅ rₖ⁻mₖ⁻ *McrB⁻*) *thi* Δ(/*ae-proAB*) was diluted to an OD₆₀₀ = 0.05-0.1 in 500 ml 2xYT, then grown at 37°C in 4 liter Ehrlenmyer flasks to an OD₆₀₀ = 0.5-0.6. The culture was poured into pre-chilled centrifuge bottles and incubated on ice for 30 min prior to centrifugation at 2000 x g for 30 min (2°C). The supernatant was poured off and the cell pellet was resuspended in a total of 400 ml of ice cold sterile distilled water. The process of centrifugation and resuspension was repeated 2 times. After the last centrifugation, the pellet was resuspended in a total of 25 ml of ice cold water containing 10% glycerol. The cell suspension was transferred to pre-chilled 35 ml centrifuge bottles, and was then pelleted at 2000 x g for 10 min at 4°C. The cells were then suspended in 0.3 ml of the same 10% glycerol solution, aliquotted into smaller tubes, and snap-frozen on dry ice. The aliquots were stored at -80°C.

To amplify the library, the transformants were inoculated into 4 1 of 2xYT-AG medium and allowed to grow until the A₆₀₀ increased approximately 400 times. The cells were pelleted by centrifugation at 3000 x g for 20 min, then resuspended in 40 ml 2xYT-AG to which glycerol was added to a final concentration of 8%. The library was stored at -80°C.

### C. Phage Rescue

This process was carried out using the standard phage preparation protocol with the following changes. Five individual recombinant cell libraries, with a total diversity of 1.6 x 10¹⁰, were combined and grown to OD₆₀₀ = 0.5 in 2xYT-AG at 30°C with shaking (250 rpm). Helper phage (M13K07) was then added (multiplicity of infection (MOI) = 15), and the cells were incubated for 30 min at 37° C without shaking, followed by 30 min at 37°C with shaking (250 rpm). Following infection, cells were pelleted and the supernatant containing the helper phage was discarded. The cell pellet was resuspended in the initial culture volume of 2xYT-A (no glucose) containing 50 mg/ml kanamycin and grown overnight at 30°C with shaking (250 rpm). The cells from the overnight culture were pelleted at 3000 x g for 30 min at 4°C and the supernatant containing the phage was recovered. The solution was adjusted to 4% PEG, 500 mM NaCl and chilled on ice for 1 h. The precipitated phage were pelleted by centrifugation at 10,000 x g for 30 min, then resuspended in phosphate-buffered saline (1/100 of the initial culture volume) and passed through a 0.45 µm filter. The phage were titered by infecting TG1 cells. The phage titer for the 40mer peptide library was 4 x 10¹³ cfu/ml. The phage titer for the 20mer library was 3 x 10⁻³.

To amplify the library, the transformants were inoculated into 4 I of 2xYT-AG medium and allowed to grow until the OD₆₀₀ increased approximately 400 times. The cells were pelleted by centrifugation at 3000 x g for 20 min, then resuspended in 40 ml 2xYT-AG to which glycerol was added to a final concentration of 8%. The library was stored at -80°C.

### Example 2:

### A. Panning IGF-1R

A standard method was used to coat and block all microtiter plates. The soluble IGF-1R ("sIGF-1R") was diluted to 1 mg/ml in 50 mM sodium carbonate buffer, pH 9.5. One hundred microliters of this solution was added to an appropriate number of wells in a 96-well microtiter plate (MaxiSorp plates, Nunc) and incubated overnight at 4°C. Wells were then blocked with MPBS (PBS buffer pH 7.5 containing 2% Carnation® non-fat dry milk) at room temperature (RT) for 1 h.

Eight wells were used for each round of panning. The phage were incubated with MPBS for 30 min at RT, then 100 µl was added to each well. For the first round, the input phage titer was 4 x 10¹³ cfu/ml. For rounds 2 and 3, the input phage titer was approximately 10¹¹ cfu/ml. Phage were allowed to bind for 2 to 3 h at RT. The wells were then quickly washed 13 times with 200 µl/well of MPBS. Bound phage were eluted by incubation with 100 µl/well of 20 mM glycine-HCl, pH 2.2 for 30 s. The resulting solution was then neutralized with Tris-HCl, pH 8.0. Log phase TG1 cells were infected with the eluted phage, then plated onto two 24 cm x 24 cm plates containing 2xYT-AG. The plates were incubated at 30°C overnight. The next morning, cells were removed by scraping and stored in 10% glycerol at -80°C. For subsequent rounds of affinity enrichment, cells from these frozen stocks were grown and phage were prepared as described above. A minimum of 72 clones were picked at random from the second, third, and fourth rounds of panning and screened for binding activity. DNA sequencing of the clones revealed the abundance of sequences as summarized in Figure 18. Some of the clones (Figure 19) were frameshifted, that is, the relevant peptide sequence was encoded not in the FLAG frame, but in either frame + 1 or - 1.

### B. ELISA Analyses of Phage

For phage pools, cells from frozen stocks were grown and phage were prepared as described above. For analysis of individual clones, colonies were picked and phage prepared as described above. Subsequent steps are the same for pooled and clonal phage. Microtiter wells were coated and blocked as described above. Wells were coated with either IGF-1 R or a control IgG mAb. Phage resuspended in MPBS were added to duplicate wells (100 µl/well) and incubated at RT for 1 h. The phage solution was then removed, and the wells were washed 3 times with PBS at RT. Anti-M13 antibody conjugated to horseradish peroxidase (Pharmacia) was diluted 1:3000 in MPBS and added to each well (100 µl/well). Incubation was for 1 h at RT, followed by PBS washes as described. Color was developed by addition of ABTS solution (100 µl/well; Boehringer). Color development was stopped by adjusting each well to 0.5% SDS. Plates were analyzed at 405 nm using a SpectraMax 340 plate reader (Molecular Devices Corp., Sunnyvale CA) and SoftMax Pro software. Data points were averaged after subtraction of appropriate blanks. A clone was considered "positive" if the A₄₀₅ of the well was ≥ 2-fold over background.

For IC₅₀ determinations in a competitive ELISA, microtiter plates were coated with IGF-1R and blocked as described. Phage were prepared as described. Prior to addition of phage to plates, the peptide or recombinant variable antibody or fragment ("rVab"), or an appropriate control, was diluted in PBS and added to duplicate wells (100 µl/well). After incubation for 1 h at RT, the prepared phage were added to each well (100 µl/well) without removing the peptide or rVab solution. After incubation for 1 h at RT, the wells were washed and the color developed as described above.

The clones were next analyzed for binding to the receptor's active site (Figures 20A and 20B). Competitions of phage binding were done with the cognate ligand (i.e., IGF-1). All four phage clones tested, B6, F6, C6 and E5, bound to same site as IGF-1 since the binding of the clones to the immobilized IGF-1R could be inhibited with IGF-1.

To determine the rank order for phage peptides, the human IGF-1R (25 g/ml) was immobilized onto a CM-5 (BIAcore) sensor chip using amino coupling chemistry and the manufacturer's recommended protocol. The final surface density was 1000 RU. A monoclonal antibody was immobilized onto another flow cell as a control surface. Phage were directly injected (30-100 µl) with a buffer flow rate of 1 µl/min. Background binding to the control surface was subtracted prior to further analysis.

### C. Phage Sequence Analysis

Sequence analysis of several clones shows that there are two distinct populations, designated as Class 1 (Formula motif 2) and motif 2) and class II (Formula motif 1; Figure 21). Several of these have been chemically synthesized for subsequent testing. Class I peptides contain the consensus sequence D-x-F-Y-x-x-L-s-x-L, and are shown to be antagonistic in cell-based assays (Figure 22). Class II peptides contain the consensus N-F-Y-D-W-F-V, and are shown to be agonistic in cell-based assays (Figure 23). Neither of these consensus sequences have any significant linear sequence similarities greater than 2 or 3 amino acids with mature IGF-1.

### Example 3: Assays with Synthetic Peptides

Four synthetic peptides, 5.1, 5.2, 5.3 and 5.4 (Figure 21) were made to study the properties of the artificial peptide ligands from phage display.

Synthetic peptides were obtained from a commercial supplier (Anaspec). The peptides were supplied greater than 90% pure by HPLC. The molecular weights of the peptides as determined by mass spectroscopy agreed with the expected values.

IGF-1R (100 µg/ml) was immobilized onto one flow-cell of a CM-5 sensor chip (Biosensor) using amine coupling chemistry and the manufacturer's recommended protocol. An unrelated IgG was immobilized in the same manner to another flow cell of the same chip as a control surface. Increasing concentrations of synthetic peptide were injected over both surfaces, and the binding responses were allowed to come to equilibrium. After subtraction of background binding from the control surface, the results were used to derive an equilibrium dissociation constant using Scatchard analysis (Figure 24A).

In another experiment, IGF-1R (100 µg/ml) was immobilized onto a CM-5 sensor chip as described above, and an unrelated IgG was immobilized in the same manner to another flow cell of the same chip. IGF-1 alone, peptide 5.1 alone (corresponding to the B6 phage clone), or different mixes of the two, were injected over the derivatized chip surfaces. The results shown in Figure 24B indicate that the 5.1 peptide inhibits the binding of IGF-1, and the inhibition is increased by increasing amounts of the peptide. The results support the idea of an overlap of the peptide 5.1 binding site and the IGF-1 binding site on IGF-1R.

### Example 4: Construction of Secondary Phage Libraries

Two phage libraries were designed on the basis of the sequences of the Class II binders known to possess agonistic properties in cell-based assays. The goal was to bring the affinity into a range that would allow the peptide to be used in a receptor binding assay and tested in a cell based assay for activity. Among several available mutagenesis methods, we chose one based on gene synthesis and phage display. In this method a library of doped oligonucleotides carrying several mutations in any single DNA molecule is used to obtain a pool of mutant genes, the expression products of which are phage displayed.

### A. Phage Library A6L

The approach used was the doped synthesis of the oligonucleotide encoding the sequence of the peptide. The sequence encoding the peptide and the sequence of the synthetic oligonucleotide made are shown in Figures 25A-25B. The amino acid residues belonging to the consensus sequence were kept constant and were not mutated. The ratio of nucleosides in each condensation was chosen to provide an average of 6 nucleotide sequence changes at the DNA level and 4-5 mutations at the amino acid level over the length of the peptides. The regions corresponding to the FLAG, *Sfi*l and *Not*l sites were not mutated.

The DNA sequence encoding the A6 peptide was optimized for E. *coli* codon usage by replacing a total of 24 nucleotides as shown in Figure 25A. The TAG stop codons (suppressed in the TG1 E. *coli* strain used) were replaced with CAG (glutamine). Then, the oligonucleotide sequence was designed to include doped nucleosides at positions corresponding to the coding region for the A6 peptide, except for the consensus NFYDWFV (Figure 25A). This synthetic oligonucleotide (Figure 25B) was then used as a template in a PCR reaction. The product of this PCR reaction was then purified, cut with *Sfi*l and *Not*l restriction enzymes and cloned into the pCANTAB5E vector as described for the original peptide library. Over 10¹⁰ different clones were obtained in the final library.

### B. Phage Library A6S

While the consensus sequence NFYDWFV was kept constant in the A6S library, the flanking regions were randomized in the A6S library as shown in Figure 26A. The codons in the random region were of the NNK type to reduce the frequency of stop codons (N = A, C, G, or T; K = G or T). The sequence of the synthetic oligonucleotide made is given in Figure 26B. This synthetic oligonucleotide was then used as a template in a PCR reaction. The product of this PCR reaction was then purified, cut with Sfi I and *Not* l restriction enzymes and cloned into the pCANTAB5E vector as described for the original peptide library. Over 10⁹ different clones were obtained in the final library.

### C. Secondary Phage Library Based on Clone H5

Peptide H5 (LCQRLGVGWPGWLSGWCA) was identified in an independent experiment as a binder to the rat growth hormone binding protein. This peptide and four other H5-like peptides, including 2C3-60 (Figure 27), were found in cell culture experiments to possess agonistic activity toward IGF-1R⁺ cells, but not against IGF-1R⁻ cells. Further, subsequent *in vitro* experiments showed that the H5-like peptides are not competed by IGF. This suggests that these peptides recognize a second allosteric site on IGF-1R. BIAcore analysis showed that binding of the 2C3-60 peptide to IGF-1R is ~20 µM. Subsequently, a phage library of mutants of the H5 sequence was constructed and used for panning against IGF-1R.

Gene synthesis to introduce mutations and phage display were used to construct an H5 secondary library. In this method a library of doped oligonucleotides carrying several mutations in any single DNA molecule is used to obtain a pool of mutant genes which a phage displayed. This method allowed the encoding of both the original H5 peptide as control as well as versions containing high numbers of mutations per peptide in a very large library (> 10¹⁰).

Therefore, the H5 secondary mutant library was designed to contain an average of four amino acid changes (mutations) per peptide. The number of possible mutant H5 peptide sequences having four mutations is 1.0 x 10¹⁰ and is equivalent to the actual size of the secondary phage library. Sequence analysis indicates that of these peptides 30% have 3-4, 33% have 1-2 and 32 % have 5-6 mutations. There also was a small percent with 7-8 mutations and 5% clones without any mutation.

An oligonucleotide based on the DNA sequence encoding the H5 peptide was synthesized. The sequence of the oligonucleotide is:

5'-CTACAAAGACCTGTGTTAGAGTTTGGGGGTTACGTATCCGGGTTGGT
TGGCGGGGTGGTGTGCGGCGGCCGCAGTGTGA-3'

The underlined base positions were synthesized as mixtures of four nucleosides as follows:
A = 90% A; 3.3% C; 3.3% G; and 3.3% T
C = 3.3% C; 90% C; 3.3% G; and 3.3% T
G = 3.3% C; 3.3% C; 90% G; and 3.3% T
T = 3.3% C; 3.3% C; 3.3% G; and 90% T
Using this oligonucleotide as a template, the H5 secondary library was constructed, electroporated, amplified, and rescued essentially as described for the original peptide library. The final diversity of this secondary library was ~10¹⁰.

### D. Characterization of Libraries

Forty-eight randomly picked clones from each of the secondary libraries (Round 0, before panning) were rescued and the phage was assayed in an ELISA for binding to the anti-E-tag mAb, as well as for binding to IGF-1 R (E-tag is used as an indicator of expression of displayed peptides on phage surfaces). The results showed that although most of the clones in the two libraries (70%) display a peptide (i.e., are positive for E-tag), only about 6% of the clones from the A6 long (A6L) library bind to IGF-1 R by phage ELISA, and none of the 24 clones tested from the A6 short (A6S) library bind to IGF-1R. This indicates that the most common outcome of random mutagenesis is the loss of IGF-1R affinity. Nevertheless, some mutants do retain their binding properties and some have improved affinities (see below).

### E. Panning with the Secondary Libraries

The two secondary libraries of Example 4 were used in a panning experiment against IGF-1 R. Approximately 50 clones from each four rounds of panning were analyzed in a phage ELISA to identify the clones that bind to the receptor. The positive clones were subjected to DNA sequencing and protein sequence comparison. Figure 28 provides a listing of different sequences obtained from panning with the A6S library. The results show that a variety of phage peptide sequences can bind to IGF-1 R, while the consensus sequence NFYDWFV is preserved in the majority of instances.

The H5 secondary phage library was panned against IGF-1R to find H5-like peptides with higher affinities for IGF-1R

The H5 Library has a diversity of ~2.6 x 10¹⁰ clones with a phage titer of 1.0 x 10¹³ phage ml⁻¹. A total of three rounds of panning were performed. Table 2 summarizes the results from the three rounds of panning and shows the ELISA results for the individual clones selected from each round, the number of clones examined in each round of panning, as well as the number and percentage of E-Tag⁺ clones and IGF-1R⁺ clones.

### F.

**TABLE 2: Results of panning with the H5 secondary phage library.**

| **Round** | **Total** | **E-Tag^{+,a}** | | **IGF-1R^{+,b}** | |
|---|---|---|---|---|---|
| | | **Number** | **%** | **Number** | **% Total** |
| **0** | 32 | 22 | 69 % | 0 | 0 % |
| **1** | 128 | 116 | 91 % | 1 | 1 % |
| **2** | 128 | 108 | 84 % | 2 | 2 % |
| **3** | 160 | 116 | 91 % | 65 | 51 % |

| | | | | | |
|---|---|---|---|---|---|
| ^{a}E-Tag⁺ means ELISA absorbance values >2X background. ^{b}IGF-1R⁺ means ELISA Absorbance >2X background. Background absorbance values are 0.05 to 0.075. | | | | | |

Each of the IGF-1R⁺ clones were sequenced, as were 15 IGF-1R⁻clones with high E-Tag values (Absorbance >1.0). These sequences are shown in Figure 29. There is no discernible difference between binding sequences and the non-binding sequences with the exception that all of the binding sequences hold the Gly at position 6 constant. All sequences, binding and non-binding, hold the TAG stop codon constant at position 3 (the E. *coli* strain used in phage production contains the *supE44* mutation, therefore Gin replaces the TAG and it denoted in Figure 29 by Q). This suggests TAG stop codon is required for phage production and not binding.

### Example 5: Construction of the rVab

### Recombinant Antibody Variable Region library

The design, expression and purification of single-chain antibodies has been reviewed (Rader and Barbas, 1997; Hoogenboom, 1997). Briefly, the variable portion of the heavy chain (V_{H}) is linked to the variable portion of the light chain (V_{L}) by a flexible peptide linker. Random combinations of V_{H} and V_{L} genes can be genetically combined to provide some of the diversity required for a library of recombinant variable region antibodies (rVabs) (Figure 30). In our library, further diversity is provided by full randomization of the 6-12 amino acids comprising the V_{H} CDR3 (indicated as "D" in Figure 30).

A total of 49 human genomic vₕ genes and ten human genomic vₗ genes (Figure 31) were isolated from total human genomic DNA by PCR. The other genetic components of the library (Vₕ, CDR3, jₕ, linker, and jₗ) were derived from synthetic oligonucleotides. Assembly of these components was done using directional cloning as outlined in Figure 32 and Figure 33.

### A. Ligations

The general schematic for the assembly of the rVab library ("GRABLIB^{™")} is provided in Figure 30. Four gene fragments (VH, VHCDR3/JH/LINKER, VL and JL) were ligated together in the proper orientation and cloned into pCANTAB 5E (Pharmacia). Directional cloning was achieved using the *Bsr*DI restriction enzyme (Figure 32). Forty-nine germline VH segments and ten VL segments encoding many of the genes from the human VH and VL repertoire were isolated (Figure 31) using the polymerase chain reaction. VH CDR3 (ranging from 6 to 12 amino acids) /JH/Linker fragments were generated by ligation of four oligonucleotides (WM 2.1, 2.2, 2.3 and 2.4) and cloning the resulting fragment into the plasmid pUC18 previously cut with *Kpn*I and *Hind*III*.* The insert was then amplified using PCR and oligonucleotide primers to introduce a synthetic D-segment of 6 to 12 amino acids having a random sequence and the *Bsr*DI restriction site. The JL gene fragments were assembled as a result of annealing of two synthetic oligonucleotides. The assembled fragments (200 ng) were used as template in a PCR amplification along with two shorter oligonucleotide primers, both of which were biotinylated at their 5' ends. The resulting 800 bp product was purified and concentrated with QIAquick spin columns (QIAGEN), then digested with the *Sfi*l and *Not*l restriction enzymes. Streptavidin-agarose (GibcoBRL) was added to the digestion mixture to remove the cleaved ends of the PCR product as well as any uncut DNA. The resulting 800 bp fragment was purified by passing DNA over QIAquick spin columns.

Phagemid pCANTAB 5E (Pharmacia) was digested with the *Sfi*l and *Not*l restriction enzymes, which was followed by the alkaline phosphatase treatment to dephosphorylate the ends of the restriction fragments generated. The digested DNA was purified by running the digested plasmid DNA on a 1 % agarose gel, followed by the DNA purification using the QIAEX II (QIAGEN) column. The vector and insert DNA were ligated overnight at 16°C. The ligation product was purified using QIAquick spin columns (QIAGEN) and electroporations were performed at 1500 v in a electroporation cuvette (0.1 mm gap; 0.5 ml volume, BTX, Inc.). The amount of DNA in one electroporation was 12.5 µg per 500 µl of TG1 electrocompetent cells. Immediately after the pulse, 12.5 ml of a pre-warmed (40°C) 2xYT medium containing 2% glucose (2xYT-G) was added, and the transformants were grown at 37°C for 1 h. The transformants were pooled, the volume measured, and an aliquot was plated onto the 2xYT-G medium containing 100 µg/ml ampicillin (2xYT-AG) plates to determine the total number of transformants. The number of different transformants and the diversity of the library was 3 x 10¹⁰.

The electrocompetent cell preparation, phage library amplification, library phage rescue, phage preparations and coating of microtiter plates were done as described above for the peptide library.

### B. Panning for IGF-1R Binders with rVab Antibody Library

### 1. Panning Procedure

Panning of the antibody library was done essentially as described for the peptide library, for a total of four rounds. Of the 200 clones tested, approximately 10% bound specifically to sIGF-1 R. Among these specific binders, 40% can be competed by IGF-1 for receptor binding. The clonal analysis and DNA sequencing (Figures 31-39) followed by ELISA and cell-based assays (Figures 40-46) have shown that two clones, 43G7 and M100, are agonistic with ED₅₀ values of approximately 20 nM (a plot for the 43G7 antibody is shown in Figure 41). Two other rVabs, 1G2P and 39F7, have been shown to be antagonistic, with IC₅₀ values of approximately 20 nM (Figure 42).

Microtiter wells were coated with IGF-1R as described above, with eight wells being used for each round of panning. The phage were incubated with MPBS for 30 min at RT, then 100 µl of the phage suspension was added to each well. For the first round, the input phage titer was 8 x 10¹³ cfu/ml. For rounds 2 and 3, the input phage titer was approximately 10¹¹ cfu/ml. Phage were allowed to bind for 2 to 3 h at RT. The wells were then quickly washed 13 times with 200 µl /well of MPBS. Bound phage were eluted by incubation with 100 µl/well of 20 mM glycine-HCl, pH 2.2 for 30 s. The resulting solution was then neutralized with Tris-HCl, pH 8.0. Log phase TG1 cells were infected with the eluted phage, then plated onto two 4 cm x 4 cm plates containing 2XYT-AG. The plates were incubated at 30°C overnight. The next morning, cells were removed by scraping and stored in 10% glycerol at -80°C. For subsequent rounds of affinity enrichment, cells from these frozen stocks were grown and phage were prepared as described above.

### 2. Elisa Analyses Of Phage Pools

To prepare the phage pools, cells from frozen stocks were grown and phage were prepared as described above. Microtiter wells were coated and blocked as described above. The wells were coated with either IGF-1R (R&D Systems, Inc.) or with control BSA. Phage resuspended in MPBS were added to duplicate wells (100 µl/well) and incubated at RT for 1 h. The phage solution was then removed, and the wells were washed 3 times with PBS at RT. Anti-M13 antibody conjugated to horseradish peroxidase (Pharmacia) was diluted 1:3000 in MPBS and added to each well (100 µl/well). Incubation was for 1h at RT, followed by PBS washes as described. Color was developed by addition of ABTS solution (100 µl/well; Boehringer). Color development was stopped by adjusting each well to 0.5% SDS. Plates were analyzed at 405 nm using a SpectraMax 340 plate reader (Molecular Devices) and SoftMax Pro software. Data points were averaged after a subtraction of appropriate blanks. Phage pools was considered "positive" if the A₄₀₅ of the well was > 2-fold over background.

### 3. Competition ELISAs

For IC₅₀ determinations, microtiter plates were coated with IGF-1R and blocked as described. Phage and soluble rVabs were prepared as described above. Prior to addition of phage or soluble rVabs to the plates, IGF-1 solution in PBS (1 µg/ml) was added to duplicate wells (100 µl/well). After incubation for 1 h at RT, the prepared phage were added to each well (100 µl/well) without removing the IGF-1 solution. After incubation for 1 h at RT, the wells were washed and the color was developed as described above.

Six rVab clones bound specifically to IGF-1 R. The sequences of the clones are shown in Figure 34-39.

### 4. Expression And Purification Of Soluble rVabs

*E*. *coli* HB2151 carrying the rVab genes on the pCANTAB5E plasmid (Pharmacia) were grown in 2xYT supplemented with 100 µg/ml ampicillin and 1% glucose at 37° C overnight and then subcultured in the absence of glucose at an OD₆₀₀ of 0.1, and grown at 21° C until OD₆₀₀ was 1.0. Expression was induced by the addition of IPTG to 1 mM and the cells were grown for 16 h at 30° C. The cells and culture supernatant were separated by centrifugation and samples of the cell pellet and supernatant were analyzed on a 15% SDS-PAGE gel followed by the Western blot analysis using the mouse monoclonal antibody anti-E-Tag-HRP conjugate (Pharmacia) to visualize the expressed product. The expressed rVabs were purified from the supernatant by precipitation with ammonium sulphate (which was added to 70% saturation) at 21°C, followed by centrifugation at 10,000 g for 15 min. The aqueous phase was discarded, and the pellet resuspended and dialyzed in PBS (phosphate buffered saline, pH 7.4) at 4° C overnight. Insoluble material was removed by centrifugation at 10,000 g, and the supernatant was filtered through a 0.22 µm membrane and purified on an anti-E-Tag antibody affinity column (Pharmacia). The affinity resin was equilibrated in TBS (0.025 M Tris-buffered saline, pH 7.4) and the bound protein was eluted with the Elution buffer (100 mM glycine, pH 3.0). The rVab was concentrated to 1 mg/ml, dialyzed against TBS and stored at 4° C. The SDS-PAGE, Western blot analysis and N-terminal sequence analysis of the affinity purified material were performed according to standard protocols.

### 5. Size Exclusion FPLC Chromatography

The affinity purified rVabs were fractionated by size exclusion FPLC on a Superdex 75 HR10/30 column (Pharmacia) to determine the molecular size and aggregation state of the rVabs. For calibration of the column, High and Low Molecular Weight Gel Filtration Calibration Kits (Pharmacia) were used. Fractions from several chromatographic separations corresponding to a molecular weight of 30 kDa were pooled and concentrated to 0.7-1.0 mg/ml using Amicon XM10 membranes. Protein concentrations were determined using the BCA protein assay kit (Pierce Chemical Co., Rockford, IL).

### 6. BIAcore Analyses

IGF-1 R was immobilized onto one flow cell of a CM-5 sensor chip (Biosensor) using amine coupling chemistry and the manufacturer's recommended protocols. BSA was immobilized in the same manner to another flow cell of the same chip as a control surface. Increasing concentrations of the affinity-purified rVabs were injected over both surfaces, and the binding responses were allowed to come to equilibrium. After a subtraction of the background binding (from the control surface), the equilibrium dissociation constant was derived using Scatchard analysis.

### 7. Time-Resolved Fluorescence Assay

We have selected the basic format of an *in vitro* competitive receptor binding assay as the basis of a heterogeneous screen for small organic molecular replacements for IGF-1. In the present assay, occupation of the active site of IGF-1 receptor is quantified by time-resolved fluorometric detection (TRFD) with streptavidin-labeled europium (saEu) complexed to biotinylated peptides (bP). In this assay, saEu forms a ternary complex with bP and IGF-1 receptor (i.e., IGF-1 R:bP:saEu complex). The TRFD assay format is well established, sensitive, and quantitative (Tompkins et *al.,* 1993). We demonstrate the assay using 43G7 rVab or a biotinylated peptide. Furthermore, we show that both assay formats faithfully report the competition of the biotinylated ligands binding to the active site of IGF-1R by IGF-1.

In these assays, soluble IGF-1 receptor is coated on the surface of microtiter wells, blocked by PBS containing milk and BSA, and then incubated with biotinylated peptide or rVab. Unbound bP is then washed away and saEu is added to complex with receptor-bound bP. Upon addition of the acidic enhancement solution, the bound europium is released as free Eu⁺³ which rapidly forms a highly fluorescent and stable complex with components of the enhancement solution. The IGF-1 R:bP bound saEu is then converted into its highly fluorescent state and detected by TRFD.

### a. Preparation of [Eu3+]-Labeled rVab 43G7

One milligram of rVab 43G7 (the sequence is provided in Figure 34) was added to 300 nmol Eu³⁺-chelated N¹(P-isothiocyanatobenzyl)-diethylenetriamine-N¹,N²,N³-tetracetic acid (Wallac). The reaction was conducted at pH 8.5. The tube was mixed gently and placed at ambient temperature. When the reaction was complete (16 h), the sample was diluted 10-fold into the Tris-buffered saline.(TBS), pH 7.5, and the separation of the labeled rVab from the unlabeled rVab and free-Eu³⁺ was achieved by using the PD-10 column. The protein concentration and labeling efficiency were determined using a Europium standard solution (Wallac).

### b. Assay Method

IGF-1R (5 µg/ml in 50 mM NaHCO₃) was coated onto low-fluorescence MaxiSorp (Nunc) plates (100 µl/well) overnight at 4°C. The plates were blocked with PBS containing 2% non-fat milk and 0.05% BSA for 2 h at RT, followed by three PBS washes. For competitive ELISA, serial dilutions of unlabelled IGF-1 (0.1 nM-100 µM) were added to the plates (100 µl/well) and incubated at RT for 1-2 h. 100 µl [Eu3⁺] rVab 43G7 in Wallac's DELFIA assay buffer (100 mM Tris-HCl, pH 7.8; 150 mM NaCl; 0.5% BSA, 0.05% bovine lg; 0.05% NaN₃; 0.01% Tween-20) was added and incubated for 1.5 h at RT. The plates were then washed 5 times with TTBS (TBS buffer containing Tween-20; Wallac) and tapped dry. Subsequently, 100 µl of DELFIA enhancement solution (100 mM acetone-potassium hydrogen phtalate, pH 3.2; 15 mM 2-naphtyltrifluoroacetate; 50 mM tri(n-octyl)-phosphine oxide; 0.1 % Triton X-100) was added to each well, and the plates were shaken for 10 min at RT. Fluorescence of each sample well was measured at 615 nm using a DELFIA 1234 fluorometer (EG&G Wallac).

The dose response of TRFD of Eu was studied in microtiter wells. Detection is linear over the range 0.2 to 200 fmol with a limit of detection (twice background) of 0.05 fmol. There are 6010 fluorescent units (FU) per fmol of Eu. Binding and detection of Eu-SA, (4.7 mol Eu/mol streptavidin) to wells coated with biotinylated BSA (bBSA) (6 mol biotin/mol BSA) is linear over the entire range tested. The specific fluorescent activity of streptavidin Eu-SA (with 4.7 mol Eu/mol SA) is 28 kfu/fmol and the limits of detection (i.e., twice background) are 0.030 fmol. Coating with IGF-1R was linear up to inputs of 200 ng/well and thereafter appeared to saturate at about 660 ng blGF-1 (biotinylated IGF-1) per well. This is the expected amount based on the manufacturer's information about protein saturation densities of these wells (Nunc manual). These studies show a limit of detection of blGF-1 (i.e., twice background) of 0.05 fmol blGF-1. The ability of this assay format to detect specifically bound blGF-1 (or bPeptides) to IGF-1 R coated wells was determined.

### 8. Elisa Analyses

ELISA was performed on selected rVabs. We found that the native IGF-1 ligand inhibits the binding of peptide 5.1 (the sequence of which originates from the phage clone B6) as shown in Figure 43. The detection of the peptide involved a sandwich configuration with the Eu-labeled streptavidin. It was determined that the binding of Eu-labeled rVab 43G7 to IGF-1R is inhibited by IGF-1 with an IC₅₀ of approximately 2 nM, as shown in Figure 44. The binding of the biotinylated peptide 5.1 is inhibited by rVab 43G7 with an IC₅₀ of about 10 nM (Figure 45), indicating that both the peptide and rVab bind to the same site on the IGF-1 R molecule.

Figures 46A-46D demonstrates the binding properties of the 43G7 antibody. The binding of the Eu-labeled 43G7 antibody is competed by peptide 5.1 (clone B6) (Figure 46A) and by the non-labeled 43G7 (Figure 46B), as well as by rVab 39F7 (Figure 46C) and rVab 1G2P (Figure 46D). The sequences of rVabs 1G2P and 39F7 are provided in Figure 35 and Figure 36, respectively.

### C. Conclusions

The above results support the use of this assay procedure as a high throughput screen for agents, with affinities for sites on the human IGF-1 R which bind IGF-1. The studies show the lGF-1-specific peptides bind in a dose-dependent, saturable manner and are blocked from binding by agents known to bind to the active site of the receptor. This competition is reproducible and easily quantified. Furthermore, the TRFD assay, which is automatable, is much more sensitive than is an ELISA.

### Example 6: Agonistic and Antagonistic Activity of IGF-1R-Binding Peptides

Agonistic and antagonistic activities of the IGF-1-specific peptides were tested in FDCP2 cells (NIH) which express IGF-1R. The cell line requires either IL-3 or IGF-1 for growth, and the cells were maintained in RPMI 1640 medium containing 15% FCS (fetal calf serum). Agonism activity assays were performed in a total volume of 100 µl in 96 well plates (flat bottom). Cells were seeded at 30,000 cells/well in 50 µl RPMI 1640 (without IL-3) medium containing 15% FCS in triplicate wells. To each well, 50 µl of a solution containing either IGF-1, rVabs or peptides at different concentrations was added, followed by incubation for 42 h in a CO₂ incubator at 37°C.

Assays to measure the antagonistic activity were performed in a total volume of 100 µl in 96 well plates. An IGF-1-specific peptide, rVab or an appropriate control was added to wells containing 0.003 µM of human IGF-1 and incubated at 37°C for 18 h in CO₂ incubator. Proliferation assays were performed using WST-1 reagent. The WST-1 tetrazolium salt (slightly red) is cleaved to formazan (dark red) by the succinate-tetrazolium reductase system, which is active only in viable cells. An increase in the number of cells results in an increase in the overall activity of the dehydrogenase which results in a higher absorbance at 450 nm. Ten microliters of WST-1 reagent was added to each well and the plates incubated for 1-4 h at 37°C. Proliferation was measured by absorbance at 450 nm. Both 5.3 and 5.4 peptides showed an agonistic activity at the 10 µM concentration (Figure 23). Peptides 5.1 and 5.2 showed a significant antagonistic activity in the 3-30 µM concentration range (Figure 22). Control peptide showed no antagonistic activity at the concentrations tested.

The results described demonstrate the feasibility of both the chemical synthesis of and construction of a recombinant expression vector to make sufficient soluble peptide (free or as fusion with some carrier protein) or rVab for testing agonist and antagonist activities. The results provide peptide-receptor pairs to be used in a site directed competition binding assay wherein IGF-1R can be used as one member of the pair, with the peptide or a rVab as the other member. Labeling of each member, and detection of pair formation, using either member in radioactive or nonradioactive labeled forms, is possible by a variety of methods known to those skilled in the art of building competition binding assays. This assay provides a high throughput screening assay to identify small organic molecules which bind to the active site of IGF-1R.

### Example 7: Phage Library B6-2

This library was designed based on the "core" sequence of the Class I binders Site 1 (B6) which posses antagonistic activities in a cell proliferation assay. The core sequence was determined as DPFYHKLSEL, where the residues F (position 3, X₆ of Formula 2), Y (position 4, corresponding to X₇ of Formula 2), L (position 7, corresponding to X₁₀ of Formula 2) and L (position 10, corresponding to X₁₃ of Formula 2) were the only residues observed at those positions. The purpose of this library was to test the possibility that some binders will show deviations from the core sequence, especially at the positions where substitutions had not previously been observed. The library was therefore made from doped oligonucleotides so that, on average, half of the amino acid residues were altered per peptide. The library was made as described in the original B6 library, i.e., synthetic oligonucleotides were first amplified in a PCR reaction. The resultant products were cloned into pACANTAB5E (Pharmacia) via *Sfi*l and Noel restriction sites as previously described. Over 10¹⁰ different clones were obtained in the final library.

### A. Random 20mer Library

### 1. Panning with the B6-2 and Random 20mer Libraries

The libraries were affinity selected against IGF-1R 96 clones from round 3 of panning from B6-2 library and 96 clones from round 4 from the random 20mer library were analyzed in a phage ELISA to identify binders. The DNA of binders was then determined. The results from both libraries show that positions other than positions 3, 4, 7 and 10 as described above can vary relatively at ease (see tables below), while variability at positions 3, 4, 7 and 10 is much more restricted. The results from the B6-2 library show that the restricted core residues were maintained in all binders except one, which happened only in one instance, L (position 7) can be substituted by another hydrophobic residue, M, at that position. The result from the random 20 library panning revealed that another aliphatic amino acid residue, I can substitute for L at position 7. In addition, the restricted residue at position 10 (L) can also be substituted with amino acid residue M. Thus, 2 of the previously identified restricted residues (L at positions 7 and 10) are not absolute, even though L is preferred at these positions. It should be noted that the failure to observe a substitution at a particular residue position does not necessarily indicate that substitutions cannot be made without losing activity, rather such an absence of substitution is indicative of a preference or an aversion for substitution. The findings are summarized below:

### B. Results

Combined results from binding clones isolated from B6-2 (doped core) and random 20 libraries of the Formula 2 motif are shown below in Table 3. Sequences from 25 clones from B6-2 and 29 clones from the random 20mer library were analyzed. Numbers adjacent the amino acid residues represent the frequency with which a specific amino acid was observed at the corresponding position.

**TABLE 3**

| B6 CORE | D37 | P34 | ***F54** | ***Y54** | H12 | K15 | ***L46** | S16 | E27 | ***L53** | L30 |
|---|---|---|---|---|---|---|---|---|---|---|---|
| | E | A | | | A4 | A10 | I7 | A5 | A6 | M | A6 |
| | G3 | D4 | | | D7 | G7 | M | D3 | D3 | | I |
| | K4 | E | | | E9 | I | | E4 | G2 | | K |
| | R2 | G10 | | | G | L8 | | F5 | K | | S2 |
| | S5 | L | | | K3 | M3 | | G4 | L | | T |
| | T | Q | | | L2 | N | | H | Q3 | | V13 |
| | V | S | | | M | Q | | L6 | R6 | | |
| | | T | | | N | R5 | | M | S4 | | |
| | | | | | Q7 | | | N | V | | |
| | | | | | R4 | V | | Q2 | | | |
| | | | | | S | W | | R2 | | | |
| | | | | | T | | | T2 | | | |
| | | | | | V | | | Y2 | | | |

Based on the substitutions observed above, the following preferences shown in Table 4 are preferred for substitutions in the amino acid sequence of Formula 2 for binding to IGF-1R.

**TABLE 4**

| 25X98 | X99 | X6 | X7 | X8 | X9 | X10 | X11 | X12 | X13 | X100 |
|---|---|---|---|---|---|---|---|---|---|---|
| 1 (D) | 2(P) | 3(F) | 4(Y) | 5(H) | 6(K) | 7(L) | 8(S) | 9(E) | 10(L) | 11(L) |
| no aromatics; no large aliphatics; no c | no aromatics; no c; no + charged | | | no aromatics; no C; no P; no I | no aromatics, except W; no - charged; no C; no P | | no C; noP | no aromatics; no C; no P | | no aromatics; no aliphatics; no C; no P |

### Example 8:

A composite of amino acid residues observed in sequences of random 20mer, 40mer and A6 (Formula 1) clones is illustrated below:

| A6 CORE | **N** | **F** | **Y** | **D** | **W** | **F** |
|---|---|---|---|---|---|---|
| | D6 | | | | A | |
| | E | | | | E9 | |
| | G6 | | | | G2 | |
| | H3 | | | | Q4 | |
| | K | | | | R | |
| | P | | | | S | |
| | Q8 | | | | | |
| | S | | | | | |
| | T | | | | | |
| | V | | | | | |

A summary of preferences for A6 residues is shown in Table 5 below. An illustration of residues which are characteristic of IGF-1R binding sequences (above parental sequence) and those which are not typically associated with binding sequences (below parental sequence). Table 6.

**TABLE 5**

| | X1 | X2 | X3 | X4 | X5 |
|---|---|---|---|---|---|
| 1 (N) | 2(F) | 3(Y) | 4(D) | 5(W) | 6(F) |
| no aromatics; no large aliphatics; no C; no P | | | no hydrophobics, except tiny; no C; no P | | |

**TABLE 6**

| | | | | | | | | | | | | | | | | | | | | |
|---|---|---|---|---|---|---|---|---|---|---|---|---|---|---|---|---|---|---|---|---|
| | | | | | | | | | | | | | | | | M | | | | |
| | | | | | | | | | | Y | | | | | | K | | | | |
| | | | | | | | | | | G | | | | | | R | | | | |
| Characteristic of IGF-1R Binding Sequences | | | | | | | | | | T | | | | | | S | | | | |
| | | | | | | | | | | K | | | | | | D | | | | |
| | | | | | | | | | | S | | | A | | | L | | | | |
| | | | | | | | | | | H | | | G | | | A | | | | |
| | | | | | | | | | | Q | | | Q | | | E | | | | |
| | | | | | | | | D | | D | Y | | E | | | Q | | | | |
| Parental | D | Y | K | D | G | F | R | E | G | N | F | Y | D | W | F | V | A | Q | V | T |
| | G | | | | | L | | | | T | Q | Q | A | R | L | L | | P | | |
| | | | | | | | | | | V | L | F | E | G | V | E | | | | |
| Uncharacteristic of IGF-1R Binding Sequences | | | | | | | | | | E | V | L | G | P | W | M | | | | |
| | | | | | | | | | | G | * | E | R | Q | C | D | | | | |
| | | | | | | | | | | A | Y | S | H | C | | | | | | |
| | | | | | | | | | | Q | S | I | | S | | | | | | |
| | | | | | | | | | | I | | N | | * | | | | | | |
| | | | | | | | | | | Y | | K | | | | | | | | |
| | | | | | | | | | | K | | * | | | | | | | | |

### Example 9: Panning the Insulin Receptor

A standard method was used to coat and block all microtiter plates. IR (prepared according to Bass *et al.,* 1996) was diluted to 2 µg/ml in PBS. Fifty microliters of this solution was added to an appropriate number of wells in a 96-well microtiter plate (MaxiSorp plates, Nunc) and incubated overnight at 4°C. Wells were then blocked with a solution of 2% non-fat milk in PBS (MPBS) at RT for at least 1 h.

### A. Two-Day Panning Procedure

Eight wells coated with IR were used for each round of panning. One hundred microliters of phage were added to each well. For the first panning round, the input phage titer was 4 x10¹³ cfu/ml. For subsequent rounds, the input phage titer was approximately 10¹¹ cfu/ml. Phage were allowed to bind for 2-3 h at RT. The wells were then quickly washed 13 times with 300 µl/well of PBS containing 0.5% Tween-20 (PBST). Bound phage were eluted by incubation with 150 µl/well of 50 mM glycine-HCl, pH 2.0 for 15 min. The resulting solution was pooled and then neutralized with Tris-HCl, pH 8.0. An equal volume of log-phase TG1 cells were infected with the eluted phage, then plated onto two 24 cm x 24 cm plates containing 2xYT-AG. The plates were incubated at 30°C overnight. The next morning, cells were removed by scraping and stored in 10% glycerol at -80°C. For subsequent rounds of affinity enrichment, cells from these frozen stocks were grown and phage were prepared as described above. A total of 216 clones from the 20mer library and 120 clones from the 40mer library were picked at random from the third and fourth rounds of panning and screened for IR binding activity. DNA sequencing of the clones revealed the abundance of sequences as summarized in Figures 1A, 1B, 2A, 2C, 10A and 10B.

### B. One-Day Panning Procedure

Log phase TG1 cells were infected with the eluted phage, amplified in the 2xYT medium for 1 h at 37°C prior to the addition of helper phage, ampicillin and glucose (2% final concentration). After incubation for 1 h at 37°C, the cells were spun down and resuspended in the 2xYT-AK medium. The cells were then returned to the shaker and incubated overnight at 37°C. The overnight phage was then precipitated and subjected to the next round of panning. A total of 96 clones were picked at random from rounds 3 and 4 and screened for binding activity.

To isolate specific binders, each library was panned against a soluble form of the human IR. This IR is composed of the extracellular domains of both the α and β chains of the natural receptor, as well as the constant domain from immunoglobulin Fc, retaining the β-a-a-β structure described above. Because the IR is expressed in a eukaryotic system, disulfide bond formation and glycosylation patterns should mimic the wild-type receptor. The details of this recombinant protein construct are described in Bass *et al.* (1996).

In panning with the peptide library, the IR was immobilized directly onto a protein-binding plastic surface, and four rounds of panning and enrichment were carried out. Analysis of phage clones from rounds three and four showed that 114 of the 216 clones from the 20mer random peptide library and 17 of the 120 from the 40mer random peptide library bound to IR (Figures 1A, 1B, 2A, 2C, 4A, 6A, 10A and 10B). Of those clones tested competitively against insulin for receptor binding, all were blocked by the presence of natural ligand. This result indicated that these phage clones and insulin bind to the same site (or at least overlapping sites) on IR.

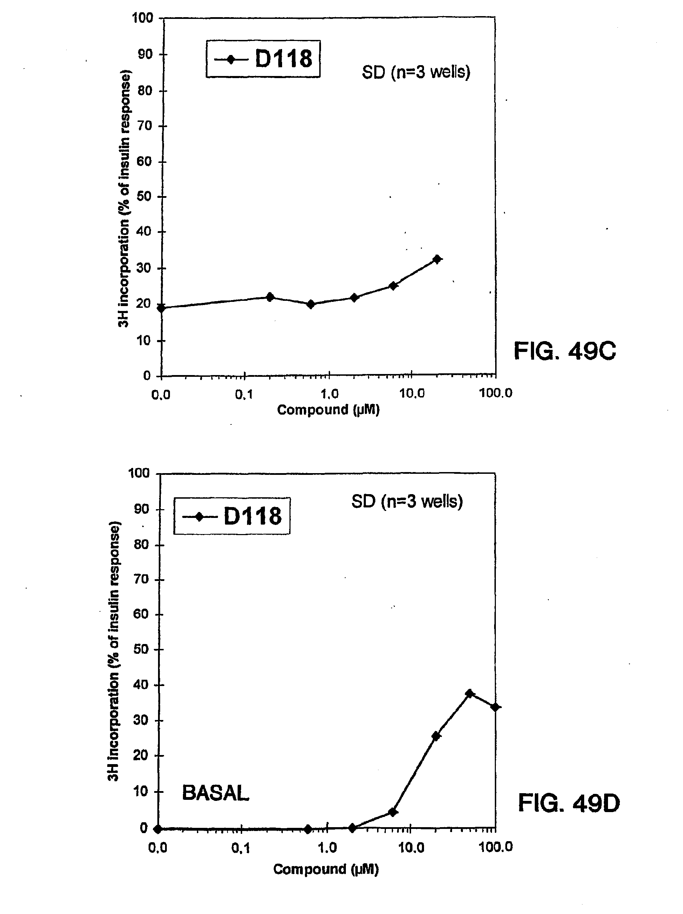

Sequence analysis of several clones shows that there are several distinct populations, designated as Groups 1 through 8 (Figures 1-8) (Figures 47 and 48). Several of the peptides based on the sequences for these groups have been chemically synthesized for subsequent testing. Group 1 (Formula 1 motif) peptides contain the consensus sequence FYxWF, and are believed to be agonistic in cell-based assays. Group 2 (Formula 6 motif) is composed of two peptides having a consensus sequence VYGR and two cysteine residues each. Thus, Group 2 peptides are capable of forming a cyclic peptide bridged with a disulfide bond. Group 3 (Formula 2 motif) peptides comprise the preferred consensus sequence F-Y-x-A/G-L/l-x-x-L (A/G denotes the alanine or glycine residue, and L/l denotes the leucine or isoleucine residue). Certain Group 3 peptides exhibit agonistic activity in cell-based assays (Figure 49). Neither of these consensus sequences have any significant linear sequence similarities greater than 2 or 3 amino acids with mature insulin.

Group 7 (Formula 4 motif) is composed of two exemplary peptides which do not have any significant sequence homology, but have two cysteine residues 13-14 residues apart, thus being capable of forming a cyclic peptide with a long loop anchored by a disulfide bridge.

### Example 10: ELISA Analyses of Phage

This series of experiments was designed to help characterize the different groups of consensus sequences found during the biopanning of IR. Phage were prepared from each group (two unique sequences each were attempted). Each phage was bound to insulin receptor and competition experiments were performed.

*Phage Production.* Each phage culture was started by the addition of 30 µl of the master stock to 20 ml 2xYT-AG in 50 ml centrifugation tubes. Cultures were incubated at 37°C until OD₆₀₀ ∼0.6-1.0. M13K07 helper phage were added to a concentration of ∼5 x 10¹⁰ cfu ml⁻¹ and incubated at RT for 30 min. The cultures were centrifuged at ∼2500g and 4°C for 20 min. The bacterial pellet was resuspended in 30 ml 2xYT-AK. The culture was transferred into 250 ml bottles and incubated O/N at 37°C. The culture was centrifuged at ∼2500g and 4°C for 20 min (in 50 ml centrifuge tubes). The supernatant was transferred to new 50 ml centrifuge tubes.

*Phage ELISA.* Each well of the Nunc-Immuno™ plates with the MaxiSorp™ surface were coated with either 50 µl of 2 ng/µl either IR or sIGF-1R in PBS overnight at 4°C. The wells were blocked with 200 µl of MPBS for 1.5 h at RT. Phage were added at 100 µl per well. Peptides were added as noted below and allowed to incubate at RT for 3 h. The plates were washed 3 times with PBST. A solution of 1:3000 diluted HRP:Anti-M13 conjugate at 100 µl per well of was added for 1 h. Following a repeat of the washing, 100 µl of ABTS was added for 15-30 min. The OD was measured using a SpectraMax 340 Microplate Spectrophotometer (Molecular Devices) at 405 nm.

*Peptide Competition.* Competition of phage displayed peptides by the addition of soluble peptides was carried out using the phage ELISA as described above. Twenty microliters of the stock synthetic-peptide solution was added to Row A. A series of 20 µl into 100 µl dilutions were performed until Row G. Twenty microliters were discarded from Row G to maintain 100 µl per well. Row H was reserved as no peptide wells. The starting concentration of the B6 peptide was 68 µM for both receptors. For IR, the starting concentration for the C1 peptide was 48.5 µM. Only 2 µl of the C1 peptide were added to Row A of wells containing IGF-1R. Therefore, the starting concentration was 4.9 µM. The volume was maintained by the addition of 18 µl of the phage solution to Row A.

*Natural Ligand Competition.* The "Phage First" experiments were performed by adding 10 µl of 5.5 µM, 550 nM, or 55 nM insulin or IGF-1 in PBS to phage-containing wells in the phage ELISA. The working concentrations were 500 nM, 50 nM, and 5 nM. The volume of no ligand wells was maintained by the addition of 10 µl PBS.

The "Ligand First" experiments were performed by added 50 µl of 2 µM, 200 nM, or 20 nM insulin or IGF-1 in PBS containing 0.5% Tween-20 to non-phage containing wells and allowed to incubate 15 min. Fifty microliters of the phage solution was then added to the wells and mixed well. The mixture was allowed to incubate for 2 h at RT and continue with the phage ELISA.

The data are provided in Table 7 and Figures 50A-50D. Sequences were confirmed on all clones by DNA sequencing.

**TABLE 7: Phage Characterization Summary**

| | Absorbance Values | | | IR | | sIGF-1R | |
|---|---|---|---|---|---|---|---|
| | | | | Competitions | | Competitions | |
| | NFM | sIGF-1R | IR | C1 | B6 | C1 | B6 |
| Group 1 | | | | | | | |
| *20D3* | 0.09 | 2.26 | 1.29 | Y | Y | - | |
| B8 | 0.10 | 2.55 | 1.30 | Y | Y | - | |
| Group 2 | | | | | | | |
| *20A4* | 0.15 | 0.21 | 1.61 | N | N | - | - |
| D8 | 0.09 | 2.19 | 1.42 | N | N | Y | Y |
| Group 3 | | | | | | | |
| *20E2* | 0.11 | 2.15 | 1.01 | Y | Y | - | |
| Group 4 | | | | | | | |
| *D10* | 0.12 | 0.14 | 0.73 | N* | N | - | - |
| A2 | 1.35 | 2.00 | 1.79 | N | N | N | |
| Group 5 | | | | | | | |
| *D9-2* | 1.02 | 2.53 | 1.64 | N | N | - | - |
| H4 | 1.16 | 1.14 | 1.41 | N* | N | - | - |
| Group 6 | | | | | | | |
| *E8* | 0.10 | 2.00 | 1.34 | Y | Y | - | |
| F2 | 0.09 | 2.08 | 1.43 | Y | Y | - | |
| Group 7 | | | | | | | |
| *F8* | 0.14 | 2.06 | 1.49 | N | N | Y | Y |
| Group 8 | | | | | | | |
| 40A2 | 0.56 | 0.55 | 1.90 | Y* | Y | - | - |
| 40H4 | 0.75 | 0.83 | 0.84 | - | - | - | - |

| | | | | | | | |
|---|---|---|---|---|---|---|---|
| NFM = Non-fat milk C1 peptide (D112) has the FYX₃WF Formula 1 motif and an amino acid sequence of DYKDCWARPCGDAANFYDWFVQQASKK B6 peptide has the FYX₈X₉LX₁₁X₁₂L Formula 2 motif and an amino acid sequence of WNNDPFYHKLSELLREKK | | | | | | | |

### Observations and Conclusions

1. The C1 and B6 peptides bind to IR. The C1 and B6 peptides expressed as phage-displayed peptides are negatively charged.
2. Groups 1, 3, and 6 (Formulas 1, 2 and 10, respectively), appear to be inhibited by both the C1 and B6 peptides when binding to IR and IGF-1R. All three groups behave with similar characteristics and similar affinities. They all bind to a common site, (Site 1) as shown by competition data.
3. Group 2 (Formula 6 motif) phage clones have different properties despite their sequence similarity. The phage 20A4 is an IR-specific clone. Its binding to IR is not inhibited by C1 or B6 peptides and therefore binds to Site 2. The phage D8 binds to both IR and IGF-1R. Inhibition by C1 peptide and B6 peptide occurs only when binding to IGF-1 R. D8 is more sensitive to C1 and B6 peptide inhibition than Group 1, 3, and 6, suggesting an allosteric competition.
4. Some phage appear to have a plastic-binding component (binding to the wells of microtiter plates) in their sequences when high amounts of phage are used. The phage A2, D9-2, H4, 40F10, 40A2, and 40H4 have a significant background to their signals. With the exception of 40H4, all signals increase over this background signal in the presence of IR. The signals for phage A2 and D9-2 also increase over background for IGF-1 R. It should be noted the phage for the IGF-1R binder B6 shows this similar characteristic.
5. The Group 2 phage 20A4 and Group 4 phage D10 are specific for IR -there is no detectable binding to IGF-1R. D10 may be inhibited by C1 peptide to a small extent.
6. The phage for Group 7, F8 (Formula 4 motif) has characteristics similar to Group 2, D8 (Formula 6 motif). This clone binds to both IR and IGF-1 R, but the C1 and B6 peptides only affect D8 binding when bound to IGF-1R. F8 is more sensitive to C1 and B6 peptide inhibition than Group 1, 3, and 6, (Formula 1, 2 and 10 motifs, respectively).

### Example 11: Cross-Reactivity Studies

Phage ELISA experiments show that the IGF-1 R peptides H2 and E4 have detectable binding to IR while expressed as a phage fusion. Other IGF-1 R-specific peptides such A6, C1, B6, and JBA5 do not have detectable binding to IR when expressed as phage.

### A. Experimental Procedures

*Phage Production.* Each phage culture was started by the addition of 40 µl of the MASTER stock to 20 ml 2xYT-AG in 50 ml centrifugation tubes. Cultures were incubated at 37°C until OD₆₀₀ ∼0.6-1.0. M13K07 helper phage were added to a concentration of ∼5 x 10¹⁰ cfu/ml and incubated at RT for 30 min. The cultures were centrifuged at ∼2500g and 4°C for 20 min. The bacterial pellet was resuspended in 20 ml 2xYT-AK and incubated O/N at 37°C. The culture was centrifuged at ∼2500 x g and 4°C for 20 min. The supernatant was transferred to new 50 ml centrifuge tubes

*Phage ELISA.* Each well of the Nunc-Immuno™ plates with the MaxiSorp™ surface were coated with 50 µl of 2 ng/µl either IR or IGF-1 R in PBS O/N at 4°C. The wells were blocked with 200 µl of 2% (w/v) Carnation non-fat dry milk in PBS for 1.5 h at RT. Phage were added at 100 µl per well. Peptides were added as noted below and allowed to incubate at RT for 3 h. The plates were washed 3X with PBST. A solution of 1:3000 diluted HRP:Anti-M13 Conjugate at 100 µl per well of was added for 1 h. Following a repeat of the washing, 100 µl of ABTS was added for 15-30 min. The OD₄₀₅ was measured using a SpectraMax 340 Microplate Spectrophotometer.

*Peptide Competition.* Peptide Competition Curves were produced during the phage ELISA across rows in triplicate. The stock synthetic peptide solution was added to Column 12 so that the total volume totaled 150 µl (additional phage solution was added when necessary). A serial dilution was made by transferring 50 µl from Column 12 into 100 µl in Column 11, 50 µl from Column 11 into 100 µl in Column 10, and continuing the serial dilution until Column 2. Fifty microliters were discarded from Column 2 to maintain 100 µl per well. Column 1 was reserved as no peptide wells. The starting working concentrations for each peptide was: H2 - 50 µM; H2C - 100 µM; C1C -100 µM; D2C -100 µM; E4 - 33.3 µM; C1 - 50 µM; A6 - 100 µM; and p53 - 100 µM.

### B. IGF-1 R Peptide Competition

An experiment was designed to ascertain whether IGF-1 R peptides have the ability to compete phage that bind to IR. Competition will occur in either IR- or IGF-1R-coated wells. The IGF-1R peptides H2, H2C, C1C, D2C, E4, C1, and A6 were tested for competition with two separate phage. The first, 20D3, (Figures 51A, 51C) is a phage discovered during panning of IR, but is also positive for binding to IGF-1R. The second, H2, (Figures 51B, 51D) is a phage found during panning of the IGF-1R, but is also positive for binding to IR. A p53-like peptide that binds to MDM2 was used as a negative control.

The Hill Plot data are provided in Table 8 below, and presented graphically in Figures 52A-52D.

**TABLE 8: Hill Plot Data**

| | IGF Receptor | | | | | | Insulin Receptor | | | | | |
|---|---|---|---|---|---|---|---|---|---|---|---|---|
| | 20D3 Phage | | | H2 Phage | | | 20D3 Phage | | | H2 Phage | | |
| Pept. | n | K_{d} | r² | n | K_{d} | r² | n | K_{d} | r² | n | K_{d} | r² |
| H2 | 1.29 | 4958 | 0.991 | 1.21 | 9812 | 0.979 | 1.07 | 1133 | 0.978 | 0.71 | 762 | 0.981 |
| H2C | 0.81 | 5055 | 0.975 | 1.02 | 3720 | 0.987 | 1.03 | 564 | 0.976 | 0.62 | 480 | 0.926 |
| C1 | 1.37 | 19 | 0.988 | 0.96 | 40 | 0.976 | 0.83 | 324 | 0.999 | 0.46 | 132 | 0.922 |
| C1C | 1.32 | 13475 | 0.990 | 1.00 | 34198 | 0.945 | 0.70 | 1190 | 0.988 | 0.53 | 532 | 0.956 |
| D2C | 1.50 | 12454 | 0.995 | 1.34 | 33124 | 0.999 | 0.81 | 2491 | 0.995 | 0.96 | 2964 | 0.983 |
| E4 | 1.53 | 6522 | 0.995 | 1.11 | 5868 | 0.961 | 0.79 | 1435 | 0.979 | 0.71 | 387 | 0.994 |

### C. Observations and Conclusions

a. These peptides can bind to IR and inhibit binding of phage found by either panning IR (20D3) or IGF-1R (H2). This crossover event between the two receptors occurs despite negative results of many of these same phage-displayed peptides.
b. Although the C1 peptide is the most potent inhibitor of phage binding, C1 peptide loses much of its potency advantage over the other peptides binding IR instead of IGF-1 R. In addition, A6 gains potency when binding to IR relative to the other peptides. Combined, this suggests that the adjacent surfaces to this active site of the receptors are sufficiently different that peptides and small organic molecules specific for either receptor can be found.
c. The Hill Coefficient of the peptides binding to IGF-1 R is always 1.5 to 2-fold higher than the same phage and peptide binding to IR.

### Example 12: Competition of Phage Binding with Insulin

Many different peptides isolated from the random peptide libraries were tested for the ability to compete the natural ligand insulin. Clones tested were B8 (D103) (Formula motif 1), F4 (Formula motif 1), A7 (D122) (20A4) (Formula motif 6), D8 (D123; data not shown) (Formula motif 6), C6 (Formula motif 2), E8 (Formula motif 10), H4 (group 5; data not shown), A4 (group 6), G8 (group 7), G7 (Fc binder). H4 most likely binds non-specifically to the material from which the microtiter plate is made.

### A. Insulin Competition Procedure

Receptors were coated at 100 µg/ml, 50 µl/well. After blocking with MPBS and washing 3x with PBST, insulin was added in the presence of 0.1 % Tween-20 at 2 µM, 100 nM, and 5 nM for 15 min before the addition of IR binding phage. The final concentration of insulin was 1 µM, 50 nM and 2.5 nM. Reaction was incubated at RT for 1 h and wells were washed 3x with PBST (PBS with 0.05% Tween-20). Anti-M13 HRP was added and incubated for 1 h at RT. Wells were washed 3x with PBST before the addition of ABTS. Plates were read at 405 nm.

### B. Results

At high insulin dosage, all clones, except F4, G7, and H4 # (not shown), were inhibited; B8 showed the best inhibition, >50%. The apparent lack of binding of F4 (group 1) might be due to the insufficient level of phage present. G7 is a Fc binding phage is should not by inhibited by insulin. H4 is suspected to be a plastic-binding phage. The results are presented in Figure 54.

### C. Conclusions

Insulin competition with a representative member from each group indicated that almost all of the groups competed with insulin; only the "plastic binders" and Fc binding phage did not compete. Different degrees of inhibition by these peptides (phage) imply that the peptides recognize different epitopes on or in the close proximity of the receptor active site.

### Example 13: Synthetic Peptide 20A4 Competition Results

This experiment was performed as in Example 12. The 20A4 peptide (D122) starting concentration was 58 µM.

Results. The results are included in Table 7. The peptide 20A4 (D122) (A7) competes with Group 2 members (Formula 6 motif), Group 4 member (miscellaneous) D10, and Group 7 member (Formula 4 motif) F8 (D124). There is a partial inhibition of Group 6 member F2. The data is consistent with the conclusion that the site for 20A4 binding is different from the site for Group 1, Group 3, and Group 6.

### Example 14: Peptide Fusions to the Maltose Binding Protein - Construction, Purification and Characterization of the Binding to the Insulin Receptor

### A. Cloning

The transfer of interesting peptide sequences from phage display to display as maltose binding protein (MBP) -fusions is desirable for several reasons. First, to obtain a more sensitive affinity estimate, the polyvalency of phage display peptides should be converted to a monovalent system. For this purpose, the peptide sequences are fused to MBP that generally exists as a monomer with no cysteine residues. Second, competition experiments can be carried out with the same or different peptides, one phage displayed and the other fused to MBP. Lastly, purified peptides can be obtained by cleavage of the fusion protein at a site engineered in the DNA sequence.

Figure 55 shows a schematic drawing of the MBP-peptide construct. In the construct, the N-terminus of the peptide sequence is fused to the C-terminus of the MBP. Two peptide-flanking epitope tags are included, a shortened-FLAG at the N-terminus and E-Tag at the C-terminus. The corresponding gene fusion was generated by ligating a vector fragment encoding the MBP in frame with a PCR product encoding the peptide of interest. The vector fragment was obtained by digesting the plasmid pMAL-c2 (New England Biolabs) with *Eco*RI and *Hind*III and then treating the fragment with shrimp alkaline phosphatase (SAP; Amersham). The digested DNA fragment was resolved on a 1 % agarose gel, excised, and purified by QIAEXII (QIAGEN). The 20-amino acid peptide sequences of interest were initially encoded in the phage display vector pCANTAB5E (Pharmacia). To obtain these sequences, primers were synthesized which anneal to sequences encoding the shortened FLAG or E-Tag epitopes and also contain the required restriction enzyme sites *Eco*RI and *Hind*III*.* PCR products were obtained from individual phage clones and digested with restriction enzymes to yield the insert fragment. The vector and insert were ligated overnight at 15°C. The ligation product was purified using QIAquick spin columns (QIAGEN) and electroporations were performed at 1500 v in an electroporation cuvette (0.1 mm gap; 0.5 ml volume) containing 10 ng of DNA and 40 µl of *E*. *coli* strain ER2508 (RR1 *lon:mini*Tn*10*(Tet^{r}) (*malB*) (*argF-lac*)*U169* Pro⁺ *zjc*::Tn5(Kan^{r}) *fhuA2*) electrocompetent cells (New England Biolabs). Immediately after the pulse, 1 ml of pre-warmed (40°C) 2xYT medium containing 2 % glucose (2xYT-G) was added and the transformants were grown at 37°C for 1 h. Cell transformants were plated onto 2xYT-AG plates and grown overnight at 37°C. Sequencing confirmed the clones contained the correct constructs.

### B. Small-Scale Expression of Soluble MBP-Peptide Fusion Proteins

*E. coli* ER2508 (New England Biolabs) carrying the plasmids encoding MBP-peptide fusion proteins were grown in 2xYT-AG at 37°C overnight (250 rpm). The following day the cultures were used to inoculate media (2x YT containing-G) to achieve an OD₆₀₀ of 0.1. When the cultures reached an OD₆₀₀ of 0.6, expression was induced by the addition of IPTG to a final concentration of 0.3 mM and then cells were grown for 3 h. The cells were pelleted by centrifugation and samples from total cells were analyzed by SDS-PAGE electrophoresis. The production of the correct molecular weight fusion proteins was confirmed by Western blot analysis using the monoclonal antibody anti-E-Tag-HRP conjugate (Pharmacia).

### C. Large-Scale Expression of Soluble MBP-Peptide Fusion Proteins

*E.* coli ER2508 carrying plasmids encoding the MBP-peptide fusion proteins were grown in 2xYT-AG media for 8 h (250 rpm, 37°C). The cultures were subcultured in 2xYT-AG to achieve an OD₆₀₀ of 0.1 and grown at 30°C overnight. This culture was used to inoculate a fermentor with medium of following composition (g/l):

| | |
|---|---|
| Glucose | 3.00 |
| (NH₄)₂SO₄ | 5.00 |
| MgSO₄ •7H20 | 0.25 |
| KH₂PO₄ | 3.00 |
| Citric Acid | 3.00 |
| Peptone | 10.00 |
| Yeast extract | 5.00 |
| pH 6.8 | |

The culture was grown at 700 rpm, 37°C until the glucose from the medium was consumed (OD₆₀₀ = ∼6.0 - 7.0). Expression of the fusion protein was induced by the addition of 0.3 mM IPTG and the culture was grown for 2 h in fed-batch mode fermentation with feeding by 50 % glucose at a constant rate of 2 g/l/h. The cells were removed from the medium by centrifugation. Samples of the cell pellet were analyzed by SDS-PAGE followed by the Western blot analysis using the mouse monoclonal antibody anti-E-Tag-HRP conjugate (Pharmacia) to visualize the expressed product.

### D. Purification

The cell pellets were disrupted mechanically by sonication or chemically by treatment with the mild detergent Triton X-100. After removal of cell debris by centrifugation, the soluble proteins were prepared for chromatographic purification by dilution or dialysis into the appropriate starting buffer. The MBP fusions were initially purified either by amylose affinity chromatography or by anion exchange chromatography. Final purification was performed using anti-E-Tag antibody affinity columns (Pharmacia). The affinity resin was equilibrated in TBS (0.025 M Tris-buffered saline, pH 7.4) and the bound protein was eluted with Elution buffer (100 mM glycine, pH 3.0). The purified proteins were analyzed for purity and integrity by SDS-PAGE and Western blot analysis according to standard protocols.

For BIAcore analysis of fusion protein and synthetic peptide binding to insulin receptor, insulin (50 µg/ml in 10 mM sodium acetate buffer pH 5) was immobilized on the CM5 sensor chip (Flowcell-2) by amine coupling. Flowcell-1 was used for background binding to correct for any non-specific binding. Insulin receptor (450 nM) was injected into the flow cell and the binding of IR to insulin was measured in resonance units (RUs). Receptor bound to insulin gave a reading of 220 RU. The surface was regenerated with 25 mM NaOH. Pre-incubation of receptor with insulin in a tube at RT completely abrogated the response units to 16 RU. Thus, the system was validated for competition studies. Several maltose-binding fusion proteins, peptides and rVabs were pre-incubated with insulin receptor before injecting over the insulin chip for competition studies. The decrease in binding/resonance units indicates that several MBP-fusion proteins can block the insulin binding site. The results are shown in Tables 9 and 10. The amino acid sequences referred to in the tables are identified in Figures 47 and 48, except the 447 and 2A9 sequences, which are shown below.

**TABLE 9:BIAcore Results―Fusion Proteins Compete for Binding to IR**

| | **Incubation Mixtures** | **Result (RUs)** | **Sequence Type** |
|---|---|---|---|
| **Controls** | Insulin Receptor (IR) 450 nM | 220 | Positive Control |
| | Insulin (8.7 µM) | 16 | Negative Control |
| **MBP Fus. Prots.** | A7 (20A4)-MBP (4.1 µM) + IR | 43 | Formula 6 Motif |
| | D8-MBP (1.6 µM) + IR | 56 | Formula 6 Motif |
| | D10-MBP (3.4 µM) + IR | 81 | Formula 11 Motif |
| | 447-MBP (11.5 µM) + IR | 195 | hGH Pept. Fus. |
| | MBP (13 µM) + IR | 209 | Negative Control |

**TABLE 10:BIAcore Results―Synthetic peptides compete for binding to IR**

| **Incubation Mix** | **% Binding** | **Result (RUs)** | **Sequence Type** |
|---|---|---|---|
| IR | 100 | 128 | Positive control |
| IR + 20D1 | 41 | 51.8 | Formula 1 Motif |
| IR + D8 | 33 | 41.6 | Formula 6 Motif |
| IR + 20C11 | 38 | 49 | Formula 2 Motif (bkg high) |
| IR + H2 | 27 | 34.6 | IGF (phosphorylated band) |
| IR + 2A9 | 100 | 128 | IGF(bkg high) |
| IR + 20A4 | 33 | 41.8 | Formula 6 Motif |
| IR + p53wt | 97 | 124.5 | P53 wild type |

The concentration of each peptide was about 40 µM and the concentration of IR was 450 nM. The 447 peptide sequence is: LCQRLGVGWPGWLSGWCA. The 2A9 peptide sequence is: LCQSWGVRIGWLTGLCP.

### Example 15: Insulin Receptor Competition ELISA Using Phage Displayed Peptides and MBP-Peptide Fusion Proteins

To determine whether the binding sites (contact sites) on the insulin receptor for the various peptides are similar, the purified fusion proteins were used in ELISA competition experiments with phage displayed peptides from various groups. Phage-displayed peptides, which were able to bind to IR, were classified into various groups according to consensus sequences identified (see Figures 47 and 48). Peptide sequences of interest were fused to the C-terminus of MBP as previously described. The protein fusion constructs were expressed as soluble proteins, purified, and the protein concentrations were determined. The purified fusion proteins were used in ELISA competition experiments with phage displayed peptides from the various groups as shown in Table 11.

As expected, the fusion proteins containing A7 (20A4), D8, D10, and F8 peptides were able to compete the corresponding identical peptide sequence displayed on phage in the range of 28-54% of the control value. The fusion protein, MBP-A7, was able to significantly compete (<54%) phage-displayed peptides D8, D10, and F8. The other fusion protein from Group 2 (Formula 6 motif), MBP-D8, was able to compete A7 and D10 peptides displayed on phage. Furthermore, the Group 7 (Formula 4 motif) fusion protein MBP-F8 competed A7 and D10 phage displayed peptides. Figures 56A and 56B show the plotted data from Table 11. In Figure 56A, a clear pattern is seen where significant (≤ 54%) competition reactions occur between fusion proteins and phage-displayed peptides which have in common the presence of at least two cysteine residues (see Figures 47 and 48 for peptide sequences).

Also striking is the observation that the cysteine containing fusion proteins were not able to compete phage displayed peptides from Group 1 (Formula 1 motif), which contain the consensus (IGF A6-like) sequences and are without cysteine residues (Figure 56A). In Figure 56B, the fusion proteins containing the Group 1 (Formula 1 motif) consensus sequences were not able to compete to a significant extent any of the phage-displayed peptides from any of the groups. It should be noted that the corresponding identical phage from Group 1 was not tested. The data support the conclusion that the cysteine-containing peptides bind to a contact site (Site 2) which is different than the contact site (Site 1) required for the consensus containing peptides (Group 1, (Formula 1 motif)) to bind the insulin receptor.

**TABLE 11**

| **Phage Displayed** | | Group 1 | | Group 2 | | Group 4 | Group 7 | Control |
|---|---|---|---|---|---|---|---|---|
| **Peptides** | | MBP-E7 | MBP-H8 | MBP-A7 | MBP-D8 | MBP-D10 | MBP-F8 | MBP-447 |
| | | 1.6 µM | 1.6 µM | (20A4) | 2 µM | 4 µM | 2.8 µM | 14 µM |
| | | | | 5µM | | | | |
| Group 1 | B8 | 265 | 264 | 329 | 267 | 274 | 240 | 299 |
| | 20 D3 | 196 | 196 | 250 | 170 | 218 | 208 | 186 |
| Group 2 | D8 | 138 | 135 | 53 | 54 | 129 | 111 | 160 |
| | A7 | 133 | 103 | 28 | 54 | 125 | 21 | 116 |
| | (20A4) | | | | | | | |
| Group 3 | 20 E2 | 80 | 106 | 100 | 69 | 84 | 161 | 100 |
| Group 4 | A2 | 92 | 92 | 88 | 74 | 105 | 98 | 79 |
| | D10 | 92 | 60 | 20 | 20 | 36 | 20 | 117 |
| Group 6 | F2 | 91 | 97 | 88 | 83 | 92 | 83 | 101 |
| | E8 | 86 | 75 | 164 | 99 | 94 | 86 | 110 |
| Group 7 | F8 | 99 | 93 | 44 | 63 | 82 | 43 | 138 |
| Group 8 | 40 A2 | 80 | 74 | 118 | 84 | 95 | 80 | 90 |

Data reported in the table above was obtained as follows: IR was coated on a 96-well plate with 50 µl of 2 ng/µl IR and incubated overnight at 4°C. The wells were then blocked with MPBS for 1 h. The fusion proteins (mixed #1:5 with MPBS) were added to the wells and incubated at RT for 30 min. An equal volume of phage (displaying various peptides from each of the groups) was then added and incubated for 1.5 h. The control well contained only phage and an equal volume of buffer. The plate was washed 3 times in PBST and then incubated with HRP/anti-M13 conjugate for 45 min. The plate was washed again and then the ABTS substrate added. The values indicate readings taken at OD₄₀₅ which were normalized as percent control. The control fusion protein MBP-447 contains a peptide that binds the growth hormone receptor. Peptides in bold type contain cysteine residues. Underlined and in bold are values which are ≤ 54% of control values.

### Example 16: Biopanning the rVab Library

The same rVab library described in Example 5 and panned for members that bound IGF-1R was also panned for members that bind IR. Human insulin receptor was diluted to 1 mg/ml in 50 mM sodium carbonate buffer, pH 9.5. One hundred microliters of this solution was added to an appropriate number of wells in a 96-well microtiter plate (MaxiSorp plates, Nunc) and incubated overnight at 4°C. The wells were then blocked by adding 100 µl of MPBS to each well and incubating at RT for 1 h.

The phage were incubated with MPBS for 30 min at RT, then 100 µl of the phage solution were added to each well and incubated for 2 h at RT. In the first round, the input phage titer was about 10¹³ cfu/ml. The input phage titer was about 10¹¹ cfu/ml in subsequent rounds.

The wells were washed 13 times with 200 µl/well of MPBS, then washed once with PBS (200 µl/well). The bound phage were eluted by adding to each well 100 µl of 20 mM glycine-HCl, pH 2.2. After 30 s, the phage was transferred to an Eppendorf tube and the solution was neutralized by adding 50 µl of 1 M Tris-HCl, pH 8.0, per volume from each well.

TG1 cells were grown to the mid-log phase (OD₆₀₀ = 0.5). Equal volumes of the TG1 cell culture and the neutralized phage solution were mixed together, incubated for 1 h at 37°C without shaking, and then plated onto two 24 cm x 24 cm 2xYT-AG agar plates. The next morning, cells were removed by scraping the surface of the agar plates, and were then suspended in 24 ml 2xYT and stored in 10% glycerol at -80°C.

The input phage for the subsequent rounds of biopanning was prepared by growing 100 µl of the cells from these frozen stocks, followed by phage preparation according to the Protocol Preparation of Phage described below.

### Protocol: Preparation of Phage

The general protocol for phage preparations used to prepare phage displayed rVabs is described below.
1. Phagemid-containing TG1 cells were grown to OD₆₀₀ = 0.5 in 2xYT-AG media at 37°C with shaking (250 rpm).
2. M13K07 helper phage were then added (at MOI = 20), and the cells were incubated for 1 h at 37°C with gentle shaking (150 rpm).
3. Following infection, cells were pelleted by centrifugation at 1,000 g for 20 min and the supernatant containing the helper phage were discarded.
4. The cell pellet was resuspended in the initial culture volume in 2xYT-AK and grown overnight at 30°C with shaking (250 rpm).
5. The cells from the overnight culture were pelleted at 3,000 g for 30 min at 4°C and the supernatant containing the phage was recovered.
6. The supernatant was adjusted to contain 4% PEG, 500 mM NaCl and chilled on ice for 1 h. The precipitated phage was pelleted by centrifugation at 10,000 x g for 30 min. The pellet was resuspended in MPBS.

### Example 17: Expression and Characterization of IR Binding rVab Clones

### A. Infection of E. coli HB2151 Cells

a. To prepare the log-phase cells, 2xYT media was inoculated with *E*. *coli* strain HB2151 cells (genotype) from a fresh minimal medium plate, and the cells were grown to OD₆₀₀ = 0.5 at 37°C with shaking (250 rpm).
b. Fifty microliters of the pool phage from biopanning round 3 (or round 4) were transferred to 2 ml of the log phase HB2151 cells. The cells were incubated with gentle shaking for 1 h at 37°C.
c. The cells were diluted appropriately with the 2xYT media, plated on 2xYT-AGN plates and incubated overnight at 30°C.

### B. Preparation of Soluble Antibodies for Screening IGF Repetition

a. Four hundred microliters of 2xYT-AG media were added to each cluster tube (in a rack of 96 tubes in a microtiter format, Costar #4411).
b. The media in cluster tubes were inoculated by transferring the individual well-isolated colonies from the 2xYT-AGN plates using sterile toothpicks; the cluster tubes were then incubated overnight at 30°C with shaking (250 rpm). The array of bacterial cultures in cluster tubes constitutes the Master Plate.
c. The next day, the Master Plate was duplicated by transferring 40 µl of the saturated culture from each tube of the Master Plate to 400 µl of 2xYT-AG medium in a new set of cluster tubes. The new array of duplicated cultures in the microtiter plate format was labeled S1.
d. Plate S1 was incubated for 2 h at 30°C with shaking (250 rpm), and then centrifuged at 1,000 X g for 20 min at RT in a centrifuge equipped with microtiter plate adapters.
e. The supernatant was carefully removed from each cluster tube and discarded to an appropriate waste container. Four hundred microliters of the 2xYT-Al medium (no glucose added) was added to each tube in plate S1, and the plate was incubated overnight at 30°C with shaking (250 rpm).
f. Plate S1 was centrifuged as described above, and 320 µl of each supernatant (containing the soluble recombinant antibodies) was carefully transferred to a corresponding tube in a new set of 96 cluster tubes. The new plate was labeled S2.
g. Eighty microliters of the MPBS blocking buffer was added to each tube of plate S2 (already containing 320 µl of the supernatant) and incubated for 10 min at RT. This rVab preparation was now ready to be used in an ELISA performed described above.

### C. Detection of rVab Binding Using HRP/Anti-E-Tag Conjugate

a. A microtiter plate was coated with the target protein and blocked as previously described. Some of the wells of the microtiter plate were coated with an unrelated antigen to serve as a negative control.
b. The rVab preparation prepared above was diluted two-fold with the MPBS blocking buffer. Two hundred microliters of this solution was added to a set of antigen-coated and control wells.
c. The plate was incubated for 2 h at RT, and then washed 3 times with PBST.
d. The HRP/Anti-E-Tag conjugate was diluted 1:4,000 in the MPBS blocking buffer. Two hundred microliters of the diluted conjugate was added to each well, and the plate was incubated for 1 h at RT.
e. The microtiter plate was washed 3 times with PBST.
f. Two hundred microliters of the ABTS solution was added to each well, the microtiter plate was incubated for 20 min at RT, and the absorbance of each well was read at 405 nm in an appropriate microtiter plate reader.

### D. Production of Soluble rVabs

a. A suitable rVab clone in HB2151 cells was transferred from a 2xYT plate to 3 ml of 2xYT-AG media, and the culture was incubated overnight at 30°C with shaking (250 rpm).
b. Part of the overnight culture (2.5 ml) was added to 25 ml of the 2xYT media and incubated for 1 h at 30°C with shaking (250 rpm).
c. The culture was centrifuged at 1000 g for 20 min at RT, and the supernatant was removed from the pelleted cells and discarded. The pelleted cells were resuspended in 25 ml of 2xYT-Al media (no glucose is added) and were incubated overnight at 30°C with shaking (250 rpm).

### E. Purification of rVabs

The Pharmacia RPSA Purification Module kit was used (Cat. #17-1362-01), and purification was performed according to the manufacturer's directions.
a. A syringe was filled with the Elution Buffer (100 mM glycine, pH 3.0).
b. The stopper on the top of the anti-E-Tag column was removed and a drop of the Elution Buffer was added to the top of the column. The syringe was connected to the column with the Luer adapter. The connection was "drop to drop" to avoid introducing air into the column.
c. The twist-off end was removed and the column was washed with 15 ml of the Elution Buffer at a flow rate of 5 ml/min, followed immediately by 25 ml Binding Buffer (10X Binding Buffer: 0.20 M Phosphate Buffer, 0.05% NaN₃, pH 7.0).
d. Sample was applied with a peristaltic pump P-1 (Pharmacia, Cat. #19-4611-02) at a flow rate of 5 ml/min at 4°C.
e. The column was washed with 25 ml of the Binding Buffer at a flow rate of 5 ml/min to remove unbound *E. coli* proteins.
f. Bound rVab was eluted from the anti-E-Tag column with the Elution Buffer. The first 4.5 ml of material eluted from the column was discarded.
g. The next 5 ml (containing the purified E-tagged rVab) was collected in either one or several fractions.
h. The column was immediately re-equilibrate with 25 ml of the Binding Buffer for use with the next sample.

### Example 18: Competition ELISA with rVabs

For IC₅₀ determinations, microtiter plates were coated with IR and blocked as in Example 9. Soluble rVabs were prepared as described in Example 9. Prior to addition of soluble rVabs to the plates, 100 l/well of 100 nM insulin solution in PBS was added to duplicate wells. After incubation for 1 h at RT, the prepared soluble rVabs were added to each well (100 µl/well) without removing the insulin solution. After incubation for 1 h at RT, the wells were washed and the color was developed as described in Example 9.

### Example 19: Activities of rVabs in the Cell-Based Assay

Agonistic and antagonistic activities of IR-specific soluble rVabs were tested in 969 cells stably transfected with the gene encoding the human IR and IRS-1 (insulin receptor substrate). The resulting cell line requires IL-3, IL-4, or insulin for growth. Negative control cell lines do not require IRS-1 for growth. The cells were grown in RPMI 1640 media containing 10% FCS and 20 units of IL-3 per ml. Cells were seeded at 30,000 cells/well in 50 µl PRMI1640 (without IL-3) media containing horse serum instead of FCS to reduce the background. Fifty microliters of either insulin or soluble rVabs at different concentrations were added to duplicate wells, followed by incubation for 18 h in a CO₂ incubator. The cell proliferation assays were performed using WST-1 reagent. The WST-1 tetrazolium salt is cleaved to form formazan by the succinate-tetrazolium reductase system that is active only in viable cells. An increase in the number of cells results in an increase of the overall enzymatic activity of the dehydrogenase that results in a higher absorbance at 450 nm. Ten microliters of WST-1 reagent were added and the plate was incubated for 1-4 h at 36°C. Figure 60 shows the results of these studies. As can be seen, rVab 12h10 was able to induce an agonist response in 32D cells expressing IR with an ED₅₀ of approximately 50 nM.

### Example 20: IR Activation Assays

The kinase receptor activation ELISA is a functional assay based on the ability of a sample to stimulate or inhibit autophosphorylation of the insulin receptor construct that has been transfected into 32D cells (Wang *et al.,* 1993; clone 969). The assay procedure begins with the cell stimulation. The IR transfected 32D cells were seeded at 5 x 10⁶ cells/well in 96-well tissue culture plates and incubated overnight at 37°C. Samples were diluted 1:10 in the stimulation medium (PRIM1640 with 25 nM HEPES pH 7.2) plus or minus insulin. The culture media was decanted from the cell culture plates, and the diluted samples were added to the cells. The plates were incubated at 37°C for 30 min. The stimulation medium was decanted from the plates, and cell lysis buffer (50 mM HEPES pH 7.2, 150 mM NaCl, 0.5% Triton X-100, 1 mM AEBSF, 10 KIU/ml aprotinin, 50 µM leupeptin, and 2 mM sodium orthovanadate) was added. The cells were lysed for 30 min.

In the ELISA portion of the assay, the cell lysates were added to the BSA-blocked anti-IR unit mAb (Upstate Biotechnology, Lake Placid, NY) coated ELISA plates. After a 2 h incubation, the plates were washed 6 times with PBST and biotinylated anti-phosphotyrosine antibody (Upstate Biotechnology) is added. After another 2 h incubation, the plates were again washed 6 times. Streptavidin-Eu was then added, and the plates were incubated for 1 h. After washing the plates again, EG&G Wallac enhancement solution (100 mM acetone-potassium hydrogen pthalate, pH 3.2; 15 mM 2-naphtyltrifluoroacetate; 50 mM tri(n-octyl)-phosphine oxide; 0.1 % Triton X-100) was added into each well, and the plates were placed onto a shaker for 20 min at RT. Fluorescence of samples in each well was measured at 615 nm using a VICTOR 1420 Multilabel Counter (EG&G Wallac).

Alternatively, IR autophosphorylation was determined using a holoenzyme phosphorylation assay. In accordance with this assay, 1 µl of purified insulin receptor (isolated from a Wheat Germ Agglutinin Expression System) was incubated with 25 nM insulin, or 10 or 50 µM peptide in 50 µl autophosphorylation buffer (50 mM HEPES pH. 8.0, 150 mM NaCl, 0.025% Triton-X-100, 5 mM Mn₂Cl, 50 µM sodium orthovanadate) containing 10 µM ATP for 45 min at 22°C. The reaction was stopped by adding 50 µl of gel loading buffer containing β-mercaptoethanol (Bio-Rad Laboratories, Inc., Hercules, CA). The samples were run on 4-12% SDS-polyacrylamide gels. Western Blot analysis was performed by transferring the proteins onto nitrocellulose membrane. The membrane was blocked in PBS containing 3% milk overnight. The membrane was incubated with anti-phosphotyrosine 4G10 HRP labeled antibody (Upstate Biotechnology) for 2 h. Protein bands were visualized using SuperSignal West Dura Extended Duration Substrate Chemiluminescence Detection System (Pierce Chemical Co.).

### Example 21: Development of IR Assays Using Soluble rVab Antibodies and Blotinylated Peptides

a. Heterogeneous Time-Resolved Fluorescence Assay. Sixty microliters of insulin receptor (60 ng/well) was coated onto 96-well low-fluorescence MaxiSorp (Nunc) plates overnight at 4°C. The plates were blocked with TBS containing 2% milk and 0.5% BSA for 1 h at RT followed by three TBS washes. To test binding of peptides to insulin receptor, serial dilutions of biotinylated peptides were added to IR coated plates for 2 h to overnight. After TBS wash, europium-labeled streptavidin at 1 µg/ml in assay buffer (100 mM Tris-HCl, pH 7.8; 150 mM NaCl; 0.5% BSA; 0.05% bovine lg; 0.05% NaN₃; 0.01 % Tween-20) was added to the plates and incubated for 1 h. To test binding of rVab antibodies to IR, Eu-labeled rVab antibodies in assay buffer were added to the plates and incubated for 2 h to overnight. After incubation with Eu-labeled streptavidin (for peptide test) or europium-labeled rVabs, the plates were washed 5 times with Tris-buffered saline (pH 7.5) containing 0.1 % Tween-20 (TTBS) and tapped dry. Sixty microliters of EG&G Wallac enhancement solution (100 mM acetone-potassium hydrogen pthalate, pH 3.2; 15 mM 2-naphtyltrifluoroacetate; 50 mM tri(n-octyl)-phosphine oxide; 0.1% Triton X-100) was added into each well, and the plates were placed onto a shaker for 20 min at RT. Fluorescence of samples in each well was measured at 615 nm using a VICTOR 1420 Multilabel Counter (EG &G Wallac).
b. Homogeneous Time-Resolved Fluorescence Assay. A mixture of 27 nM Cy5-labeled rVab 43G7 and 6-8 nM LANCE-labeled IGF-1R (EG&G Wallac) in Tris-buffered saline containing 0.1 % BSA is added to 96-or 384-well white low-fluorescence plates (Nunc) for 2 h or overnight. For library screening, 20 µM of small organic molecules in 2 % DMSO are included in the mixture. Unlabeled rVab 43G7 at 50 nM or IGF at 3 µM are used as positive controls. Fluorescence of samples in each well is measured at both 615 nm and 665 nm using a VICTOR 1420 Multilabel Counter (EG &G Wallac).

### Example 22: Binding of Synthetic Peptides to Insulin Receptor

A series of synthetic peptides were synthesized and biotinylated (Anaspec, Inc., San Jose, CA). The binding affinities of these peptides to IR and IGF-1R were tested. Most of these peptides bind to IR at micromolar range (Figure 63). Comparison of binding of biotinylated C1 peptide to IGF-1 R and IR is shown in Figure 64, which indicates that binding of C1 to IGF-1 R is at the nM range while binding to IR is at the micromolar range. A series of unlabeled peptides or soluble rVab were added to test competition binding to IR (Figure 65). H2C peptide at 30 µM appears to compete for binding to IR with biotinylated peptides from group 1 (Formula 1 motif) (20D1 and 20D3) and the two A6-based peptides (C1 and H2) but not compete with peptides from group 2 (Formula 6 motif) (20A4 and D8), group 3 (Formula 2 motif) (20C11) or the IGF peptide A9. The 33 F7 soluble rVab antibody competes with group 1 and 2 peptides as well as C1 peptide, however, it does not compete with 20C11 or 2A9. Figure 66 shows that H2C competition with biotinylated peptides, 20D3, H2, and C1, binding to IR is dose-dependent. C1C peptide also competes with C1 for IR binding (Figure 67).

### Example 23: Competition for Binding to rVab 12H10 by Peptides and MBP-Peptide Fusion Proteins

Several peptides and four MBP-peptide fusion peptides were tested for competition of binding to IR with soluble rVab 12H10. Figure 68 shows that C1 and H2C at 30 µM inhibit binding to 40-50% of control and C1C at 30 µM inhibit to 60%. B6 and growth hormone do not compete with binding of 12H10 to IR. Four MBP-peptide fusion proteins (D10, 20A4, E7 and H8) all inhibit binding of 12H10 to IR to 20-30 % of control (Figure 69).

### Example 24: Effects of Small Organic Molecules on IR Phosphorylation

Organic molecules positive for binding to IGF-1 R and negative controls can be tested for their effects on phosphorylation of insulin receptor.

### Example 25: Method for Determination of Insulin Receptor Binding of Peptides

In other insulin binding assays, IR was incubated with ¹²⁵l-labeled insulin at various concentrations of test substance and the K_{d} was calculated. According to this method, human insulin receptor (HIR) or human IGF-1 receptor (HIGF-1R) was purified from transfected cells after solubilization with Triton X-100. The assay buffer contains 100 mM HEPES (pH 7.8), 100 mM NaCl, 10 mM MSG, 0.5% human serum albumin, 0.2% gammaglobulin and 0.025% Triton X-1 00. The receptor concentration was chosen to give 30-60% binding of 2000 cpm (3 pM) of its ¹²⁵l-labeled ligand (TyrA14- ¹²⁵ l-HI or Tyr31-¹²⁵l-IGF1) and a dilution series of the substance to be tested was added. After equilibration for 2 days at 4°C, each sample (200 µl) was precipitated by addition of 400 µl 25% PEG 6000, centrifuged, washed with 1 ml 15% PEG 6000, and counted in a gamma-counter.

The insulin/IGF-1 competition curve was fitted to a one-site binding model and the calculated parameters for receptor concentration, insulin affinity, and non-specific binding were used in calculating the binding constants of the test substances. Representative curves for insulin and IGF-1 are shown in Figures 71A-71N.

The sequences of certain peptides analyzed are shown in Table 12, except for peptides D125 and D126. Synthetic peptides are numbered D1XX. D117K is an analog of D117 with an extra N-terminal lysine added for facilitate solubility. Peptides produced recombinantly by phage are indicated as D1XXA.

The peptides are all biotinylated in the side chain of the C-terminal lysine (except D117A). The peptides produced recombinantly are C-terminal acids, whereas the synthetic peptides are C-terminal amides.

The results of the binding and phosphorylation assays are shown in Table 13.

**TABLE 12**

| **Name** | **Sequence** | **Motif** |
|---|---|---|
| D101 | KIGGQGQHQDGNFYDWFVEALAKK | 1 |
| D102 | KVLQARHGCDSVSDCFYEWFAKK | 1 |
| D103 | KWSALLSVMDTGFYAWFDDAVKK | 1 |
| D104 | KGHSWALVRHVDRLFYEWFDLKK | 1 |
| D105 | KRDKPTDQEEQNWSFYEWFRHKK | 1 |
| D106 | KVFWNCRSQQLDFYEWFEQAAKK | 1 |
| D107 | KLESH\/VPQAALDRLFYSWFSKK | 1 |
| D108 | KFYGWFSRQLSLTPRDDWGLPKK | 1 |
| D109 | KSAPGLVSNKQDGLFYSWFREKK | 1 |
| D110 | KRGGGTFYEWFESALRKHGAGKK | 1 |
| D111 | KDPERMQSDVGFYEWFRAAVGKK | 1 |
| D112 | DYKDCWARPCGDAANFYDWFVQQASKK | 1 |
| D113 | DYKDVTFTSAVFHENFYDWFVRQVSKK | 1 |
| D114 | SAKNFYDWFVKK | 1 |
| D115 | ADKNFYDWFMAAKK | 1 |
| D116 | DYKDLCQSWGVRIGWLAGLCPKK | 9 |
| D117 | FHENFYDWFVRQVSKK | 1 |
| D117K | KFHENFYDWFVRQVSKK | 1 |
| D118 | DYKDFYDAIDQLVRGSARAGGTRDKK | 2 |
| D119 | KDRAFYNGLRDLVGAVYGAWDKK | 2 |
| D120 | KVRGFQGGTVWPGYEWLRNAAKK | 10 |
| D121 | KSMFVAGSDRWPGYGVLADWLKK | 10 |
| D122 | KEIEAEWGRVRCLVYGRCVGGKK | 10 |
| D123 | KWLDQEWAWVQCEVYGRGCPSKK | 6 |
| D124 | KHLCVLEELFWGASLFGYCSGKK | 4 |
| | | |
| D101A | KIGGQGQHQDGNFYDWFVEALAKK | 1 |
| D102A | KVLQARHGCDSVSDCFYEWFAKK | 1 |
| D112A | DYKDCWARPCGDAANFYDWFVQQASKK | 1 |
| D113A | DYKDVTFTSAVFHENFYDWFVRQVSKK | 1 |
| D117A | FHENFYDWFVRQVSKK | 1 |
| D119A | KDRAFYNGLRDLVGAVYGAWDKK | 2 |
| D122A | KEIEAEWGRVRCLVYGRCVGGKK | 10 |
| D123A | KWLDQEWAWVQCEVYGRGCPSKK | 6 |
| D124A | KHLCVLEELFWGASLFGYCSGKK | 4 |

**TABLE 13**

| **Name** | **K_{d} (µM) HIR** | **K_{d}** (**µM) HIGF1R** | **Ratio** | **Autophosph. Blot** |
|---|---|---|---|---|
| D101 | 0.51 | 13 | 25 | - |
| D102 | 1.2 | 7.4 | 6.2 | - |
| D103 | 0.74 | 15 | 20 | - |
| D104 | 20 | >20 | | - |
| D105 | 2.8 | 12 | 4.3 | - |
| D106 | 0.97 | 6.2 | 6.4 | - |
| D107 | 1.1 | 9.7 | 8.8 | + |
| D108 | 2.3 | 19 | 8.3 | - |
| D109 | 3.6 | 12 | 3.3 | - |
| D110 | 0.84 | 1.4 | 1.7 | - |
| D111 | 0.62 | 3.2 | 5.2 | - |
| D112 | 0.49 | 0.05 | 0.1 | - |
| D113 | 0.75 | 5.4 | 7.2 | - (prec) |
| D114 | 8.1 | >20 | >2.5 | 0 |
| D115 | 8.1 | >20 | >2.5 | 0 |
| D116 | 4.4 | 8.1 | 1.8 | 0 |
| D117 | 0.70 | 6.1 | 8.6 | + |
| D117K | 0.82 | 9.1 | 11.1 | |
| D118 | 0.25 | 1.3 | 5.2 | + |
| D119 | 4.5 | 13 | 2.9 | + |
| D120 | 0.37 | 2.2 | 5.9 | - |
| D121 | 1.1 | 7.4 | 6.7 | - |
| D122 | 1.2 | >20 | >17 | 0 |
| D123 | 0.55 | 16 | 29 | 0 |
| D124 | 0.04* | 8.2 | 200 | - |
| | | | | |
| D101A | 0.27 | 11.0 | 41 | |
| D102A | 0.97 | 16.0 | 16 | |
| D112A | 0.19 | 0.02* | 0.1 | |
| D113A | | | | |
| D117A | 0.60 | 5.1 | 8.5 | |
| D119A | 3.0 | 2.5 | 0.8 | |
| D122A | 1.0 | >20 | >20 | |
| D123A | 1.3 | >20 | >15 | |
| D124A | 0.09* | >20 | >200 | |
| D125A | 2.6 | >20 | >8 | |
| D126A | 1.4 | 18 | 13 | |

### Example 26: Determination of Insulin Agonist Activity Based On ³H-Glucose Uptake into Adipocytes

Insulin increases uptake of ³H glucose into adipocytes and its conversion into lipid. Incorporation of ³H into the lipid phase was determined by partitioning of lipid phase into a scintillant mixture, which excludes watersoluble ³H products. The effect of compounds on the incorporation of ³H glucose at a sub-maximal insulin dose was determined, and the results expressed as increase relative to full insulin response. The method was adapted from Moody *et al.* (1974).

Mouse epididymal fat pads were dissected out, minced into degradation buffer (Krebs-Ringer 25 mM HEPES, 4% HSA, 1.1 mM glucose, 0.4 mg/ml Collagenase Type 1, pH 7.4), and degraded for up to 1.5 h at 36.5°C. After filtration, washing (Krebs-Ringer HEPES , 1% HSA) and resuspension in assay buffer (Krebs-Ringer HEPES, 1% HSA), cells were pipetted into 96-well Picoplates (Packard), containing test solution and approximately an ED₂₀ insulin. The assay was started by addition of ³H glucose (Amersham TRK 239), in a final concentration of 0.45 mM glucose. The assay was incubated for 2 h, 36.5°C, in a Labshaker incubation tower, 400 rpm, then terminated by the addition of Permablend/Toluene scintillant (or equivalent), and the plates sealed, before standing for at least 1 h and detection in a Packard Top Counter or equivalent. A full insulin standard curve (8 dose) was run as control on each plate. Data are presented graphically, as effect of compound on an (approx) ED₂₀ insulin response, with data normalized to a full insulin response. The assay can also be run at basal or maximal insulin concentration. Representative dose-response curves for insulin and IGF-1 are shown in figures 71A-71Z; 71A2-71Z2; 71A3-B3. Qualitative references are shown in Table 14.

**TABLE 14**

| **Comp.** | **Resp.** | **#expts** | **ED₅₀** | **Comments** |
|---|---|---|---|---|
| **1** | **2** | **3** | **4** | **5** |
| D101 | 0 | 4 | | |
| D102 | 0 | 2 | | Precipitates |
| D103 | 0 | 2 | | |
| D104 | 0 | 2 | | Precipitates |
| D105 | 0 | 2 | | |
| D106 | 0 | 2 | | Precipitates |
| D107 | -2 | 2 | | |
| D108 | -1 | 2 | | |
| D110 | -1 | 2 | | |
| D110 | -2 | 4 | | |
| D111 | 0 | 2 | | |
| D112 | 0 | 5 | | Precipitates |
| D113 | +2 | 7 | Approx 20 µM | Insoluble, especially afterfreeze-thaw, resulting in inconsistent results. Some response at basal insulin. |
| D114 | 0 | 2 | | |
| D115 | 0 | 3 | | |
| D116 | +2 | 4 | >20µM | Slight effect at basal insulin |
| D117 | +2 | 8 | Approx 20 µM | Precipitates. Under assay conditions, soluble at least up to 20µM (no ppt in microscope, low magnification). Some response at basal insulin. |
| D117K | +2 | 2 | >20µM | |
| D118 | +2 | 5 | Approx 20 µM | Biphasic dose response curve (needs repeating) |
| D119 | +1 | 2 | | |
| D120 | -1 | 4 | | |
| D121 | -1 | 3 | | |
| D122 | -1 | 6 | | |
| D123 | -1 | 5 | | Precipitates |
| D124 | 0 | 5 | | Precipitates |
| D125 | 0 | 2 | | |
| D126 | 0 | 2 | | |

| | | | | |
|---|---|---|---|---|
| ¹ Includes series "A" e.g. D101A ² Subjective ranking, on a scale of -2 (antagonist) to +2 (agonist) ³ Includes experiments run at basal and sub maximal insulin concentrations ⁴ Estimated, not calculated values. ⁵ "Precipitates" indicates precipitate in diluted stock prior to adding to assay. May be soluble under assay conditions | | | | |

### Results:

The binding assays showed that most of the peptides completely inhibited insulin binding to HIR with lC₅₀-values ranging from 0.3 to 20 µM. One peptide (D124) was active at lower concentration but only displaced insulin partially (see Figure 71). One peptide (D112) had high affinity for HIGF-1R, but all the others showed 2-20 fold selectivity for HIR (see Figure 71).

In the effect assay (FFC), several of the peptides had no effect, some were antagonists, and a few were agonists reaching a response comparable to that of full insulin stimulation. The ED₅₀ for the best peptides (D113 and D117) was around 20-30 µM.

Despite a right shifted does response curve relative to insulin, these peptides represent the first non-insulin compounds ever found to elicit a maximal insulin response by binding to the insulin receptor. Such peptides may be useful for development as therapeutics themselves.

The peptides could also be useful as leads for further characterization of molecular requirements for binding to and activation of IR, and/or as tools for identification of the mechanisms involved in the activation.

Analysis of affinity and activity of another group of peptides is shown in Table 15. In addition to presenting data on the single chain or looped peptide, Table 15 also reports data showing high affinity binding of certain dimers.

**TABLE 15**

| **Name** | **Sequence** | **HIR affinity mol/l** | **FFC** |
|---|---|---|---|
| S105 | FHENFYDWFVRQVAKK-NH₂ | 3.1*10⁻⁷ | ++ |
| S106 | FHENFYDWFVRQASKK-NH₂ | 4.2* 10⁻⁷ | ++ |
| S107 | FHENFYDWFVRAVSKK-NH₂ | 10.0*10⁻⁷ | + |
| S108 | FHENFYDWFVAQVSKK-NH₂ | 7.5* 10⁻⁷ | + |
| S109 | FHENFYDWFARQVSKK-NH₂ | 2.3*10⁻⁷ | ++ |
| S110 | FHEAFYDWFVRQVSKK-NH₂ | 2.2*10⁻⁷ | ++ |
| S111 | FHANFYDWFVRQVSKK-NH₂ | 3.3* 10⁻⁷ | 0 |
| S112 | FAENFYDWFVRQVSKK-NH₂ | 6.1*10⁻⁷ | + |
| S113 | AHENFYDWFVRQVSKK-NH₂ | 5.9*10⁻⁷ | + |
| S114 | fhenfydwfvrqvskk | 8.3*10⁻⁶ | 0 |
| S115 | EFHENFYDWFVRQVSEE | 6.5*10⁻⁷ | + |
| S116 | FHENFYGWFVRQVSKK | 1.4*10⁻⁶ | ++ |
| S117 | HETFYSMIRSLAK | 2.7*10⁻⁶ | 0 |
| S118 | SDGFYNAIELLS | 2.4*10⁻⁶ | + |
| S119 | SLNFYDALQLLAKK | 1.8*10⁻⁶ | 0 |
| S120 | HDPFYSMMKSLLK | 2.0*10⁻⁶ | 0 |
| S121 | NSFYEALRMLSSK | 3.1*10⁻⁶ | 0 |
| S122 | HPTSKEIYAKLLK | 9.3*10⁻⁶ | 0 |
| S123 | HPSTNQMLMKLFK | 1.6*10⁻⁵ | 0 |
| S124 | HPPLSELKLFLIKK | 2.3*10⁻⁵ | 0 |
| S125 | HAPLSVLVQALLKK | | 0 |
| S126 | HPSLSDMRWILLK | | |
| S127 | WSDFYSYFQGLD | 1.2*10⁻⁶ | 0 |
| S128 | D117-Dap(D117) | 1.1*10⁻⁶ | ++ |
| S129 | SSNFYQALMLLS | 2.9*10⁻⁶ | 0 |
| S131 | D117-Dap(CO-CH₂-O-NH₂) | 1.2*10⁻⁶ | + |
| S137 | HENFYGWFVRQVSKK | 7.7*10⁻⁷ | 0 |
| S145 | D117-Lys(D117) | 1.5*10⁻⁶ | ++ |
| S147 | D117-b-Ala-Lys(D117) | 9.3*10⁻⁷ | ++ |
| S148 | D117-b-Ala-Dap(b-Ala-D117) | 1.1*10⁻⁶ | ++ |
| S149 | D117-Gly-Lys(Gly-D117) | 2.0*10⁻⁶ | ++ |
| S150 | D117-b-Ala-Lys(b-Ala-D117) | 6.2*10⁻⁷ | ++ |
| S152 | D117-Dab(D117) | 5.2*10⁻⁵ | + |
| S153 | D117-Orn(D117) | 3.9*10⁻⁶ | + |
| S154 | D117-Dap(b-Ala-D117) | 3.6*10⁻⁶ | + |
| S155 | D117-b-Ala-Orn(b-Ala-D117) | 2.5*10⁻⁶ | ++ |
| S156 | 1-(Thia-b-Ala-D117)₂ | | |
| S157 | FHENFYDWFVRQVS | | |
| S158 | FHENFYDWFVRQVSK | 8.1*10⁻⁷ | + |
| S159 | FHENFYDWFVQVSK | | |
| S160 | FHENFYDWFWSK | | |
| S161 | FHENFYDWFVSK | | |
| S162 | FHENFYDWFVK | | |
| S165 | FYDWF-NH₂ | >2*10⁻⁵ | 0 |
| S166 | FYDWFKK-NH₂ | >2*10⁻⁵ | 0 |
| S167 | AFYDWFAKK-NH₂ | >2*10⁻⁵ | (-) |
| S168 | AAAAFYDWFAAAAAKK-NH₂ | 3.8*10⁻⁶ | 0 |
| S169 | (D117)₂⁻12 | 5.8*10⁻⁷ | ++ |
| S170 | (Cys-Gly-D117)₂ | 7.0*10⁻⁷ | +++ |
| S171 | Cys-Gly-D117 | 2.9*10⁻⁶ | +++ |
| S172 | (D117)₂-14 | 4.8*10⁻⁶ | +++ |
| S173 | LDALDRLMRYFEERPSL-NH₂ | 1.2*10⁻⁶ | 0 |
| S174 | PLAELWAYFEHSEQGRSSAH-NH₂ | 1.6*10⁻⁵ | 0 |
| S175 | GRVDWLQRNANFYDWFVAELG-NH₂ | 2.3*10⁻⁷ | +++ |
| S176 | NGVERAGTGDNFYDWFVAQLH-NH₂ | 4.7*10⁻⁷ | + |
| S177 | EHWNTVDPFYFTLFEWLRESG-NH₂ | 2.7*10⁻⁶ | 0 |
| S178 | EHWNTVDPFYQYFSELLRESG-NH₂ | 1.3*10⁻⁷ | ++ |
| S179 | QSDSGTVHDRFYGWFRDTWAS-NH₂ | 5.4*10⁻⁷ | + |
| S180 | AFYDWFAK-NH₂ | >2*10⁻⁵ | 0 |
| S181 | AFYDWFA-NH₂ | >2*10⁻⁵ | 0 |
| S182 | AFYDWF-NH₂ | >2*10⁻⁶ | 0 |
| S183 | FYDWDA-NH₂ | >2*10⁻⁵ | 0 |
| S184 | Ac-FYDWF-NH₂ | >2*10⁻⁵ | 0 |
| S203 | Lig-FHENFYDWFVRQVSKK | | |
| S204 | Lig-GGGFHENFYDWFVRQVSKK | | |
| S205 | FHENFYDWFVRQVSKKGGG-Lig | | |
| S206 | Lig-CAWPTYWNCG | | |
| S207 | ACAWPTYWNCG-Lig | | |
| S208 | ACAWPTYWNCGGGG-Lig | | |
| S209 | Lig-SDGFYNAIELLS | | |
| S210 | SDGFYNAIELLS-Lig | | |
| S211 | SDGFYNAIELLSGGG-Lig | | |
| S212 | KHLCVLEELFWGASLFGYCSGKK-Lig | | |
| S213 | AFYDWFAKK-Lig | | |
| S214 | AFYEWFAKK-NH₂ | >2*10⁻⁵ | 0 |
| S215 | AFYGWFAKK-NH₂ | >2* 10⁻⁵ | 0 |
| S216 | AFYKWFAKK-NH₂ | >2*10⁻⁵ | 0 |
| S217 | (SDGFYNAIELLS-Lig)₂-14 | 3.9*10⁻⁸ | ++ |
| S218 | (AFYDWFAKK-Lig)₂-14 | 1,1*10⁻⁵ | 0 |
| S219 | FHENAYDWFVRQVSKK | >2*10⁻⁵ | 0 |
| S220 | FHENFADWFVRQVSKK | >2*10⁻⁵ | 0 |
| S221 | FHENFYAWFVRQVSKK | 1.1*10⁻⁶ | (+) |
| S222 | FHENFYDAFVRQVSKK | >2*10⁻⁵ | 0 |
| S223 | FHENFTDWAVRQVSKK | >2* 10⁻⁵ | 0 |
| S224 | FQSLLEELVWGAPLFRYGTG | >2*10⁻⁵ | 0 |
| S225 | PLCVLEELFWGASLFGQCSG | | |
| S226 | QLEEEWAGVQCEVYGRECPS | 1.6*10⁻⁶ | |
| S227 | Cys-(Gly)₂-D117 | 5.1*10⁻⁷ | ++ |
| S228 | (Cys-(Gly)₂-D117)₂ | 3.6* 10⁻⁷ | ++ |
| S229 | (S210)-14-(S212) | 4.4*10⁻⁹ | 0 |
| S230 | (S131)-14-(S212) | | |
| S231 | (S205)₂-14 | 2.7*10⁻⁷ | + |
| S232 | (S204)₂-14 | 3.8*10⁻⁷ | +++ |
| S233 | (S131)-14-(S210) | 2.6*10⁻⁷ | + |
| S234 | RVDWLQRNANFYDWFVAELG | 1.3*10⁻⁷ | ++ |
| S235 | VDWLQRNANFYDWFVAELG | 5.3*10⁻⁸ | ++ |
| S236 | DWLQRNANFYDWFVAELG | 1.0*10⁻⁷ | ++ |
| S237 | WLQRNANFYDWFVAELG | 8.5*10⁻⁷ | 0 |
| S238 | LQRNANFYDWFVAELG | 8.5*10⁻⁷ | 0 |
| S239 | QRNANFYDWFVAELG | 1.3*10⁻⁶ | 0 |
| S240 | RNANFYDWFVAELG | 1.4*10⁻⁶ | |
| S241 | NANFYDWFVAELG | 1.6*10⁻⁶ | |
| S242 | ANFYDWFVAELG | 2.0*10⁻⁶ | |
| S243 | NFYDWFVAELG | 2.0*10⁻⁶ | |
| S244 | GRVDWLQRNANFYDWFVAELG-Lig | 2.2* 10⁻⁷ | ++ |
| S245 | Lig-GRVDWLQRNANFYDWFVAELG | 2.2*10-⁻⁷ | + |
| S246 | (S208)-14-(S131) | 5.0*10⁻⁶ | |
| S247 | (S208)-14-(S209) | | |
| S248 | GRVDWLQRNANFYDWFVAEL | 6.3*10⁻⁸ | ++ |
| S249 | GRVDWLQRNANFYDWFVAE | 7.4*10⁻⁷ | 0 |
| S250 | GRVDWLQRNANFYDWFVA | 8.9*10⁻⁶ | 0 |
| S251 | GRVDWLQRNANFYDWFV | 5.6* 10⁻⁶ | |
| S252 | 14-(SDGFYNAIELLS-Lig)₂ | 4.4* 10⁻⁷ | 0 |
| S253 | (GRVDWLQRNANFYDWFVAELG)-14 | 2.2*10⁻⁸ | ++ |
| S254 | 14-(GRVDWLQRNANFYDWFVAE LG) | | |
| S255 | (SDGFYNAIELLSGGG)₂-14 | 1.6*10⁻⁶ | 0 |
| S256 | H-Acy-CLEE-w-GASL-Tic-QCSG-NH₂ | 9.0*10⁻⁶ | (-) |
| S257 | RWPNFYGYFFSLLTHFS-NH₂ | 1.4*10⁻⁵ | 0 |
| S258 | HYNAFYEYFQVLLAETW- NH₂ | | |
| S259 | EGWDFYSYFSGLLASVT-NH₂ | 7.7*10⁻⁶ | 0 |
| S260 | LDRQFYRYFQDLLVGFM-NH₂ | 2.3*10⁻⁶ | 0 |
| S261 | WGRSFYRYFETLLAQGI-NH₂ | >2*10⁻⁵ | 0 |
| S262 | PLCFLQELFGGASLGGYCSG-NH₂ | 1.9*10⁻⁵ | 0 |
| S263 | WLEQERAWIWCEIQGSGCRA-NH₂ | >2*10⁻⁵ | 0 |
| S264 | IQGWEPFYGWFDDWAQMFEE-NH₂ | 1.9*10⁻⁷ | 0 |
| S265 | TGHRLGLDEQFYWWFRDALSG-NH₂ | 1.1*10⁻⁷ | 0 |
| S266 | H-Abu-CLEE-w-GASL-Tic-QCSG-NH₂ | >2* 10⁻⁵ | 0 |
| S267 | 14-(Dap-CAWPTYWNCG)₂ | | |
| S268 | RDHypFYDWFDDi-NH₂ | 4.5*10⁻⁷ | 0 |
| S273 | S131-14-S209 | 1.5*10⁻⁶ | + |
| S274 | S294-14-S210 | | |
| S275 | S295-14-S210 | | |
| S276 | S294-14-204 | | |
| S277 | S295-14-S204 | | |
| S278 | GFREGQRWYWFVAQVT-NH₂ | >2*10⁻⁵ | 0 |
| S279 | VASGHVLHGQFYRWFVDQFALEE-NH₂ | | |
| S280 | VGDFCVSHDCFYGWFLRESMQ-NH₂ | | |
| S281 | DLRVLCELFGGAYVLGYCSE-NH₂ | 1.1*10⁻⁵ | 0 |
| S282 | HLSVGEELSWWVALLGQWAR-NH₂ | >2*10⁻⁵ | 0 |
| S283 | APVSTEELRWGALLFGQWAG-NH₂ | >2*10⁻⁵ | 0 |
| S284 | ALEEEWAWVQVRSIRSGLPL-NH₂ | >2*10⁻⁵ | 0 |
| S285 | WLEHEWAQIQCELYGRGCTY-NH2 | 8.3*10⁻⁷ | |
| S286 | AAVHEQFYDWFADQYEE-NH₂ | | |
| S287 | QAPSNFYDWFVREWDEE-NH₂ | 5.9*10⁻⁶ | 0 |
| S288 | QSFYDYIEELLGGEWKK-NH₂ | 4.3*10⁻⁶ | 0 |
| S289 | DPFYQGLWEWLRESGEE-NH₂ | >2*10⁻⁵ | 0 |
| S290 | (S204)₂-7 | 9.0*10⁻⁷ | ++ |
| S291 | (s204)₂₋9 | 1.2*10⁻⁶ | ++++ |
| S292 | (s204)₂-12 | 7.5*10⁻⁷ | ++ |
| S293 | (S204)₂-13 | 1.2*10⁻⁷ | ++ |
| S294 | DWLQRNANFYDWFVAEL-Lig | 1.3*10⁻⁷ | ++ |
| S295 | Lig-DWLQRNANFYDWFVAEL | 4.8*10⁻⁷ | + |
| S296 | (S209)₂-9 | | |
| S297 | (S210)₂-9 | | |
| S298 | LigKHLCVLEELFWGASLFGYCSGKKKK | | |
| S299 | KHLCVLEELFWGASLFGYCSGKKKK-Lig | | |
| S300 | (S294)₂-14 | 5.0*10⁻⁸ | +++ |
| S301 | (S295)₂-14 | 6.4*10⁻⁷ | + |
| S302 | S-D-G-F-Y-N-A-Acy-E-L-L-S | | |
| S303 | S-G-P-F-Y-E-E-Acy-E-L-L-W-Aib | | |
| S304 | G-G-S-F-Y-D-D-Acy-E Aib-L-W-Aib | | |
| S305 | N-Aib-P-F-Y-D-E-Acy-D-E-Cha-W-Aib | | |
| S306 | GRVDWLQRNANFYDWFVAEAcyG-NH₂ | | |

| | | | |
|---|---|---|---|
| 7, 9, 12, 13, and 14 represent specific chemical linkers (see Table 18) FFC: 0 is no effect, + is agonist, - is antagonist | | | |

### Example 27: Formula 8 synthetic Peptides with Their Affinities for the Human Insulin Receptor (HIR)

A commercial phage display peptide library (New England Biolabs Ph.D.-C7C Disulfide Constrained Peptide Library) was screened for members which bind to IR.

### A. Identification of IR Binding Phage

Binding of phage with displayed peptides was detected by ELISA assay. Plates were coated with anti-FC antibody for 2 h at RT or overnight at 4°C. Nonspecific sites were blocked with skim milk (2%) for 1 h at RT. 'slR-Fc, a modified form of IR in which the cytoplasmic region is substituted with an IgG-Fc fragment (Bass *et al.,* 1990), was then added to the wells for 2 h at RT. Phage were then added to wells and incubated with or without competing peptides for 2 h at RT. Binding was detected with an anti-phage HRP antibody which was added to the wells and incubated for 2.5 h. at RT. OPD (o-phenylenediame) color reaction was detected between 5 and 10 min.

### B. Characterization of Phage Displayed Peptides

Fifteen different phage were isolated from a linear 12-mer peptide library (New England Biolabs) panned against a dimer of the LI portion of IR (IR Δ703) (Kristensen *et al.,* 1998) Table 16. The displayed sequences were divided into three groups based on their consensus sequences which correspond to Formula motifs 1, 2 and 7. As can be seen in Table 16, the peptides of motif 7 bind strongly to sIR but not sIGF-1 R-FC.

The ability of certain peptides identified in the phage library to compete with other peptides is shown in Table 17 below.

J101 (see Figure 8), the peptide expressed by phage CP42, and containing the Formula 8 motif was found to displace insulin from IR with an IC₅₀ of about 5 µm and to be an antagonist in the receptor autophosphorylation and fat cell assays. J101 also does not bind the IR Δ703 construct and is not displaced from IR by insulin. Accordingly, J101, may bind IR outside of the insulin binding site. J101, which contains two cysteine residues is likely to have a cyclic structure.

Phage displaying IR binding peptides were also identified by binding phage to plates coated with sIR-Fc as discussed above and washing away non-binding phages. Binding phage were eluted with glycine-HCI, pH 2.2 for 10 min.

The sequences of the displayed peptides which bind IR are shown in Figure 8.

A few of the peptides (e.g. J101 and J115) (Figure 8) were tested in the fat cell assay and all were full antagonists.

**TABLE 16**

| | | | | | **Relative binding to** | | **Formula** |
|---|---|---|---|---|---|---|---|
| IM no. | Isolate | Displayed peptide sequence | No. Found | | **sIR** | **slGF-1 R-Fc** | **Motif** |
| IM445 | Δ-12-3#6: | APTFYAWFNQQT-GGGS. | 1 | [∼J229] IC₅₀~2,4 µm | +++ | + | 1 |
| IM447 | Δ-12-3#45: | SFYEAIHQLLGV-GGGS. | 23 | [∼J227] | +++ | +++ | 2 |
| IM460 | Δ-12-3*#76: | NSFYEALRMLSS-GGGS. | 2 | lC₅₀-6,4 µm | ++ | (+) | 2 |
| IM453 | Δ-12-3#112: | SLNFYDALQLLA-GGGS. | 1 | | ++++ | ++ | 2 |
| IM466 | Δ-12-3* #146 | SSNFYQALMLLS-GGGS. | 1 | | +++ | ++ | 2 |
| IM448 | Δ-12-3#40 | SDGFYNAIELLS-GGGS. | 3 | | + | (+) | 2 |
| IM446 | Δ-12-3#24 | HETFYSMIRSLA-GGGS. | 60 | | ++++ | +++ | 2 |
| IM455 | Δ-1 2-3* #1 | HDPFYSMMKSLL-GGGS. | 1 | | +++ | ++ | 2 |
| IM465 | Δ-12-3#193 | WSDFYSYFQGLD-GGGS. | 1 | | +++ | (+) | 2 |
| | | | | | | | |
| ≥50% Consensus: . | | __FY_ Al__L_ | | | | | |
| | | | | | | | |
| IM452 | Δ-12-3#23: | HPPLEHLKAFLL-GGGS. | 4 | [∼J228] | +++++ | ÷ | 7 |
| IM451 | Δ-12-3#34: | HPPLSELKLFLI-GGGS.. | 33 | lC₅₀∼24 µm S124 | +++++ | ÷ | 7 |
| IM459 | Δ-12-3*#60: | HPSLSDMRWILL-GGGS. | 2 | | ++++ | ÷ | 7 |
| IM458 | Δ-12-3*#30: | HAPLSVLQALL-GGGS. | 2 | | ++ | ÷ | 7 |
| IM449 | Δ-12-3#43: | HPTSKEIYAKLL-GGGS. | 14 4 | | ++ | ÷ | 7 |
| IM450 | Δ-12-3#28: | HPSTNQMLMKLF-GGGS. | 40 | | ++ | ÷ | 7 |
| | | | | S122 | | | |
| ≥50% Consensus: | | HPPLS__L_LL | | 2123 | | | |

**TABLE 17**

| Phage | Sequence | D103 | D118 | D119 | D120 | D121 | D122 | D123 | D124 | Insulin |
|---|---|---|---|---|---|---|---|---|---|---|
| | Formula Motif | 1 | 2 | 2 | 10 | 10 | 10 | 6 | 4 | |
| IM332(~J101) | Cyclic | % 85 | %100 | % 100 | % 100 | % 100 | % 100 | % 100 | +71 | % 98 |
| IM445(~J229) | APTFYAWFNQQT | ++ 0 | ++ 2 | ++0 | ++ 0 | ++ 0 | %100 | % 100 | + 68 | ++ 11 |
| IM447(~J227) | SFYEAIHQLLGV | ++ 0 | ++0 | ++0 | ++ 0 | ++ 0 | %85 | + 58 | + 46 | ++3 |
| IM452(~J228) | HPPLEHLKAFLL | ++ 0 | ++ 0 | ++ 10 | ++ 0 | ++0 | % 95 | nd | % 84 | +26 |
| IM242(~ILPI) | ILPI | +37 | ++ 17 | +46 | +30 | +66 | +57 | +55 | ++19 | ++ 0 |

| | | | | | | | | | | |
|---|---|---|---|---|---|---|---|---|---|---|
| % :>80% signal (not displaced) +: 20-70% signal ++: <20% signal (fully displaced) | | | | | | | | | | |

### EXAMPLE 28: PREPARATION OF THE DIMERS

### A. Materials

Generally, suitably protected N-Fmoc (fluorenylmethoxycarboxyl)-amino acids were purchased from Novabiochem (Switzerland), 1-hydroxy-7-azabenzotriazole (HOAt) from Perspective Biosystems and *N,N'-*diisopropylcarbodiimide (DIC) from Fluka. The molecular weights of the peptides were determined using matrix-assisted laser desorption time of-flight mass spectroscopy (MALDI-MS), recorded on a Voyager-DE (Perseptive Biosystems). A matrix of sinapinic acid was used. Analytical and semi-preparative high-pressure liquid chromatography (HPLC) were performed using a Waters RCM 8 x 10 module and with a C-18 column (19 x 300 mm) and a C-18 column (25 x 300 mm), respectively, at 40°C. The solvent system for both analytical and semi-preparative HPLC was buffer A; 0.1 % TFA in water and buffer B; 0.07% TFA in 100% and UV detection was at 215 nm. The gradient for analytical HPLC (1.5 ml/min); a linear gradient of 5-90% buffer B over 25 min and semi-preparative HPLC (4 ml/min); an isocratic gradient of 20% buffer B over 5 min, followed by a linear gradient of 20-60% buffer B over 40 min.

### B. Solid-Phase Peptide Synthesis and Analysis of the D117 Monomer(FHENFYDWFVRQVSKK-Dap(CO-CH2-O-NH2)

The peptide monomer available for ligation was synthesized manually in plastic syringes using a preloaded Rink amide linker (RAM)-TentaGel (0.26 mmol/g). Fully protected N-Fmoc amino acids (3 equiv.) were used and the temporary Fmoc protecting group was removed after each cycle by 30% piperidine in *N-*methylpyrrolidone (NMP). The natural amino acids were coupled as their free acids in NMP using DIC (3 mol equiv.) and HOAt (3 mol equiv.) as coupling additive.

First, Fmoc-Dap(Alloc) was coupled as described above. The alloc group was then removed by Pd(0) (3 mol equiv.) in CHCl₃/AcOH/N-methylmorpholine (37:2:1, v/v/v) under helium. After 2 h. at RT, the resin was washed with 5% in NMP containing 2% diethyldithiocarbamide, Na salt. Finally, the resin was washed with NMP containing HOBt (hydroxybenzotriazole). The protected oxyamino acetic acid (3 mol equiv.) was then coupled on the side-chain of Dap (diaminopropionic acid). The completion of all the acylation reactions was monitored visually by the use of bromophenol blue. Between the Fmoc-deprotection and the acylation reaction, the resin was washed with NMP (x 6).

After synthesis, the peptide was washed with DCM (dichloromethane) (x 3). The peptides were cleaved simultaneously from the resin and the side-chain protecting groups were removed by treatment with 95% aqueous TFA containing triisopropylsilan (TIS) (4 molar equiv.) for 1.5 h. The resin was rinsed with 95% aqueous acetic acid (x 4). Both TFA and acetic acid were evaporated and the peptide was finally precipitated in diethyl ether and lyophilized overnight. The peptide was both analyzed by analytical HPLC and MALDI-MS. Analysis by MALDI-MS; m/z 2287.5 (M + H)+ (requires m/z, 2288.3) confirmed the expected product.

To the peptide monomer, FHENFYDWFVRQVSKK-Dap(CO-CH2-O-NH2) (9.1 mg, 3.9 mol) was added the dialdehyde linker (0.81 mol) dissolved in 80% DMSO (aqueous) (28 I). The pH was then adjusted to 5 with solid sodium acetate. The solution was left overnight at 37°C and progress of the reaction was monitored by RP-HPLC. The formed dimer (see Table 18) was purified by semi-preparative HPLC. Analysis by MALDI-MS confirmed the expected product (see Table 18). The molecular weights and inter peptide distance of various linkers is shown below.

**TABLE 18**

| **Structure** | **Number** | **MW** | **MW (- 2H₂O)** |
|---|---|---|---|
| 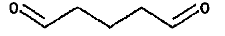 | **1** | **100.1** | **64.1** |
| 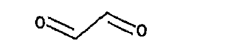 | **2** | **58.04** | **22.04** |
| 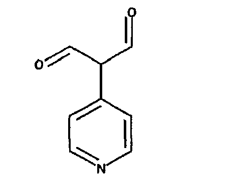 | **3** | **149.15** | **113.15** |
| 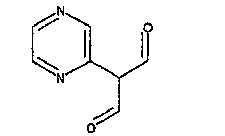 | **4** | **150.14** | **114.14** |
| 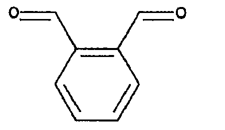 | **5** | **134.13** | **98.13** |
| 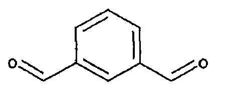 | **6** | **134.13** | **98.13** |
| 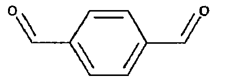 | **7** | **134.13** | **98.13** |
| 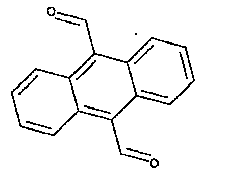 | **8** | **234.25** | **198.25** |
| 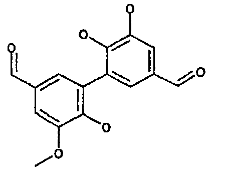 | **9** | **302.3** | **266.3** |
| 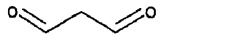 | **10** | **72.06** | **36.06** |
| 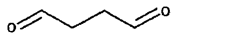 | **11** | **86.09** | **50.09** |
| 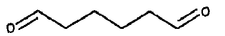 | **12** | **114.14** | **78.14** |
| 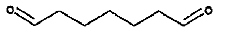 | **13** | **128.08** | **92.08** |
| 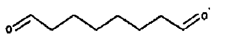 | **14** | **142.19** | **106.19** |

Dimers were prepared by ligation chemistry (oxime bond in the ligation site) (attached through C-terminal domain).

### C. Binding of Dimers to Different IR Constructs Indicates Peptides Bind to Two Independent Sites

Table 19 summarizes the results of binding of phages of D117 (Formula 1 Motif), D123 (Formula 6 motif), D124 (Formula 4 motif), and CP42 (phage expressing peptide J101, Formula 8 motif) monomer to constructs of IR consisting of the L1-cys-L2 region, L1-cys-L2-FnIIIα region and L2-FnIIIα region.

**TABLE 19**

| **IR Construct** | **Peptides Bound** | **Motif** |
|---|---|---|
| L1-cys-L2 | D117 | A6 Only |
| L1-cys-L2-FnIIIα | D117, CP42, D123, 124 | A6, D8, F8 |
| L2-FnIIIα | CP42, D123, D124 | D8, F8 |

The data above is consistent with a conclusion that the A6 (Formula 1 motif) and F8 (Formula 4 Motif) motifs are physically distinct and on separate parts of IR. Competition data, supra, further indicates that the binding site for the B6 (Formula 2 motif) is on the same subunit as that for the A6 motif.

As shown below, BIAcore competition studies are consistent with the separation of Sites 1 (A6, B6) and 2 (D8, F8, J101).

### D. Competition of Site 1 and Site 2 Phage Displayed Peptides with Recombinant Cleaved Di-Peptides

Insulin receptor was coated on a 96-well plate with 50 µl of a 2 ng/µl solution of IR and incubated overnight at 4°C. The wells were then blocked with MPBS for 1 h.

Dimers were prepared by expressing them as MBP fusion products. See, Table 1, supra. The sequences of the MBP- cleaved dimers are shown below (core peptide sequences are underlined):

### Cleaved Dimer Sequences

**#426 (D8)**
   AQPAMAWLDQEWAWVQCEVYGRGCPSAAAGAPVPYPDPLEPRAA.
**#429(D8-6-D8)** 
AQPAMAWLDQEWAWVQCEVYGRGCPSGGSGGSWLDQEWAWVQCEVY
GRGCPSAAAGAPVPYPDPLEPRAA.
**#459 (short flag RB6)**
   ISEFGSADYKDLDALDRLMRYFEERPSLAAAGAPVPYPDPLEPRAA.
**#430 (H2C-4-RB6)** 
DYKDDDDKFHENFYDWFVRQVSGSGSLDALDRLMRYFEERPSLAAAGAP
VPYPDPLEPRAA.
**#464 (H2C)**
   DYKDDDDFHENFYDWFVRQVSAAAGAPVPYPDPLEPRAA.
**#446 (F8)**
   DYKDDDDHLCVLEELFWGASLFGYCSGAAAGAPVPYPDPLEPRAA.
**#431 (H2C-6-F8)** 
DYKDDDDKFHENFYDWFVRQVSGGSGGSHLCVLEELFWGASLFGYCSG
AAAGAPVPYPDPLEPRAA.
**#433 (H2C-9-F8)** 
DYKDDDDKFHENFYDWFVRQVSGGSGGSGGSHLCVLEELFWGASLFGY
CSGAAAGAPVPYPDPLEPRAA.
**#432 (H2C-12-F8)** 
DYKDDDDKFHENFYDWFVRQVSGGSGGSGGSGGSHLCVLEELFWGASL
FGYCSGAAAGAPVPYPDPLEPRAA.
**#452 (G3)**
   AQPAMARGGGTFYEWFESALRKHGAGAAAGAPVPYPDPLEPRAA.
**#427 (G3-6-G3)** 
AQPAMARGGGTFYEWFESTLRKHGAGGGSGGSRGGGTFYEWFESALRK
HGAGAAAGAPVPYPDPLEPRAA.
 (* A TO T CHANGE)
**#428 (G3-12-G3)** 
AQPAMARGGGTFYEWFESALRKHGAGGGSGGSGGSGGSRGGGTFYEW
FESALRKHGAGAAAGAPVPYPDPLEPRAALTN.
**#434 (G3-12-G3)** 
ISEFIEVRAQPAMARGGGTFYEWFESALRKHGAGGGSGGSGGSGGSRG
GGTFYEWFESALRKHGAGAAAGAPVPYPDPLEPRAA.
**#437 (H2C)**
   AQPAMAFHENFYDWFVRQVSAAAGAPVPYPDPLEPRAA.
**#463 (H2C-3-H2C)** 
AQPAMAFHENFYDWFVRQVSGGSFHENFYDWFVRQVSAAAGAPVPYPD
PLEPRAA.
**#435 (H2C-3-H2C-3-H2C)** 
AQPAMAFHENFYDWFVRQVSGGSFHENFYDWFVRQVSGGSFHENFYD
WFVRQVSAAAGAPVPYPDPLEPRAA.
**#439 (H2C-6-H2C)** 
AQPAMAFHENFYDWFVRQVSGGSGGSFHENFYDWFVRQVSAAAGAPVP
YPDPLEPRAA.
**#436 (H2C-9-H2C)** 
AQPAMAFHENFYDWFVRQVSGGSGGSGGSFHENFYDWFVRQVSAAAG
APVPYPDPLEPRAA.
**#449 (H2C-12-H2C)** 
AQPAMAFHENFYDWFVRQVSGGSGGSGGSGGSFHENNFYDWFVRQVS
AAAGAPVPYPDPLEPRAA.
**MBP***

ISEFGSSRVDLQASLALAVVLQRRDWENPGVTQLNRLAAHPPFASWRNSEEA
RTDRPSQQLRSLNGEWQLGCFGG

The MBP- cleaved fusion protein mixtures were appropriately diluted, added to the wells, and incubated at RT for 30 min. An equal volume of F8 or H2C phage displayed peptide was then added to each well and incubated for 1 h. The control wells (100% phage binding) contained only phage and an equal volume of buffer. The control cleaved fusion protein mixture contains a peptide derived from the lacZ gene. The plate was washed 3 times in PBST and then incubated with HRP/anti-M13 conjugate for 45 min. The plate was washed again and then the ABTS substrate added. The values indicate readings taken at OD₄₀₅. Figure 72A shows competition between cleaved monomers and dimers and F7 phage for binding to Site 2 of IR. Figure 72B shows competition for binding to Site 1 between H2C and the cleaved and uncleaved monomers and dimers. IC₅₀ values are shown in Table 20.

**TABLE 20**

| **Dimers Site 1/Site 2 IC₅₀ Values** | | | | |
|---|---|---|---|---|
| **Phage Signal** | **H2C** | | **F8** | |
| **Cleavage** | - | + | - | + |
| **Dimers** | | | | |
| LF-H2C(6)F8 | 0.2 | 0.19 | 0.3 | 5 |
| LF-H2C(9)F8 | 0.4 | 0.11 | 3 | 15 |
| LF-H2C(12)F8 | 0.3 | 0.19 | >16 | 16 |
| LF-F8 mono | - | - | >20 | 12 |
| LF-H2C mono | 0.145 | 0.11 | >1 | >1 |
| H2C mono | 0.3 | 0.2 | >0.5 | >0.5 |
| MBP-lacZ control | - | - | - | - |

| | | | | |
|---|---|---|---|---|
| - = uncleaved + = cleaved | | | | |

### E. Stimulation of autophosphorylation of IR by MBP-Fusion Peptides

Fusion peptides were prepared as described above, and then assayed for IR activation (see Example 20). The results of these experiments shown in Figure 74 indicate that the H2C monomer and H2C-H2C homodimers stimulate autophosphorylation of IR *in vivo.*

H2C dimers (Site 1-Site 1) with a 6 amino acid linker (H2C-6-H2C) were most active in the autophosphorylation assay. Other active dimers are also shown in Figure 74, particularly H2C-9-H2C, H2C-12-H2C, H2C-3-H2C, and F8.

### Example 29: IGF-1R Peptide Assays

### A. IC₅₀ Determinations

Peptides that meet the proper criteria of affinity, selectivity, and activity may be used to develop site-directed assays to identify active molecules which bind to sites on IGF-1R. Assays have been developed using Time-Resolved Fluorescence Resonance Energy Transfer (FRET). These assays are not radioactive, homogeneous (no wash steps), and can be rapidly carried out in 96- or 384-well microtiter plate format facilitating their use in high-throughput screening assays for small organic molecules.

This assay can be used to assess the affinity of peptides for IGF-1R or can be used to find small organic molecule leads in a high-throughput capacity. The determination of the IC₅₀ for several peptides is described below.

### 1. Assay Components

IGF-1 R was purchased from R&D System, Cat. # 391-GR/CF. The IGF-1R was labeled with Europium (Eu) by EG&G Wallac. Ten milligrams of IGF-1R was sent to Wallac and the IGF-1R was labeled with Wallac's W-1024 Eu-chelate.

The Streptavidin-Allophycocynanin (SA-APC) was obtained from Prozyme Cat. # PJ25S. The biotinylated 20E2 [DYKDFYDAIDQLVRGSARAGGTRDKK(ε-biotin)] ("b-20E2") was synthesized by Novo Nordisk or by PeptidoGenic Research & Co., Inc. The IGF-1 was commercially available from PeproTech Cat. # 100-11.

### 2. Assay Method

a. Preparation of the Assay Mix. A 2X concentration of Assay Mix consisting of 4 nM Eu-labeled IGF-1 R, 30 nM b-20E2, 4 nM SA-APC, and 0.1 % BSA was prepared. This mixture was allowed to pre-incubate at RT in the dark for 1-2 h before competitor was added.
b. Dilutions of the Competitors were carried out on a 96-well microtiter plate (Costar Cat. #3912). 100 µl of Buffer (TBS pH 8.0 + 0.1 % BSA) were dispensed to wells in columns 1 through 11. Competitors and Buffer were added to Column 12 wells so that the total volume is 150 µl.
c. To identify small organic compounds which also bind the active sites of IGF-1R, dilutions of the small organic compounds are also performed on a 96-well microtiter plate (Costar Cat. #3912). Compounds are dissolved in 100% DMSO. Therefore, 100 µl of Buffer (TBS pH 8.0 + 0.1 % BSA) with 4% DMSO are dispensed to wells in columns 1 through 10. Column 11 contains 100 µl of Buffer with 2.7% DMSO. Compounds (6 µl) are added into 144 µl Buffer (No DMSO) to Column 12 wells.
d. Dilutions were performed across columns on the plate. Once competitors were dispensed into Column 12 and mixed, 50 µl of the solution Column 12 were transferred to wells in 11 and mixed. 50 µl of the Column 11 mixture was transferred to Column 10 wells. This was repeated until 50 µl of Column 3 mixture was transferred to Column 2 wells. Once accomplished to Column 2, 50 µl from Column 2 were removed and discarded. Column 1 wells were reserved for No Competitor Wells. 100 µl volume was therefore maintained across all columns.
e. 50 µl of the Assay Mix was dispensed into wells on a new 96-well microtiter plate. 50 µl from the Dilutions Plate were then added to this plate.
f. 30 µl from Assay Mix Plate were transferred from the 96-well in duplicate on a 384-well microtiter plate (Nunc Cat. # 264512). This covered plate was allowed to incubate at RT overnight.
g. Binding was measured using Wallac's Victor II fluorometer by excitation at 340 nm and measuring emission at 665 and 615 nm.
h. The working concentrations of this assay were 2 nM Eu-labeled IGF-1R, 15 nM b-20E2, 2 nM SA-APC, and 0.1% BSA. Peptides were normally diluted starting from 100 µM, where IGF-1 begins at 30 µM. Compounds begin at 200 µM in a working concentration of 2% DMSO. Controls also contained 2% DMSO.

### 3. Results

The IC₅₀ and holoenzyme phosphorylation activity (see Example 20) values for certain peptides are shown below.

| Peptide Data | | | |
|---|---|---|---|
| Name | Sequence | IGF-1R IC₅₀ | Holo. Phos. |
| IGF-1 | Natural Ligand | ~1-10 nM | |
| C1 | A6S-4-C1-IGFR or D112 | ~10 nM | |
| RP9 | H2C Design | 33 nM | ++ |
| 20E2 | R20a-3-20E2-IR or D118 | ~100 nM | |
| G8 | 20E2B-3-C6-IGFR | 139 nM | - |
| RP2 | HZCB-3-B9-IR | 163 nM | + |
| E8 | R20b-4-E8-IR or D120 | 175 nM | |
| G33 | H2CA-4-G9-IGFR | 178 nM | +++ |
| RP6 | 20C-4-G3-IGFR | 184 nM | +++++ |
| RP14 | H2CA-4-H8-IGFR | 225 nM | |
| S178 | B6C-3-C10-IR | 240 nM | |
| RP10 | 20E2 Design | 315 nM | + |
| S176 | A6S-4-G1-IR | 418 nM | |
| H2C | A6S-4-H2-IGFR or D117 | ~600 nM | + |
| B6 | R40-3-B6-IGFR | 631 nM | |
| RP13 | H2CA-4-H6-IGFR | 818nM | |
| G8 | 20E2B-3-C6-IGFR | 1330 nM | - |
| S174 | R20-4-F9-IGFR | 1460 nM | |
| RP8 | 20E2 Design | 1800 nM | + |
| S177 | B6C-3-C7-IR | 2040 nM | |
| S175 | A6S-3-E12-IR | 2050 nM | ++ |
| RP1 | H2CB-4-G11-IR | 2790 nM | + |
| bS175 | A6S-3-E12-IR | 3230 nM | |
| NG C2 | 20E2-3-C2-IGFR | 4020 nM | |
| S179 | H2CBa-3-B12-IR | 5350 nM | |
| S173 | rB6-4-A12-IR | 5620 nM | |
| RP5 | 20E2B-3-B3-IR | 7450 nM | - |
| G9 | 20E2B-1-A6-IGFR | 7550 nM | - |
| RP4 | 20E2A-4-F9-IR | 8110 nM | + |
| D8 (B12) | D820-4-B12-IR | 11300 nM | |
| RP24 | R20b-4-A4-IR | 17800 nM | |
| RP11 | A6S Design | 18800 nM | + |
| DB | R20b-4-D8-IR | 21650 nM | |
| A6 | R40-3-A6-IGFR | 46600 nM | |
| RP17 | R20b-4-A6-IR | 50000 nM | |
| S167 | Short A6 | ~100 µM | |
| RP3 | 20E2A-3-B11-IR | ~100 µM | - |
| KC F9 | D820-4-F9-IR | ~100 µM | |
| JB3 | CONTROL | ~100 µM | |
| KC G1 | D820-4-F10-IGFR | ~100 µM | |
| C3-MDM2 | CONTROL | >100 µM | |
| RP21. | 40F-4-C1-IGFR | >100 µM | |
| RP22 | 40F-4-D10-IGFR | >100 µM | |
| RP23 | 40F-4-C1-IR | >100 µM | |
| KC G2 | D820-4-F10-IGFR | >100 µM | |
| KC G7 | F815-4-G7-IGFR | >100 µM | |

### B. IGF-1R Peptide Assay Competition Dissociation

A competition dissociation experiment was performed to determine if any peptides altered the dissociation rate of the 20E2 (B6 motif) peptide in the IGF-1R Peptide Assay. An alteration of the dissociation rate suggests the peptide used in the competition binds to a second site on IGF-1R thus enhancing or slowing the 20E2 dissociation rate through an allosteric interaction.

### 1. Materials

IGF-1R was purchased from R&D System, Cat. # 391-GR/CF. The IGF-1R was labeled with Europium (Eu) by EG&G Wallac. Ten milligrams of IGF-1R was sent to Wallac and the IGF-1R was labeled with Wallac's W-1024 Eu-chelate.

The Streptavidin-Allophycocynanin (SA-APC) obtained from Prozyme Cat. # PJ25S. The biotinylated 20E2 [DYKDFYDAIDQLVRGSARAGGTRDKK(ε-biotin)] was synthesized by Novo Nordisk or by PeptidoGenic Research & Co., Inc. The IGF-1 was commercially available from PeproTech Cat. # 100-11.

### 2. Methods

a. Preparation of the Assay Mix. A 1.25X concentration of Assay Mix consisting of 2.5 nM Eu-labeled IGF-1R, 18.75 nM b-20E2, 2.5 nM SA-APC, and 0.1 % BSA was prepared. This mixture was allowed to pre-incubate.
b. 20 µl of Competitor and Buffer were added to a 96-well microtiter plate (Costar Cat. #3912).
c. Wallac Victor II Fluorometer was readied to read at 665 nm only in multiple repeats (99) of only the wells containing material.
d. 80 µl of the 1.25X Assay Mix was added to the 96-well microtiter plate and promptly placed onto the Victor II for readings.
e. After the original 99 repeat readings were taken, periodic readings were taken until equilibrium had been established.
   NOTE: Different conditions can be used for these experiments. For example, a 1.1X concentration of assay mix can be initially made. Then first add 10 µl of Competitor and Buffer to the microtiter plate followed by 90 µl of the Assay Mix.
f. The working concentrations of this assay were 2 nM Eu-labeled IGF-1R, 15 nM b-20E2, 2 nM SA-APC, and 0.1% BSA. Peptides were normally competed at 100 µM, whereas IGF-1 was competed at 30 µM. Results are shown in Figure 14.

### 3. Results

Figure 14 shows the results of one of the experiments. Clearly, IGF-1 and D8 (B12) cause a much slower dissociation rate than the 20E2 (motif 2), H2C (motif 1), C1 (motif 1), and RP6 (motif 2) peptides. This suggests that IGF-1 and D8 (B12) contact IGF-1R in different locations than that of 20E2, H2C, C1, and RP6.

Previous data (EXAMPLE 28) suggests that the motif 6 series binds to a location of IGF-1R that differs from motifs 1 and 2 and that these two sites are not independent of one another. The slowing of the dissociation rate by IGF-1 and D8 (B12) further suggests that there are at least two sites of binding to IGF-1R and that these two sites are not independent of one another.

The following publications, some of which have been cited herein, are cited for general background information and are incorporated by reference in their entirety.

### REFERENCES

Angelloz-Nicoud P and Binoux M (1995). Autocrine Regulation of Cell Proliferation by the Insulin-Like Growth Factor (IGF) and IGF Binding Protein-3 Protease System in a Human Prostate Carcinoma Cell Line (PC-3). Endocrinology 136:5485-5492.
Apfel SC and Kessler JA (1996). Neurotrophic Factors in the Treatment of Peripheral Neuropathy. Ciba Found. Symp. 196:98-108.
Apfel SC (1999). Neurotrophic factors in the therapy of diabetic neuropathy. Am. J. Med. 107:34S-42S.
Auer RN (1998). Insulin, blood glucose levels, and ischemic brain damage. Neurology 51:S39-43.
Bass J, Kurose T, Pashmforoush M, and Steiner DF (1996). Fusion of Insulin Receptor Ectodomains to Immunoglobulin Constant Domains Reproduces High-affinity Insulin Binding in vitro. J. Biol. Chem. 271:19367-19375.
Canalis E (1997). Insulin-Like Growth Factors and Osteoporosis. Bone 21:215-216
Carcamo J, Ravera MW, Brissette R, Dedova O, Beasley JR, Alam-Moghé A, Wan C, Blume A, and Mandecki W (1998). Unexpected Frameshifts from Gene to Expressed Protein in a Phage-displayed Peptide Library. Proc. Natl. Acad. Sci. U. S.A. 95:11146-11151.
Carroll PV, Umpleby M, Ward GS, Imuere S, Alexander E, Dunger D, Sonksen PH, and Russell-Jones DL (1997). rhlGF-I Administration Reduces Insulin Requirements, Decreases Growth Hormone Secretion, and Improves the Lipid Profile in Adults with IDDM. Diabetes 46:1453-1458.
Chan JM, Stampfer MJ, Giovannucci E, Gann PH, Ma J, Wilkinson P, Hennekens CH, and Pollak M (1998). Plasma Insulin-Like Growth Factor-I and Prostate Cancer Risk: A Prospective Study. Science 279:563-566. Chen YCJ., Delbrook K, Dealwis C, Mimms L. Mushawar IK, and Mandecki W (1996). Discontinuous Epitopes of Hepatitis B Surface Antigen derived from a Filamentous Phage Peptide Library. Proc. Natl. Acad. Sci. U.S.A. 93:1997-2001.
Clark R (1997). The Somatogenic Hormones and Insulin-Like Growth Factor-1: Stimulators of Lymphopoiesis and Immune Function. Endocr. Rev. 18:157-179.
Cohen P, Graves HC, Peehl DM, Kamarei M, Giudice LC, and Rosenfeld RG (1992). Prostate-Specific Antigen (PSA) is an Insulin-Like Growth Factor Binding Protein-3 Protease Found in Seminal Plasma. J. Clin. Endocrinol. Metab. 75:1046-1053.
Cohen P, Peehl DM, Graves HC and Rosenfeld RG (1994). Biological Effects of Prostate Specific Antigen as an Insulin-Like Growth Factor Binding Protein-3 Protease. J. Endocrinol. 142:407-415.
Conover CA (1996). Regulation and Physiological Role of Insulin-Like Growth Factor Binding Proteins. Endocr. J. 43S:S43-S48.
Crowne EC, Samra JS, Cheetham T, Watts A, Holly JM, Dunger DB (1998). Recombinant Human Insulin-Like Growth Factor-I Abolishes Changes in Insulin Requirements Consequent Upon Growth Hormone Pulsatility in Young Adults with Type I Diabetes Mellitus. Metabolism 47:31-38.
Cwirla SE, Balasubramanian P, Duffin DJ, Wagstrom CR, Gates CM, Singer SC, Davis AM, Tansik RL, Mattheakis LC, Boytos CM, Schatz PJ, Baccanari DP, Wrighton, NC, Barrett RW, and Dower WJ (1997). Peptide Agonist of the Thrombopoietin Receptor as Potent as the Natural Cytokine. Science 276:1696-1698.
De Meyts P, Wallach B, Christoffersen CT, Ursø B, Grønskov K, Latus L, Yakushiji F, Ilondo M, and Shymko RM. (1994). The Insulin-Like Growth Factor-1 Receptor Structure, Ligand-Binding Mechanism and Signal Transduction. Horm. Res. 42:152-169*.*
Feld SM and Hirschberg R (1996). Insulin-Like Growth Factor-I and Insulin-Like Growth Factor-Binding Proteins in the Nephrotic Syndrome. Pediatr. Nephrol. 10:355-358.
Figueroa JA, Lee AV, Jackson JG, and Yee D (1995). Proliferation of Cultured Human Prostate Cancer Cells is Inhibited by Insulin-Like Growth Factor (IGF) Binding Protein-1: Evidence for an IGF-II Autocrine Growth Loop. J. Clin. EndocrinoL Metab. 80:3476-3482.
Garrett, TPJ, McKern NM, Lou M, Frenkel MJ, Bentley JD, Lovrecz GO, Elleman TC, Cosgrove LJ, and Ward CW(1998). Crystal Structure of the First Three Domains of the Type-1 Insulin-like Growth Factor Receptor. Nature 394:395-399.
Grihalde ND, Chen YC, Golden A, Gubbins E, and Mandecki W (1995). Epitope Mapping of Anti-HIV and Anti-HCV Monoclonal Antibodies and Characterization of Epitope Mimics using a Filamentous Phage Peptide Library. Gene 166:187-195.
Hoogenboom HR (1997) Designing and optimizing library selection strategies for generating high-affinity antibodies. Trends Biotechnol. 15, 62-70.
Hopp TP, Prickett KS, Price V, Libby RT, March CJ, Cerretti P, Urdal DL, and Conlon PJ (1988). A Short Polypeptide Marker Sequence useful for Recombinant Protein Identification and Purification. Bio/Technology 6:1205-1210.
Hubbard SR, Wei L, Ellis L, Hendrickson WA (1994). Crystal structure of the tyrosine kinase domain of the human insulin receptor. Nature 372:746-754.
Kay BK, Adey NB, He YS, Manfredi JP, Mataragnon AH, and Fowlkes DM (1993). An M13 Phage Library Displaying Random 38-amino-acid Peptides as a Source of Novel Sequences with Affinity to Selected Targets. Gene 128:59-65.
Kristensen C, Wiberg FC, Schaffer L, Andersen AS (1998). Expression and characterization of a 70-kDa fragment of the insulin receptor that binds insulin. Minimizing ligand binding domain of the insulin receptor. J. Biol. Chem. 273:17780-6.
Lai EC, Felice KJ, Festoff BW, Gawel MJ, Gelinas DF, Kratz R, Murphy MF, Natter HM, Norris FH, and Rudnicki SA (1997). Effects of Recombinant Human Insulin-Like Growth Factor-I on Progression of ALS. A Placebo-Controlled Study. The North America ALS/IGF-I Study Group. Neurology 49:1621-1630.
Lee J and Pilch PF (1994). The Insulin Receptor: Structure, Function, and Signaling. Am. J. Physiol. 266:C319-C334.
Lilja H (1995). Regulation of the Enzymatic Activity of Prostate-Specific Antigen and its Reactions with Extracellular Protease Inhibitors in Prostate Cancer. Scand. J. Clin. Lab. Invest. Suppl. 220:47-56.
Livnah O, Stura EA, Johnson DL, Middleton SA, Mulcahy LS, Wrighton NC, Dower WJ, Jolliffe LK, and Wilson IA (1996). Functional Mimicry of a Protein Hormone by a Peptide Agonist: the EPO Receptor Complex at 2.8 A. Science 273:464-71.
Mandecki W, Brissette R, Carcamo J, Cheng W, Dedova O, Hsiao KC, Moghe A, Ravera M, Shen H, Tang P, and Blume A (1997). Display Technologies - Novel Targets and Strategies. P. Guttry (Ed.). International Business Communications, Inc., Southborough, MA, pp. 231-254. Mandecki W, Brissette R, Carcamo J, Cheng W, Dedova O, Hsiao KC, Moghe A, Ravera M, Shen H, Tang P, and Blume A (1997). Display Technologies- Novel Targets and Strategies. P. Guttry (ed). International Business Communications, Inc., Southborough, MA, pp. 231-254. Mynarcik DC, Williams PF, Schaffer L, Yu GQ, and Whittaker J (1997). Identification of Common Ligand Binding Determinants of the Insulin and Insulin-Like Growth Factor 1 Receptors. Insights in Mechanisms of Ligand Binding. J. Biol. Chem. 272:18650-18655.
Rader C and Barbas CF III (1997). Phage Display of Combinatorial Antibody Libraries. Curr. Opin. Biotechnol. 8:503-508.
Rajaram S, Baylink DJ, and Mohan S (1997). Insulin-Like Growth Factor-Binding Proteins in Serum and other Biological Fluids: Regulation and Functions. Endocr. Rev. 18:801-831.
Ravera MW, Carcamo J, Brissette R, Alam-Moghe A, Dedova O, Cheng W, Hsiao KC, Klebanov D, Shen H, Tang P, Blume A, and Mandecki W (1998). Identification of an Allosteric Binding Site on the Transcription Factor p53 Using a Phage-Displayed Peptide Library. Oncogene 16:1993-1999. Renschler MF, Bhatt RR, Dower WJ, and Levy R (1994). Synthetic Peptide Ligands of the Antigen Binding Receptor induce Programmed Cell Death in a Human B-cell Lymphoma. Proc. Natl. Acad. Sci. U.S.A. 91:3623-3627. Scott JK and Smith GP (1990). Searching for Peptide Libraries with an Epitope Library. Science 249:386-390.
Smith LE, Shen W, Perruzzi C, Soker S, Kinose F, Xu X, Robinson G, Driver S, Bischoff J, Zhang B, Schaeffer JM, Senger DR (1999). Regulation of vascular endothelial growth factor-dependent retinal neovascularization by insulin-like growth factor-1 receptor. Nat. Med. 5:1390-5
Tompkins SM, Rota PA, Moore JC, and Jensen PE (1993). A Europium Fluoroimmunoassay for Measuring Binding of Antigen to Class II MHC Glycoproteins. J. Immunological Methods 163:209-216.
Torring N, Vinter-Jensen L, Pedersen SB, Sorensen FB, Flybjerg A, Nexo E (1997). Systemic Administration of Insulin-Like Growth Factor I (IGF-I) Causes Growth of the Rat Prostate. J. Urol. 158:222-227.
Wang LM, Myers MG Jr, Sun XJ, Aaronson SA, White M, Pierce JH (1993) IRS-1: essential for insulin- and IL-4-stimulated mitogenesis in hematopoietic cells. Science 261:1591-1594.
Ward CW, Hoyne PA, and Flegg RH (1995). Insulin and Epidermal Growth Factor Receptors contain the Cysteine Repeat Motif found in the Tumor Necrosis Factor Receptor. Protein Struct. Funct Genet. 22:141-153. Wrighton NC, Farrell FX, Chang R, Kashyap AK, Barbone FP, Mulcahy LS, Johnson DL, Barrett RW, Jolliffe LK, and Dower WJ (1996). Small Peptides as Potent Mimetics of the Protein Hormone Erythropoietin. Science 273:458-464.
Yanofsky SD, Balldwin DN, Butler JH, Holden FR, Jacobs JW, Balsubramanian P, Cinn JP, Cwirla SE, Petter-Bhatt E, Whitehorn EA, Tate EH, Akeson A, Bowlin TL, Dower WJ, and Barrett RW (1996). High affinity Type I Interleukin 1 Receptor Antagonists discovered by Screening Recombinant Peptide Libraries. Proc. Natl. Acad. Sci. U.S.A. 93:7381-7386.

### Aspects of the disclosure

48. A method of modulating insulin activity in mammalian cells, said method comprising administering to said cells an amino acid sequence which binds IR and comprises the sequence of amino acids X₆X₇X₈X₉X₁₀X₁₁X₁₂X₁₃ wherein X₆ and X₇ are aromatic amino acids or glutamine, X₈, X₉, X₁₁ and X₁₂ may be any amino acid, X₁₀ and X₁₃ are hydrophobic amino acids.
49. The method according to aspects of the disclosure 48 wherein X₆ and X₇ are selected from group consisting of phenylalanine and tyrosine, and X₁₀ and X₁₃ are selected from group consisting of leucine, isoleucine, tryptophan, phenylalanine methionine and valine.
50. The method according to aspects of the disclosure 48 wherein X₆ is phenylalanine and X₇ is tyrosine.
51. The method according to aspects of the disclosure 50 wherein X₁₀ is isoleucine.
52. The method according to aspects of the disclosure 50 wherein X₁₀ is leucine.
53. The method according to aspects of the disclosure 50 wherein X₁₃ is leucine.
54. The method according to aspects of the disclosure 50 wherein X₉ is tyrosine and X₁₀ is phenylalanine.
55. The method according to aspects of the disclosure 50 wherein the amino acid sequence is selected from FYX₈X₉LX₁₁X₁₂L, FYX₈X₉I X₁₁X₁₂L and FYX₈YFX₁₁X₁₂L.
56. The method according to aspects of the disclosure 5 wherein the amino acid sequence comprises FYX₈X₉LX₁₁X₁₂L.
57. The method according to aspects of the disclosure55 wherein the amino acid sequence comprises FYX₈YFX₁₁X₁₂L.
58. The method according to aspect 48 wherein the amino acid sequence X₆X₇X₈X₉X₁₀X₁₁X₁₂X₁₃ further comprises amino acids X₉₈ and X₉₉ at the amino terminal end and X₁₀₀ at the carboxy terminal end to form X₉₈X₉₉ X₆X₇X₈X₉X₁₀X₁₁X₁₂X₁₃X₁₀₀ and wherein X₉₈ is optionally aspartic acid and X₉₉ is independently an amino acid selected from the group consisting of glycine, glutamine and proline, and X₁₀₀ is a hydrophobic amino acid.
59. The method according to aspects 58 wherein X₁₀₀ is an aliphatic amino acid.
60. The method according to aspect 59 wherein X₁₀₀ is leucine.
61. The method according to aspect 48 wherein the amino acid sequence binds to the insulin receptor with an affinity of at least about 10⁻⁵M.
62. The method according to aspect 61 wherein the affinity is between about 10⁻⁷M.
63. The method according to aspect 48 wherein the amino acid sequence comprises DYKDFYDAmQLVRGSARAGGTRD or KDRAFYNGLRDLVGAVYGAWD.
64. The method according to aspect 48 wherein the amino acid sequence is selected from the group of amino acid sequences listed in Figures 2A through 2P.
65. An amino acid sequence comprising X₆X₇X₈X₉X₁₀X₁₁X₁₂X₁₃ wherein X₆ and X₇ are aromatic amino acids or glutamine, X, ₈X₉, X₁₁ and X₁₂ may be any amino acid, X₁₀ and X₁₃ are hydrophobic amino acids and wherein said amino acid sequence binds to IGF-1R.
66. The amino acid sequence according to aspect 65 wherein the binding occurs at an affinity (K_{d}) of at least about 10⁻⁵ M.
67. The amino acid sequence according to aspect 66 wherein the binding occurs at an affinity (K_{d}) of at least about 10⁻⁷ M.
68. The amino acid sequence according to aspect 65 wherein X₆ and X₇ are phenylalanine or tyrosine, and X₁₀ and X₁₃ are leucine, isoleucine, tryptophan, phenylalanine or methionine.
69. The amino acid sequence according to aspect 68 wherein X₆ is phenylalanine and X₇ is tyrosine.
70. The amino acid sequence according to aspect 68 wherein X₁₀ is isoleucine.
71. The amino acid sequence according to aspect 68 wherein X₁₀ is leucine.
72. The amino acid sequence according to aspect 69 wherein X₁₀ is leucine.
73. The amino acid sequence according to aspect 69 wherein X₉ is tyrosine and X₁₀ is phenylalanine.
74. The amino acid sequence according to aspect 68 wherein the amino acid sequence comprises an amino acid sequence selected from FYX₈X₉LX₁₁X₁₂L, FY X₈X₉I X₁₁X₁₂L and FYX₈YFX₁₁X₁₂L.
75. The amino acid sequence according to aspect 74 wherein the amino acid sequence comprises FY X₈X₉I X₁₁X₁₂L.
76. The amino acid sequence according to aspect 74 wherein the amino acid sequence comprises FYX₈X₉L X₁₁X₁₂L.
77. The amino acid sequence according to aspect 74 wherein the amino acid sequence is FY X₈YF X₁₁X₁₂L.
78. The amino acid sequence according to aspect 65 wherein the amino acid sequence X₆X₇X₈X₉X₁₀X₁₁X₁₂X₁₃ further comprises amino acids X₉₈ and X₉₉ at the amino terminal end and X₁₀₀ at the carboxy terminal end to form X₉₈ X₉₉ X₆X₇X₈X₉X₁₀X₁₁X₁₂X₁₃X₁₀₀ and wherein X₉₈ is optionally aspartic acid and X99 is independently an amino acid selected from the group consisting of glycine, glutamine and proline, and X₁₀₀ is a hydrophobic amino acid.
79. The amino acid sequence according to aspect 78 wherein X₁₀₀ is an aliphatic amino acid.
80. The amino acid sequence according to aspect 79 wherein X₁₀₀ is leucine.
81. The amino acid sequence according to aspect 68 wherein the amino acid sequence comprises DYKDFYDAIDQLVRGSARAGGTRD or KDRAFYNGLRDLVGAVYGAWDKK.
82. The sequence according to aspect 81 wherein the amino acid sequence comprises DYKDFYDAIDQLVRGSARAGGTRD.
83. An amino acid sequence comprising an amino acid sequence selected from the group consisting of amino sequences listed in Figures 2A through 2P.
84. An amino acid sequence comprising a sequence selected from the group consisting of
   SFYEAIHQLLGV,
   NSFYEALRMLSS,
   SLNFYDALQLLA,
   SSNFYQALMLLS,
   SDGFYNAIELLS,
   HETFYSMIRSLA,
   HDPFYSMMICSLL and
   WSDFYSYFQGLD.
85. The amino acid sequence according to aspect 65 wherein the sequence comprises the amino acid sequence
   X₁₁₅X₁₁₆X₁₁₇X₁₁₈FYX₈YFX₁₁X₁₂LX₁₁₉X₁₂₀X₁₂₁X₁₂₂ wherein X₁₁₅ is selected from the group consisting of trytophan, glycine, aspartic acid, glutamic acid and arginine, X₁₁₆ is selected from the group consisting of aspartic acid, histidine, glycine and asparagine, X₁₁₇ and X₁₁₈ are selected from the group consisting of glycine, aspartic acid, glutamic acid, asparagine, and alanine, X₈ is selected from the group consisting of arginine, glycine, glutamic acid and serine, X₁₁ is selected from the group consisting of glutamic acid, asparagine, glutamine and tryptophan, X₁₂ is selected from the group consisting of aspartic acid, glutamic acid, glycine, lysine, and glutamine, X₁₁₉ is selected from the group consisting of glutamic acid, glycine, glutamine, aspartic acid and alanine, X₁₂₀ is selected from the group consisting of glutamic acid, aspartic acid, glycine and glutamine, X₁₂₁ is selected from the group consisting of tryptophan, tyrosine, glutamic acid, phenylalanine, histidine and aspartic acid, and X₁₂₂ is selected from the group consisting of glutamic acid, aspartic acid, and glycine.
86. The amino acid sequence according to aspect 85 wherein X₁₁₅ is is tryptophan, X₁₁₇ is selected from glycine, aspartic acid, glutamic acid and asparagine; X₁₁₈ is selected from glycine, aspartic acid, glutamic acid and alanine; X₁₁, X₁₁₉, X₁₂₀, and X₁₂₂ are glutamic acid; X₁₂ is aspartic acid, and X₁₂₁ is tryptophan or tyrosine.
87. An amino acid sequence comprising X₆X₇X₈X₉X₁₀X₁₁X₁₂X₁₃ wherein X₆ and X₇ are aromatic amino acids or glutamine, X₈, X₉, X₁₁ and X₁₂ may be any amino acid, X₁₀ and X₁₃ are hydrophobic amino acids and wherein said amino acid sequence binds to IR such that binding to IGF-1R is at or below background.

## Claims

1. An insulin-like growth factor receptor (IGFR) binding peptide which comprises the amino acid sequence X₆X₇X₈X₉X₁₀X₁₁X₁₂X₁₃, wherein X₆, and X₇ are aromatic amino acids, X₈, X₉, X₁₁, and X₁₂ are any amino acid, and X₁₀, and X₁₃ are hydrophobic amino acids.

2. The peptide of claim 1, wherein the amino acid sequence further comprises a terminal extension of one or two amino acids at either its amino terminus adjacent to X₆ or at its carboxy terminus adjacent to X₁₃, represented as X₉₈ X₉₉ B6 X₁₀₀ wherein "B6" is the amino acid sequence X₆X₇X₈X₉X₁₀X₁₁X₁₂X₁₃, and wherein X₉₈ is aspartic acid and X₉₉ is independently selected from the group consisting of glycine, glutamine, and proline, and wherein X₁₀₀ is a hydrophobic or aliphatic amino acid.

3. The peptide of claim 1 or 2 wherein the peptide has an affinity (K_{d}) for the IGFR of from 10⁻⁵ M to 10⁻¹² M_{.}

4. The peptide of any one of claims 1-3, wherein the peptide is an antagonist of the IGFR.

5. The peptide of claim 1, wherein the amino acid sequence is selected from the amino acid sequences listed in Figures 2A through 2P.

6. The peptide of claim 1, wherein the amino acid sequence is selected from the group consisting of: D 118, D119, S287, S173, S174, S177, S178, S257, S258, and S259.

7. The peptide of claim 1, wherein the amino acid sequence comprises D118 (DYKDFYDAIDQLVRGSARAGGTRD) or D119 (KDRAFYNGLRDLVGAVYGAWDKK).

8. The peptide of claim 1, wherein the amino acid sequence comprises an amino acid sequence selected from FYX₈X₉LX₁₁X₁₂L, FY X₈X₉I X₁₁X₁₂L and FYX₈YFX₁₁X₁₂L.

9. A multivalent ligand comprising the peptide of any one of claims 1-8.

10. A nucleic acid sequence encoding an amino acid sequence comprising the peptide according to any one of claims 1-8.

11. An in vitro method of modulating insulin activity in mammalian cells, said method comprising administering to said cells the peptide of any one of claims 1-8, wherein the peptide is an antagonist of the IGFR.

12. A kit for identifying a compound which binds to the IGFR comprising an IGFR and a peptide of any one of claims 1-8.

13. A pharmaceutical composition comprising the peptide of any one of claims 1-8, and a pharmaceutically acceptable carrier.

14. The peptide of any one of claims 1-8 for use as a medicament.

15. A method of screening for a compound which binds to IGFR comprising:
i) immobilizing IGF-1R, or a fragment thereof, on a surface;
ii) incubating the IGFR, or fragment thereof, with a known amount of a labelled peptide according to any one of claims 1-8;
iii) measuring the amount of labelled peptide bound to IGFR;
iv) determining from the amount of bound labelled peptide whether the compound has competitively bound to IGFR.
